(19) Europäisches Patentamt
European Patent Office
Office européen des brevets

(11) EP 4 509 507 A1

(12) EUROPEAN PATENT APPLICATION
published in accordance with Art. 153(4) EPC

(43) Date of publication:
**19.02.2025 Bulletin 2025/08**

(21) Application number: **23787628.9**

(22) Date of filing: **10.04.2023**

(51) International Patent Classification (IPC):
***C07D 471/00*** *(2006.01)* ***C07D 471/04*** *(2006.01)*
***A61K 31/517*** *(2006.01)* ***A61K 31/519*** *(2006.01)*
***A61P 35/00*** *(2006.01)*

(52) Cooperative Patent Classification (CPC):
**A61K 31/517; A61K 31/519; A61K 31/5383; A61K 31/5386; A61K 31/675; A61K 31/69; A61P 35/00; A61P 35/02; C07D 471/00; C07D 471/04; C07D 519/00; C07F 5/02; C07F 9/6561**

(86) International application number:
**PCT/CN2023/087259**

(87) International publication number:
**WO 2023/197984 (19.10.2023 Gazette 2023/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **11.04.2022 CN 202210383719**
**13.01.2023 CN 202310039419**
**20.03.2023 CN 202310270906**

(71) Applicants:
- **Chengdu Hyperway Pharmaceuticals Co., Ltd.**
  **Chengdu, Sichuan 610041 (CN)**
- **Shenzhen Hyperway Pharmaceuticals Co., Ltd.**
  **Shenzhen, Guangdong 518116 (CN)**

(72) Inventors:
- **REN, Junfeng**
  **Chengdu, Sichuan 610041 (CN)**
- **LIU, Guanfeng**
  **Chengdu, Sichuan 610041 (CN)**
- **WU, Xiancai**
  **Chengdu, Sichuan 610041 (CN)**
- **LI, Shai**
  **Chengdu, Sichuan 610041 (CN)**
- **SONG, Zhiquan**
  **Chengdu, Sichuan 610041 (CN)**
- **YI, Shoubing**
  **Chengdu, Sichuan 610041 (CN)**
- **ZHUO, Guoqing**
  **Chengdu, Sichuan 610041 (CN)**
- **CHEN, Lin**
  **Chengdu, Sichuan 610041 (CN)**
- **YUAN, Chenguang**
  **Chengdu, Sichuan 610041 (CN)**
- **LI, Yingfu**
  **Chengdu, Sichuan 610041 (CN)**

(74) Representative: **Boult Wade Tennant LLP**
**Salisbury Square House**
**8 Salisbury Square**
**London EC4Y 8AP (GB)**

# (54) FUSED RING COMPOUND, PHARMACEUTICAL COMPOSITION CONTAINING SAME, AND USE OF FUSED RING COMPOUND

(57) The present invention relates to the technical field of medicines, and provides a fused ring compound, a pharmaceutical composition containing the same, and use of the fused ring compound. The compound has a structure as shown in formula (I), or a tautomer, a mesomer, a racemat, an enantiomer, a diastereoisomer or a mixture thereof, a metabolite, a metabolic precursor, an isotope substitution form, a pharmaceutically acceptable salt, a hydrate, a solvate, a polymorph or a cocrystal thereof. The compound provided by the present invention can be used for treating cancers caused by KRAS mutation. The cancers caused by KRAS mutation are selected from one or more cancers caused by KRAS G12C, KRAS G12V, KRAS G12A and G12D mutations. In particular, the compound can serve as a G12D inhibitor and has relatively high inhibitory activity.

EP 4 509 507 A1

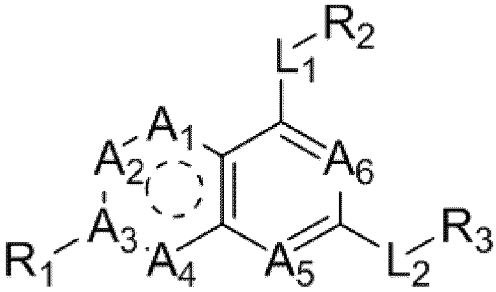

(I)

## Description

This application claims the priority of Chinese Patent Application No. 202210383719.2, filed with the China National Intellectual Property Administration on April 11, 2022 and titled with "FUSED RING COMPOUND, PHARMACEUTICAL COMPOSITION CONTAINING SAME, AND USE OF FUSED RING COMPOUND", Chinese Patent Application No. 202310039419.7, filed with the China National Intellectual Property Administration on January 13, 2023 and titled with "FUSED RING COMPOUND, PHARMACEUTICAL COMPOSITION CONTAINING SAME, AND USE OF FUSED RING COMPOUND", and Chinese Patent Application No. 202310270906.4, filed with the China National Intellectual Property Administration on March 20, 2023 and titled with "FUSED RING COMPOUND, PHARMACEUTICAL COMPOSITION CONTAINING SAME, AND USE OF FUSED RING COMPOUND", which are hereby incorporated by reference in entirety.

## FIELD

The present disclosure relates to the field of pharmaceutical technology, in particular to a fused ring compound, a pharmaceutical composition containing the same, and use thereof.

## BACKGROUND

The Kirsten rat sarcoma viral oncogene homologue (KRAS) gene is a member of the RAS family, which includes two other genes, HRAS and NRAS, with up to 85% homology between these genes. KRAS is a monomeric G-protein located on both sides of the cell membrane and has GTPase activity. The encoded protein consists of 188-189 amino acids with a molecular weight of 21 KD. KRAS plays an important role in regulating signaling pathways involved in tumor cell growth, angiogenesis, and other processes. Under normal conditions, KRAS can control the pathway that regulates cell growth. However, under abnormal conditions, it could lead to continued cellular growth and hinder cell self-destruction. After mutated, the KRAS gene is irreversibly activated, persistently stimulating cell growth. This, in turn, results in disrupted intracellular signaling, uncontrolled cell proliferation, and ultimately, the development of cancer.

KRAS is the most commonly mutated gene in human cancers. Approximately 30% of all cancers are associated with the activation of KRAS mutations, including 95% of pancreatic ductal adenocarcinoma (PDAC), 45% of colon and rectal cancer (CRC), and 35% of non-small cell lung cancer (NSCLC). Due to the structural characteristics of proteins, KRAS was previously considered an "undruggable" target in the context of tumor drug development. However, as the research progressed, researchers found that certain mutant KRAS proteins were capable of forming a site that could interact with drugs. Once bound to small molecule drugs, KRAS is maintained in the inactive KRAS protein-GDP state, which inhibits the activation of the KRAS pathway and thus initiates the anti-tumor effect. In recent years, several KRAS G12C candidate drugs have entered clinical trials. Among these, Amgen's AMG 510 and Mirati Therapeutics' MRTX 849 have made the most rapid progress, with the former receiving approval from the FDA for marketing. Nevertheless, G12C is not the most prevalent KRAS mutant subtype.

## SUMMARY

In view of this, the present disclosure provides a fused ring compound, a pharmaceutical composition comprising the same, and use thereof. The compound provided by the present disclosure is useful in the treatment of cancer caused by KRAS mutation, wherein the cancer caused by KRAS mutation is one or more of the cancers caused by KRAS G12C, KRAS G12V, KRAS G12A, and G12D mutations, and is useful as, in particular, a G12D inhibitor. The compound provided by the present disclosure has high inhibitory activity and good availability, is useful in the manufacture of a medicament for preventing and/or treating a disease mediated by KRAS G12D, and has promising applications.

The present disclosure provides a compound represented by formula (I), or a tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof:

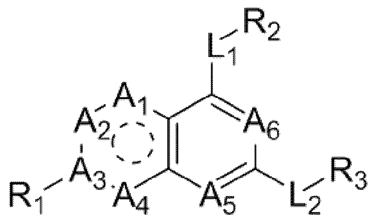

(I)

wherein,

$A_1$, $A_2$, $A_3$, $A_4$, $A_5$ and $A_6$ are each independently selected from the group consisting of C-$R_4$ and N,

$R_1$ is selected from the group consisting of substituted or unsubstituted aryl, and substituted or unsubstituted heteroaryl,

$R_2$ is selected from the group consisting of substituted or unsubstituted cycloalkyl, and substituted or unsubstituted heterocycloalkyl,

$R_3$ is selected from the group consisting of substituted or unsubstituted cycloalkyl, and substituted or unsubstituted heterocycloalkyl,

$R_4$ in $A_1$, $A_2$, $A_3$, $A_4$, $A_5$, and $A_6$ is independently selected from the group consisting of hydrogen, deuterium, halogen, substituted or unsubstituted alkyl or heteroalkyl, substituted or unsubstituted cycloalkyl or heterocycloalkyl, substituted or unsubstituted unsaturated cyclyl or heterocyclyl, substituted or unsubstituted aryl or heteroaryl, hydroxy, cyano, amino, ester, nitro, thiol, amido, sulfonyl, phosphoryl, alkylphosphinoyl, alkylsulfonyl and alkylsulfinyl;

$L_1$ and $L_2$ are absent or independently selected from the group consisting of -CH=CH-, -N(Ra)-, -N(Ra)-(CRaRb)n -, -O-(CRaRb)n-, and -(CRaRb)n-;

Ra and $R_b$ are each independently selected from the group consisting of hydrogen, deuterium, C1-C6 alkyl, C1-C6 heteroalkyl, and C3-C6 cycloalkyl.

Preferably, the compound has a structure represented by formula (II):

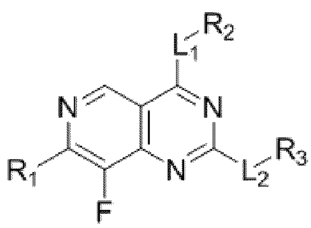

(II);

wherein,

$R_1$ is selected from the group consisting of substituted or unsubstituted aryl, and substituted or unsubstituted heteroaryl,

$R_2$ is selected from the group consisting of substituted or unsubstituted cycloalkyl, and substituted or unsubstituted heterocycloalkyl,

$R_3$ is selected from the group consisting of substituted or unsubstituted cycloalkyl, and substituted or unsubstituted heterocycloalkyl,

$L_1$ is absent or selected from the group consisting of -CH=CH-, -N(Ra)- and -N(Ra)-(CRaRb)n-, $L_2$ is selected from the

group consisting of -O-(CRaRb)n-, -CH=CH-, and -(CRaRb)n-, and

Ra and $R_b$ are each independently selected from the group consisting of hydrogen, deuterium, C1-C6 alkyl, C1-C6 heteroalkyl, and C3-C6 cycloalkyl. Preferably, $L_1$ is absent or selected from the group consisting of -N(Ra)- and -N(Ra)-(CRaRb)n-, and $L_2$ is -O-(CRaRb)n- or -(CRaRb)n-.

Preferably, the compound has a structure represented by formula (III) or formula (IV):

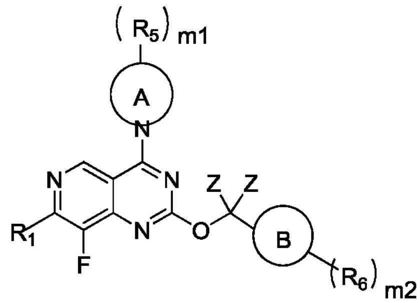

(III),

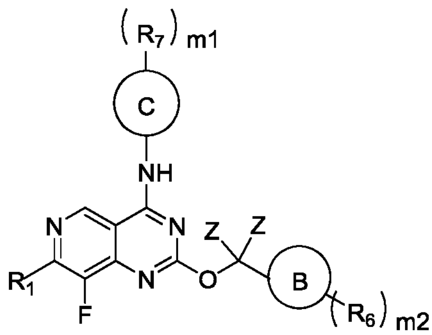

(IV),

wherein,

ring A is a 4- to 12-membered heterocyclic ring with N and/or O as a heteroatom;

ring B is a 4 to 12-membered heterocyclic ring with N as a heteroatom,

the ring A and ring B are ach independently selected from the group consisting of saturated or partially saturated monocycle, fused ring, bridged ring, and spiro ring,

Z is selected from the group consisting of hydrogen and deuterium;

$R_1$ is selected from the group consisting of substituted or unsubstituted aryl, and substituted or unsubstituted heteroaryl,

$R_5$, $R_6$, and $R_7$ are each independently selected from the group consisting of hydrogen, deuterium, halogen, substituted or unsubstituted alkyl or cycloalkyl, substituted or unsubstituted heteroalkyl or heterocycloalkyl, hydroxy, cyano, amino, ester, amido, aryl, heteroaryl, acylguanidino, sulfonyl, phosphoryl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidino, sulfonyl, phosphoryl, sulfonate, and phosphate,

m1 is an integer of 0 to 6, and

m2 is an integer of 0 to 6.

Preferably, the halogen is F.

Preferably, ring B is selected from the group consisting of:

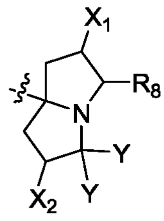 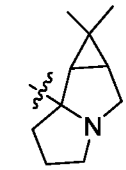 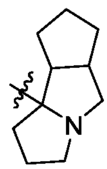 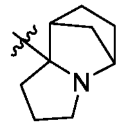 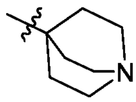

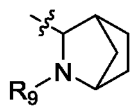 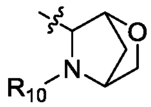 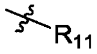 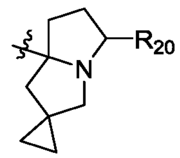 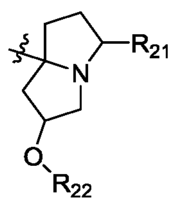

wherein,

$X_1$ and $X_2$ are independently selected from the group consisting of hydrogen, deuterium, and halogen; Y is selected from the group consisting of hydrogen and deuterium;

$R_8$, $R_{20}$, and $R_{21}$ are each independently selected from the group consisting of hydrogen, substituted or unsubstituted aryl or heteroaryl, substituted or unsubstituted alkyl or cycloalkyl, and substituted or unsubstituted heteroalkyl or heterocycloalkyl;

$R_9$, $R_{10}$, and $R_{22}$ are each independently selected from the group consisting of hydrogen, substituted or unsubstituted alkyl or cycloalkyl, substituted or unsubstituted heteroalkyl or heterocycloalkyl, ester, amido, aryl, heteroaryl, acylguanidinyl, sulfonyl, phosphonyl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidinyl, sulfonyl, phosphonyl, sulfonate, and phosphate; and

$R_{11}$ is substituted monocyclic heteroalkyl with nitrogen and/or oxygen as a heteroatom.

Preferably, $R_9$ and $R_{10}$ are independently selected from the group of consisting of

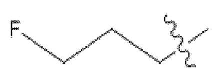 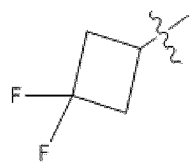

Preferably, ring A is selected from the group consisting of:

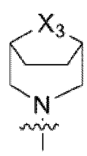 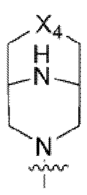 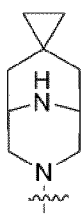 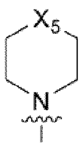 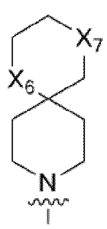 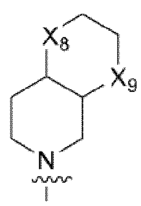 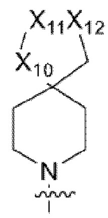 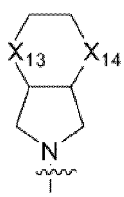

;

ring C is

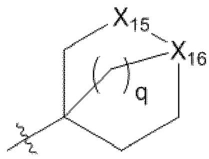

;

wherein,

$X_3$ to $X_{16}$ are each independently selected from the group consisting of $-NR_{12}-$, -O-, -CO-, and $-CR_{14}R_{15}-$;

$R_{12}$, $R_{14}$, and $R_{15}$ are each independently selected from the group consisting of hydrogen, deuterium, halogen, substituted or unsubstituted alkyl or cycloalkyl, substituted or unsubstituted heteroalkyl or heterocycloalkyl, hydroxy, cyano, amino, ester, amido, aryl, heteroaryl, acylguanidino, sulfonyl, phosphoryl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidino, sulfonyl, phosphoryl, sulfonate, and phosphate; and

q is an integer of 0 to 3, and q=0 indicates this chemical bond is absent.

Preferably, $R_1$ is selected from the group consisting of:

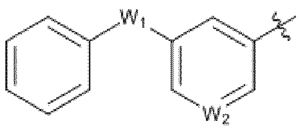
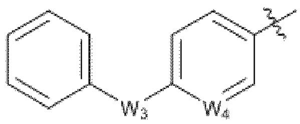
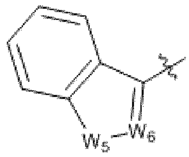
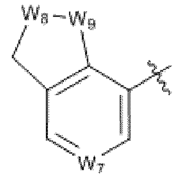

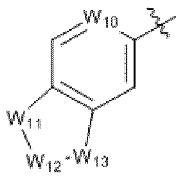
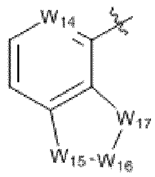
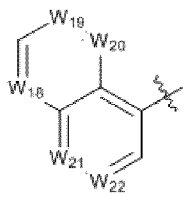
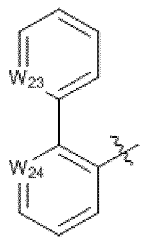

, and

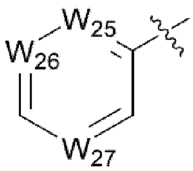

;

wherein, $W_1$, $W_3$, $W_5$, $W_8$, $W_9$, $W_{11}$, $W_{12}$, $W_{13}$, $W_{15}$, $W_{16}$, and $W_{17}$ are each independently selected from the group consisting of $-NR_{12}-$, -O-, -CO-, $-CR_{17}R_{18}-$, and $-SO_2-$; $W_2$, $W_4$, $W_6$, $W_7$, $W_{10}$, $W_{14}$, $W_{18}$, $W_{19}$, $W_{20}$, $W_{21}$, $W_{22}$, $W_{23}$, $W_{24}$, $W_{25}$, $W_{26}$, and $W_{27}$ are each independently selected from the group consisting of N atom, and C atom with $R_{16}$ as a substituent; when $W_{23}$ and $W_{24}$ are C atoms with $R_{16}$ as a substituent, the two carbon atoms are optionally connected by a chemical bond to form a saturated or unsaturated 5-membered or 6-membered ring; and

$R_{12}$, $R_{16}$, $R_{17}$, and $R_{18}$ are each independently selected from the group consisting of hydrogen, deuterium, halogen, substituted or unsubstituted alkyl or cycloalkyl, substituted or unsubstituted heteroalkyl or heterocycloalkyl, hydroxy,

alkynyl, cyano, amino, ester, amido, aryl, heteroaryl, acylguanidino, sulfonyl, phosphoryl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidino, sulfonyl, phosphoryl, sulfonate, and phosphate.

Preferably, the compound has a structure represented by formula (V), formula (VI) or formula (VII):

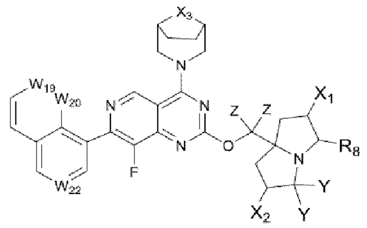

(V)

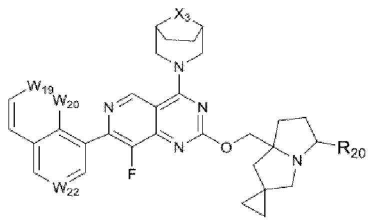

(VI)

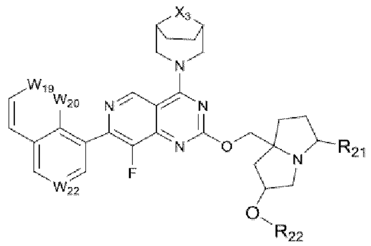

(VII)

wherein,

$X_1$ and $X_2$ are independently selected from the group consisting of hydrogen, deuterium, and halogen; Y is selected from the group consisting of hydrogen and deuterium; Z is selected from the group consisting of hydrogen and deuterium;

$R_8$, $R_{20}$, and $R_{21}$ are each independently selected from the group consisting of hydrogen, substituted or unsubstituted aryl or heteroaryl, substituted or unsubstituted alkyl or cycloalkyl, and substituted or unsubstituted heteroalkyl or heterocycloalkyl;

$R_{22}$ is selected from the group consisting of hydrogen, substituted or unsubstituted alkyl or cycloalkyl, substituted or unsubstituted heteroalkyl or heterocycloalkyl, ester, amido, aryl, heteroaryl, acylguanidinyl, sulfonyl, phosphonyl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidinyl, sulfonyl, phosphonyl, sulfonate, and phosphate;

$X_3$ is selected from the group consisting of $-NR_{12}-$, -O-, -CO-, and $-CR_{14}R_{15}-$; and

$W_{19}$, $W_{20}$, and $W_{22}$ are independently selected from the group consisting of N atom, and C atom with $R_{16}$ as a substituent.

Preferably, the compound has a structure represented by formula (V).

Preferably, the compound has a structure represented by formula (VIII), formula (IX) or formula (X):

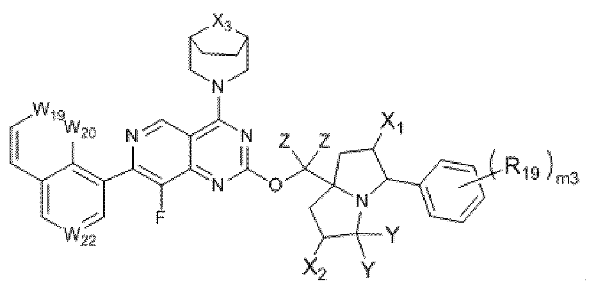

(VIII)

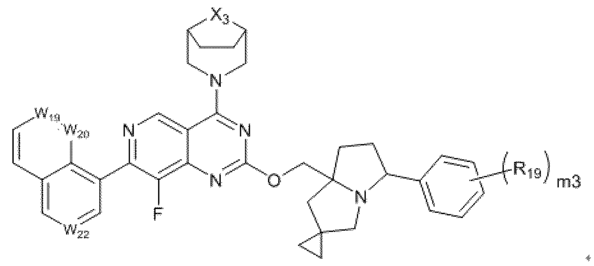

(IX)

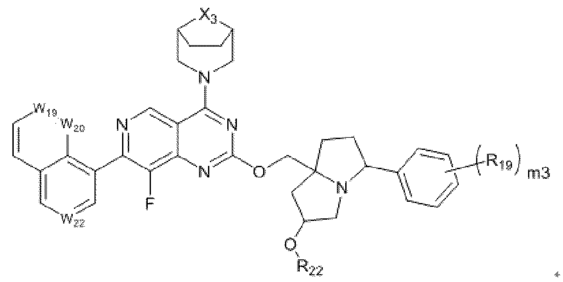

(X)

wherein, $X_1$ and $X_2$ are independently selected from the group consisting of hydrogen, deuterium, and halogen; Y is selected from the group consisting of hydrogen and deuterium; Z is selected from the group consisting of hydrogen and deuterium;

$X_3$ is selected from the group consisting of $-NR_{12}-$, -O-, -CO-, and $-CR_{14}R_{15}-$;

$W_{19}$, $W_{20}$, and $W_{22}$ are each independently selected from the group consisting of N atom, and C atom with $R_{16}$ as a substituent;

$R_{19}$ is selected from the group consisting of -H, -OH, halogen, $-NO_2$, -CN, $-CF_3$, $-C_2F_5$, $-OCF_3$, $-OCHF_2$, $-OCH_2F$, substituted or unsubstituted alkyl or cycloalkyl, substituted or unsubstituted heteroalkyl or heterocycloalkyl, alkoxy, alkynyl, ester, amido, aryl, heteroaryl, acylguanidinyl, sulfonyl, phosphonyl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidinyl, sulfonyl, phosphonyl, sulfonate, and phosphate;

$R_{22}$ is selected from the group consisting of hydrogen, substituted or unsubstituted alkyl or cycloalkyl, substituted or unsubstituted heteroalkyl or heterocycloalkyl, alkoxy, alkynyl, ester, amido, aryl, heteroaryl, acylguanidinyl, sulfonyl, phosphonyl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidinyl, sulfonyl, phosphonyl, sulfonate, and phosphate; and

m3 is an interger of 0 to 5, and when m3 greater than or equal to 2, $R_{19}$ is optionally a 4-to 7-membered ring connected in parallel with a benzene ring.

Preferably, the compound has a structure represented by (VIII).

In a preferred embodiment of the present disclosure, the compound has a structure represented by formula (XI) or formula (XII):

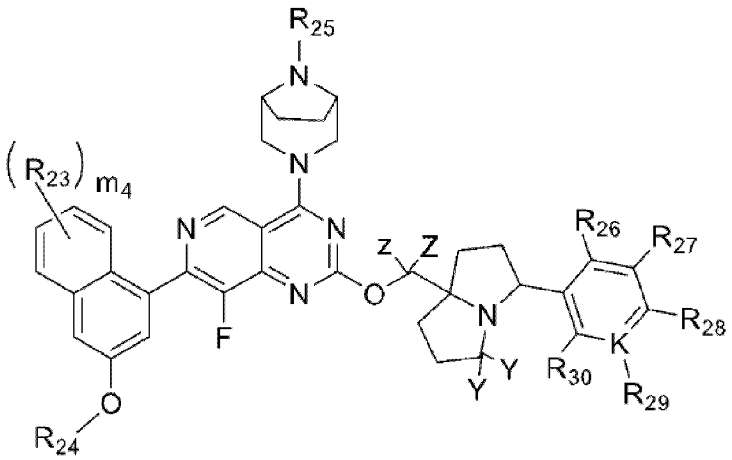

(XI)

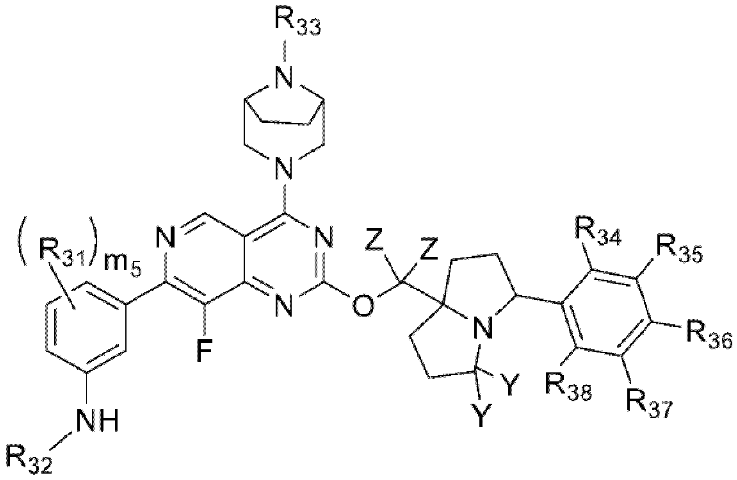

(XII)

Y is selected from the group consisting of hydrogen and deuterium; Z is selected from the group consisting of hydrogen and deuterium;

wherein $R_{23}$ in formula (XI) is independently selected from the group consisting of -H, -F, -Cl, -CN, $-CF_3$, alkenyl, alkynyl, $C_{1-3}$ linear alkyl, and $C_{3-6}$ cycloalkyl;

$R_{24}$ is selected from the group consisting of -H, $C_{1-5}$ alkyl or heteroalkyl, and $-COR_{55}$, and $R_{55}$ is selected from the group consisting of -H, $C_{1-10}$ linear/branched alkyl, $C_{1-10}$ linear/branched heteroalkyl, $C_{3-6}$ cycloalkyl, and $C_{3-6}$ heterocycloalkyl;

$R_{25}$ is selected from the group consisting of -H, -CO($C_{0-10}$ alkyl), -COO($C_{0-10}$ alkyl), $-COO-CH_2-OCO$($C_{0-10}$ alkyl), and $-COO-CH(CH_3)-OCO$($C_{0-10}$ alkyl).

K is selected from the group consisting of C and N, when K is N, $R_{29}$ is absent. $R_{26}$, $R_{27}$, $R_{28}$, $R_{29}$, $R_{30}$ are independently selected from the group consisting of -H, -OH, halogen, $-NO_2$, -CN, $-CF_3$, $-C_2F_5$, $-OCF_3$, $-OCHF_2$, $-OCH_2F$, phosphate, monomethylphosphate, dimethylphosphate, $C_{1-5}$ linear/branched alkyl, $C_{1-5}$ alkoxy, $C_{3-10}$ cycloalkyl, $C_{3-10}$ heterocycloalkyl, -N($C_{0-10}$ alkyl)($C_{0-10}$ alkyl), -S($C_{0-10}$ alkyl), -OCON($C_{0-10}$ alkyl)($C_{0-10}$ alkyl), $C_{5-6}$ aryl, five-membered heteroaryl, alternatively, $R_{27}$, $R_{28}$ and the carbon atom between $R_{27}$ and $R_{28}$ form saturated or unsaturated $C_{3-8}$ cycloalkyl, saturated or unsaturated $C_{3-8}$ heterocycloalkyl, saturated or unsaturated cyclic lactone, $C_{5-6}$ aryl, bridged cycloalkyl, or spirocycloalkyl, wherein H thereof is optionally substituted with -D, -OH, -F, $-NO_2$, -CN, $-CF_3$, $-C_2F_5$, $C_{1-3}$ alkyl, or amido;

m4 is an interger of 0 to 4.

Wherein, $R_{31}$ in formula (XII) is independently selected from the group consisting of -F, -Cl, $-NH_2$, -CN, $-CF_3$, $-C_2F_5$, $-OCF_3$, alkenyl, alkynyl, $C_{1-3}$ linear/branched alkyl, $C_{1-3}$ linear/branched alkoxy, $C_{3-6}$ cycloalkyl, aryl, and heteroaryl;

$R_{32}$ is selected from the group consisting of -H, $C_{1-5}$ linear/branched alkyl, and $C_{3-6}$ cycloalkyl;

$R_{33}$ is selected from the group consisting of -H, $-CO(C_{0-10}$ alkyl), $-COO(C_{0-10}$ alkyl), $-COO-CH_2-OCO(C_{0-10}$ alkyl), and $-COO-CH(CH_3)-OCO(C_{0-10}$ alkyl);

$R_{34}$, $R_{35}$, $R_{36}$, $R_{37}$, and $R_{38}$ are independently selected from the group consisting of -H, -OH, halogen, $-NO_2$, -CN, $-CF_3$, $-C_2F_5$, $-OCF_3$, $-OCHF_2$, $-OCH_2F$, $C_{1-5}$ linear/branched alkyl, $C_{1-5}$ alkoxy, $C_{3-10}$ cycloalkyl, and $C_{3-10}$ heterocycloalkyl; alternatively, $R_{35}$, $R_{36}$ and the carbon atom between $R_{35}$ and $R_{36}$ form saturated or unsaturated $C_{3-8}$ cycloalkyl or saturated or unsaturated $C_{3-8}$ heterocycloalkyl, wherein H thereof is optionally substituted with -D, -OH, -F, $-NO_2$, -CN, $-CF_3$, $-C_2F_5$, $C_{1-3}$ alkyl, or amido; and

m5 is an integer of 0 to 4.

More preferably, the compound has the following structure:

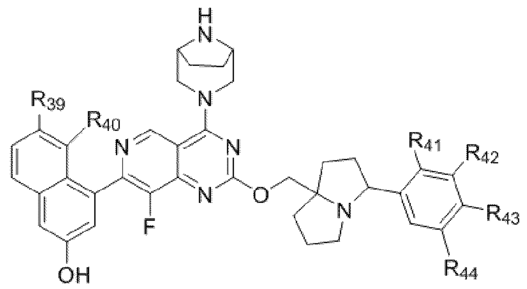

(XIII),

or

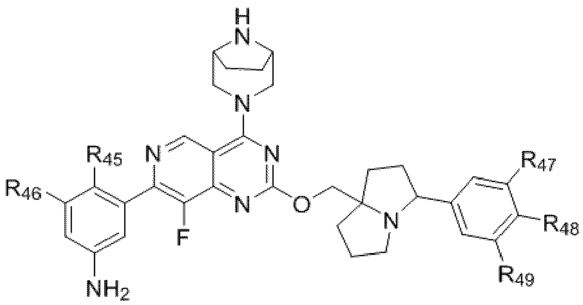

(XIV)

wherein, $R_{39}$ and $R_{40}$ are independently selected from the group consisting of -H, -F, -Cl, -CN, alkynyl, and $C_{1-3}$ linear alkyl; $R_{41}$, $R_{42}$, $R_{43}$, and $R_{44}$ are independently selected from the group consisting of -H, -OH, -F, -Cl, -CN, $-CF_3$, $-C_2F_5$, $-OCF_3$, monomethylphosphoryl, dimethylphosphoryl, $C_{1-5}$ linear/branched alkyl, $C_{1-5}$ alkoxy, $C_{3-10}$ cycloalkyl, $C_{3-10}$ heterocycloalkyl, $-S(C_{0-10}$ alkyl), $-OCON(C_{0-10}$ alkyl)($C_{0-10}$ alkyl), $C_{5-6}$ aryl, five-membered heteroaryl, and six-membered heteroaryl; alternatively, $R_{42}$, $R_{43}$ and the carbon atom between $R_{42}$ and $R_{43}$ form saturated or unsaturated $C_{3-8}$ cycloalkyl, saturated or unsaturated $C_{3-8}$ heterocycloalkyl, five-membered cyclic lactone, $C_{5-6}$ aryl, bridged cycloalkyl, or spirocycloalkyl, wherein H thereof is optionally substituted with -D, -OH, -F, $-NO_2$, -CN, $-CF_3$, $-C_2F_5$, $C_{1-3}$ alkyl, or amido; and

$R_{45}$ and $R_{46}$ are independently selected from the group consisting of -H, -F, -Cl, $-NH_2$, -CN, $-CF_3$, $-C_2F_5$, $-OCF_3$, alkynyl, $C_{1-3}$ linear alkyl, $C_{1-3}$ alkoxy, $C_{3-6}$ cycloalkyl, aryl, and heteroaryl; $R_{47}$, $R_{48}$, and $R_{49}$ are independently selected from the group consisting of -H, -F, -Cl, -CN, $-CF_3$, and $-C_2F_5$; alternatively, $R_{47}$, $R_{48}$ and the carbon atom between $R_{47}$ and $R_{48}$ form saturated or unsaturated $C_{3-8}$ cycloalkyl or saturated or unsaturated $C_{3-8}$ heterocycloalkyl, wherein H thereof is optionally substituted with -D, -OH, -F, -CN, $-CF_3$, or $C_{1-3}$ alkyl.

Wherein, $R_{42}$, $R_{43}$ and the carbon atom between $R_{42}$ and $R_{43}$ on the benzene ring in formula (XIII) form saturated or unsaturated $C_{3-8}$ cycloalkyl, saturated or unsaturated $C_{3-8}$ heterocycloalkyl, five-membered cyclic lactone, $C_{5-6}$ aryl, bridged cycloalkyl, or spirocycloalkyl, selected from the group consisting of:

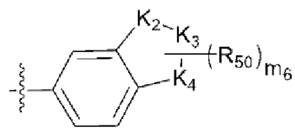 、 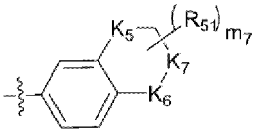 、 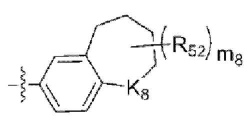 、 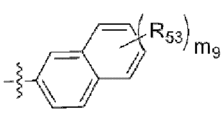 、

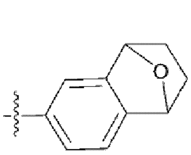 、 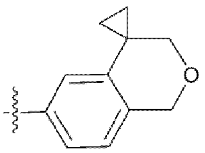 、 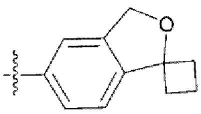 、 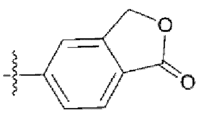 ;

;

wherein, $K_2$, $K_3$, and $K_4$ are fused with the benzene ring to form a saturated or unsaturated five-membered ring, $K_2$, $K_3$, and $K_4$ are independently selected from the group consisting of C, N, O, and S; $K_5$, $K_6$, and $K_7$ are independently selected from the group consisting of C, N, O, and S; $K_8$ is independently selected from the group consisting of C, N, O, and S; $R_{50}$, $R_{51}$ , $R_{52}$, and $R_{53}$ are independently selected from the group consisting of -H, -D, -OH, -F, $-NO_2$, -CN, $-CF_3$, $-C_2F_5$, $C_{1-3}$ alkyl, $C_{1-3}$ alkoxy, and $C_{3-6}$ cycloalkyl; m6, m7, m8, and m9 are each an integer of 0 to 6;

$R_{47}$, $R_{48}$ and the carbon atom between $R_{47}$ and $R_{48}$ on the benzene ring in formula (XIV) form saturated or unsaturated $C_{3-8}$ cycloalkyl or saturated or unsaturated $C_{3-8}$ heterocycloalkyl, selected from the group consisting of::

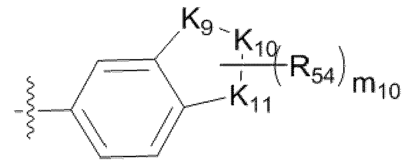

$K_9$, $K_{10}$, and $K_{11}$ are independently selected from the group consisting of C, N, and O; $R_{54}$ is independently selected from the group consisting of -H, -F, -CN, $-CF_3$, $-C_2F_5$, $C_{1-3}$ alkyl, $C_{1-3}$ alkoxy, and $C_{3-6}$ cycloalkyl; and m10 is an integer of 0 to 4.

Preferably, the structure represented formula (I) is selected from the group consisting of:

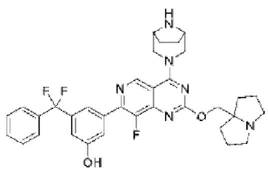 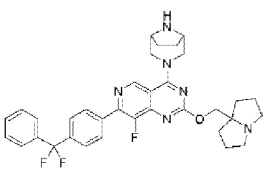 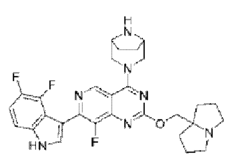 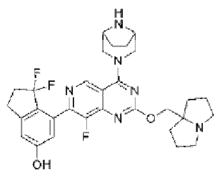 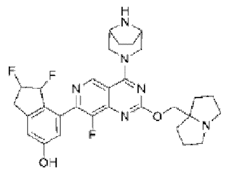

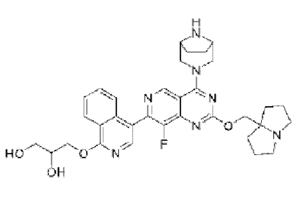 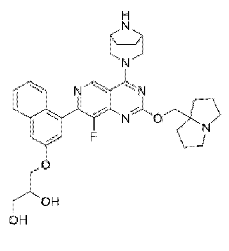 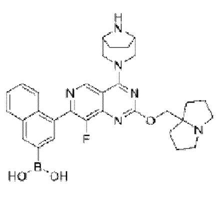 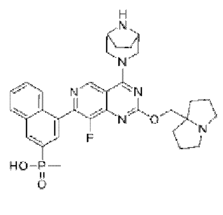 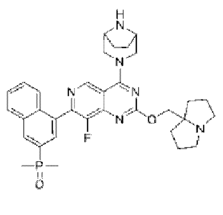

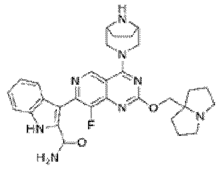 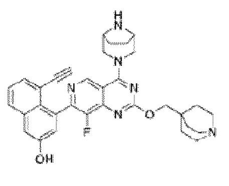 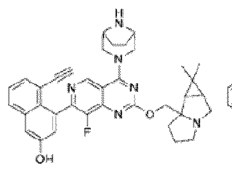 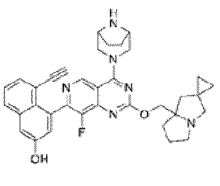 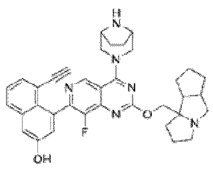

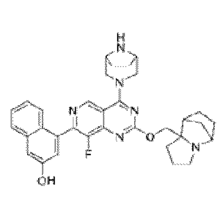
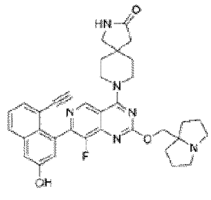
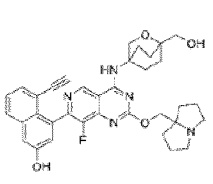
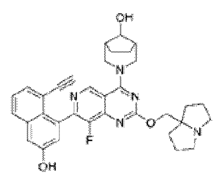
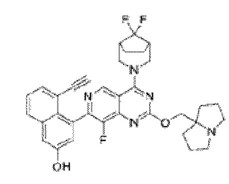

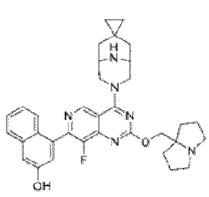
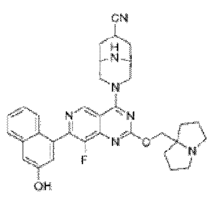
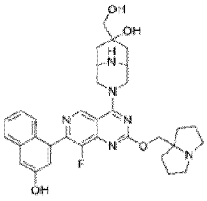
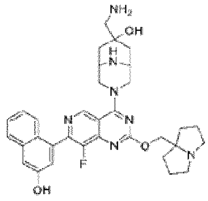
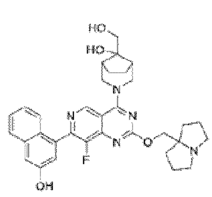

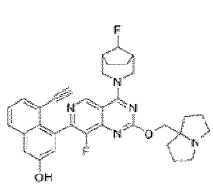
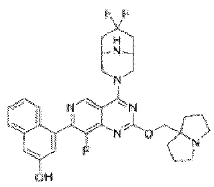
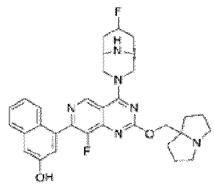
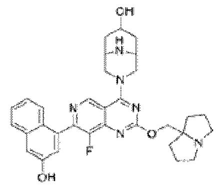
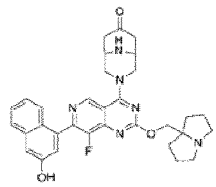

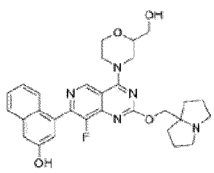
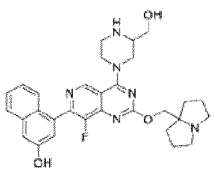
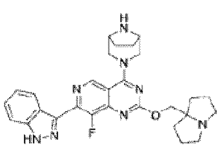
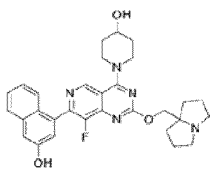
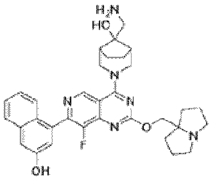

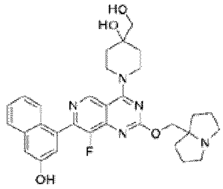
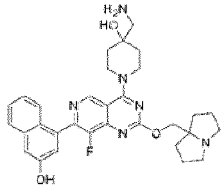
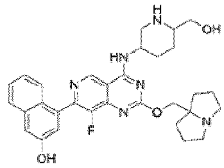
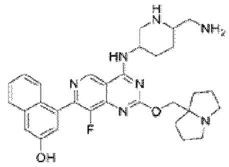
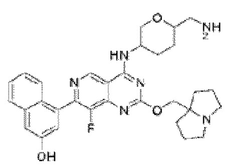

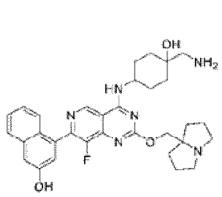
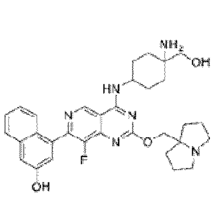
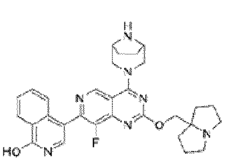
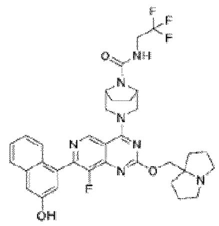
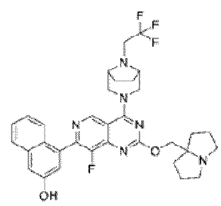

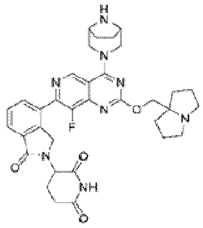
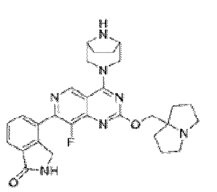
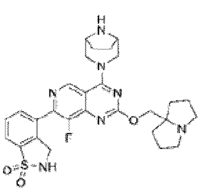
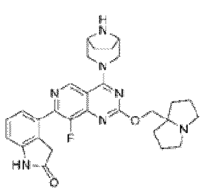
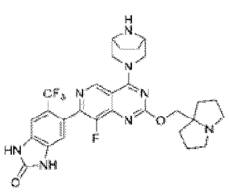

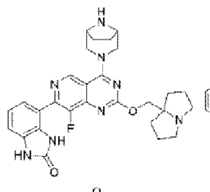 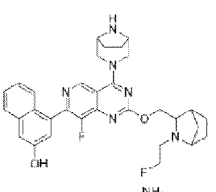 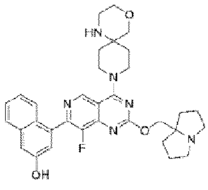 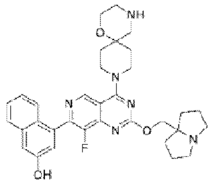 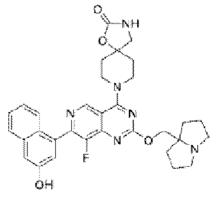

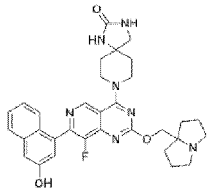 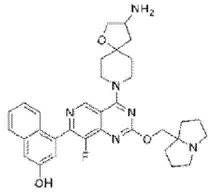 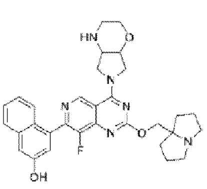 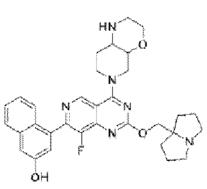 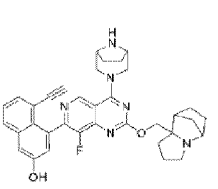

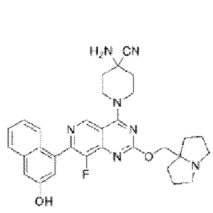 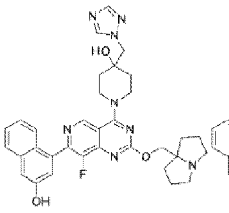 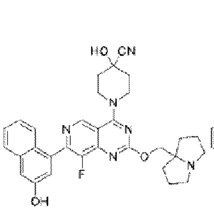 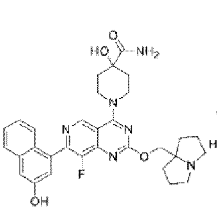 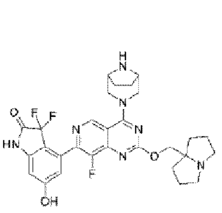

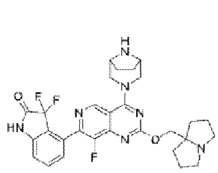 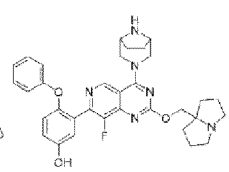 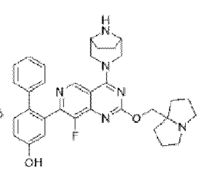 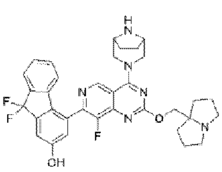 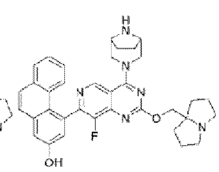

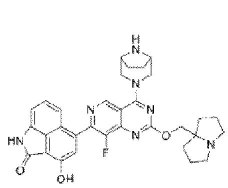 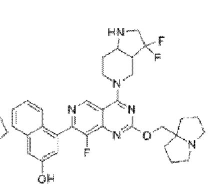 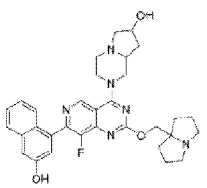 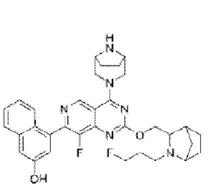 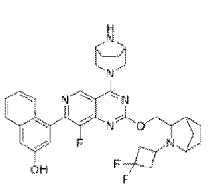

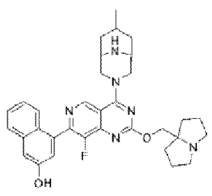 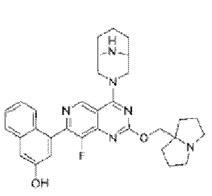 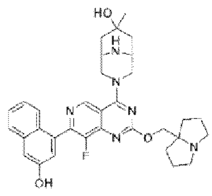 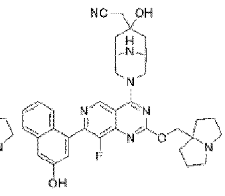 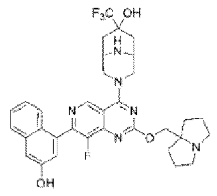

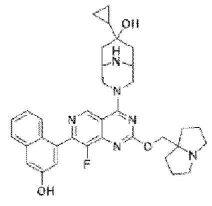 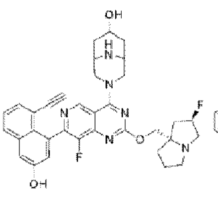 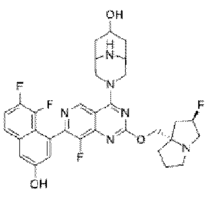 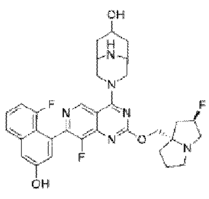 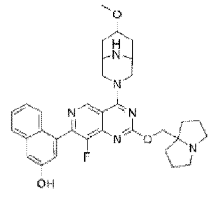

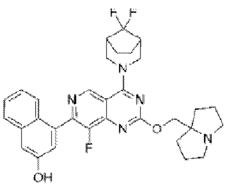 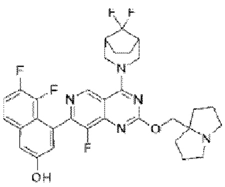 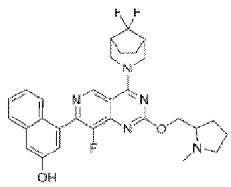 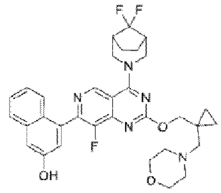 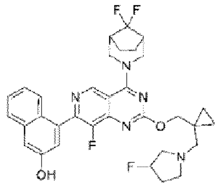

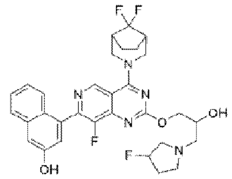 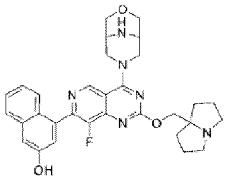 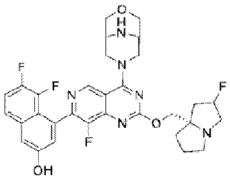 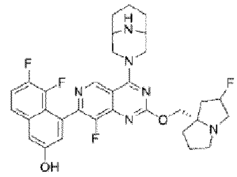 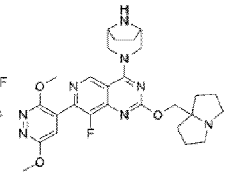

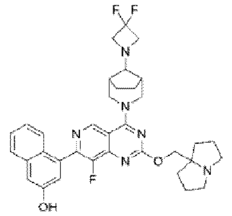 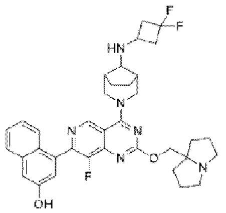 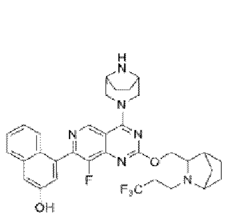 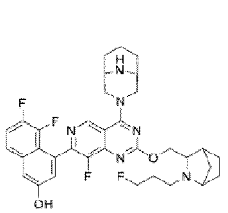 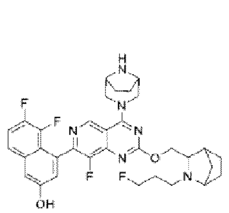

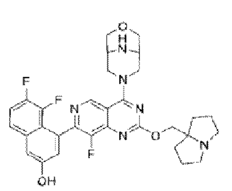 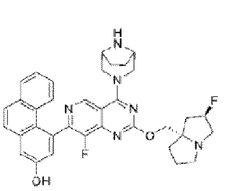 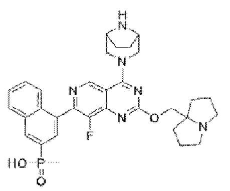 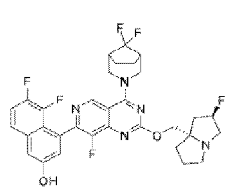 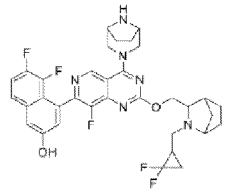

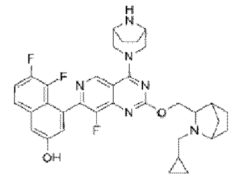 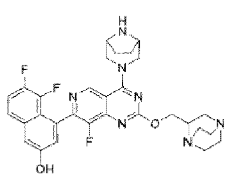 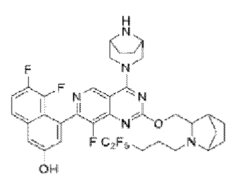 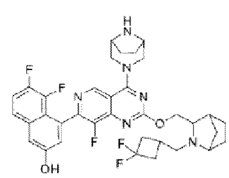 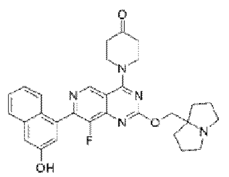

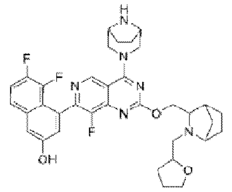 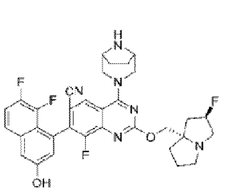 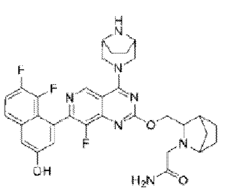 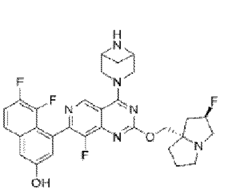 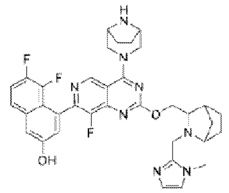

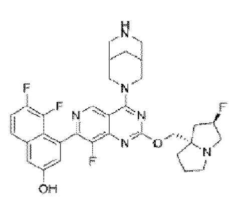 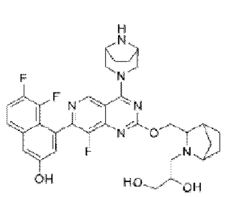 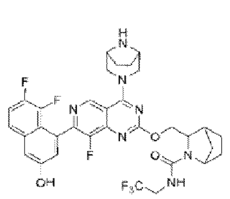 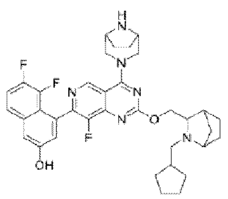 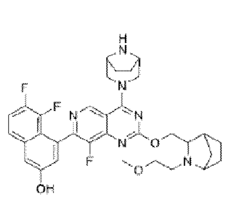

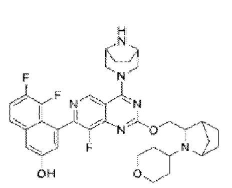 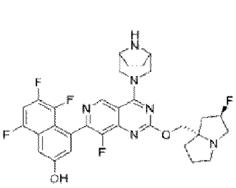 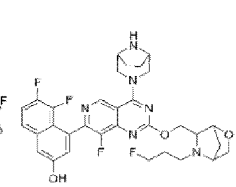 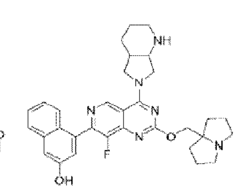 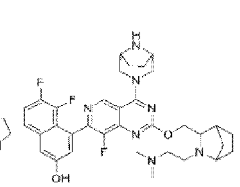

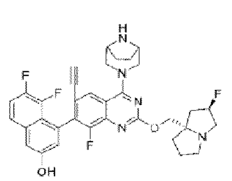 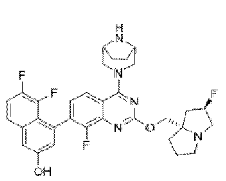 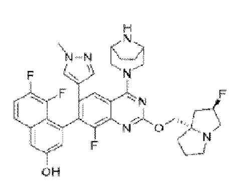 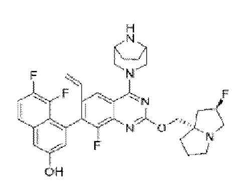 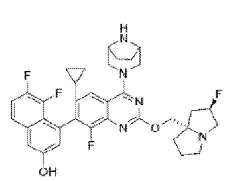

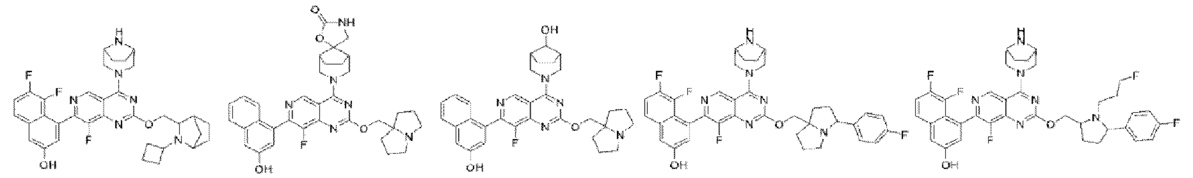

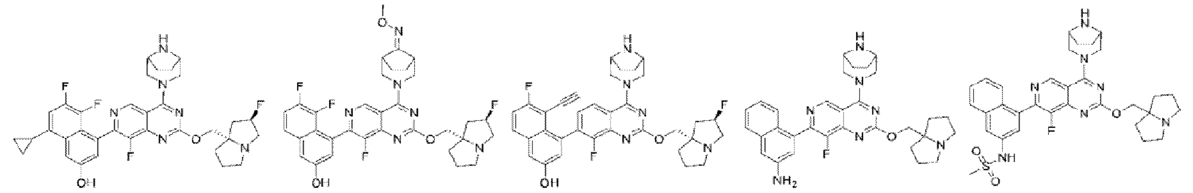

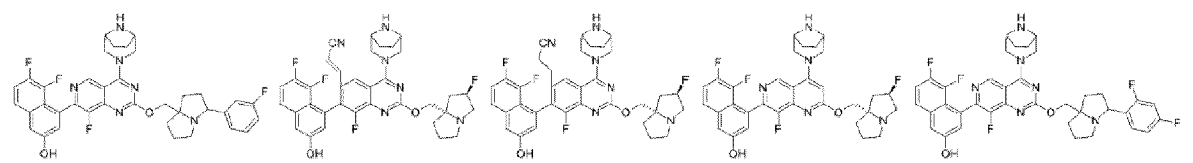

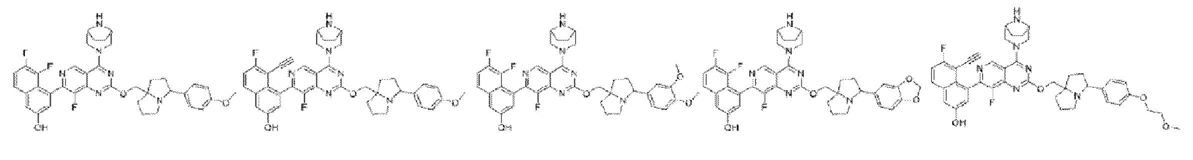

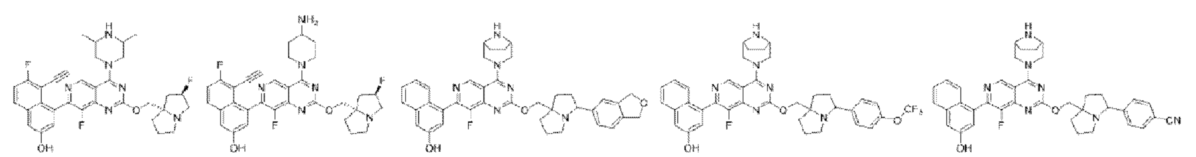

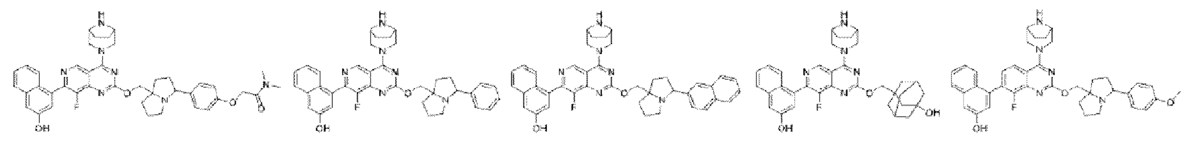

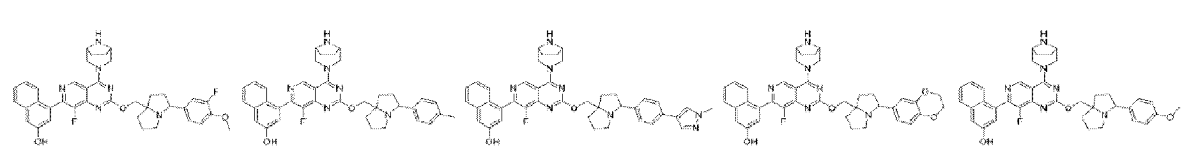

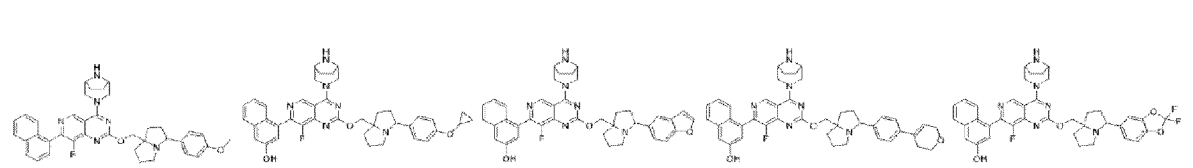

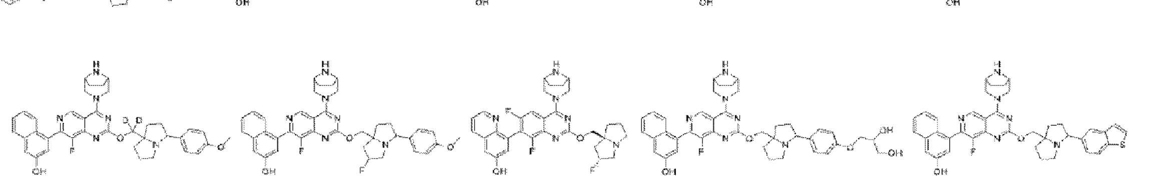

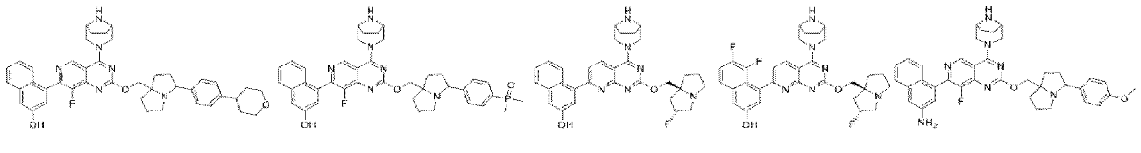

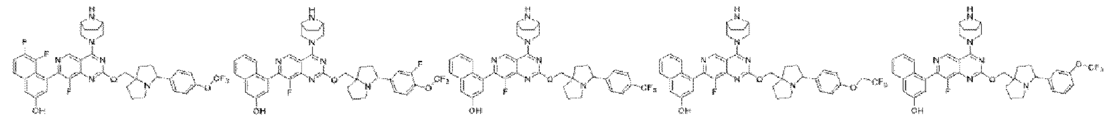

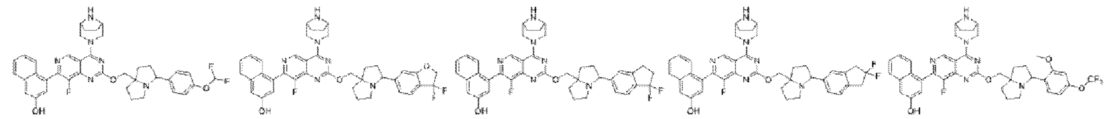

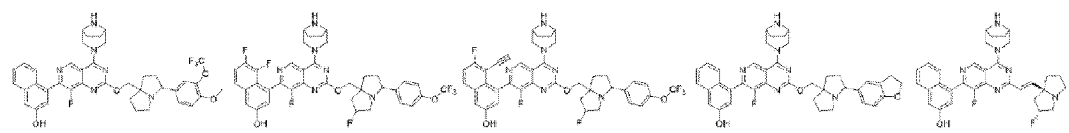

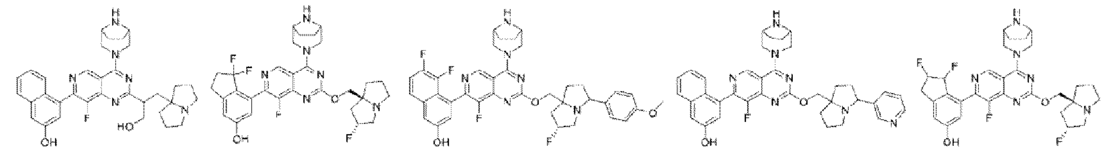

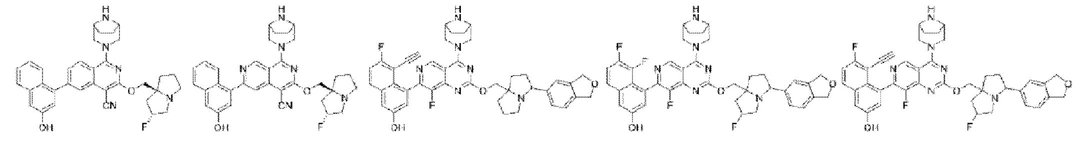

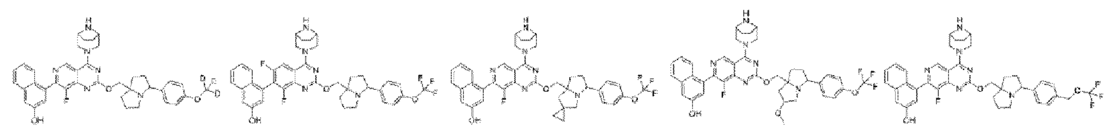

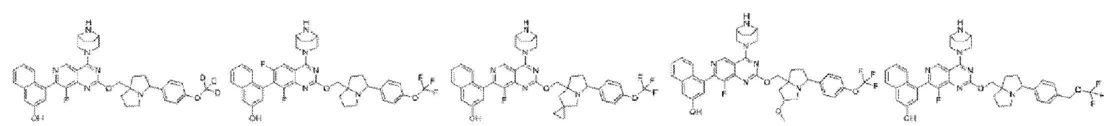

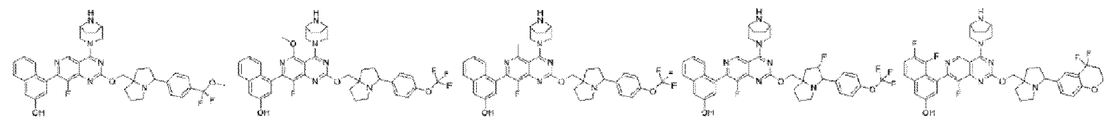

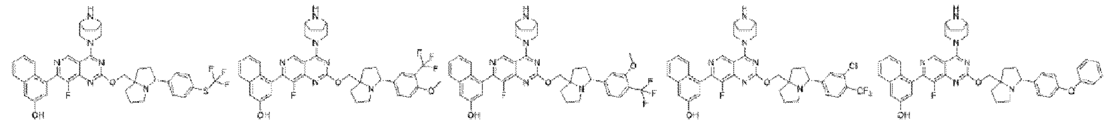

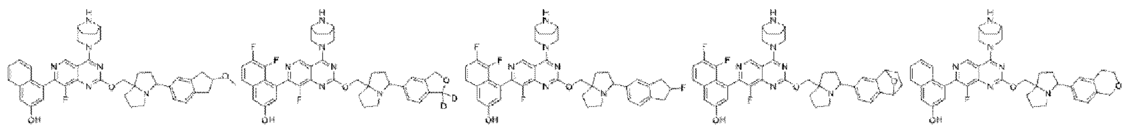

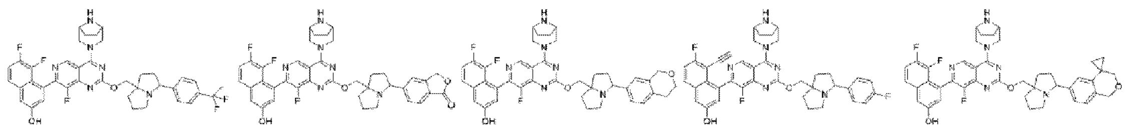

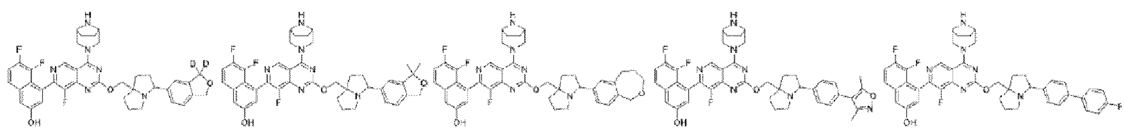

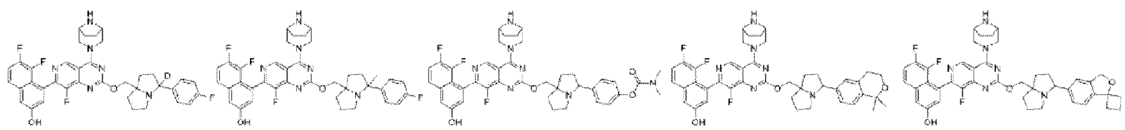

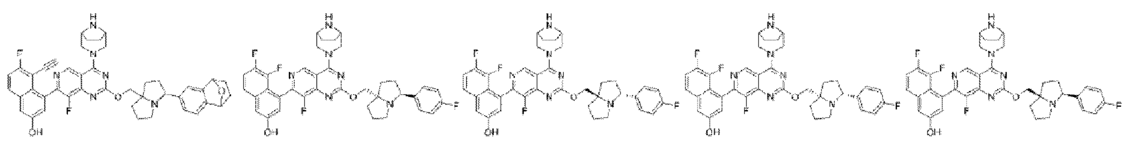

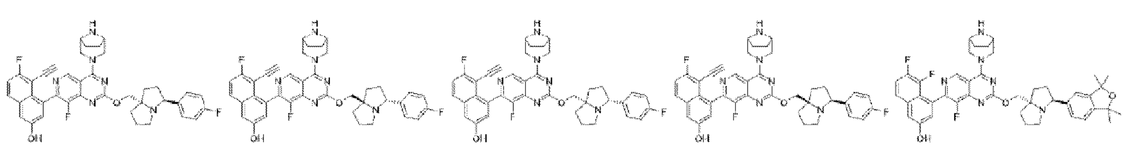

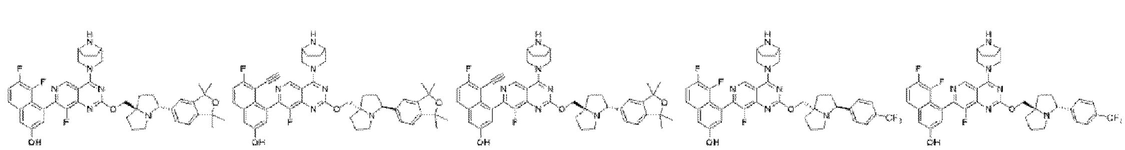

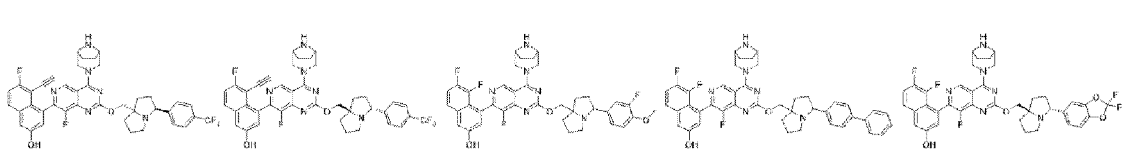

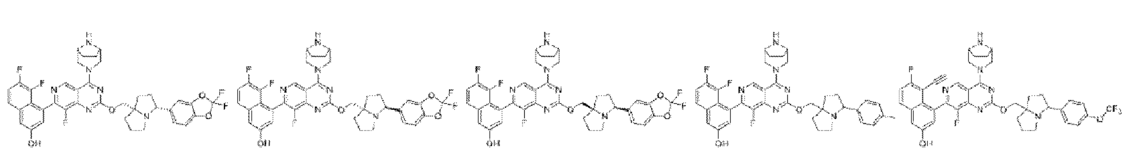

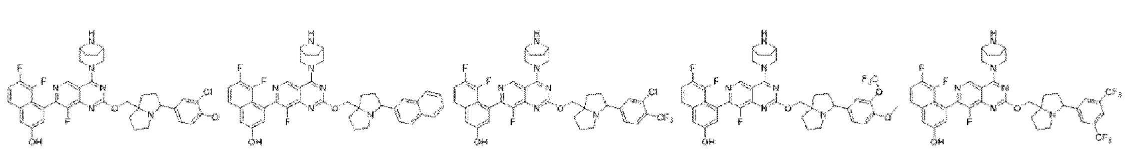

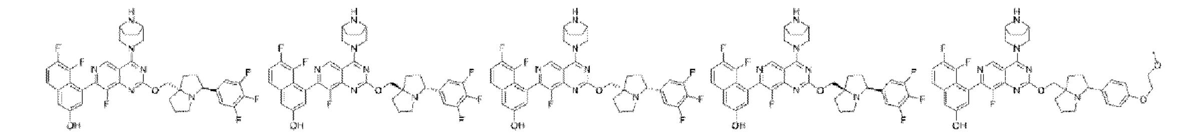

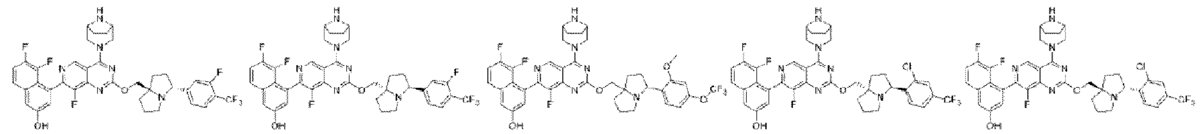

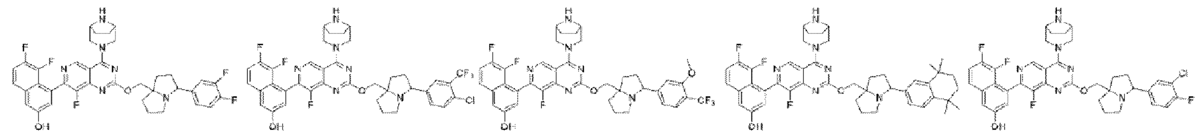

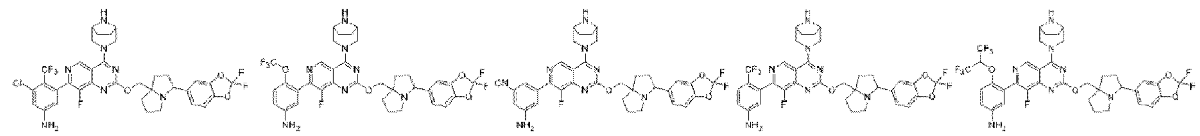

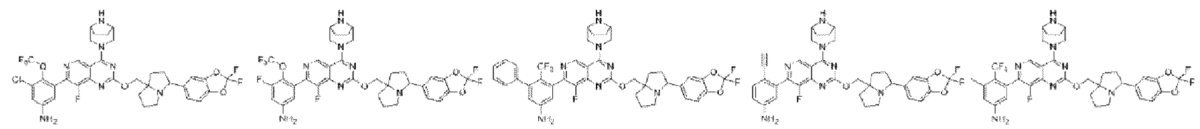

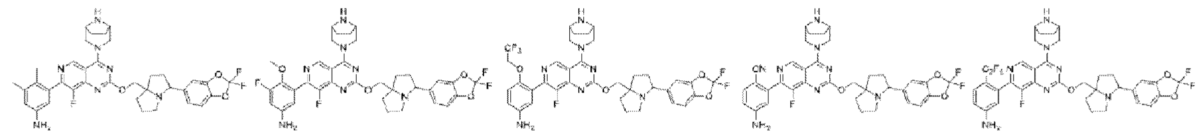

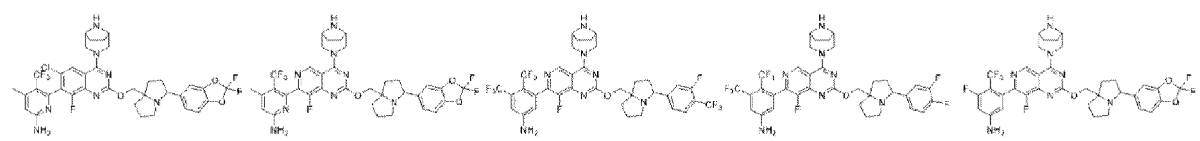

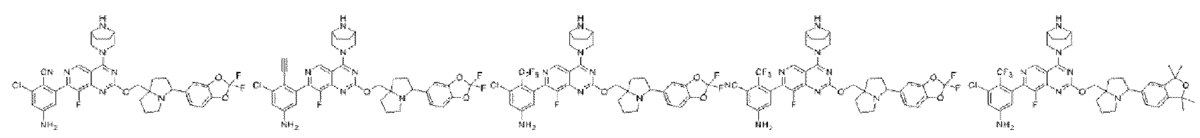

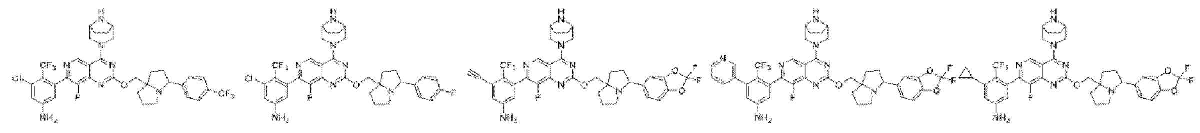

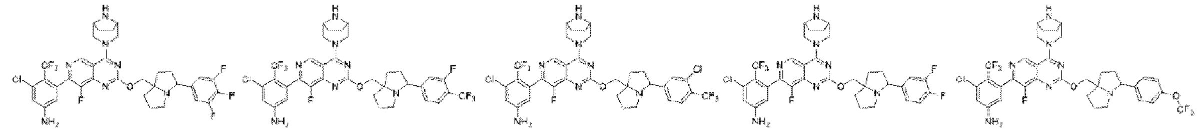

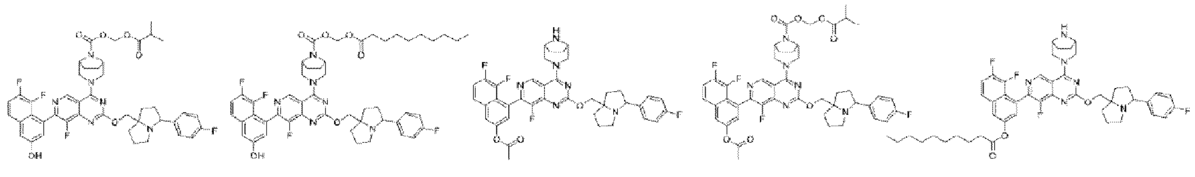

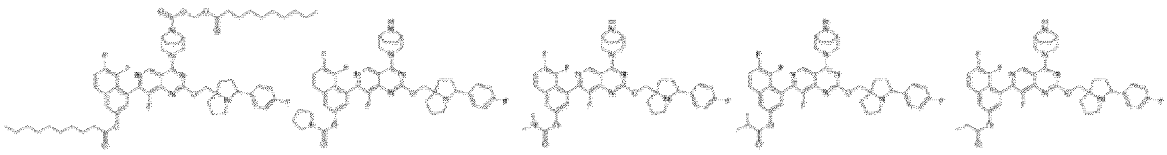

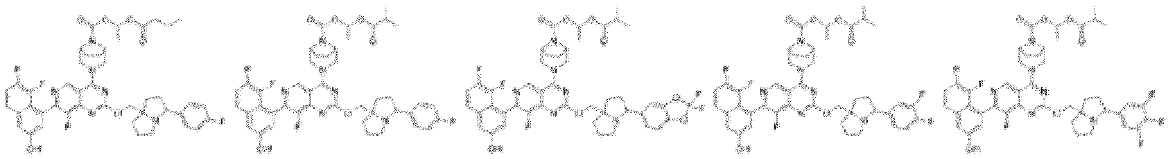

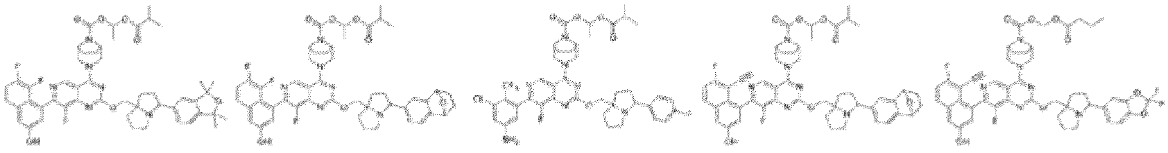

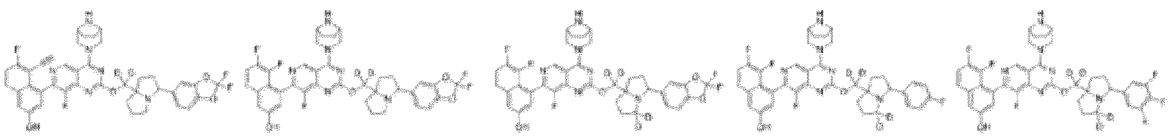

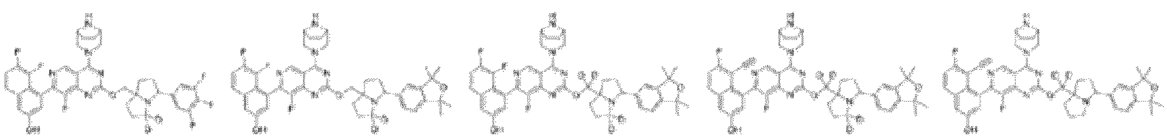

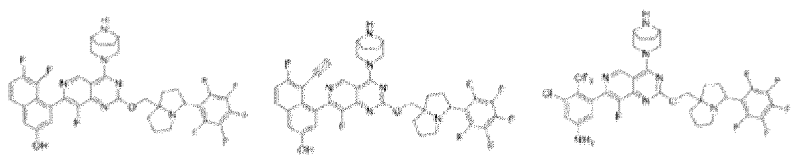

The present disclosure provides a pharmaceutical composition an active compound selected from the group consisting of the compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to the above, and a combination thereof.

Preferably, the pharmaceutical composition further comprises a pharmaceutically acceptable adjuvant. The pharmaceutically acceptable adjuvant is selected from the group consisting of a carrier, diluent, excipient and a combination thereof. In addition, the present disclosure has no special restrictions on the formulation type of the pharmaceutical composition.

The present disclosure provides use of the compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to the above in the manufacture of a medicament for a KRAS inhibitor.

Preferably, the present disclosure provides use of the compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to the above in the manufacture of a

medicament for KRAS G12D mutation.

The present disclosure provides use of the compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to the above in the manufacture of a medicament for treating, alleviating or preventing a disease or condition associated with Noonan syndrome, LEOPARD syndrome, leukaemia, neuroblastoma, melanoma, oesophageal cancer, head and neck tumors, breast cancer, lung cancer, pancreatic cancer, pancreatic ductal adenocarcinoma, colorectal and colon cancer.

For a clearer description of the present disclosure, the terms involved are defined below.

The term "pharmaceutically acceptable" as used herein refers to any substance that does not interfere with effectiveness of biological activity of active ingredients and is not toxic to the host to which it is administered.

The "pharmaceutically acceptable adjuvant" as used herein is a general term for all additional materials in the drug other than the active pharmaceutical ingredient. The adjuvants should have the following properties: (1) no toxicity to the human body and almost no side effects; (2) stable chemical properties insusceptible to temperature, pH, and storage time, etc.; (3) no incompatibility with the active pharmaceutical ingredient and no impact on its efficacy and quality analysis; (4) no interaction with packaging materials. The adjuvants in the present disclosure include but are not limited to fillers (diluents), lubricants (glidants or anti-adhesive agents), dispersants, wetting agents, binders, regulators, solubilizers, antioxidants, antibacterial agents, emulsifiers, and disintegrants, etc. Binders include syrup, gum arabic, gelatin, sorbitol, tragacanth, cellulose and its derivatives (such as microcrystalline cellulose, sodium carboxymethyl cellulose, ethyl cellulose or hydroxypropyl methyl cellulose), gelatin slurry, syrup, starch slurry or polyvinylpyrrolidone, etc. Fillers include lactose, powdered sugar, dextrin, starch and its derivatives, cellulose and its derivatives, inorganic calcium salts (such as calcium sulfate, calcium phosphate, calcium hydrogen phosphate, precipitated calcium carbonate, etc.), sorbitol or glycine, etc. Lubricants include micropowder silica gel, magnesium stearate, talc, aluminum hydroxide, boric acid, hydrogenated vegetable oil, polyethylene glycol, etc. Disintegrants include starch and its derivatives (such as sodium carboxymethyl starch, sodium starch glycolate, pregelatinized starch, modified starch, hydroxypropyl starch, corn starch, etc.), polyvinyl pyrrolidone or microcrystalline cellulose, etc. Wetting agents include sodium dodecyl sulfate, water or alcohol, etc. Antioxidants include sodium sulfite, sodium bisulfite, sodium metabisulfite, dibutyl benzoic acid, etc. Antibacterial agents include 0.5% phenol, 0.3% cresol, 0.5% trichlorobutanol, etc. Regulators include hydrochloric acid, citric acid, potassium hydroxide (sodium), sodium citrate and buffers (including sodium dihydrogen phosphate and disodium hydrogen phosphate), etc. Emulsifiers include polysorbate-80, sorbitan oleate, Pluronic F-68, lecithin, soybean lecithin, etc. Solubilizers include Tween-80, bile, glycerol, etc.

There are no particular limitations on the administration method of the compound or pharmaceutical composition of the present disclosure, and representative administration methods include (but are not limited to): oral, parenteral (intravenous, intramuscular or subcutaneous), and topical administration.

Solid dosage forms for oral administration include capsules, tablets, pills, powders and granules. In these solid dosage forms, the active compound is mixed with at least one conventional inert excipient (or carrier), such as sodium citrate or dicalcium phosphate, or with the following ingredients: (a) fillers or extenders, for example, starches, lactose, sucrose, glucose, mannitol, and silicic acid; (b) binders, for example, hydroxymethylcellulose, alginates, gelatin, polyvinyl pyrrolidone, sucrose, and gum arabic; (c) humectants, for example, glycerol; (d) disintegrants, for example, agar, calcium carbonate, potato starch or tapioca starch, alginic acid, certain complex silicates, and sodium carbonate; (e) retarders, for example, paraffin; (f) absorption accelerators, for example, quaternary ammonium compounds; (g) wetting agents, for example, cetyl alcohol and glyceryl monostearate; (h) adsorbents, for example, kaolin; and (i) lubricants, for example, talc, calcium stearate, magnesium stearate, solid polyethylene glycols, sodium lauryl sulfate, or mixtures thereof. In capsules, tablets and pills, the dosage forms may also comprise buffering agents.

Solid dosage forms such as tablets, sugar pills, capsules, pills and granules can be prepared using coatings and shell materials, such as enteric coatings and other materials known in the art. They may contain opacifiers, and the release of the active compound or compounds in such compositions may be delayed in a certain part of the digestive tract. Examples of embedding components that can be used are polymeric substances and waxes. If necessary, the active compound can also be formed into microencapsulated form with one or more of the above-mentioned excipients.

Liquid dosage forms for oral administration include pharmaceutically acceptable emulsions, solutions, suspensions, syrups or tinctures. In addition to the active compound, the liquid dosage form may contain an inert diluent conventionally used in the art, such as water or other solvents, solubilizers and emulsifiers, for example, ethanol, isopropanol, ethyl carbonate, ethyl acetate, propylene glycol, 1,3-butylene glycol, dimethylformamide and oils, in particular cottonseed oil, peanut oil, corn germ oil, olive oil, castor oil and sesame oil or mixtures thereof, etc.

In addition to these inert diluents, the compositions may also comprise adjuvants such as wetting agents, emulsifiers and suspensions, sweeteners, flavorings, and fragrances.

In addition to the active compounds, the suspension may comprise suspending agents such as ethoxylated isooctadecanol, polyoxyethylene sorbitol and sorbitan esters, microcrystalline cellulose, methanolic aluminum and agar, or mixtures thereof.

Compositions for parenteral injection may comprise physiologically acceptable sterile aqueous or anhydrous solutions, dispersions, suspensions or emulsions, as well as sterile powders for reconstitution into sterile injectable solutions or dispersions. Suitable aqueous and non-aqueous carriers, diluent, and solvent or excipients include water, ethanol, polyols, and mixtures thereof.

Dosage forms for topical administration of the compounds of the present disclosure include ointments, powders, patches, sprays and inhalants. The active ingredient is mixed under sterile conditions with a physiologically acceptable carrier and any preservatives, buffers, or propellants that may be required.

The compounds of the present disclosure can also be used in injection preparations, wherein the injection is selected from liquid injection (water injection), sterile powder for injection (powder injection) or injection tablets (referring to molded tablets or machine-pressed tablets made by aseptic operation of drugs, dissolved in water for injection before use, for subcutaneous or intramuscular injection).

The powder for injection comprises at least an excipient in addition to the above compound. The excipient in the present disclosure is a component intentionally added to the drug and should not have pharmacological properties in the amount used. However, the excipient can contribute to the processing, dissolution, or delivery of the drug through a targeted administration route, or can contribute to stability.

The term "substitution" refers to the replacement of a hydrogen atom in a molecule by another atom or molecule.

The term "member" refers to the number of backbone atoms constituting the ring.

The term "one bond" as used herein refers to only one linkage bond therein, which can also be understood as "none".

The term "alkyl" refers to an aliphatic hydrocarbon group, that is, a saturated hydrocarbon group. The alkyl moiety may be a linear alkyl group or a branched alkyl group. Typical alkyl groups include, but are not limited to, methyl, ethyl, propyl, isopropyl, butyl, isobutyl, tert-butyl, pentyl, hexyl, and the like.

C1-Cn as used herein includes C1-C2, C1-C3, ......, C1-Cn, where n is an integer greater than 1, and is used as a prefix of a substituent to indicate the minimum and maximum number of carbon atoms in the substituent. For example, "C1-C6 alkyl" refers to a linear or branched alkyl group containing one to six carbon atoms.

The term "heteroalkyl" refers to alkyl group containing a heteroatom, including alkoxy.

The term "alkenyl" refers to an aliphatic hydrocarbon group having at least one carbon-carbon double bond. The alkenyl may be linear or branched.

The term "alkynyl" refers to an aliphatic hydrocarbon group having at least one carbon-carbon triple bond. The alkynyl may be linear or branched.

The term "amido" is a chemical structure having the formula -C(O)NHR or -NHC(O)R, wherein R may be selected from alkyl, heteroalkyl, cycloalkyl, heterocycloalkyl, aryl, heteroaryl, and the like.

The term "sulfonyl" is a chemical structure having the formula $-S(=O)_2R$, including sulfonamido, wherein R may be selected from alkyl, heteroalkyl, cycloalkyl, heterocycloalkyl, aryl, heteroaryl, amino, and the like.

The term "phosphoryl" is a chemical structure having the formula -P(=O)RR', wherein R and R' are independently selected from alkyl, heteroalkyl, cycloalkyl, heterocycloalkyl, aryl, heteroaryl, hydroxy, amino, and the like.

The term "ester" is a chemical structure having the formula -COOR, wherein R is selected from alkyl, heteroalkyl, cycloalkyl, heterocycloalkyl, aryl, heteroaryl, and the like.

The term "acyl" is a chemical structure having the formula -C(O)R, wherein R is selected from alkyl, heteroalkyl, cycloalkyl, heterocycloalkyl, aryl, heteroaryl, and the like.

The term "cycloalkyl" refers to a saturated or unsaturated cyclic hydrocarbon substituent.

The term "heterocycloalkyl" refers to cycloalkyl having at least one heteroatom in the ring backbone.

Heteroatoms include but are not limited to O, S, N, P, Si, and the like.

The term "ring" refers to any covalent closed structure, including, for example, a carbocyclic ring (e.g., aryl or cycloalkyl), a heterocyclic ring (e.g., heteroaryl or heterocycloalkyl), an aromatic group (e.g., aryl or heteroaryl), a non-aromatic group (e.g., cycloalkyl or heterocycloalkyl). The "ring" as used herein may be a monocyclic ring or a polycyclic ring, and may be a fused ring, a spirocyclic ring, or a bridged ring

Typical cycloalkyl groups include but are not limited to:
cyclopropyl, cyclobutyl, cyclopentyl, cyclohexyl,

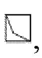, 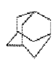,

and the like.

Typical heterocycloalkyl groups include but are not limited to:

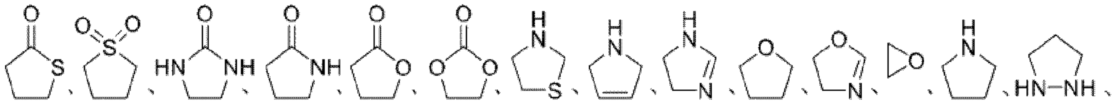

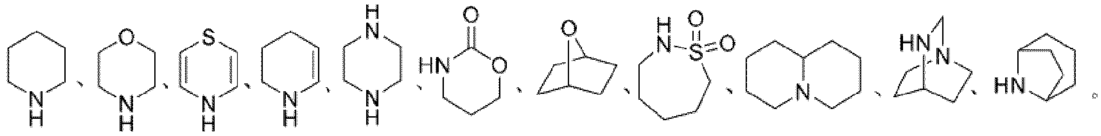

The term "aryl" refers to a planar ring having a delocalized π-electron system and containing 4n+2π electrons, where n is an integer. The aryl ring may comprise five, six, seven, eight, nine or more than nine atoms. Aryl group include but are not limited to phenyl, naphthyl, phenanthryl, anthryl, fluorenyl, and indenyl.

Typical heteroaryl groups include but are not limited to:

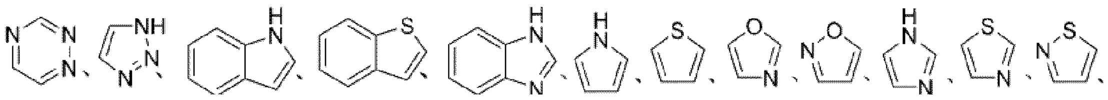

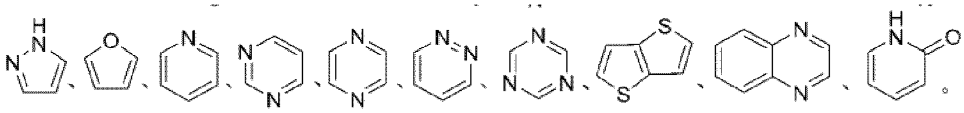

The term "halogen" or "halo" refers to fluorine, chlorine, bromine or iodine.

The term "deuterium" refers to an isotope of hydrogen (H), also known as heavy hydrogen, generally with the elemental symbol D or $^2$H.

The alkyl, heteroalkyl, cyclyl, heterocyclyl, amino, ester, carbonyl, amido, sulfonyl, phosphonyl, boric acid, borate, guanidino, acylguanidino, aryl, heteroaryl, and imino, as used herein, may be non-substituted alkyl, heteroalkyl, cyclyl, heterocyclyl, amino, ester, carbonyl, amido, sulfonyl, phosphonyl, boronic acid, borate, guanidino, acylguanidine, aryl, heteroaryl, and imino, or may be substituted alkyl, heteroalkyl, cyclyl, heterocyclyl, amino, ester, carbonyl, amido, sulfonyl, phosphonyl, boronic acid, borate, guanidino, acylguanidine, aryl, heteroaryl, and imino.

In the above, except where otherwise indicated, the term "substituted" means that the group mentioned may be substituted by one or more additional groups, each of which is independently selected from alkyl, cycloalkyl, aryl, carboxyl, heteroaryl, heterocycloalkyl, hydroxy, alkoxy, alkylthio, aryloxy, O=, guanidinyl, cyano, nitro, acyl, halogen, haloalkyl, amino, and the like.

The term "pharmaceutically acceptable salt" refers to a salt formed by the compound of the present disclosure and an acid or base suitable for use as a drug. The above-mentioned acid and base are Lewis acids and bases in a broad sense. Acids suitable for forming salts include but are not limited to: inorganic acids such as hydrochloric, hydrobromic, hydrofluoric, sulfuric, nitric, and phosphoric acids; organic acids such as formic, acetic, propionic, oxalic, malonic, succinic, fumaric, maleic, lactic, malic, tartaric, citric, picric, methanesulfonic, benzenemethanesulfonic, and benzenesulfonic acids; and acidic amino acids such as aspartic and glutamic acids.

The term "optional" or "optionally" means that the subsequently described event or circumstance may but does not necessarily occur, and the description includes instances where the event or circumstance occurs or does not occur. For example, "heterocycly optionally substituted with alkyl" means that alkyl may but is not necessarily be present, and the description includes instances where the heterocycly is substituted with alkyl and instances where the heterocycly is not substituted with alkyl.

Most KRAS missense mutations are found at codon 12, resulting in the conversion of glycine to other amino acids. The KRAS G12D and KRAS G12V mutations predominate, both of which are found in 90% of pancreatic cancers. Among them, KRAS G12D is also the most common KRAS mutation in colon cancer. Unfortunately, because the amino acid residues at the mutation site are difficult to be chemically bound, there are still no clinical or marketed drugs for KRAS G12D and KRAS G12V.

Mirati Therapeutics disclosed the first KRAS G12D inhibitor compound, MRTX1133 (WO2021041671), which can selectively bind to KRAS G12D mutant proteins and inhibit downstream ERK phosphorylation with high activity. Sustained dose-dependent inhibition of KRAS-dependent signals was shown in animal models of pancreatic cancer and colorectal cancer. However, the compound was originally scheduled to be launched in a clinical trial starting this year, and this candidate drug was previously expected to be a traditional oral or intravenous formulation, while Mirati has switched to

a long-acting formulation that requires a smaller infusion. Therefore, MRTX1133 may still have some undisclosed deficiencies, such as toxic and side effects. In addition, TAIHO PHARMACEUTICAL CO., LTD discloses a compound with KRAS G12D inhibitory activity (WO 2021/106231), which is still in the early stages of research.

Other strategies for the development of KRAS inhibitors include targeting upstream and downstream targets and indirectly targeting KRAS. However, these strategies may have problems such as bypass activation and inhibition of normal KRAS activity, and there has not been much research progress so far.

Therefore, it is of great clinical significance to find and develop new and efficient inhibitors of KRAS G12D mutation.

The present disclosure is described in detail below as compared to the prior art.

The present disclosure provides a novel class of compounds targeting KRAS G12D with unexpectedly excellent activity and pharmacokinetic properties.

The compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof of the present disclosure can be used to manufacture a medicament for treating, alleviating or preventing a disease or condition associated with Noonan syndrome, LEOPARD syndrome, leukaemia, neuroblastoma, melanoma, oesophageal cancer, head and neck tumors, breast cancer, lung cancer, pancreatic cancer, pancreatic ductal adenocarcinoma, colorectal and colon cancer.

The compound provided by the present disclosure can be used to treat a cancer caused by KRAS mutation, wherein the cancer caused by KRAS mutation is one or more of the cancers caused by KRAS G12C, KRAS G12V, KRAS G12A, and G12D mutations.

The compound provided by the present disclosure can effectively block the binding of KRAS G12D protein to SOS1, and the compound of the present disclosure show a significant improvement in KRAS G12D inhibitory activity, liver microsomal stability, and pharmacokinetics in rat.

The synthetic method of the present disclosure is easy to operate and has a high yield.

## DETAILED DESCRIPTION

For a better understand of the technical solutions of the present disclosure by those skilled in the art, the present disclosure will be further described in detail below in conjunction with specific embodiments.

In the present disclosure, the structures of the compounds are determined by mass spectrometry (MS) and/or nuclear magnetic resonance ($^1$H NMR) equipment. Chemical abbreviations have the following meanings:

DMF: N,N-dimethylformamide

THF: Tetrahydrofuran

DIPEA: N,N-diisopropylethylamine

PE: Petroleum ether

EA: Ethyl acetate

DCM: Dichloromethane

HATU: O-(7-azabenzotriazol-1-yl)-N,N,N',N'- tetramethylurea

DMSO: Dimethyl sulfoxide

### Synthesis of intermediates

**Preparation of intermediate 1:** Tert-butyl (1R,5S)-3-(7-chloro-8-fluoro-2-((tetrahydro-1H-pyrrolizin-7a(5H)-yl)methoxy)pyrido[4,3-d]pyri midin-4-yl)-3,8-diazabicyclo[3.2.1]octan-8-formate

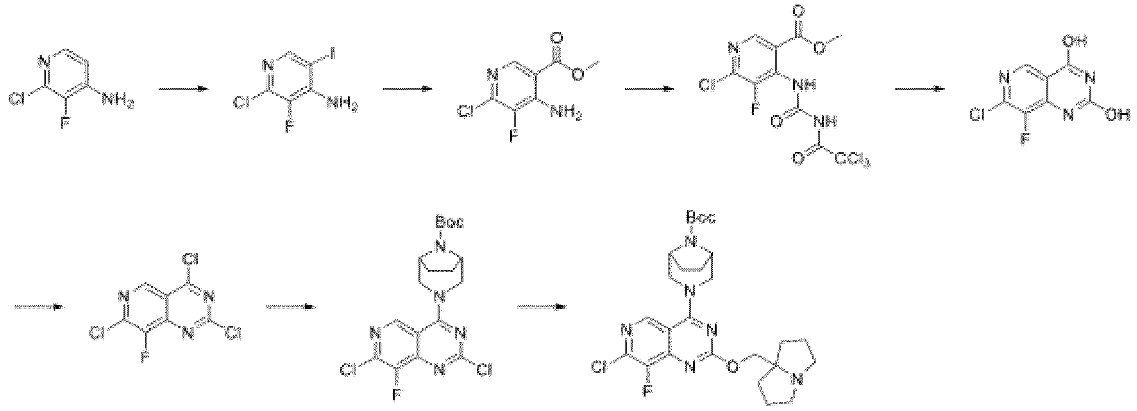

Step 1: Synthesis of 2-chloro-3-fluoro-5-iodopyridin-4-amine

2-Chloro-3-fluoro-4-aminopyridine (14.60 g, 100 mmol) was dissolved in 200 mL of acetonitrile, p-toluenesulfonic acid (0.86 g, 5 mmol) was added at room temperature, and then NIS (24.75 g, 110 mmol) was added in batches. Afterwards, this reaction system was stirred at 70°C overnight. The reaction system was poured into 1000 mL of water, and extracted with ethyl acetate. The organic phase was backwashed twice with an aqueous solution of sodium dithionite followed by saturated brine. After dried and evaporated under reduced pressure, it was purified by FCC (PE/EA=3/1) to obatin 25.00 g of yellow solid, with a yield of 92%.

Step 2: Synthesis of methyl 4-amino-6-chloro-5-fluoronicotinate

To a reaction flask, 2-chloro-3-fluoro-5-iodopyridin-4-amine (25.00 g, 91.9 mmol), triethylamine (30.30 g, 300 mmol) and 300 mL of methanol were added. After replacement with nitrogen, Pd(dppf)$Cl_2$ (3.66 g, 5 mmol) was added. Under carbon monoxide atmosphere (2 atmospheres), the reaction mixture was heated to 80°C and stirred overnight. The reaction system was cooled down to room temperature, poured into 1000 mL of water, and extracted with ethyl acetate. The organic phase was dried, evaporated under reduced pressure and purified by silica gel column chromatography (PE/EA=3/1) to obtain 14.50 g of yellow solid, with a yield of 78%.

Step 3: Synthesis of methyl 6-chloro-5-fluoro-4-(3-(2,2,2-trichloroacetyl)ureido)nicotinate

At room temperature, to a solution of methyl 4-amino-6-chloro-5-fluoronicotinate (6.3 g, 30.9 mmol) in 100 mL of THF, trichloroacetyl isocyanate (5.8 g, 30.9 mmol) was added and the reaction solution was stirred for 1 h at room temperature. The reaction system was evaporated under reduced pressure and the crude product was pulped with methyl tert-butyl ether to obtain 12.5 g of yellow solid, with a yield of 100%.

Step 4: Synthesis of 7-chloro-8-fluoropyrido[4,3-d]pyrimidin-2,4-diol

At room temperature, to a solution of methyl 6-chloro-5-fluoro-4-(3-(2,2,2-trichloroacetyl)ureido)nicotinate (12.5 g, 31.8 mmol) in 100 mL of methanol was added an ammonia/methanol solution (7 M, 20 mL), and the reaction solution was stirred for 1 h at room temperature. The reaction system was evaporated under reduced pressure, and the crude product was purified by FCC (PE/EA=3/1) to obtain 6.4 g of yellow solid with a yield of 94%.

Step 5: Synthesis of 2,4,7-trichloro-8-fluoropyrido[4,3-d]pyrimidine

At room temperature, 7-chloro-8-fluoropyrido[4,3-d]pyrimidin-2,4-diol (6.4 g, 29.8 mmol) was dissolved in 40 mL of phosphorus oxychloride, DIPEA (19.3 g, 150 mmol) was added, and the reaction solution was stirred at 100°C for 1 h. The reaction system was evaporated under reduced pressure, and the crude product was directly used for next step.

Step 6: Synthesis of tert-butyl (1R,5S)-3-(2,7-dichloro-8-fluoropyrido[4,3-d]pyrimidin-4-yl)-3,8-diazabicyclo [3.2.1]octan-8-formate

To a reaction flask, 2,4,7-trichloro-8-fluoropyrido[4,3-d]pyrimidine (crude product obtained in the previous step), DIPEA (25.8 g, 200 mmol) and 100 mL of dichloromethane were added, and tert-butyl (1R,5S)-3,8-diazabicyclo[3.2.1]octan-8-carboxylate (6.6 g, 31 mmol) was added at -40°C. The reaction system was stirred for 1 h at -40°C, then poured into 1000 mL of water, and extracted with ethyl acetate. The organic phase was dried, evaporated under reduced pressure and purified by silica gel column chromatography (PE/EA=3/1) to obtain 8.1 g of yellow solid with a yield of 64%.

Step 7: Synthesis of tert-butyl (1R,5S)-3-(7-chloro-8-fluoro-2-((tetrahydro-1H-pyrrolizin-7a(5H)-yl)methoxy)pyrido[4,3-d]pyri midin-4-yl)-3,8-diazabicyclo[3.2.1]octan-8-formate

To a reaction flask, tert-butyl (1R,5S)-3-(2,7-dichloro-8-fluoropyrido[4,3-d]pyrimidin-4-yl)-3,8-diazabicyclo [3.2.1]octan-8-for mate (500 mg, 1.2 mmol), DIPEA (313 mg, 2.5 mmol), (tetrahydro-1H-pyrrolizin-7a(5H)-yl) methanol (282 mg, 2.0 mmol) and 5 mL of dioxane were added, and the mixture was stirred at 80°C overnight. The reaction solution was poured into 20 mL of water, and extracted with ethyl acetate. The organic phase was dried, evaporated under reduced pressure and purified by silica gel column chromatography (DCM/MeOH=20/1) to obtain 250 mg of yellow solid with a yield of 40%.

Preparation of intermediate 2: 2-(3-(methoxymethoxy)naphthalen-1-yl)-4,4,5,5-tetramethyl-1,3,2-dioxaborolane

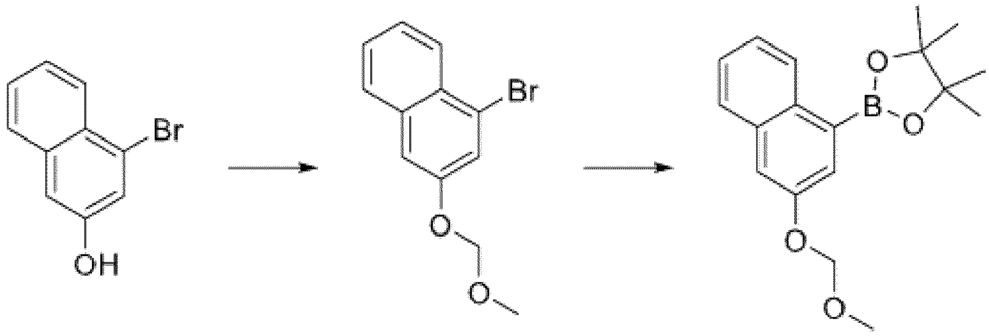

Step 1: Synthesis of 1-bromo-3-(methoxymethoxy)naphthalene

At 0°C, to a solution of 4-bromonaphthalen-2-ol (10.0 g, 44.8 mmol) and DIPEA (17.4 g, 134.5 mmol) in 100 mL of dichloromethane, chloromethyl methyl ether (3.6 g, 45.0 mmol) was added dropwise, and then the mixture was stirred at room temperature for 1 h. The reaction solution was poured into 500 mL water and extracted with ethyl acetate. The organic phase was dried, evaporated under reduced pressure, and then purified by silica gel column chromatography (PE/EA=20/1) to obtain 11.5 g of yellow oil with a yield of 96%.

Step 2: Synthesis of 2-(3-(methoxymethoxy)naphthalen-1-yl)-4,4,5,5-tetramethyl-1,3,2-dioxaborolane

To a reaction flask, 1-bromo-3-(methoxymethoxy)naphthalene (10.0 g, 37.5 mmol), bis(pinacolato)diboron (10.2 g, 40 mmol), potassium acetate (7.8 g, 80 mmol) and 100 mL of dioxane were added. After replacement with nitrogen, Pd(dppf)$Cl_2$ (1.5 g, 2 mmol) was added. Under the protection of nitrogen, the reaction mixture was heated to 80°C and stirred overnight. The reaction system was cooled down to room temperature, then poured into 500 mL water and extracted with ethyl acetate. The organic phase was dried, evaporated under reduced pressure, and then purified by silica gel column chromatography (PE/EA=10/1) to obtain 10.5 g of white solid with a yield of 89%.

**Preparation of intermediate 3:** triisopropyl((6-(methoxymethoxy)-8-(4,4,5,5-tetramethyl-1,3,2-dioxaborolan-2-yl) naphthalen-1-yl)ethynyl)silane

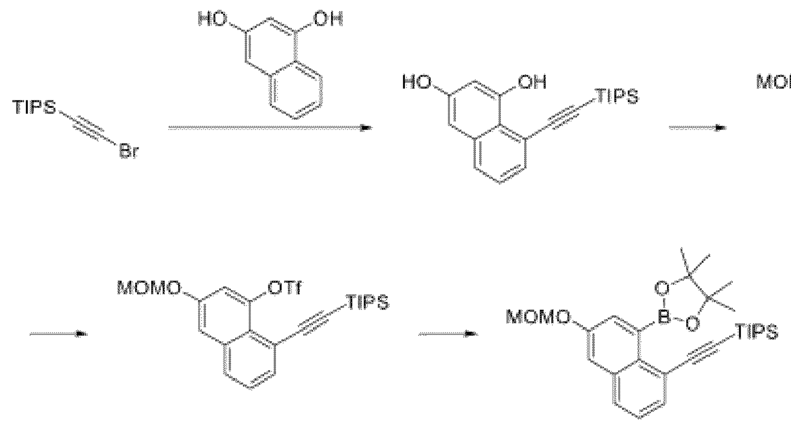

Step 1: Synthesis of 8-((triisopropylmethylsilyl)ethynyl)naphthalen-1,3-diol

To a reaction flask, naphthoresorcinol (10.0 g, 62.5 mmol), (bromoethynyl)triisopropylsilane (19.6 g, 75.0 mmol), potassium acetate (12.3 g, 125.0 mmol) and 100 mL of dioxane were added. After replacement with nitrogen, dichlorobis(4-methylisopropylphenyl)ruthenium (II) (3.8 g, 6.3 mmol) was added. Under the protection of nitrogen, the reaction mixture was heated to 100°C and stirred overnight. The reaction system was cooled down to room temperature, poured into 500 mL of water, and extracted with ethyl acetate. The organic phase was dried, evaporated under reduced pressure and purified by silica gel column chromatography (PE/EA=50/1) to obtain 11.5 g of yellow oil with a yield of 89%.

Step 2: Synthesis of 3-(methoxymethoxy)-8-((triisopropylmethylsilyl))ethynyl)naphthalen-1-ol

At 0°C, to a solution of 8-((triisopropylmethylsilyl)ethynyl)naphthalen-1,3-diol (11.5 g, 34.0 mmol) and DIPEA (17.4 g, 134.5 mmol) in 100 mL of dichloromethane, chloromethyl methyl ether (2.7 g, 34.0 mmol) was added dropwise, and then this mixture was stirred at room temperature for 1 h. The reaction solution was poured into 500 mL of water, and extracted with dichloromethane. The organic phase was dried, evaporated under reduced pressure, and then purified by silica gel column chromatography (PE/EA=50/1) to obtain 8.6 g of yellow oil with a yield of 66%.

Step 3: Synthesis of 3-(methoxymethoxy)-8-((triisopropylmethylsilyl))ethynyl)naphthalen-1-yl-trifluoromethanesulfo nate

At 0°C, to a solution of 3-(methoxymethoxy)-8-((triisopropylmethylsilyl)ethynyl)naphthalen-1-ol (8.6 g, 22.4 mmol) and DIPEA (5.8 g, 45.0 mmol) in 100 mL of dichloromethane, trifluoromethanesulfonic anhydride (7.0 g, 24.8 mmol) was added dropwise, and then this mixture was stirred at room temperature for 1 h. The reaction solution was poured into 500 mL of water, and extracted with dichloromethane. The organic phase was dried, evaporated under reduced pressure, and then purified by silica gel column chromatography (PE/EA=50/1) to obtain 5.5 g of yellow solid with a yield of 48%.

Step 4: Synthesis of triisopropyl ((6-(methoxymethoxy)-8-(4,4,5,5-tetramethyl-1,3,2-dioxaborolan-2-yl)naphthalen-1-yl)ethynyl)si lane

To a reaction flask, 3-(methoxymethoxy)-8-(triisopropylmethylsilyl)ethynyl)naphthalen-1-yltrifluoromethanesulfonat e (4.8 g, 9.3 mmol), bis(pinacolato)diboron (5.0 g, 19.7 mmol), potassium acetate (2.9 g, 29.6 mmol) and 50 mL of dioxane were added. After replacement with nitrogen, Pd(dppf)$Cl_2$ (0.7 g, 1 mmol) was added. Under the protection of nitrogen, the reaction mixture was heated to 100°C and stirred overnight. The reaction system was cooled down to room temperature, then poured into 200 mL of water, and extracted with ethyl acetate. The organic phase was dried, evaporated under reduced pressure, and then purified by silica gel column chromatography (PE/EA=20/1) to obtain 2.1 g of white solid with a yield of 46%.

**Preparation of intermediate 4:** ((2-fluoro-6-(methoxymethoxy)-(4,4,5,5-tetramethyl-1,3,2-dioxaborolan-2-yl)naphthalen-1-yl)eth ynyl) triisopropylsilane

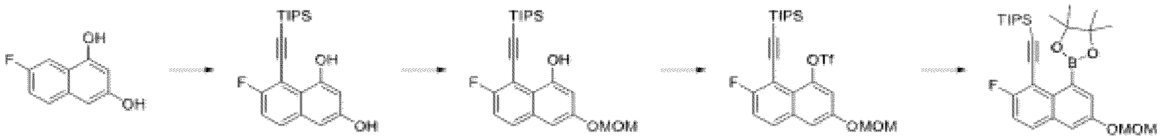

Step 1: Synthesis of 7-fluoro-8-((triisopropylmethylsilyl)ethynyl)naphthalen-1,3-diol

To a reaction flask, 7-fluoro-1,3-naphthalenediol (178 mg, 1.0 mmol), (bromoethynyl)triisopropylsilane (260 mg, 1.0 mmol), potassium acetate (196 mg, 2.0 mmol) and 5 mL of dioxane were added. After replacement with nitrogen, dichlorobis(4-methylisopropylphenyl)ruthenium(II) (60 mg, 0.1 mmol) was added. Under the protection of nitrogen, the reaction mixture was heated to 100°C and stirred overnight. The reaction system was cooled down to room temperature, then poured into 500 mL of water, and extracted with ethyl acetate. The organic phase was dried, evaporated under reduced pressure, and then purified by silica gel column chromatography (PE/EA=10/1) to obtain 295 mg of yellow oil with a yield of 82%.

Step 2: Synthesis of 7-fluoro-3-(methoxymethoxy)-8-((triisopropylmethylsilyl))ethynyl)naphthalen-1-ol

At 0°C, to a solution of 7-fluoro-8-((triisopropylsilyl)ethynyl)naphthalen-1,3-diol (295 mg, 0.84 mmol) in dichloromethane (5 mL), DIPEA (319 mg, 2.47 mmol) was added, and MOMCl (67 mg, 0.84 mmol) was added dropwise. After 2 h of reaction at 0°C, the system was slowly heated to room temperature. After reaction, the system was evaporated under reduced pressure and purified on a silica gel column (PE/EA=20/1) to obtain 210 mg of light yellow solid with a yield of 62%.

Step 3: Synthesis of 7-fluoro-3-(methoxymethoxy)-8-((triisopropylmethylsilyl)ethynyl)naphthalen-1-yltrifluorometha nesulfonate

At -78°C, to a solution of 7-fluoro-3-(methoxymethoxy)-8-((triisopropylmethylsilyl)ethynyl)naphthalen-1-ol (210 mg, 0.52 mmol) in dichloromethane, DIPEA (194 mg, 1.50 mmol) was added and trifluoromethanesulfonic anhydride (169 mg, 0.60 mmol) was slowly added dropwise. This system was stirred for 1 h at this temperature, then added with water to quench reaction, and extracted with ethyl acetate. The organic phases was combined, backwashed with water followed by saturated brine, dried over anhydrous sodium sulfate, evaporated under reduced pressure, and purified by Prep-TLC (PE/EA=20/1) to obtain 255 mg of light yellow solid with a two-step yield of 81%.

Step 4: Synthesis of ((2-fluoro-6-(methoxymethoxy)-8-(4,4,5,5-tetramethyl-1,3,2-dioxaborolan-2-yl)naphthalen-1-yl) ethynyl) triisopropylsilane

To a reaction flask, -fluoro-3-(methoxymethoxy)-8-((triisopropylmethylsilyl)ethynyl)naphthalen-1-yltrifluoromethan esulfonate (225 mg, 0.42 mmol), bis(pinacolato)diboron (127 mg, 0.50 mmol), potassium acetate (98 mg, 1.00 mmol) and 5 mL of dioxane were added. After replacement with nitrogen, Pd(dppf)$Cl_2$ (15 mg, 0.02 mmol) was added. Under the protection of nitrogen, the reaction mixture was heated to 100°C and stirred overnight. The reaction system was cooled down to room temperature, poured into 10 mL of water, and extracted with ethyl acetate. The organic phase was dried, evaporated under reduced pressure, and then purified by Prep-TLC (PE/EA=20/1) to obtain 140 mg of white solid with a yield of 65%.

**Preparation of intermediate 5:** 2-(7,8-difluoro-3-(methoxymethoxy)naphthalen-1-yl)-4,4,5,5- tetramethyl-1,3,2-dioxaborolane

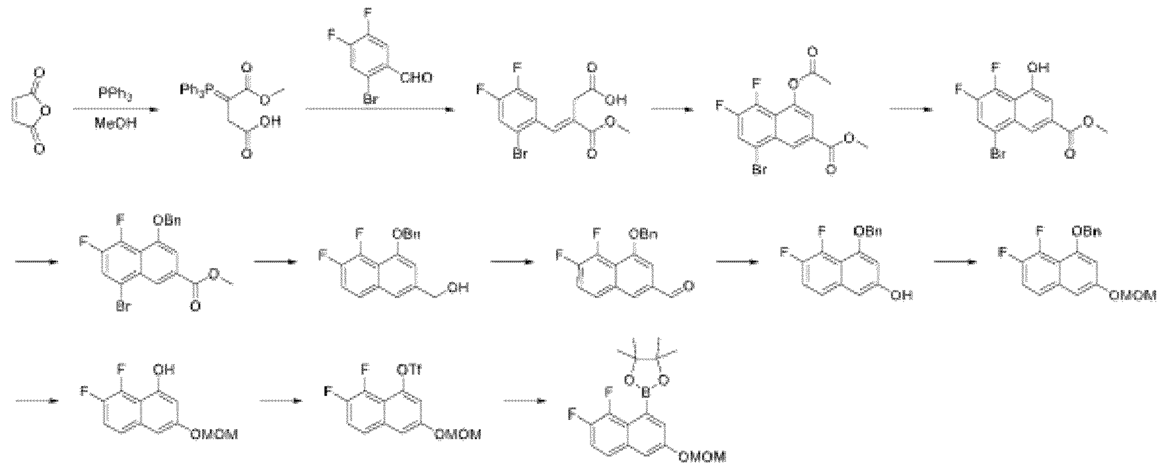


Step 1: Synthesis of 4-methoxy-4-oxo-3-(triphenyl-phosphinylidene) butanoic acid

Triphenylphosphine (52.46 g, 200 mmol) was dissolved in acetonitrile (800 mL), and a solution of maleic anhydride (19.5 g, 200 mmol) in acetonitrile was added dropwise at room temperature. After 30 min of dropwise addition, the reaction was carried out at room temperature for 3 h. Yellow solid precipitated. After filteration, the filter cake was collected, evaporated under reduced pressure, added with 800 mL of methanol, stirred at room temperature for 16 h of reaction. After all the solids were dissolved to obtain a clear solution, the reaction system was evaporated under reduced pressure to obtain 55 g of fluffy yellow solid with a yield of 84%.

Step 2: Synthesis of 4-(2-bromo-4,5-difluorophenyl)-3-(methoxycarbonyl)but-3-enoic acid

4-Methoxy-4-oxo-3-(triphenyl-phosphinylidene) butanoic acid (8.9 g, 22.73 mmol) was dissolved in a mixed solution of toluene (50 mL) and dichloromethane (10 mL). Under the protection of nitrogen, a solution of 2-bromo-4,5-difluorobenzaldehyde (5 g, 22.73 mmol) in dichloromethane was added dropwise for 16 h of reaction at room temperature. After reaction, the system was slowly added with sodium hydroxide aqueous solution, stirred for 5 min, and then extracted with ethyl acetate for three times. The water phase was collected, adjusted with an acid and extracted with ethyl acetate. The organic phases were combined and backwashed with sodium hydroxide solution. Again, the water phase was collected, adjusted with an acid and extracted. The resulting organic phase was dried and evaporated under reduced pressure to obtain 5.5 g of off-white solid with a yield of 72%.

Step 3: Synthesis of methyl 4-acetoxy-8-bromo-5,6-difluoro-2-naphthoate

To a single-necked flask, 4-(2-bromo-4,5-difluorophenyl)-3-(methoxycarbonyl)but-3-enoic acid (12 g, 35.9 mmol), sodium acetate (3.54 g, 43.2 mmol), and acetic anhydride (120 mL) were added, and the reaction was carried out at 140°C for 6 h. After reaction, the system was evaporated under reduced pressure at 70°C to remove acetic anhydride and the flash column was rinsed with petroleum ether: ethyl acetate = 1:1 to obtain 13.96 g of a black crude product, which was used directly for next step.

Step 4: Synthesis of methyl 8-bromo-5,6-difluoro-4-hydroxy-2-naphthoate

Potassium carbonate (10.9 g, 76.8 mmol) was added to a solution of the crude product obtained in the previous step in methanol (200 mL), and stirred at room temperature for 1 h of reaction. After reaction, the system was evaporated under reduced pressure and the crude product was directly used for next step.

Step 5: Synthesis of methyl 4-(benzyloxy)-8-bromo-5,6-difluoro-2-naphthoate

Benzyl bromide (8 g, 46.7 mmol) was slowly added dropwise to a solution of the crude product obtained in the previous step in DMF. Afterwards, the system was stirred at room temperature for 0.5 h. After reaction, the system was added with water and extracted with ethyl acetate for several times. The organic phases were combined, backwashed with water followed by saturated brine, and dried over anhydrous sodium sulfate. After filtration, the filtrate was evaporated under reduced pressure and purified by silica gel column (PE/EA=50/1) to obtain 9.5 g of yellow solid, with a three-step yield of 65%.

Step 6: Synthesis of (4-(benzyloxy)-5,6-difluoronaphthalen-2-yl)methanol

At -78°C, to a solution of methyl 4-(benzyloxy)-8-bromo-5,6-difluoro-2-naphthoate (9.5 g, 23.4 mmol) in tetrahydrofuran (100 mL), lithium aluminium hydride (1.3 g, 35.2 mmol) was added under the protection of nitrogen. The reaction was carried out at low temperature for 1 h. Afterwards, the reaction sysytem was slowly heated to room temperature for reaction overnight at room temperature. After reaction, the system was cooled down to 0°C, added with tetrahydrofuran for dilution, added slowly with water (2 mL), 15% sodium hydroxide solution (2 mL) and water (6 mL) sequentially, heated to room temperature and stirred for 15 min. The system was added with appropriate amount of anhydrous sodium sulfate, stirred at room temperature for 15 min, and filtered through a pad of celite. The resulting filtrate was evaporated under reduced pressure to obtain 7.5 g of light yellow solid. This crude product was used directly for next step.

Step 7: Synthesis of 4-(benzyloxy)-5,6-difluoro-2-naphthaldehyde

To a solution of (4-(benzyloxy)-5,6-difluoronaphthalen-2-yl)methanol (7.5 g, 25 mmol) in dichloromethane, manganese dioxide (20 g, 141 mmol) was added and the reaction was carried out at room temperature overnight. After reaction, the system was filtered through a pad of celite. The filtrate was evaporated under reduced pressure and purified on a silica gel column (PE/EA=20/1) to obtain 5.5 g of white solid with a yield of 73%.

Step 8: Synthesis of 4-(benzyloxy)-5,6-difluoronaphthalen-2-ol

To a solution of 4-(benzyloxy)-5,6-difluoro-2-naphthaldehyde (5.4 g, 18.1 mmmol) in dichloromethane (54 mL), meta-chloroperoxybenzoic acid (11 g, 70 mmol) was added under the protection of nitrogen and the reaction was carried out at room temperature overnight. After reaction, the system was filtered directly, and the filter cake was washed with a small amount of dichloromethane. The filtrate was evaporated under reduced pressure, dissolved in ethyl acetate, then added dropwise to an aqueous sodium carbonate solution, stirred for 1 h, and extracted with ethyl acetate. The organic phases were combined, backwashed with water followed by saturated brine, dried over anhydrous sodium sulfate and evaporated under reduced pressure. The resulting crude product was used directly for next step.

Step 9: Synthesis of 8-(benzyloxy)-1,2-difluoro-6-(methoxymethoxy)naphthalene

At 0°C, to a solution of the crude product obtained in the previous step in dichloromethane (50 mL), DIPEA (4.7 g, 36.4 mmol) was added and MOMCl (1.2 g, 14.8 mmol) was added dropwise. After 2 h of reaction at 0°C, the system was slowly heated to room temperature. After reaction, the system was evaporated under reduced pressure and purified on a silica gel column (PE/EA=20/1) to obtain 540 mg of light yellow solid with a two-step yield of 9%.

Step 10: Synthesis of 7,8-difluoro-3-(methoxymethoxy)naphthalen-1-ol

To a solution of 8-(benzyloxy)-1,2-difluoro-6-(methoxymethoxy)naphthalene (540 mg, 1.6 mmol) in methanol, palladium carbon (10%, 50 mg) was added. After replacement with hydrogen, the reaction was carried out under hydrogen atmosphere for 3 h. After reaction, the system was filtered through a pad of celite. The filter cake was washed with methanol and the filtrate was evaporated under reduced pressure to obtain a crude product, which was not further purified.

Step 11: Synthesis of 7,8-difluoro-3-(methoxymethoxy)naphthalen-1-yl trifluoromethanesulfonate

At -78°C, to a solution of the crude product obtained in the previous step in dichloromethane, DIPEA (645 mg, 5 mmol) was added and trifluoromethanesulfonic anhydride (517 mg, 1.83 mmol) was slowly added dropwise. The reaction was stirred for 1 h at this temperature. After reaction, the system was added with water to quench reaction, and extracted with ethyl acetate. The organic phases were combined, backwashed with water followed by saturated brine, dried over anhydrous sodium sulfate, evaporated under reduced pressure, and purified on a silica gel column (PE/EA=20/1) to obtain 420 mg of light yellow solid with a two-step yield of 69%.

Step 12: Synthesis of 2-(7,8-difluoro-3-(methoxymethoxy)naphthalen-1-yl)-4,4,5,5-tetramethyl-1,3,2-dioxaborolane

To a reaction flask, 7,8-difluoro-3-(methoxymethoxy)naphthalen-1-yltrifluoromethanesulfonate (372 mg, 1.0 mmol), bis(pinacolato)diboron (254 mg, 1.0 mmol), potassium acetate (196 mg, 2.0 mmol) and 5 mL of dioxane were added. After replacement with nitrogen, Pd(dppf)$Cl_2$ (37 mg, 0.05 mmol) was added. Under the protection of nitrogen, the reaction mixture was heated to 100°C and stirred overnight. The reaction system was cooled down to room temperature,

poured into 20 mL of water, and extracted with ethyl acetate. The organic phase was dried, evaporated under reduced pressure and purified by silica gel column chromatography (PE/EA=20/1) to obtain 240 mg of white solid, with a yield of 69%.

**Preparation of intermediate 6:** tert-butyl (1R,5S)-3-(7-chloro-8-fluoro-2-(((2S,3aR)-2-fluorohexahydropenten-3a(1H)-yl)methoxy)pyrido[ 4,3-d]pyrimidin-4-yl)-3,8-diazabicyclo[3.2.1]octan-8-formate

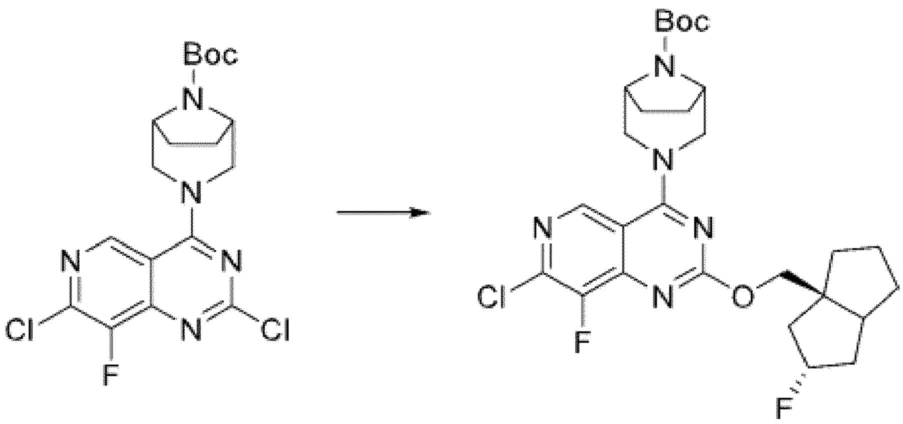

To a reaction flask, tert-butyl (1R,5S)-3-(2,7-dichloro-8-fluoropyrido[4,3-d]pyrimidin-4-yl)-3,8-diazabicyclo [3.2.1]octan-8-for mate (500 mg, 1.2 mmol), DIPEA (313 mg, 2.5 mmol), ((2R,7aS)-2-fluorotetrahydro-1H-pyrrolizin-7a(5H)-yl)methanol (318 mg, 2.0 mmol) and 5 mL of dioxane were added. This mixture was stirred at 80°C overnight. The reaction solution was poured into 20 mL of water, and extracted with ethyl acetate. The organic phase was dried, evaporated under reduced pressure and purified by silica gel column chromatography (DCM/MeOH=20/1) to obtain 275 mg of yellow solid with a yield of 43%.

**Preparation of intermediate 7:** tert-butyl (1R,5S)-3-(7-bromo-2-chloro-8-fluoroquinazolin-4-yl)-3,8-diazabicyclo [3.2.1]octan-8-carboxylat e

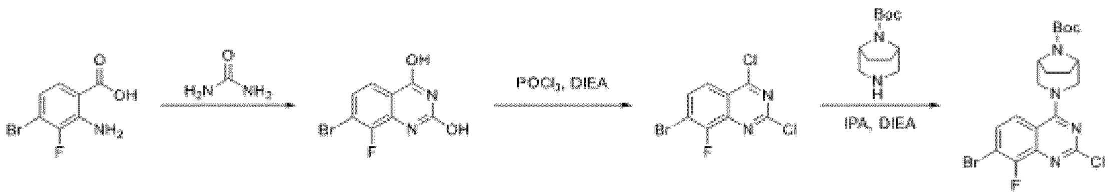


Step 1: Synthesis of 7-bromo-8-fluoroquinazolin-2,4-diol

To a reaction flask, 2-amino-4-bromo-3-fluorobenzoic acid (1.0 g, 4.27 mmol), and urea (3.0 g, 50.00 mmol) were added, and the reaction system was heated to 170°C and stirred for 4 h. The reaction system was cooled down to room temperature, and extracted three times with water and ethyl acetate. The organic phases were combined, washed with saturated brine, dried over anhydrous sodium sulfate, filtered, and the filtrate was concentrated under reduced pressure and then purified by passing through a column (PE/EA = 5/1) to obtain 675 mg of target product, with a yield of 61%.

Step 2: Synthesis of 7-bromo-2,4-dichloro-8-fluoroquinazoline

To a reaction flask, 7-bromo-8-fluoroquinazolin-2,4-diol (300 mg, 1.16 mmol), phosphorus oxychloride (1.5 g, 9.80 mmol), and DIEA (650 mg, 5.04 mmol) were added, and the reaction system was heated to 90°C and stirred for 3 h. The reaction system was cooled down to room temperature and the filtrate was concentrated under reduced pressure to obtain a crude product, which was used directly for next step.

Step 3: Synthesis of tert-butyl (1R,5S)-3-(7-bromo-2-chloro-8-fluoroquinazolin-4-yl)-3,8-diazabicyclo[3.2.1]octan-8-carboxylat e

To a reaction flask, the crude product obtained in the previous step, DIEA (650 mg, 5.04 mmol), tert-butyl (1R,5S)-3,8-diazabicyclo[3.2.1]octan-8-carboxylate (200 mg, 0.95 mmol), and 30 mL of anhydrous ethanol were added, and the reaction system was heated to 80°C and stirred for 4 h. The reaction system was cooled down to room temperature, and extracted three times with water and ethyl acetate. The organic phases were combined, washed with saturated brine, dried over anhydrous sodium sulfate, filtered, and the filtrate was concentrated under reduced pressure and purified by column chromatography (PE/EA = 8/1) to obtain 190 mg of target product, with a two-step yield of 35%.

**Example 1**

3-(4-((1R,5S)-3,8-diazabicyclo[3.2.1]octyl-3-yl)-8-fluoro-2-((tetrahydro-1H-pyrrolizin-7a(5H)-yl)methoxy)pyrido[4,3-d]pyrimidin-7-yl)-5-(difluoro(phenyl)methyl)phenol

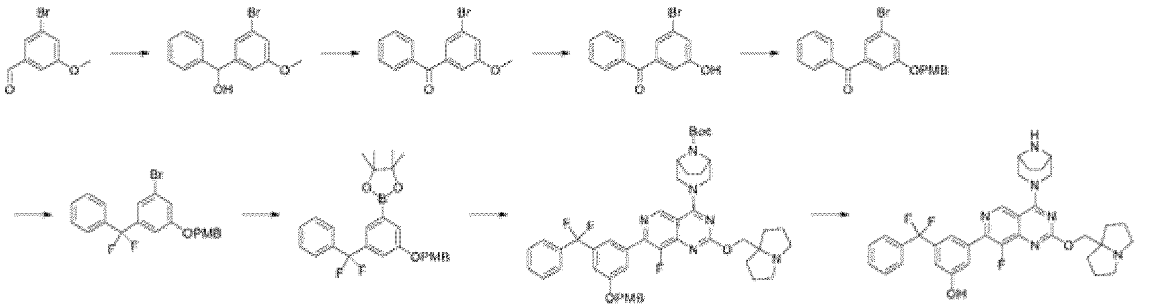

Step 1: Synthesis of (3-bromo-5-methoxyphenyl)(phenyl)methanol

At 0°C and under the protection of nitrogen, phenylmagnesium bromide (1 M, 11 mL, 11.0 mmol) was added dropwise to a solution of 3-bromo-5-methoxybenzaldehyde (2.15 g, 10.0 mmol) in 20 mL of THF, and the reaction mixture was stirred at 0°C for 1 h. The reaction system was poured into 100 mL of water, and extracted with ethyl acetate. The organic phase was dried, evaporated under reduced pressure, and purified by silica gel column chromatography (PE/EA=10/1) to obtain 2.8 g of light yellow solid, with a yield of 96%.

Step 2: Synthesis of (3-bromo-5-methoxyphenyl)(phenyl)methanone

To a solution of (3-bromo-5-methoxyphenyl)(phenyl)methanol (2.8 g, 9.6 mmol) in 50 mL of dichloromethane was added activated manganese dioxide (4.4 g, 50.6 mmol), and the reaction mixture was stirred at room temperature overnight. The reaction mixture was subjected to suction filteration, and the filtrate was evaporated under reduced pressure and purified by silica gel column chromatography (PE/EA=50/1) to obtain 2.6 g of light yellow solid with a yield of 93%.

Step 3: Synthesis of (3-bromo-5-hydroxyphenyl)(phenyl)methanone

(3-Bromo-5-methoxyphenyl)(phenyl)methanone (2.0 g, 6.9 mmol) was dissolved in 20 mL of dichloromethane, and $BBr_3$ (2.5 g, 10.0 mmol) was added at 0°C. The reaction mixture was stirred at room temperature overnight. The reaction system was poured into 100 mL of water, and extracted with ethyl acetate. The organic phase was dried, evaporated under reduced pressure, and purified by silica gel column chromatography (PE/EA=10/1) to obtain 1.2 g of yellow solid, with a yield of 63%.

Step 4: Synthesis of (3-bromo-5-((4-methoxybenzyl)oxy)phenyl)(phenyl)methanone

To a solution of (3-bromo-5-hydroxyphenyl)(phenyl)methanone (277 mg, 1.0 mmol) and potassium carbonate (276 mg, 2.0 mmol) in 50 mL of acetonitrile, para-methoxybenzoic bromide (241 mg, 1.2 mmol) was added dropwise at 0°C. Afterwards, the mixture was stirred at room temperature for 5 h. The system was subjected to suction filtration, and the filtrate was dried to obtain 370 mg of yellow solid, with a yield of 93%.

Step 5: Synthesis of 1-bromo-3-(difluoro(phenyl)methyl)-5-((4-methoxybenzyl)oxy)benzene

To a reaction flask, (3-bromo-5-((4-methoxybenzyl)oxy)phenyl)phenyl)methanone (370 mg, 0.9 mmol) and 1 mL of bis(2-methoxyethyl)aminosulfur trifluoride were added. The reaction mixture was stirred at 80°C for 2 h. The reaction system was poured into 10 mL of ice water, and extracted with ethyl acetate. The organic phase was dried, evaporated under reduced pressure, and purified by Prep-TLC (PE/EA=30/1) to obtain 80 mg of light yellow solid, with a yield of 21%.

Step 6: Synthesis of 2-(3-(difluoro(phenyl)methyl)-5-((4-methoxybenzyl)oxy)phenyl)-4,4,5,5-tetramethyl-1,3,2-dioxa borolane

To a reaction flask, 1-bromo-3-(difluoro(phenyl)methyl)-5-((4-methoxybenzyl)oxy)benzene (60 mg, 0.14 mmol), bis(pinacolato)diboron (38 mg, 0.15 mmol), potassium acetate (29 mg, 0.30 mmol) and 2 mL of dioxane were added. After replacement with nitrogen, 10 mg of $Pd(dppf)Cl_2$ was added. Under the protection of nitrogen, the reaction mixture was heated to 100°C and stirred for 6 h. The reaction system was cooled down to room temperature, poured into 10 mL of water, and extracted with ethyl acetate. The organic phase was dried, evaporated under reduced pressure and purified by Prep-TLC (PE/EA=20/1) to obtain 60 mg of yellow solid, with a yield of 90%.

Step 7: Synthesis of tert-butyl (1R,5S)-3-(7-(3-(difluoro(phenyl)methyl)-5-((4-methoxybenzyl)oxy)phenyl)-8-fluoro-2-((tetrahy dro-1H-pyrrolizin-7a(5H)-yl)methoxy)pyrido[4,3-d]pyrimidin-4-yl)-3,8-diazabicyclo[3.2.1]octan -8-formate

To a reaction flask, tert-butyl (1R,5S)-3-(7-chloro-8-fluoro-2-((tetrahydro-1H-pyrrolizin-7a(5H)-yl)methoxy)pyrido[4,3-d]pyri midin-4-yl)-3,8-diazabicyclo[3.2.1]octan-8-formate (67 mg, 0.13 mmol), 2-(3-(difluoro(phenyl)methyl)-5-((4-methoxybenzyl)oxy)phenyl)-4,4,5,5-tetramethyl-1,3,2-dioxa borolane (60 mg, 0.13 mmol), potassium carbonate (40 mg, 0.29 mmol), and dioxane/water (2 mL/0.2 mL) were added. After replacement with nitrogen, 5 mg of $Pd(dppf)Cl_2$ was added. Under the protection of nitrogen, the reaction mixture was heated to 100°C and stirred for 6 h. The reaction system was cooled down to room temperature, poured into 10 mL of water, and extracted with ethyl acetate. The organic phase was dried, evaporated under reduced pressure and purified by Prep-TLC (DCM/MeOH=20/1) to obtain 17 mg of grey solid, with a yield of 16%.

Step 8: Synthesis of 3-(4-((1R,5S)-3,8-diazabicyclo[3.2.1]octyl-3-yl)-8-fluoro-2-((tetrahydro-1H-pyrrolozin-7a(5H)-y l) methoxy)pyrido[4,3-d]pyrimidin-7-yl)-5-(difluoro(phenyl)methyl)phenol

Tert-butyl (1R,5S)-3-(7-(3-(difluoro(phenyl)methyl)-5-((4-methoxybenzyl)oxy)phenyl)-8-fluoro-2-((tetrahy dro-1H-pyrrolozin-7a(5H)-yl)methoxy)pyrido[4,3-d]pyrimidin-4-yl)-3,8-diazabicyclo[3.2.1]octa n-8-formate (17 mg, 0.02 mmol) was dissolved in 2 mL of ethyl acetate, added with HCl/EA (4 M, 1 mL) at room temperature, and then stirred for 0.5 h. The reaction system was evaporated under reduced pressure, added with 2 mL of aqueous sodium carbonate, and extracted with ethyl acetate. The organic phase was dried, evaporated under reduced pressure and purified by Prep-TLC (DCM/MeOH=8/1) to obtain 4 mg of grey solid, with a yield of 31%.
LC/MS: m/z=617.3 $[M+H]^+$.

**Example** 2

5-Ethynyl-4-(8-fluoro-4-((1-(hydroxymethyl)-2-oxabicyclo[2.2.2]octyl-4-yl)amino)-2-(( tetrahydro-1H-pyrrolizin-7a(5H)-yl)methoxy)pyrido[4,3-d]pyrimidin-7-yl)naphthalen-2-ol

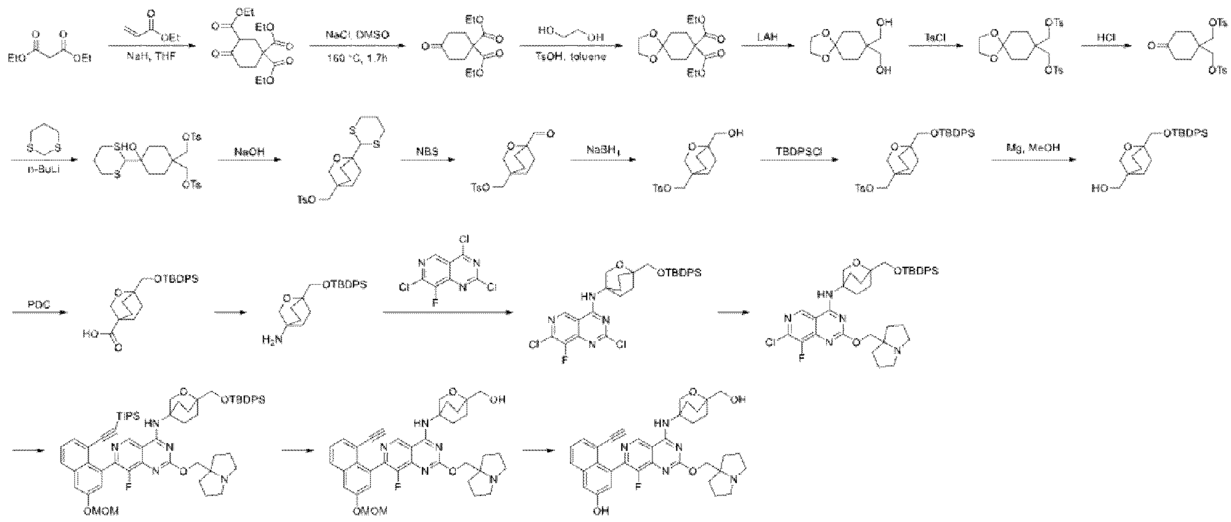


Step 1: Synthesis of triethyl 4-oxocyclohexan-1,1,3-tricarboxylate

NaH (8.56 g, 357 mmol) was suspended in anhydrous THF (80 mL), heated to 40-45°C, and added dropwise with a solution of diethyl malonate (22.85 g, 149 mmol) in THF, and then stirred at this temperature for 15 min. A solution of ethyl acrylate in THF was added dropwise, and then the reaction was continued for another 15 min. The reaction solution was cooled down to room temperature, added to ice water, adjusted to pH 3 with concentrated HCl, and extracted twice with ethyl acetate. The organic phases were combined, dried over anhydrous sodium sulfate, filtered, concentrated under reduced pressure, and purified by column chromatography to obtain colorless oil (37.6 g, 83%).

Step 2: Synthesis of diethyl 4-oxocyclohexan-1,1-dicarboxylate

Triethyl 4-oxocyclohexan-1,1,3-tricarboxylate (37.6 g, 120 mmol) and sodium chloride (20.97 g, 359 mmol) were added to DMSO (170 mL) and $H_2O$ (5 mL) for 1.7 h of reaction at 160°C. The reaction solution was cooled, added to ice water, and extracted with ethyl acetate. The organic phase was dried over anhydrous sodium sulfate, filtered, concentrated under reduced pressure, and purified by column chromatography to obtain colourless oil (21.6 g, 75%).

Step 3: Synthesis of diethyl 1.4-dioxaspiro[4.5]decan-8,8-dicarboxylate

Diethyl 4-oxocyclohexan-1,1-dicarboxylate (21.6 g, 89.2 mmol), ethylene glycol (6.64 g, 107 mmol) and p-toluenesulfonic acid-monohydrate (169 mg, 0.89 mmol) were added to toluene (180 mL) and refluxed at 120°C for 4 h. The reaction solution was cooled, added to saturated $NaHCO_3$ aqueous solution, and extracted with ethyl acetate. The organic phase was dried over anhydrous sodium sulfate, filtered, concentrated under reduced pressure, and purified by column chromatography to obtain light yellow liquid (23.7 g, 93%).

Step 4: Synthesis of (1,4-dioxaspiro[4.5]decan-8,8-diyl)dimethanol

LAH (6.47 g, 166 mmol) was added in a three-necked flask, and anhydrous THF (150 mL) was added. After replacement with nitrogen, a solution of diethyl 1,4-dioxaspiro[4.5]decan-8,8-dicarboxylate (23.7 g, 82.8 mmol) in THF (100 mL) was added dropwise under cooling in an ice-salt bath, and then slowly heated to room temperature for 5 h of reaction. Under an ice water bath, $H_2O$/THF (1:1, 30 mL) was slowly added dropwise followed by 5 N aqueous sodium hydroxide solution (8 mL), and stirred overnight at room temperature. The reaction solution was diluted by adding DCM/MeOH (5:1, 250 mL) to the reaction flask, filtered and rinsed with DCM/MeOH (5:1). The filtrate was added with 50 g of silica gel, stirred for 15 min, filtered, and rinsed. The filtrate was concentrated under reduced pressure to obtain a product (16.7 g, 99%).

Step 5: Synthesis of (1,4-dioxaspiro[4.5]decan-8,8-diyl)bis(methylene)bis(4-methylbenzenesulfonate)

(1,4-Dioxaspiro[4.5]decan-8,8-diyl)dimethanol (16.7 g, 82.6 mmol) was added in a reaction flask, pyridine (100 mL) was added, TsCl (34.6 g, 182 mmol) was added under an ice-water bath and stirred at room temperature overnight. The reaction solution was diluted by adding ethyl acetate, and washed with 10% citric acid solution and saturated sodium

chloride. The organic phase was dried over anhydrous sodium sulfate, filtered, and concentrated under reduced pressure. The resulting crude product was pulped with ethanol to obtain white solid (35 g, 83%).

Step 6: Synthesis of (4-oxocyclohexan-1,1-diyl)bis(methylene)bis(4-methylbenzenesulfonate)

(1,4-Dioxaspiro[4.5]decan-8,8-diyl)bis(methylene)bis(4-methylbenzenesulfonate) (35 g, 68.5 mmol) was added in a reaction flask, 1N HCl solution (260 mL) and THF (300 mL) was added, and the reaction was proformed at 80°C for 5 h. The reaction solution was extracted with ethyl acetate. The organic phase was washed with water, dried over anhydrous sodium sulfate, filtered, concentrated under reduced pressure, and purified by column chromatography to obtain the target product (26.2 g, 82%).

Step 7: Synthesis of ((4-(1,3-dithian-2-yl)-4-hydroxycyclohexan-1,1-diyl)bis(methylene)bis(4-methylbenzenesulfonat e)

To a reaction flask, compound 1,3-dithiane (2.1 g, 17.4 mmol) and anhydrous THF (40 mL) were added. After replacement with nitrogen, n-butyl lithium (2.5 M, 8.5 mL) was added dropwise under cooling in a dry ice-ethanol bath, and then warmed to 0°C for 1 h of reaction. After cooling in a dry ice-ethanol bath, a solution of (4-oxocyclohexan-1,1-diyl) bis(methylene)bis(4-methylbenzenesulfonate) (6.5 g, 13.9 mmol) in THF was added dropwise, and then the reaction system was heated to room temperature for 1 h of reaction. The reaction solution was added to saturated $NH_4Cl$ solution to quench reaction, and extracted with ethyl acetate. The organic phase was dried over anhydrous sodium sulfate, filtered, concentrated under reduced pressure, and purified by column chromatography to obtain the target product (6.77 g, 83%).

Step 8: Synthesis of (1-(1,3-dithian-2-yl)-2-oxabicyclo[2.2.2]octan-4-yl)methyl 4-methylbenzenesulfonate

To a reaction flask, ((4-(1,3-dithian-2-yl)-4-hydroxycyclohexan-1,1-diyl)bis(methylene)bis(4-methylbenzenesulfonat e) (6.77 g, 11.5 mmol), NaOH (1.38 g, 34.6 mmol), and THF (170 mL) were added and refluxed at 70°C overnight. The reaction solution was cooled down to room temperature, added with water, and extracted with ethyl acetate. The organic phase was dried over anhydrous sodium sulfate, filtered, concentrated under reduced pressure, and purified by column chromatography to obtain the target product (3.7 g, 77%).

Step 9: Synthesis of ((1-formyl-2-oxabicyclo[2.2.2]octyl-4-yl)methyl 4-methylbenzenesulfonate

To a reaction flask, (1-(1,3-dithian-2-yl)-2-oxabicyclo[2.2.2]octan-4-yl)methyl 4-methylbenzenesulfonate (3.7 g, 8.92 mmol), acetonitrile (50 mL), and water (12.5 mL) were added, and NBS (5.56 g, 31.2 mmol) was added under an ice-water bath. Afterwards, the reaction was carried out at room temperature for 3 h. The reaction solution was added to saturated $NaHCO_3$ solution, and extracted with ethyl acetate. The organic phases were combined, backwashed with saturated NaCl solution, dried over anhydrous sodium sulfate, and filtered. The filtrate was concentrated under reduced pressure to obtain a crude product, without further purification.

Step 10: Synthesis of (1-(hydroxymethyl)-2-oxabicyclo[2.2.2]octyl-4-yl)methyl 4-methylbenzenesulfonate

The crude product obtained above was dissolved in ethanol and added with $NaBH_4$ (508 mg, 13.4 mmol) under an ice water bath. The reaction was then carried out at room temperature for 1 h. The reaction solution was added with saturated $NH_4Cl$ solution to quench the reaction and extracted with ethyl acetate. The organic phase was dried over anhydrous sodium sulfate, filtered, concentrated under reduced pressure, and purified by column chromatography to obtain the target product (2.66 g), with a two-step yield of 91%.

Step 11: Synthesis of (1-(((tert-butyldiphenylmethylsilyl)oxy)methyl)-oxabicyclo[2.2.2]octyl-4-yl)methyl 4-methylbenzenesulfonate

To a reaction flask, (1-(hydroxymethyl)-2-oxabicyclo[2.2.2]octyl-4-yl)methyl 4-methylbenzenesulfonate (2.56 g, 7.84 mmol), DMAP (287 mg, 2.35 mmol), imidazole (1.06 g, 15.7 mmol), and DMF (15 mL) were added, and then TBDPSCl (2.59 g, 9.41 mmol) was added. The reaction was performed at room temperature for 0.5 h. The reaction solution was added to water and extracted with ethyl acetate. The organic phases were combined, backwashed with saturated NaCl solution, dried over anhydrous sodium sulfate, filtered, concentrated under reduced pressure, and purified by olumn chromatography to obtain the target product (4.0 g, 90%).

Step 12: Synthesis of (1-(((tert-butyldiphenylmethylsilyl)oxy)methyl)-2-oxabicyclo[2.2.2]octyl-4-yl)methanol

To a reaction flask, (1-(((tert-butyldiphenylmethylsilyl)oxy)methyl)-2-oxabicyclo[2.2.2]octyl-4-yl)methyl 4-methylbenzenesulfonate (4.0 g, 7.08 mmol) and methanol (120 mL) were added, and Mg (1.89 g, 77.9 mmol) was added at room temperature with stirring. After 30 min, the reaction was dramatically exothermic and the reaction system was stirred overnight. The reaction solution was added to saturated $NH_4Cl$ solution, and extracted with ethyl acetate. The organic phase was dried over anhydrous sodium sulfate, filtered, concentrated under reduced pressure, and purified by column chromatography to obtain the target product (2.4 g, 83%).

Step 13: Synthesis of 1-(((tert-butyldiphenylmethylsilyl)oxy)methyl)-2-oxabicyclo[2.2.2]octan-4-carboxylic acid

To a reaction flask, (1-(((tert-butyldiphenylmethylsilyl)oxy)methyl)-2-oxabicyclo[2.2.2]octyl-4-yl)methanol (2.4 g, 5.85 mmol) and DMF (30 mL) were added, and PDC (6.6 g, 17.6 mmol) was added under an ice-water bath. Afterwards, the reaction system was left at room temperature for 2 h. The reaction solution was diluted by adding ethyl acetate to the reaction flask, and extracted with water. The organic phases were combined, backwashed with saturated NaCl solution, dried over anhydrous sodium sulfate, filtered, concentrated under reduced pressure, and purified by column chromatography to obtain the target product (1.99 g, 80%).

Step 14: Synthesis of 1-(((tert-butyldiphenylmethylsilyl)oxy)methyl)-2-oxabicyclo[2.2.2]octyl-4-amine

To a reaction flask, 1-(((tert-butyldiphenylmethylsilyl)oxy)methyl)-2-oxabicyclo[2.2.2]octan-4-carboxylic acid (1.0 g, 2.35 mmol), DPPA (688 mg, 2.50 mmol), 2 mL of tert-butyl alcohol and 10 mL of toluene were added, and the reaction system was stirred at 100°C overnight. The reaction system was cooled down to room temperature, added with 0.5 mL of concentrated hydrochloric acid and stirred for another 10 min. The reaction system was poured into 50 mL of water, added with sodium carbonate to adjust the pH to be alkaline, and extracted with ethyl acetate. The organic phase was dried, evaporated under reduced pressure, and purified by silica gel column chromatography (PE/EA=2/1) to obtain 60 mg of yellow oil, with a yield of 6%.

Step 15: Synthesis of N-(1-(((tert-butyldiphenylmethylsilyl)oxy)methyl)-2-oxabicyclo[2.2.2]octa-4-yl)-2,7-dichloro-8-f luoropyrido[4,3-d]pyrimidin-4-amine

1-(((Tert-butyldiphenylmethylsilyl)oxy)methyl)-2-oxabicyclo[2.2.2]octyl-4-amine (50 mg, 0.13 mmol), 2,4,7-trichloro-8-fluoropyrido[4,3-d]pyrimidine (33 mg, 0.13 mmol) and DIPEA (39 mg, 0.30 mmol) were dissolved in 2 mL of dichloromethane and stirred at room temperature for 6 h. The reaction system was poured into 10 mL of water, and extracted with dichloromethane. The organic phase was dried, evaporated under reduced pressure and purified by Prep-TLC (DCM/MeOH=50/1) to obtain 60 mg of yellow solid, with a yield of 76%.

Step 16: Synthesis of N-(1-(((tert-butyldiphenylmethylsilyl)oxy)methyl)-2-oxabicyclo[2.2.2]octa-4-yl)-7-chloro-8-fluoro-2-((tetrahydro-1H-pyrrolin-7a(5H)-yl)methoxy)pyrido[4,3-d]pyrimidin-4-amine

At room temperature, NaH (60%, 8 mg, 0.20 mmol) was added to a solution of (tetrahydro-1H-pyrrolizin-7a(5H)-yl)methanol (21 mg, 0.15 mmol) in 2 mL of THF and the resulting reaction solution was stirred for 1 h. Afterwards, N-(1-(((tert-butyldiphenylmethylsilyl)oxy)methyl)-2-oxabicyclo[2.2.2]octa-4-yl)-2,7-dichloro-8-f luoropyrido[4,3-d]pyrimidin-4-amine (60 mg, 0.10 mmol) was added. This mixture was stirred at room temperature for 2 h. The reaction solution was poured into 10 mL of water, and extracted with ethyl acetate. The organic phase was dried, evaporated under reduced pressure and purified by Prep-TLC (DCM/MeOH = 20/1) to obtain 33 mg of yellow solid, with a yield of 46%.

Step 17: Synthesis of N-(1-(((tert-butyldiphenylmethylsilyl)oxy)methyl)-2-oxabicyclo[2.2.2]octyl-4-yl)-8-fluoro-7-(3-( methoxymethoxy)-8-((triisopropylmethylsilyl))ethynyl)naphthalen-1-yl)-2-((tetrahydro-1H-pyrro lin-7a(5H)-yl)methoxy)pyrido[4,3- d]pyrimidin-4-amine

To a reaction flask, N-(1-(((tert-butyldiphenylmethylsilyl)oxy)methyl)-2-oxabicyclo[2.2.2]octa-4-yl)-7-chloro-8-fluo ro-2-((tetrahydro-1H-pyrrolin-7a(5H)-yl)methoxy)pyrido[4,3-d]pyrimidin-4-amine (33 mg, 0.046 mmol), triisopropyl ((6-(methoxymethoxy)-8-(4,4,5,5-tetramethyl-1,3,2-dioxaborolan-2-yl)naphthalen-1-yl)ethynyl)si lane (25 mg, 0.051 mmol), potassium carbonate (14 mg, 0.100 mmol), and dioxane/water (2 mL/0.2 mL) were added. After replacement with nitrogen, 5 mg of $Pd(dppf)Cl_2$ was added. Under the protection of nitrogen, the reaction mixture was heated to 100°C and stirred for 6 h. The reaction system was cooled down to room temperature, poured into 10 mL of water, and extracted with ethyl acetate. The organic phase was dried, evaporated under reduced pressure and purified by Prep-TLC

(DCM/MeOH=20/1) to obtain 17 mg of grey solid, with a yield of 35%.

Step 18: Synthesis of (4-((7-(8-ethynyl-3-(methoxymethoxy)naphthalen-1-yl)-8-fluoro-2-((tetrahydro-1H-pyrrolizin-7a(5H)-yl)methoxy)pyrido[4,3-d]pyrimidin-4-yl)amino)-2-oxabicyclo[2.2.2]octyl-1-yl)methanol

N-(1-(((tert-butyldiphenylmethylsilyl)oxy)methyl)-2-oxabicyclo[2.2.2]octyl-4-yl)-8-flu oro-7-(3-(methoxymethoxy)-8-((triisopropylmethylsilyl))ethynyl)naphthalen-1-yl)-2-((tetrahydro -1H-pyrrolozin-7a(5H)-yl)methoxy)pyrido[4,3-d]pyrimidin-4-amine (10 mg, 0.0095 mmol) was dissolved in 1 mL of DMF, added with CsF (10 mg) at room temperature and stirred overnight. The reaction system was poured into 10 mL of water, and extracted with ethyl acetate. The organic phase was dried, and evaporated under reduced pressure. The resulting crude product was used directly for next step.

Step 19: Synthesis of 5-ethynyl-4-(8-fluoro-4-((1-(hydroxymethyl)-2-oxabicyclo[2.2.2]octyl-4-yl)amino)-2-((tetrahydro-1H-pyrrolizin-7a(5H)-yl)methoxy)pyrido[4,3-d]pyrimidin-7-yl)naphthalen-2-ol

The crude product obtained in the previous step was dissolved in 2 mL of ethyl acetate, added with HCl/EA (4 M, 0.5 mL) and stirred for 10 min. The reaction system was evaporated under reduced pressure and purified by Prep-HPLC to obtain 1 mg of grey solid, with a two-step yield of 17%.
LC/MS: m/z=610.3 $[M+H]^+$.

$^1$H NMR

**Example** 3

4-((1R,5S)-3,8-diazabicyclo[3.2.1]octan-3-yl)-7-(4-(difluoro(phenyl)methyl)phenyl)-8-f luoro-2-((tetrahydro-1H-pyrrolizin-7a(5H)-yl)methoxy)pyrido[4,3-d]pyrimidine

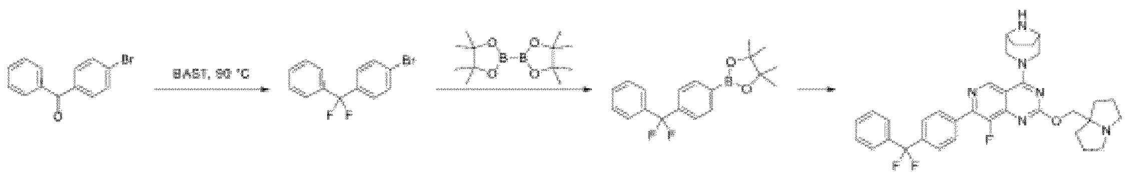


Step 1: Synthesis of 1-bromo-4-(difluoro(phenyl)methyl)benzene

(4-Bromophenyl)(phenyl)methanone (300 mg, 1.15 mmol) and BAST (3 mL) were added to a reaction tube, which was sealed and heated to 90°C for reaction overnight. The reaction solution was cooled, poured into water, and extracted twice with ethyl acetate. The organic phases were combined, washed with saturated brine, dried over anhydrous sodium sulfate, evaporated in vacuo, and purified by using a preparative silica gel plate to obtain 270 mg of a product with a yield of 83%.

Step 2: Synthesis of 2-(4-(difluoro(phenyl)methyl)phenyl)-4,4,5,5-tetramethyl-1,3,2-dioxaborolane

To a reaction flask, 1-bromo-4-(difluoro(phenyl)methyl)benzene (1.0 g, 3.5 mmol), bis(pinacolato)diboron (1.3 g, 5.1 mmol), potassium acetate (1.0 g, 10.2 mmol) and 15 mL of dioxane were added. After replacement with nitrogen, Pd(dppf)$Cl_2$ (146 mg, 0.2 mmol) was added. Under the protection of nitrogen, the reaction mixture was heated to 100°C and stirred for 6 h. The reaction system was cooled down to room temperature, poured into 100 mL of water, and extracted with ethyl acetate. The organic phase was dried, evaporated under reduced pressure and purified by silica gel column chromatography (PE/EA=20/1) to obtain 970 mg of off-white solid, with a yield of 75%.

Step 3: Synthesis of 4-((1R,5S)-3,8-diazabicyclo[3.2.1]octan-3-yl)-7-(4-(difluoro(phenyl)methyl)phenyl)-8-fluoro-2-( (tetrahydro-1H-pyrrolizin-7a(5H)-yl)methoxy)pyrido[4,3-d]pyrimidine

Using the product obtained in the previous step and the corresponding intermediates as raw materials, the target product was synthesized by a method similar to that of Example 1.

LC/MS: m/z 601.3 [M+H]+.

Example 4

4-((1R,5S)-3,8-diazabicyclo[3.2.1]octan-3-yl)-7-(4-(difluoro(phenyl)methyl)phenyl)-8-f luoro-2-((tetrahydro-1H-pyrrolizin-7a(5H)-yl)methoxy)pyrido[4,3-d]pyrimidine

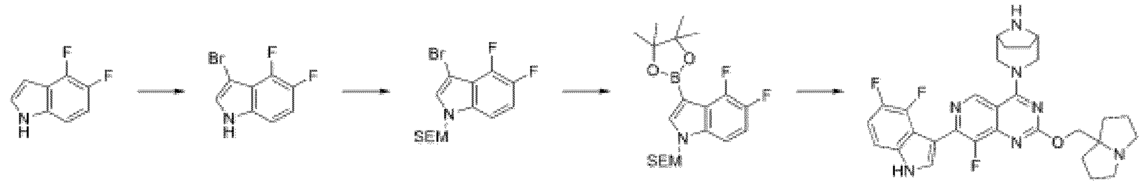

Step 1: Synthesis of 3-bromo-4,5-difluoro-1H-indole

At room temperature, NBS (356 mg, 2.0 mmol) was added to a solution of 4,5-difluoro-1H-indole (300 mg, 2.0 mmol) in 5 mL of DMF, and stirred for 6 h at room temperature. The reaction solution was poured into 50 mL of water, and extracted with ethyl acetate. The organic phase was dried and evaporated under reduced pressure to obtain a crude product.

Step 2: Synthesis of 3-bromo-4,5-difluoro-1-((2-(trimethylsilyl)ethoxy)methyl)-1H-indole

The product obtained in the previous step was dissolved in 5 mL of THF and NaH (60%, 120 mg, 3.0 mmol) was added at 0°C. After stirring at room temperature for 0.5 h, SEM-Cl (367 mg, 2.2 mmol) was added. The reaction solution was stirred at room temperature for 3 h. The reaction solution was poured into 50 mL of water and extracted with ethyl acetate. The organic phase was dried, evaporated under reduced pressure, and purified by column chromatography to obtain 400 mg of colourless oil, with a two-step yield of 85%.

Step 3: Synthesis of 4,5-difluoro-3-(4,4,5,5-tetramethyl-1,3,2-dioxaborolan-2-yl)-1-((2-(trimethylsilyl)ethoxy)methyl) -1H-indole

To a reaction flask, 3-bromo-4,5-difluoro-1-((2-(trimethylsilyl)ethoxy)methyl)-1H-indole (100 mg, 0.28 mmol), bis(pinacolato)diboron (102 mg, 0.40 mmol), potassium acetate (59 mg, 0.60 mmol) and 3 mL of dioxane were added. After replacement with nitrogen, Pd(dppf)$Cl_2$ (7 mg, 0.01 mmol) was added. Under the protection of nitrogen, the reaction mixture was heated to 100°C and stirred for 6 h. The reaction system was cooled down to room temperature, poured into 10 mL of water, and extracted with ethyl acetate. The organic phase was dried, evaporated under reduced pressure and purified by silica gel column chromatography (PE/EA=5/1) to obtain 20 mg of grey solid, with a yield of 18%.

Step 4: Synthesis of 4-((1R,5S)-3,8-diazabicyclo[3.2.1]octan-3-yl)-7-(4,5-difluoro-1H-indol-3-yl)-8-fluoro-2-((tetrahy dro-1H-pyrrolizin-7a(5H)-yl)methoxy)pyrido[4,3-d]pyrimidine

Using the product obtained in the previous step and the corresponding intermediates as raw materials, the target product was synthesized by a method similar to that of Example 1.

LC/MS: m/z 550.2 [M+H]+.

**Example 5**

4-(4-((1R,5S)-8,8-difluoro-3-azabicyclo[3.2.1]octan-3-yl)-8-fluoro-2-((tetrahydro-1H-p yrrolizin-7a(5H)-yl)methoxy)pyrido[4,3-d]pyrimidin-7-yl)-5-ethynylnaphthalen-2-ol

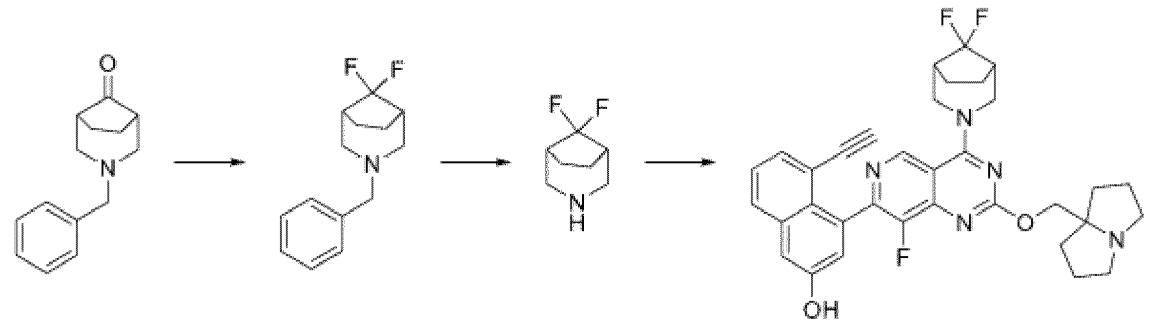

Step 1: Synthesis of (1R,5S)-3-benzyl-8,8-difluoro-3-azabicyclo[3.2.1]octane

3-Benzyl-3-azabicyclo[3.2.1]octan-8-one (250 mg, 1.2 mmol) was dissolved in 5 mL of dichloromethane, and DAST (300 mg, 1.8 mmol) was added dropwise at 0°C. Afterwards, the system was slowly heated to room temperature and stirred for 5 h. The system was added dropwise into saturated aqueous sodium bicarbonate solution, and extracted with ethyl acetate. The organic phase was dried, concentrated, and purified by silica gel column chromatography using the wet method to load column (PE/EA=20/1) to obtain 215 mg of target product as a colourless oily liquid with a yield of 78%.

Step 2: Synthesis of (1R,5S)-8,8-difluoro-3-azabicyclo[3.2.1]octane

(1R,5S)-3-benzyl-8,8-difluoro-3-azabicyclo[3.2.1]octane (200 mg, 0.8 mmol) was dissolved in 5 mL of methanol. PdOH/C (10%, 20 mg) and 1 mL solution of hydrochloric acid in methanol (4 M) were added. The reaction solution was stirred under the atmosphere of $H_2$ of 2 atmospheres at room temperature for 4 h. The reaction solution was filtered and the filtrate was evaporated under reduced pressure to obtain 140 mg of crude product with a yield of 84%.

Step 3: Synthesis of 4-(4-((1R,5S)-8,8-difluoro-3-azabicyclo[3.2.1]octan-3-yl)-8-fluoro-2-((tetrahydro-1H-pyrrolizin-7a(5H)-yl)methoxy)pyrido[4,3-d]pyrimidin-7-yl)-5-ethynylnaphthalen-2-ol

Using the product obtained in the previous step and the corresponding intermediates as raw materials, the target product was synthesized by a method similar to that of Example 2.

LC/MS: m/z 600.3 $[M+H]^+$.

## Example 6

4-(4-((1R,5S)-3,8-diazabicyclo[3.2.1]octan-3-yl)-8-fluoro-2-((hexahydro-5,8-methanoin dolizin-8a(1H)-yl)methoxy)pyrido[4,3-d]pyrimidin-7-yl)naphthalen-2-ol

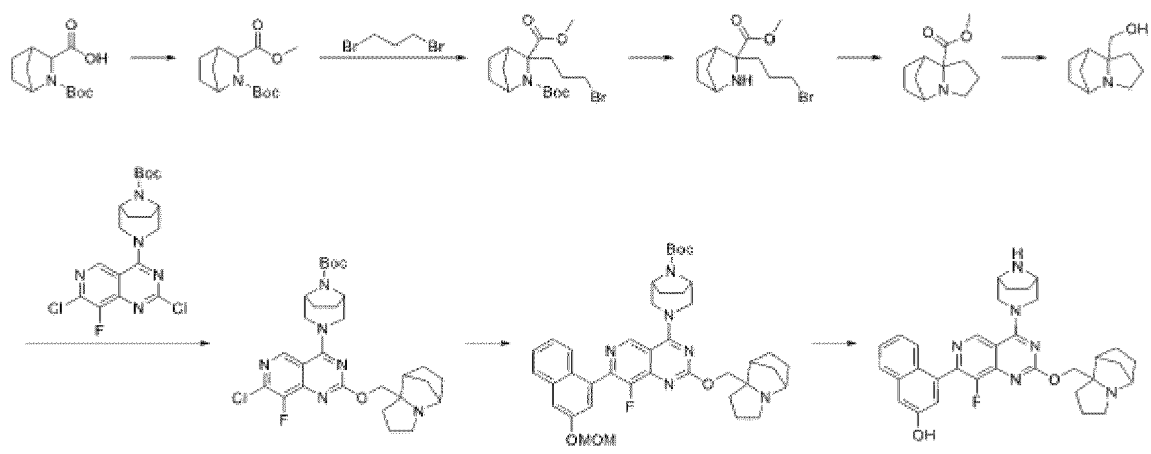

Step 1: Synthesis of 2-(tert-butyl)3-methyl 2-azabicyclo[2.2.1]heptan-2,3-dicarboxylate

To a reaction flask, 2-(tert-butoxycarbonyl)-2-azabicyclo[2.2.1]heptan-3-carboxylic acid (5000 mg, 20.7 mmol), potassium carbonate (5726 mg, 41.5 mmol) and 50 mL of DMF were added, and iodomethane (3535 mg, 24.9 mmol) was added at room temperature. The reaction mixture was stirred at room temperature for 6 h. TLC showed that the reaction was complete. The system was filtered. The filtrate was collected, added with 250 mL of water, and extracted with ethyl acetate. The organic phase was backwashed with saturated brine, dried, evaporated under reduced pressure and then purified by silica gel column chromatography (PE/EA=50/1-20/1) to obtain 5 g of target product, with a yield of 94%.

Step 2: Synthesis of 2-(tert-butyl)3-methyl 3-(3-bromopropyl)-2-azabicyclo[2.2.1]heptan-2,3-dicarboxylate

To a reaction flask, 2-(tert-butyl)3-methyl 2-azabicyclo[2.2.1]heptan-2,3-dicarboxylate (1000 mg, 3.9 mmol) and 10 mL of THF were added. The reaction mixture was cooled down to -70°C. LDA (2M, 2 mL, 4.0 mmol) was added dropwise at -70°C. Afterwards, this system was stirred for 1 h. 1,3-Dibromopropane (1188 mg, 5.9 mmol) was added dropwise to the system, stirred for 10 min, and then the system was moved at room temperature for 2 h of reaction. TLC showed that the reaction was complete. The reaction system was poured into 50 mL of water, and extracted with ethyl acetate. The organic phase was dried, evaporated under reduced pressure, and purified by silica gel column chromatography (PE/EA=20/1-5/1) to obtain 1.2 g of the target product with a yield of 82%.

Step 3: Synthesis of methyl 3-(3-bromopropyl)-2-azabicyclo[2.2.1]heptan-3-carboxylate

To a reaction flask, 2-(tert-butyl)3-methyl 3-(3-bromopropyl)-2-azabicyclo[2.2.1]heptan-2,3-dicarboxylate (1200 mg, 3.2 mmol) and a solution of hydrogen chloride in 1,4-dioxane (4 M, 12 mL) were added. The reaction mixture was stirred at room temperature for 2 h and the TLC showed that the reaction was complete. The reaction system was evaporated under reduced pressure and the hydrochloride was used directly for next step.

Step 4: Synthesis of methyl hexahydro-5,8-methanoindolizin-8a(1H)-carboxylate

To a reaction flask, methyl 3-(3-bromopropyl)-2-azabicyclo[2.2.1]heptan-3-carboxylate (the product obtained in step 3), potassium carbonate (1325 mg, 9.6 mmol) and 10 mL of acetonitrile were added, and the reaction mixture was stirred at room temperature for 4 h. TLC showed that the reaction was complete. The reaction system was filtered. The filtrate was dried, evaporated under reduced pressure and purified by silica gel column chromatography (DCM/MeOH=20/1) to obtain 611 mg of target product, with a total yield of 98% for steps 3 and 4.

Step 5: Synthesis of (hexahydro-5,8-methanoindolizin-8a(1H)-yl)methanol

Methyl hexahydro-5,8-methanoindolizin-8a(1H)-carboxylate (611 mg, 3.1 mmol) was dissolved in 5 mL of THF. The reaction mixture was cooled down to -40°C, added with lithium aluminium hydride (357 mg, 9.4 mmol) in batches under the protection of nitrogen, and was stirred at -40°C for 2 h. The reaction system was diluted by adding 5 mL of THF, then added with 0.6 mL of water and stirred for 5 min. 15% aqueous sodium hydroxide (0.6 mL) was added and stirred for 10 min, and then 1.8 mL of water was added and stirred for another 30 min. The system was dried by adding anhydrous sodium sulphate, and filtered. The filtrate was evaporated under reduced pressure and purified by silica gel column chromatography (DCM/MeOH=20/1) to obtain 96 mg of target product, with a yield of 18%.

Step 6: Synthesis of tert-butyl (1R,5S)-3-(7-chloro-8-fluoro-2-((hexahydro-5,8-methanoindolizin-8a(1H)-yl)methoxy) pyrido[4, 3-d]pyrimidin-4-yl)-3,8-diazabicyclo[3.2.1]octan-8-carboxylate

At room temperature, to a solution of (hexahydro-5,8-methanoindolizin-8a(1H)-yl)methanol (84 mg, 0.50 mmol) in 2 mL of THF was added NaH (60%, 40 mg, 1.0 mmol). The resulting reaction solution was stirred at room temperature for 1 h, and then tert-butyl (1R,5S)-3-(7-chloro-8-fluoro-2-((tetrahydro-1H-pyrrolizin-7a(5H)-yl)methoxy)pyrido[4,3-d]pyrimidin-4-yl)-3,8-diazabicyclo[3.2.1]octan-8-formate (214 mg, 0.50 mmol) was added. This mixture was stirred at room temperature overnight. The reaction solution was poured into 10 mL of water, and extracted with ethyl acetate. The organic phase was dried, evaporated under reduced pressure and purified by Prep-TLC (DCM/MeOH=20/1) to obtain 70 mg of yellow solid, with a yield of 25%.

Step 7: Synthesis of tert-butyl (1R,5S)-3-(8-fluoro-2-((hexahydro-5,8-methanoindolizin-8a(1H)-yl)methoxy)-7-(3-(methoxymet hoxy)naphthalen-1-yl)pyrido[4,3-d]pyrimidin-4-yl)-3,8-diazabicyclo[3.2.1]octan-8-carboxylate

To a reaction flask, tert-butyl (1R,5S)-3-(7-chloro-8-fluoro-2-((hexahydro-5,8-methanoindolizin-8a(1H)-yl)methoxy)pyrido[4, 3-d]pyrimidin-4-yl)-3,8-diazabicyclo[3.2.1]octan-8-carboxylate (70 mg, 0.13 mmol), 2-(3-(methoxymethoxy) naphthalen-1-yl)-4,4,5,5-tetramethyl-1,3,2-dioxaborolane (40 mg, 0.13 mmol), potassium carbonate (40 mg, 0.29 mmol), and dioxane/water (2 mL/0.2 mL) were added. After replacement with nitrogen, 5 mg of Pd(dppf)$Cl_2$ was added. Under the protection of nitrogen, the reaction mixture was heated to 100°C and stirred for 6 h. The reaction system was cooled down to room temperature, poured into 10 mL of water, and extracted with ethyl acetate. The organic phase was dried, evaporated under reduced pressure and purified by Prep-TLC (DCM/MeOH=20/1) to obtain 35 mg of grey solid, with a yield of 39%.

Step 8: Synthesis of 4-(4-((1R,5S)-3,8-diazabicyclo[3.2.1]octan-3-yl)-8-fluoro-2-((hexahydro-5,8-methanoindolizin-8 a(1H)-yl)methoxy)pyrido[4,3-d]pyrimidin-7-yl)naphthalen-2-ol

Tert-butyl (1R,5S)-3-(8-fluoro-2-((hexahydro-5,8-methanoindolizin-8a(1H)-yl)methoxy)-7-(3-(methoxymet hoxy)naphthalen-1-yl)pyrido[4,3-d]pyrimidin-4-yl)-3,8-diazabicyclo[3.2.1]octan-8-carboxylate (35 mg, 0.05 mmol) was dissolved in 2 mL of ethyl acetate, added with HCl/EA (4 M, 1 mL) at room temperature and stirred for 0.5 h. The reaction system was evaporated under reduced pressure, added with 2 mL of aqueous sodium carbonate, and extracted with ethyl acetate. The organic phase was dried, evaporated under reduced pressure and purified by Prep-TLC (DCM/MeOH=8/1) to obtain 16 mg of grey solid, with a yield of 57%.

LC/MS: m/z 567.3 $[M+H]^+$.

**Example 7**

4-(4-((1R,5S)-3,8-diazabicyclo[3.2.1]octan-3-yl)-8-fluoro-2-((tetrahydro-1H-pyrrolizin-7a(5H)-yl)methoxy)pyrido[4,3-d] pyrimidin-7-yl)isoquinolin-1-ol

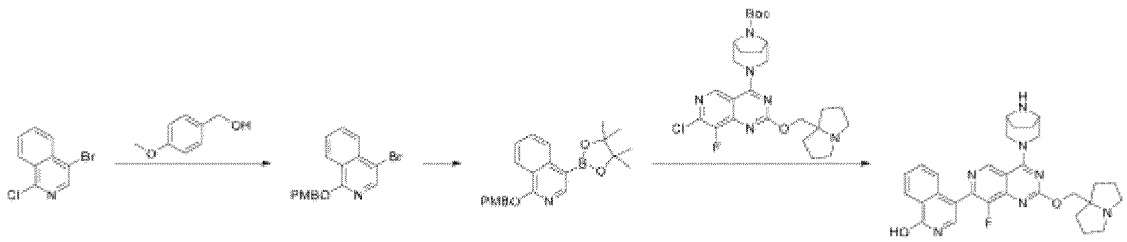

Step 1: Synthesis of 4-bromo-1-((4-methoxybenzyl)oxy)isoquinoline

To a reaction flask, 4-bromo-1-chloroisoquinoline (300 mg, 1.24 mmol), potassium tert-butanolate (279 mg, 2.48 mmol) and (4-methoxyphenyl)methanol (859 mg, 6.22 mmol) were added. The reaction mixture was heated to 80°C and stirred for 3 h. TLC showed that the reaction was complete. The reaction system was cooled down to room temperature, poured into 10 mL of water, and extracted with ethyl acetate. The organic phase was dried, evaporated under reduced pressure and purified by silica gel column chromatography (PE/EA=10/1) to obtain 400 mg of target product, with a yield of 94%.

Step 2: Synthesis of 1-((4-methoxybenzyl)oxy)-4-(4,4,5,5-tetramethyl-1,3,2-dioxolan-2-yl)isoquinoline

To a reaction flask, 4-bromo-1-((4-methoxybenzyl)oxy)isoquinoline (400 mg, 1.17 mmol), bis(pinacolato)diboron (889 mg, 3.51 mmol), potassium acetate (343 mg, 3.51 mmol), and 5 mL of 1,4-dioxane were added. After replacement with nitrogen, Pd(dppf)$Cl_2$ (170 mg, 0.23 mmol) was added. The reaction mixture was heated to 90°C under the protection of nitrogen and stirred overnight. The reaction system was cooled down to room temperature, poured into 10 mL of water, and extracted with ethyl acetate. The organic phase was dried, evaporated under reduced pressure and purified by silica gel column chromatography (PE/EA=10/1) to obtain 290 mg of target product with a yield of 64%.

Step 3: Synthesis of 4-(4-((1R,5S)-3,8-diazabicyclo[3.2.1]octan-3-yl)-8-fluoro-2-((tetrahydro-1H-pyrrolizin-7a(5H)-yl ) methoxy)pyrido[4,3-d]pyrimidin-7-yl)isoquinolin-1-ol

Using the product obtained in the previous step and the corresponding intermediates as raw materials, the target product was synthesized by a method similar to that of Example 1.
LC/MS: m/z 542.3 [M+H]+.

**Example 8**

3-((4-(4-((1R,5S)-3,8-diazabicyclo[3.2.1]octan-3-yl)-8-fluoro-2-((tetrahydro-1H-pyrroli zin-7a(5H))-yl)methoxy)pyrido [4,3-d]pyrimidin-7-yl)isoquinolin-1-yl)oxy)propan-1,2-diol

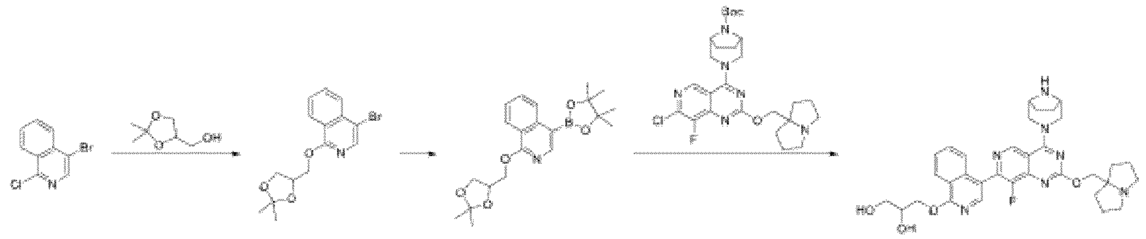

Step 1: Synthesis of 4-bromo-1-((2,2-dimethyl-1,3-dioxolan-4-yl)methoxy)isoquinoline

To a reaction flask, 4-bromo-1-chloroisoquinoline (1000 mg, 4.15 mmol), potassium tert-butoxide (929 mg, 8.30 mmol) and acetone glycidol (2.74 g, 20.75 mmol) were added. The reaction mixture was heated to 80°C and stirred for 3 h. TLC showed that the reaction was complete. The reaction system was cooled down to room temperature, poured into 50 mL of water, and extracted with ethyl acetate. The organic phase was dried, evaporated under reduced pressure and purified by silica gel column chromatography (PE/EA=10/1) to obtain 700 mg of target product with a yield of 50%.

Step 2: Synthesis of 1-((2,2-dimethyl-1,3-dioxolan-4-yl)methoxy)-4-(4,4,5,5-tetramethyl-1,3,2-dioxolan-2-yl)isoquino line

To a reaction flask, 4-bromo-1-((2,2-dimethyl-1,3-dioxolan-4-yl)methoxy)isoquinoline (200 mg, 0.59 mmol), bis(pinacolato)diboron (452 mg, 1.78 mmol), potassium acetate (174 mg, 1.78 mmol), and 5 mL of 1,4-dioxane were added. After replacement with nitrogen, Pd(dppf)$Cl_2$ (87 mg, 0.12 mmol) was added. The reaction mixture was heated to 90°C under the protection of nitrogen and stirred overnight. The reaction system was cooled down to room temperature, poured into 10 mL of water, and extracted with ethyl acetate. The organic phase was dried, evaporated under reduced pressure and purified by silica gel column chromatography (PE/EA=10/1) to obtain 180 mg of target product with a yield of 79%.

Step 3: Synthesis of 3-((4-(4-((1R,5S)-3,8-diazabicyclo[3.2.1]octan-3-yl)-8-fluoro-2-((tetrahydro-1H-pyrrolizin-7a(5 H))-yl)methoxy)pyrido[4,3-d]pyrimidin-7-yl)isoquinolin-1-yl)oxy)propan-1,2-diol

Using the product obtained in the previous step and the corresponding intermediates as raw materials, the target product was synthesized by a method similar to that of Example 1.
LC/MS: m/z 616.3 [M+H]+.

**Example 9**

4-(8-Fluoro-4-(2-(hydroxymethyl)morpholin)-2-((tetrahydro-1H-pyrrolizin-7a(5H)-yl)m ethoxy)pyrido[4,3-d]pyrimidin-7-yl)naphthalen-2-ol

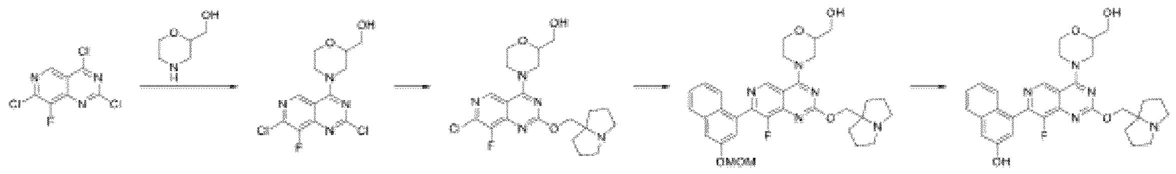

Step 1: Synthesis of (4-(2,7-dichloro-8-fluoropyrido[4,3-d]pyrimidin-4-yl)morpholin-2-yl)methanol

To a reaction flask, morpholin-2-yl methanol (59 mg, 0.50 mmol), DIPEA (155 mg, 1.20 mmol) and 5 mL of tetrahydrofuran were added, and 1.5 mL solution of 2,4,7-trichloro-8-fluoropyrido[4,3-d]pyrimidine (100 mg, 0.40 mmol) in tetrahydrofuran was added at -40°C. The reaction mixture was stirred at -40°C for 2 h. The reaction system was poured into 15 mL of water, and extracted with ethyl acetate. The organic phase was dried, evaporated under reduced pressure and purified by Prep-TLC (DCM/MeOH=50/1) to obtain 80 mg of target product with a yield of 60%.

Step 2: Synthesis of (4-(7-chloro-8-fluoro-2-((tetrahydro-1H-pyrrolin-7a(5H)-yl)methoxy)pyrido[4,3-d]pyrimidin-4-yl ) morpholin-2-yl)methanol

At room temperature, to a solution of (tetrahydro-1H-pyrrolizin-7a(5H)-yl)methanol (71 mg, 0.50 mmol) in 2 mL of THF, NaH (60%, 40 mg, 1.0 mmol) was added. The resulting reaction solution was stirred at room temperature for 1 h, and then (4-(2,7-dichloro-8-fluoropyrido[4,3-d]pyrimidin-4-yl)morpholin-2-yl) methanol (80 mg, 0.24 mmol) was added. The mixture was stirred at room temperature overnight. The reaction solution was poured into 10 mL of water, and extracted with ethyl acetate. The organic phase was dried, evaporated under reduced pressure and purified by Prep-TLC (DCM/MeOH=20/1) to obtain 63 mg of yellow solid with a yield of 60%.

Step 3: Synthesis of (4-(8-fluoro-7-(3-(methoxymethoxy)naphthalen-1-yl)-2-((tetrahydro-1H-pyrrolizin-7a(5H)-yl)me thoxy)pyrido[4,3-d]pyrimidin-4-yl)morpholin-2-yl)methanol

To a reaction flask, (4-(7-chloro-8-fluoro-2-((tetrahydro-1H-pyrrolin-7a(5H)-yl)methoxy)pyrido[4,3-d]pyrimidin-4-yl )morpholin-2-yl)methanol (44 mg, 0.10 mmol), 2-(3-(methoxymethoxy)naphthalen-1-yl)-4,4,5,5-tetramethyl-1,3,2-dioxaborolane (31 mg, 0.10 mmol), potassium carbonate (28 mg, 0.20 mmol) and dioxane/water (2 mL/0.2 mL) were added. After replacement with nitrogen, Pd(dppf)$Cl_2$ (7 mg, 0.01 mmol) was added. Under the protection of nitrogen, the reaction mixture was heated to 100°C and stirred for 6 h. The reaction system was cooled down to room temperature, poured into 10 mL of water, and extracted with ethyl acetate. The organic phase was dried, evaporated under reduced pressure and purified by Prep-TLC (DCM/MeOH=20/1) to obtain 25 mg of yellow solid with a yield of 42%.

Step 4: Synthesis of 4-(8-fluoro-4-(2-(hydroxymethyl)morpholin)-2-((tetrahydro-1H-pyrrolizin-7a(5H)-yl)methoxy)p yrido[4,3-d]pyrimidin-7-yl)naphthalen-2-ol

(4-(8-Fluoro-7-(3-(methoxymethoxy)naphthalen-1-yl)-2-((tetrahydro-1H-pyrrolizin-7a( 5H)-yl)methoxy)pyrido [4,3-d]pyrimidin-4-yl)morpholin-2-yl)methanol (25 mg, 0.04 mmol) was dissolved in 2 mL of ethyl acetate. The reaction system was added with HCl/EA (4 M, 1 mL) at room temperature and then stirred for 0.5 h. The reaction system was evaporated under reduced pressure, added with 2 mL of aqueous sodium carbonate, and extracted with ethyl acetate. The organic phase was dried, evaporated under reduced pressure and purified by Prep-TLC (DCM/MeOH=8/1) to obtain 12 mg of grey solid with a yield of 52%.

LC/MS: m/z 546.2 $[M+H]^+$.

**Example 10**

1-(8-Fluoro-7-(3-hydroxynaphthalen-1-yl)-2-((tetrahydro-1H-pyrrolizin-7a(5H)-yl)meth oxy)pyrido[4,3-d]pyrimidin-4-yl)-4-(hydroxymethyl)piperidin-4-ol

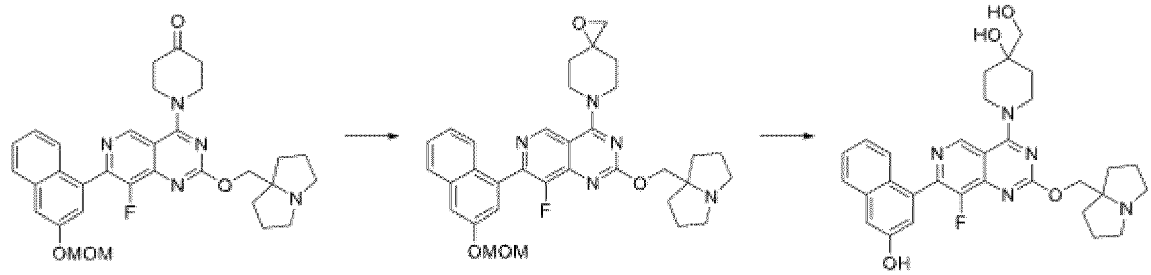

Step 1: Synthesis of 6-(8-fluoro-7-(3-(methoxymethoxy)naphthalen-1-yl)-2-((tetrahydro-1H-pyrrolizin-7a(5H)-yl)met hoxy)pyrido[4,3-d]pyrimidin-4-yl)-1-oxa-6-azaspiro[2.5]octane

To a solution of trimethylsulfoxide iodide (22 mg, 0.10 mmol) in 1 mL of DMSO, potassium tert-butoxide (13 mg, 0.12 mmol) was added and stirred at room temperature for 10 min. Then, 1-(8-fluoro-7-(3-(methoxymethoxy)naphthalen-1-yl)-2-((tetrahydro-1H-pyrrolizin-7a(5H)-yl)met hoxy)pyrido[4,3-d]pyrimidin-4-yl)piperidin-4-one (30 mg, 0.05 mmol, synthesized by a method similar to that of Example 9) was added and stirred at room temperature for 20 min. The reaction system was poured into 10 mL of water, and extracted with ethyl acetate. The organic phase was dried, evaporated under reduced pressure and purified by Prep-TLC (DCM/MeOH=20/1) to obtain 6 mg of target product with a yield of 20%.

Step 2: Synthesis of 1-(8-fluoro-7-(3-hydroxynaphthalen-1-yl)-2-((tetrahydro-1H-pyrrolizin-7a(5H)-yl)methoxy)pyrid o[4,3-d]pyrimidin-4-yl)-4-(hydroxymethyl)piperidin-4-ol

6-(8-Fluoro-7-(3-(methoxymethoxy)naphthalen-1-yl)-2-((tetrahydro-1H-pyrrolizin-7a(5 H)-yl)methoxy)pyrido[4,3-d]pyrimidin-4-yl)-1-oxa-6-azaspiro[2.5]octane (25 mg, 0.04 mmol) was dissolved in 1 mL of THF and 0.5 mL of water, and 0.2 mL of trifluoroacetic acid was added at room temperature. The reaction solution was stirred for 1 h. The reaction system was poured into 5 mL of aqueous sodium carbonate solution, and extracted with ethyl acetate. The organic phase was dried, evaporated under reduced pressure and purified by Prep-TLC (DCM/MeOH=8/1) to obtain 6 mg of grey solid with a yield of 25%.

LC/MS: m/z 560.3 $[M+H]^+$.

**Example 11**

4-(Aminomethyl)-1-(8-fluoro-7-(3-hydroxynaphthalen-1-yl)-2-((tetrahydro-1H-pyrroliz in-7a(5H)-yl)methoxy)pyrido[4,3-d]pyrimidin-4-yl)piperidin-4-ol

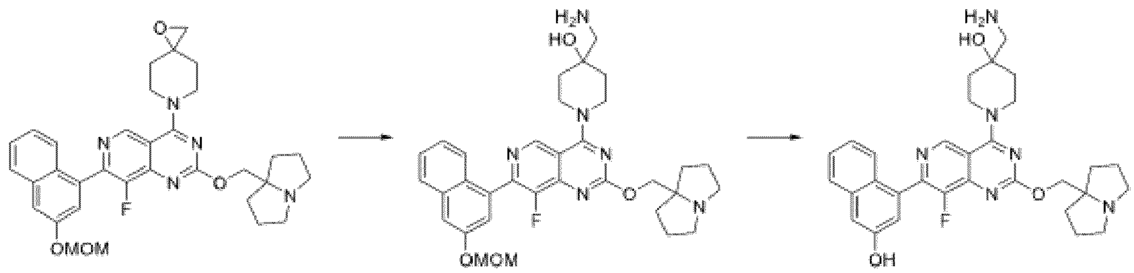

Step 1: Synthesis of 4-(aminomethyl)-1-(8-fluoro-7-(3-(methoxymethoxy)naphthalen-1-yl)-2-((tetrahydro-1H-pyrroli zin-7a(5H)-yl)methoxy)pyrido[4,3-d]pyrimidin-4-yl)piperidin-4-ol

6-(8-Fluoro-7-(3-(methoxymethoxy)naphthalen-1-yl)-2-((tetrahydro-1H-pyrrolizin-7a(5 H)-yl)methoxy)pyrido[4,3-d]pyrimidin-4-yl)-1-oxa-6-azaspiro[2.5]octane (25 mg, 0.04 mmol) was dissolved in $NH_3$/MeOH (7 M, 1 mL), and the reaction solution was stirred at 40°C for 2 h and evaporated under reduced pressure. The resulting crude product was used for next step.

Step 2: Synthesis of 4-(aminomethyl)-1-(8-fluoro-7-(3-hydroxynaphthalen-1-yl)-2-((tetrahydro-1H-pyrrolizin-7a(5H)-yl) methoxy)pyrido[4,3- d]pyrimidin-4-yl)piperidin-4-ol

The crude product obtained in the previous step was dissolved in 2 mL of EA, HCl/EA solution (4 M, 1 mL) was added at room temperature, and the reaction solution was stirred for 0.5 h. The reaction system was evaporated under reduced pressure, added with 2 mL of aqueous sodium carbonate solution, and extracted with ethyl acetate. The organic phase was dried, evaporated under reduced pressure and purified by Prep-TLC (DCM/MeOH=8/1) to obtain 5 mg of grey solid with a two-step yield of 21%.
LC/MS: m/z 559.3 $[M+H]^+$.

**Example 12**

4-(4-((1R,5S)-3,8-diazabicyclo[3.2.1]octan-3-yl)-8-fluoro-2-((2-(2-fluoroethyl)-2-azabi cyclo[2.2.1]heptan-3-yl)methoxy) pyrido[4,3-d]pyrimidin-7-yl)naphthalen-2-ol

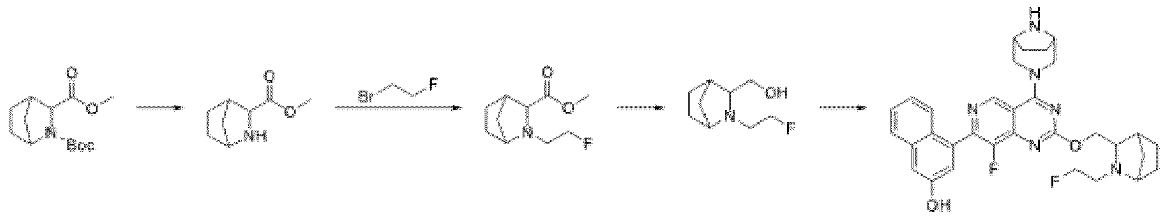

Step 1: Synthesis of methyl 2-azabicyclo[2.2.1]heptan-3-carboxylate

2-(Tert-butyl) 3-methyl 2-azabicyclo[2.2.1]heptan-2,3-dicarboxylate (1 g, 3.9 mmol) was added to 10 mL solution of hydrogen chloride in 1,4-dioxane. The reaction mixture was stirred at room temperature for 2 h. TLC showed that the reaction was complete. The reaction system was evaporated under reduced pressure to obtain the hydrochloride salt of the product, which was used directly for next step.

Step 2: Synthesis of methyl 2-(2-fluoroethyl)-2-azabicyclo[2.2.1]heptan-3-carboxylate

Methyl 2-azabicyclo[2.2.1]heptan-3-carboxylate (the product obtained in step 2) was dissolved in 10 mL of acetonitrile, and then potassium carbonate (1083 mg, 7.8 mmol) was added. Afterwards, the reaction mixture was stirred at 60°C for 24 h. The reaction system was filtered. The filtrate was dried, evaporated under reduced pressure, and then purified by silica gel column chromatography (PE/EA=20/1) to obtain 407 mg of target product, with a total yield of 52% for two steps.

Step 3: Synthesis of (2-(2-fluoroethyl)-2-azabicyclo[2.2.1]heptan-3-yl)methanol

Methyl 2-(2-fluoroethyl)-2-azabicyclo[2.2.1]heptan-3-carboxylate (407 mg, 2.0 mmol) and 5 mL of tetrahydrofuran were added to a reaction flask. The reaction mixture was cooled down to -40°C under the protection of nitrogen and stirred. Lithium aluminium hydride (231 mg, 6.087 mmol) was added in batches and stirred again for 2 hours. TLC showed that the reaction was complete. The reaction system was warmed to room temperature, and diluted by adding a small amount of THF. 0.4 mL of water was added and stirred for 10 min. 15% aqueous sodium hydroxide (0.4 mL) was added and stirred for 10 min. 1.2 mL of water was added and stirred for 30 min. The reaction system was dried by adding anhydrous sodium sulphate and then filtered. The filtrate was dried, evaporated under reduced pressure, and then purified by silica gel column chromatography (PE/EA=5/1-2/1) to obtain 160 mg of target product with a yield of 46%.

Step 4: Synthesis of 4-(4-((1R,5S)-3,8-diazabicyclo[3.2.1]octan-3-yl)-8-fluoro-2-((2-(2-fluoroethyl)-2-azabicyclo[2.2. 1] heptan-3-yl)methoxy)pyrido[4,3-d]pyrimidin-7-yl)naphthalen-2-ol

Using the product obtained in the previous step and the corresponding intermediates as raw materials, the target product was synthesized by a method similar to that of Example 6.
LC/MS: m/z 573.3 $[M+H]^+$.
$^1H$ NMR (400 MHz, $d_6$-DMSO) $\delta$ 9.98 (1H, brs), 9.16 (1H, s), 7.80 (1H, d, $J$ = 8.4 Hz), 7.56 (1H, d, $J$ = 8.4 Hz),

7.42-7.45 (1H, m), 7.22-7.29 (3H, m), 4.52-4.60 (1H, m), 4.43-4.46 (3H, m), 4.17-4.20 (1H, m), 3.93-3.98 (1H, m), 3.61 (1H, s), 3.58 (1H, s), 3.53 (2H, s), 3.25 (1H, s), 2.86-2.88 (1H, m), 2.78-2.82 (1H, m), 2.33-2.36 (1H, m), 2.26-2.27 (1H, m), 1.80-1.88 (1H, m), 1.55-1.70 (6H, m), 1.20-1.33 (4H, m).

**Example 13**

(4-(4-((1R,5S)-3,8-diazabicyclo[3.2.1]octan-3-yl)-8-fluoro-2-((tetrahydro-1H-pyrrolizin -7a(5H)-yl)methoxy)pyrido[4,3-d]pyrimidin-7-yl)naphthalen-2-yl)dimethylphosphineoxide

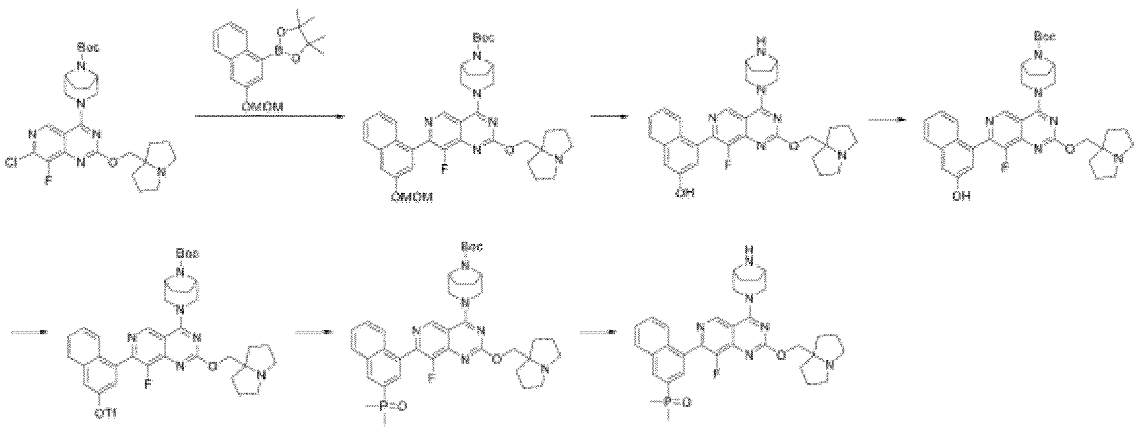

Step 1: Synthesis of tert-butyl (1R,5S)-3-(8-fluoro-7-(3-(methoxymethoxy)naphthalen-1-yl)-2-((tetrahydro-1H-pyrrolizin-7a(5H )-yl)methoxy)pyrido[4,3-d]pyrimidin-4-yl)-3,8-diazabicyclo[3.2.1]octan-8-carboxylate

To a reaction flask, tert-butyl (1R,5S)-3-(7-chloro-8-fluoro-2-((tetrahydro-1H-pyrrolizin-7a(5H)-yl)methoxy)pyrido[4,3-d]pyri midin-4-yl)-3,8-diazabicyclo[3.2.1]octan-8-formate (533 mg, 1.0 mmol), 2-(3-(methoxymethoxy)naphthalen-1-yl)-4,4,5,5-tetramethyl-1,3,2-dioxaborolane (314 mg, 1.0 mmol), potassium carbonate (276 mg, 2.0 mmol), and dioxane/water (8 mL/0.8 mL) were added. After replacement with nitrogen, Pd(dppf)$Cl_2$ (73 mg, 0.1 mmol) was added. Under the protection of nitrogen, the reaction mixture was heated to 100°C and stirred for 6 h. The reaction system was cooled down to room temperature, poured into 10 mL of water, and extracted with ethyl acetate. The organic phase was dried, evaporated under reduced pressure and purified by silica gel column chromatography (DCM/MeOH=20/1) to obtain 220 mg of grey solid with a yield of 32%.

Step 2: Synthesis of 4-(4-((1R,5S)-3,8-diazabicyclo[3.2.1]octan-3-yl)-8-fluoro-2-((tetrahydro-1H-pyrrolizin-7a(5H)-y l) methoxy)pyrido[4,3-d]pyrimidin-7-yl)naphthalen-2-ol

Tert-butyl (1R,5S)-3-(8-fluoro-7-(3-(methoxymethoxy)naphthalen-1-yl)-2-((tetrahydro-1H-pyrrolizin-7a(5H )-yl) methoxy)pyrido[4,3-d]pyrimidin-4-yl)-3,8-diazabicyclo[3.2.1]octan-8-carboxylate (220 mg, 0.32 mmol) was dissolved in 5 mL of ethyl acetate. HCl/EA (4 M, 2 mL) was added and stirred for 0.5 h at room temperature. The reaction system was evaporated under reduced pressure and the crude product was used directly for next step.

Step 3: Synthesis of tert-butyl (1R,5S)-3-(8-fluoro-7-(3-hydroxynaphthalen-1-yl)-2-((tetrahydro-1H-pyrrolizin-7a(5H)-yl) metho xy)pyrido[4,3-d]pyrimidin-4-yl)-3,8-diazabicyclo[3.2.1]octan-8-carboxylate

The crude product obtained in the previous step and 0.5 mL of DIPEA were dissolved in 5 mL of DCM. At room temperature, $(Boc)_2O$ (109 mg, 0.5 mmol) was added and stirred for 2 h. The reaction system was poured into 20 mL of water, and extracted with ethyl acetate. The organic phase was dried and evaporated under reduced pressure, and the crude product was used directly for next step.

Step 4: Synthesis of tert-butyl (1R,5S)-3-(8-fluoro-2-((tetrahydro-1H-pyrrolizin-7a(5H)-yl)methoxy)-7-(3-(((trifluoromethyl)sul fonyl)oxy)naphthalen-1-yl)pyrido[4,3-d]pyrimidin-4-yl)-3,8-diazabicyclo[3.2.1]octan-8-carboxyl ate

The crude product obtained in the previous step and 0.5 mL of DIPEA were dissolved in 5 mL of DCM.

Trifluoromethanesulfonic anhydride (141 mg, 0.5 mmol) was added at 0°C and stirred at room temperature for 4 h. The reaction system was poured into 20 mL of water, and extracted with ethyl acetate. The organic phase was dried, evaporated under reduced pressure and purified by Prep-TLC (DCM/MeOH=20/1) to obtain 85 mg of yellow solid, with a three-step yield of 34%.

Step 5: Synthesis of tert-butyl (1R,5S)-3-(7-(3-(dimethylphosphoryl)naphthalen-1-yl)-8-fluoro-2-((tetrahydro-1H-pyrrolizin-7a( 5H)-yl)methoxy)pyrido[4,3-d]pyrimidin-4-yl)-3,8-diazabicyclo[3.2.1]octan-8-carboxylate

To a reaction flask, tert-butyl (1R,5S)-3-(8-fluoro-2-((tetrahydro-1H-pyrrolizin-7a(5H)-yl)methoxy)-7-(3-(((trifluoromethyl)sul fonyl)oxy)naphthalen-1-yl)pyrido[4,3-d]pyrimidin-4-yl)-3,8-diazabicyclo[3.2.1]octan-8-carboxyl ate (77 mg, 0.1 mmol), dimethylphosphine oxide (78 mg, 1 mmol), triethylamine (101 mg, 1 mmol), xantphos (12 mg, 0.02 mmol), and 3 mL of dioxane were added. After replacement with nitrogen, $Pd_2(dba)_3$ (9 mg, 0.01 mmol) was added. Under the protection of nitrogen, the reaction mixture was heated to 110°C and stirred for 6 h. The reaction system was cooled down to room temperature, poured into 10 mL of water, and extracted with ethyl acetate. The organic phase was dried, evaporated under reduced pressure and purified by Prep-TLC (DCM/MeOH=20/1) to obtain 17 mg of target product with a yield of 24%.

Step 6: Synthesis of (4-(4-((1R,5S)-3,8-diazabicyclo[3.2.1]octan-3-yl)-8-fluoro-2-((tetrahydro-1H-pyrrolizin-7a(5H)-yl) methoxy)pyrido[4,3-d]pyrimidin-7-yl)naphthalen-2-yl)dimethylphosphine oxide

Tert-butyl (1R,5S)-3-(7-(3-(dimethylphosphoryl)naphthalen-1-yl)-8-fluoro-2-((tetrahydro-1H-pyrrolizin-7a( 5H)-yl)methoxy)pyrido[4,3-d]pyrimidin-4-yl)-3,8-diazabicyclo[3.2.1]octan-8-carboxylate (17 mg, 0.024 mmol) was dissolved in 2 mL of ethyl acetate. HCl/EA (4 M, 1 mL) was added at room temperature and stirred for 0.5 h. The reaction system was evaporated under reduced pressure, added with 2 mL of aqueous sodium carbonate, and extracted with ethyl acetate. The organic phase was dried, evaporated under reduced pressure and purified by Prep-TLC (DCM/MeOH=8/1) to obtain 8 mg of grey solid with a yield of 53%.

LC/MS: m/z 601.3 $[M+H]^+$.

**Example 14**

(1R,5S)-3-(8-fluoro-7-(3-hydroxynaphthalen-1-yl)-2-((tetrahydro-1H-pyrrolizin-7a(5H) -yl)methoxy)pyrido[4,3-d]pyrimidin-4-yl)-N-(2,2,2-trifluoroethyl)-3,8-diazabicyclo[3.2.1]octan-8 -formamide

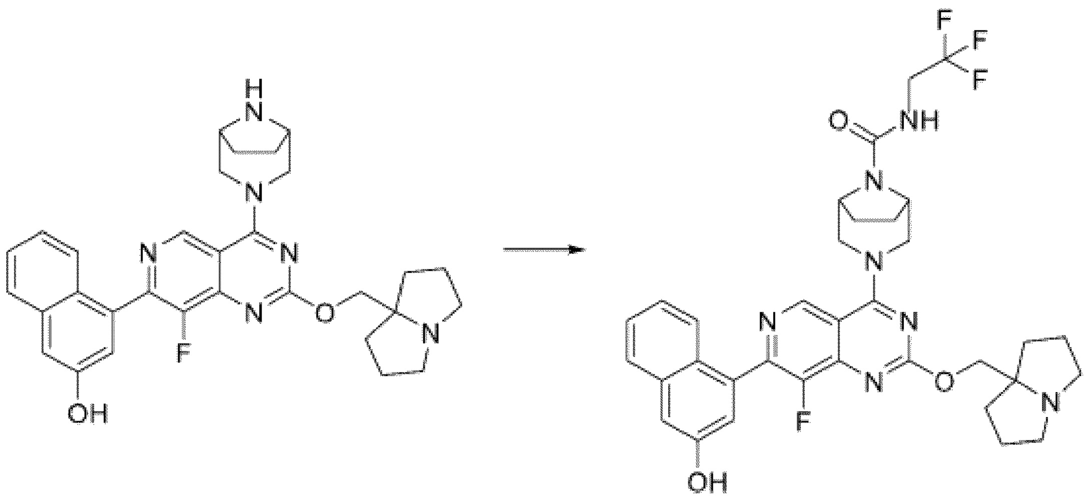

4-(4-((1R,5S)-3,8-diazabicyclo[3.2.1]octan-3-yl)-8-fluoro-2-((tetrahydro-1H-pyrrolizin-7a(5H)-yl)methoxy)pyrido[4,3-d]pyrimidin-7-yl)naphthalen-2-ol (54 mg, 0.1 mmol) and DIPEA (52 mg, 0.4 mmol) were dissolved in 2 mL of tetrahydrofuran. CDI (16 mg, 0.1 mmol) was added at room temperature, and the reaction system was stirred at room temperature for 1 h. Afterwards, 2,2,2-trifluoroethylamine (50 mg, 0.5 mmol) was added, and the mixture was stirred at 70°C for 6 h. The reaction system was poured into 10 mL of water, and extracted with ethyl acetate. The organic phase was dried, evaporated under reduced pressure and purified by TLC (DCM/MeOH=10/1) to obtain14 mg of target product as off-white solid, with a yield of 21%.

LC/MS: m/z 666.3 [M+H]+.

$^1$H NMR (400 MHz, $d_6$-DMSO) δ 9.98 (1H, s), 9.17 (1H, s), 7.80 (1H, d, *J* = 8.4 Hz), 7.54 (1H, d, *J* = 8.4 Hz), 7.42-7.44 (2H, m), 7.21-7.29 (3H, m), 4.45-4.55 (4H, m), 4.08 (2H, s), 3.87-3.95 (4H, m), 3.63-3.66 (2H, m), 2.90-2.95 (2H, m), 1.75-1.90 (10H, m), 1.52-1.50 (2H, m).

**Example 15**

4-(8-Fluoro-2-((tetrahydro-1H-pyrrolizin-7a(5H)-yl)methoxy)-4-((1R,5S)-8-(2,2,2-triflu oroethyl)-3, 8-diazabicyclo[3.2.1] octan-3-yl)pyrido[4,3-d]pyrimidin-7-yl)naphthalen-2-ol

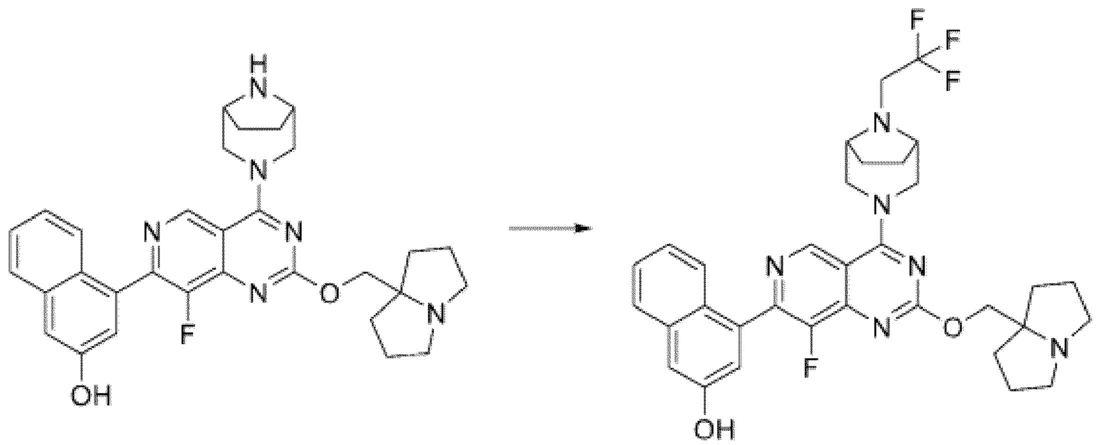

To a reaction flask, 4-(4-((1R,5S)-3,8-diazabicyclo[3.2.1]octan-3-yl)-8-fluoro-2-((tetrahydro-1H-pyrrolizin-7a(5H)-y l)methoxy)pyrido[4,3-d]pyrimidin-7-yl)naphthalen-2-ol (54 mg, 0.1 mmol), potassium carbonate (28 mg, 0.2 mmol) and 2 mL of acetonitrile were added. At room temperature, the reaction system was added with monobromotrifluoroethane (16 mg, 0.1 mmol), stirred overnight, and subjected to suction filtration. The filtrate was evaporated under reduced pressure, and purified by TLC (DCM/MeOH=8/1) to obtain 5 mg of target product as off-white solid, with a yield of 8%.

LC/MS: m/z 623.3 [M+H]+.

$^1$H NMR (400 MHz, $d_6$-DMSO) δ 9.99 (1H, s), 9.18 (1H, s), 7.80 (1H, d, *J* = 8.4 Hz), 7.54 (1H, d, *J* = 8.4 Hz), 7.42-7.46 (1H, m), 7.21-7.29 (3H, m), 4.46-4.50 (2H, m), 4.15-4.17 (2H, m), 3.69-3.72 (2H, m), 3.46 (2H, s), 3.16-3.23 (2H, m), 2.96-3.11 (2H, m), 2.55-2.70 (2H, m), 1.78-2.00 (8H, m), 1.59-1.70 (4H, m).

**Example 16**

3-(8-Fluoro-7-(3-hydroxynaphthalen-1-yl)-2-((tetrahydro-1H-pyrrolizin-7a(5H)-yl)meth oxy)pyrido[4,3-d]pyrimidin-4-yl)-3,9-diazabicyclo[3.3.1]nonan-7-one

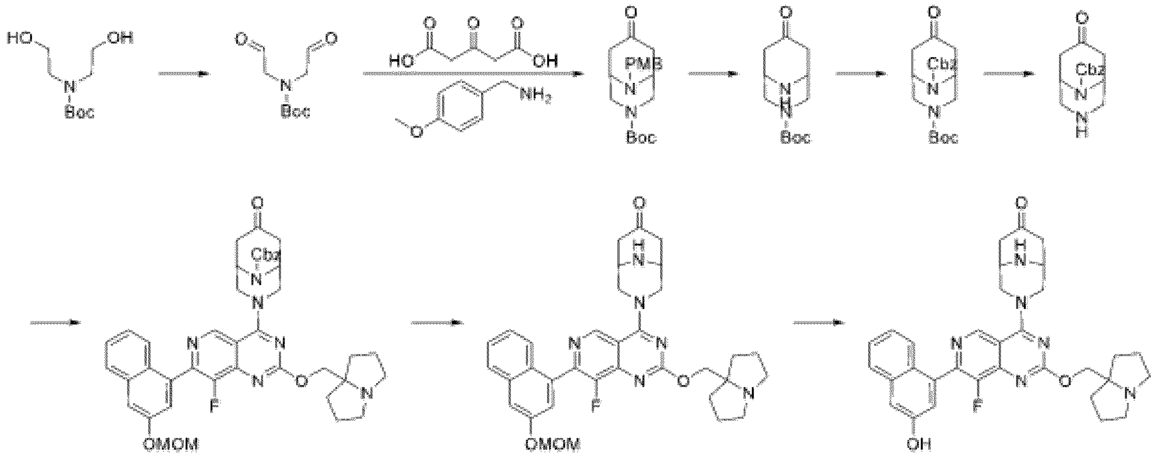

Step 1: Synthesis of tert-butyl bis(2-oxoethyl)carbamate

To a solution of tert-butyl bis(2-hydroxyethyl)carbamate (5.0 g, 24.4 mmol) in 100 mL of THF was added PDC (27.5 g, 73.2 mmol). The reaction mixture was stirred at room temperature for 6 h. The reaction system was poured into 1000 mL of water and extracted with methyl tert-butyl ether. The organic phase was backwashed three times with saturated brine, dried, and evaporated under reduced pressure. The crude product was used directly for next step.

Step 2: Synthesis of tert-butyl 9-(4-methoxybenzyl)-7-oxo-3,9-diazabicyclo[3.3.1]nonan-3-carboxylate

The product obtained in the previous step was dispersed in 50 mL of water, and 3-oxoglutaric acid (3.6 g, 24.8 mmol) and sodium acetate (2.1 g, 25.3 mmol) were added at room temperature and stirred for 0.5 h. A solution of 4-methoxybenzylamine (3.4 g, 24.8 mmol) in dilute hydrochloric acid (3 M, 8 mL) was slowly added dropwise to the reaction system, and then the reaction solution was stirred at room temperature overnight. The reaction solution was diluted by adding water, adjusted to be alkaline with sodium carbonate, and extracted with dichloromethane. The organic phase was dried, evaporated under reduced pressure and purified by silica gel column chromatography (PE/EA=5/1) to obtain 3.0 g of target product, with a two-step yield of 17%.

Step 3: Synthesis of tert-butyl 7-oxo-3,9-diazabicyclo[3.3.1]nonan-3-carboxylate

To a solution of tert-butyl 9-(4-methoxybenzyl)-7-oxo-3,9-diazabicyclo[3.3.1]nonan-3-carboxylate (300 mg, 0.83 mmol) in 5 mL of methanol, 50 mg of 20% palladium hydroxide/carbon was added. The reaction solution was stirred under hydrogen atmosphere at 45°C for 3 h. The reaction solution was subjected to suction filtration. The filtrate was evaporated under reduced pressure and the crude product was used directly for next step.

Step 4: Synthesis of 9-benzyl 3-(tert-butyl) 7-oxo-3,9-diazabicyclo[3.3.1]nonan-3,9-dicarboxylate

The crude product obtained in the previous step and 0.5 mL of DIPEA were dissolved in 5 mL of DCM, and Cbz-Cl (170 mg, 0.83 mmol) was added at 0°C and stirred at room temperature overnight. The reaction system was poured into 20 mL of water, and extracted with dichloromethane. The organic phase was dried, evaporated under reduced pressure, and purified by Prep-TLC (PE/EA=2/1) to obtain 200 mg of colourless oil, with a two-step yield of 64%.

Step 5: Synthesis of benzyl 7-oxo-3,9-diazabicyclo[3.3.1]nonan-9-carboxylate

9-Benzyl 3-(tert-butyl) 7-oxo-3,9-diazabicyclo[3.3.1]nonan-3,9-dicarboxylate (200 mg, 0.53 mmol) was dissolved in 2 mL of ethyl acetate. 2 mL of ethyl acetate solution of hydrogen chloride (4 M) was added, and stirred at room temperature for 1 h. The reaction system was evaporated under reduced pressure and the crude product was used directly for next step

Step 6: Synthesis of benzyl 3-(8-fluoro-7-(3-(methoxymethoxy)naphthalen-1-yl)-2-((tetrahydro-1H-pyrrolizin-7a(5H)-yl)met hoxy)pyrido[4,3-d]pyrimidin-4-yl)-7-oxo-3,9-diazabicyclo[3.3.1]nonan-9-carboxylate

Using the product obtained in the previous step and the corresponding intermediates as raw materials, the target

product was synthesized by a method similar to that of Example 9.

Step 7: Synthesis of 3-(8-fluoro-7-(3-(methoxymethoxy)naphthalen-1-yl)-2-((tetrahydro-1H-pyrrolizin-7a(5H)-yl)met hoxy)pyrido[4,3-d]pyrimidin-4-yl)-3,9-diazabicyclo[3.3.1]nonan-7-one

Benzyl 3-(8-fluoro-7-(3-(methoxymethoxy)naphthalen-1-yl)-2-((tetrahydro-1H-pyrrolizin-7a(5H)-yl)met hoxy) pyrido[4,3-d]pyrimidin-4-yl)-7-oxo-3,9-diazabicyclo[3.3.1]nonan-9-carboxylate (10 mg) was dissolved in 5 mL of methanol, and added with 5 mg of 10 % palladium/carbon. The reaction solution was stirred under hydrogen atmosphere at room temperature for 4 h. The reaction solution was subjected to suction filtration. The filtrate was evaporated under reduced pressure and the crude product was used directly for next step.

Step 8: Synthesis of 3-(8-fluoro-7-(3-hydroxynaphthalen-1-yl)-2-((tetrahydro-1H-pyrrolizin-7a(5H)-yl)methoxy)pyrid o[4,3-d]pyrimidin-4-yl)-3,9-diazabicyclo[3.3.1]nonan-7-one

The crude product obtained in the previous step was dissolved in 2 mL of ethyl acetate. HCl/EA (4 M, 1 mL) was added and stirred for 0.5 h at room temperature. The reaction system was evaporated under reduced pressure, added with 2 mL of aqueous sodium carbonate, and extracted with ethyl acetate. The organic phase was dried, evaporated under reduced pressure and purified by Prep-TLC (DCM/MeOH=8/1) to obtain 3 mg of grey solid, with a two-step yield of 39%.
LC/MS: m/z 569.3 $[M+H]^+$.

**Example 17**

3-(8-Fluoro-7-(3-hydroxynaphthalen-1-yl)-2-((tetrahydro-1H-pyrrolizin-7a(5H)-yl)meth oxy)pyrido[4,3-d]pyrimidin-4-yl)-3,9-diazabicyclo[3.3.1]nonan-7-ol

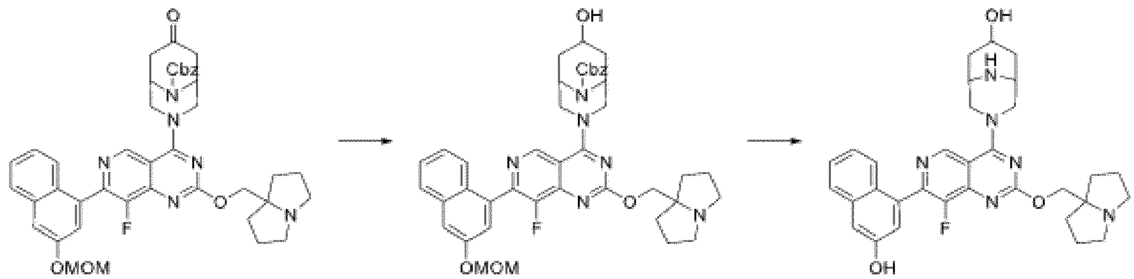

Step 1: Synthesis of benzyl 3-(8-fluoro-7-(3-(methoxymethoxy)naphthalen-1-yl)-2-((tetrahydro-1H-pyrrolizin-7a(5H)-yl) met hoxy)pyrido[4,3-d]pyrimidin-4-yl)-7-hydroxy-3,9-diazabicyclo[3.3.1]nonan-9-carboxylate

To a solution of benzyl 3-(8-fluoro-7-(3-(methoxymethoxy)naphthalen-1-yl)-2-((tetrahydro-1H-pyrrolizin-7a(5H)-yl)met hoxy)pyrido[4,3-d]pyrimidin-4-yl)-7-oxo-3,9-diazabicyclo[3.3.1]nonan-9-carboxylate (5 mg, 0.0067 mmol) in 1 mL of THF, sodium borohydride (1 mg) was added. The reaction mixture was stirred at room temperature for 0.5 h, then poured into 10 mL of water, and extracted with ethyl acetate. The organic phase was dried, evaporated under reduced pressure and purified by Prep-TLC (DCM/MeOH=30/1) to obtain 4 mg of grey solid with a yield of 80%.

Step 2: Synthesis of 3-(8-fluoro-7-(3-hydroxynaphthalen-1-yl)-2-((tetrahydro-1H-pyrrolizin-7a(5H)-yl)methoxy)pyrid o[4,3-d]pyrimidin-4-yl)-3,9-diazabicyclo[3.3.1]nonan-7-ol

Using the product obtained in the previous step as a raw material, the target product was synthesized by a method similar to that of Example 16.
LC/MS: m/z 571.3 $[M+H]^+$.

**Example 18**

8-(8-Fluoro-7-(3-hydroxynaphthalen-1-yl)-2-((tetrahydro-1H-pyrrolizin-7a(5H)-yl)meth oxy)pyrido[4,3-d]pyrimidin-4-yl)-1-oxa-3,8-diazaspiro[4.5]decan-2-one

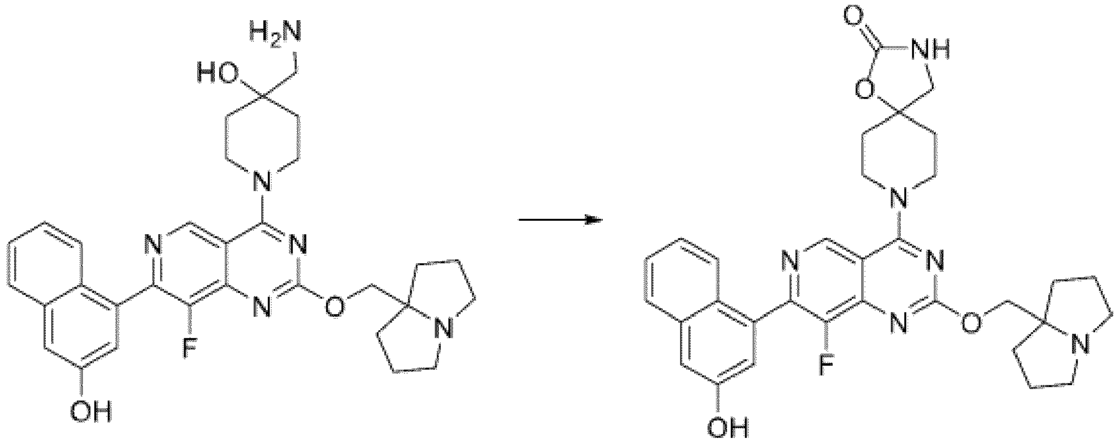

To a solution of 4-(aminomethyl)-1-(8-fluoro-7-(3-hydroxynaphthalen-1-yl)-2-((tetrahydro-1H-pyrrolizin-7a(5H)-yl)methoxy)pyrido[4,3-d]pyrimidin-4-yl)piperidin-4-ol (5 mg, 0.009 mmol) in 1 mL of THF, CDI (2 mg, 0.012 mmol) was added. The reaction mixture was stirred at room temperature for 0.5 h, then heated to 60°C and stirred for 2 h. The reaction solution was poured into 10 mL of water, and extracted with ethyl acetate. The organic phase was dried, evaporated under reduced pressure and purified by Prep-TLC (DCM/MeOH=20/1) to obtain 2 mg of grey solid, with a yield of 38%.

LC/MS: m/z 585.3 $[M+H]^+$.

**Example 19**

(4-(4-((1R,5S)-3,8-diazabicyclo[3.2.1]octa-3-yl)-8-fluoro-2-((tetrahydro-1H-pyrrolizin-7a(5H)-yl)methoxy)pyrido[4,3-d]pyrimidin-7-yl)naphthalen-2-yl)boronic acid

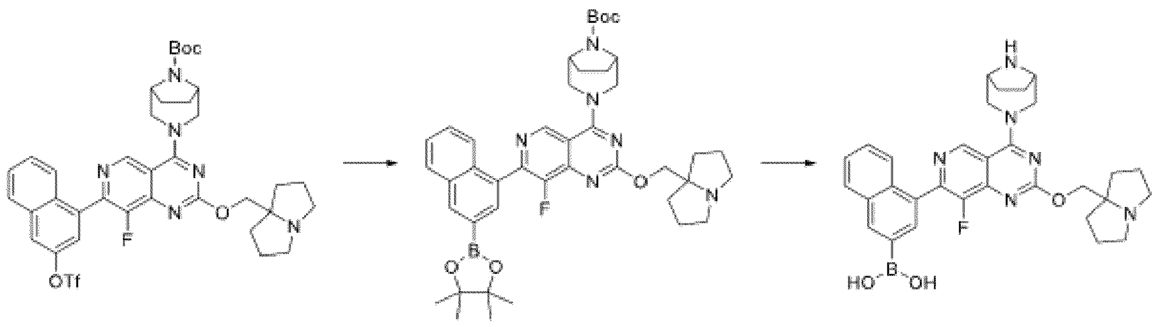

Step 1: Synthesis of (4-(4-((1R,5S)-8-(tert-butoxycarbonyl)-3,8-diazabicyclo[3.2.1]octan-3-yl)-8-fluoro-2-((tetrahydro-1H-)pyrrolizin-7a(5H)-yl)methoxy)pyrido[4,3-d]pyrimidin-7-yl)naphthalen-2-yl)boronic acid

To a solutinon of tert-butyl (1R,5S)-3-(8-fluoro-2-((tetrahydro-1H-pyrrolizin-7a(5H)-yl)methoxy)-7-(3-(((trifluoromethyl)sul fonyl)oxy)naphthalen-1-yl)pyrido[4,3-d]pyrimidin-4-yl)-3,8-diazabicyclo[3.2.1]octan-8-carboxyl ate (100 mg, 0.13 mmol), potassium acetate (38 mg, 0.39 mmol), and bis(pinacolato)diboron (66 mg, 0.26 mmol) in 5 mL of 1,4-dioxane, [1,1'-bis(diphenylphosphino)ferrocene]dichloropalladium (II) (20 mg, 0.027 mmol) was added. The reaction mixture was stirred under the protection of nitrogen at 90°C for 2 h. The reaction solution was poured into 10 mL of water, and extracted with ethyl acetate. The organic phase was dried, evaporated under reduced pressure and purified by column chromatography (DCM/MeOH=20/1) to obtain 6 mg of yellow solid with a yield of 38%.

Step 2: Synthesis of (4-(4-((1R,5S)-3,8-diazabicyclo[3.2.1]octa-3-yl)-8-fluoro-2-((tetrahydro-1H-pyrrolizin-7a(5H)-yl ) methoxy)pyrido[4,3-d]pyrimidin-7-yl)naphthalen-2-yl)boronic acid

(4-(4-((1R,5S)-8-(tert-butoxycarbonyl)-3,8-diazabicyclo[3.2.1]octan-3-yl)-8-fluoro-2-((t etrahydro-1H-)pyrrolizin-7a(5H)-yl)methoxy)pyrido[4,3-d]pyrimidin-7-yl)naphthalen-2-yl)boron ic acid (6 mg) was dissolved in 2 mL of ethyl acetate, and HCl/EA (4 M, 0.5 mL) was added and stirred for 1 h. The reaction system was poured into 10 mL of water, added with sodium bicarbonate to adjust the pH to be weak alkaline, and extracted with ethyl acetate. The organic phase was dried, evaporated under reduced pressure and purified by Prep-TLC (DCM/MeOH=5/1) to obtain 1 mg of light yellow solid, with a two-step yield of 22%.

LC/MS: m/z 569.3 $[M+H]^+$.

**Example 20**

4-(8-Fluoro-4-(3-(hydroxymethyl)piperazin-1-yl)-2-((tetrahydro-1H-pyrrolizin-7a(5H)-yl)methoxy)pyrido[4,3-d]pyrimidin-7-yl)naphthalen-2-ol

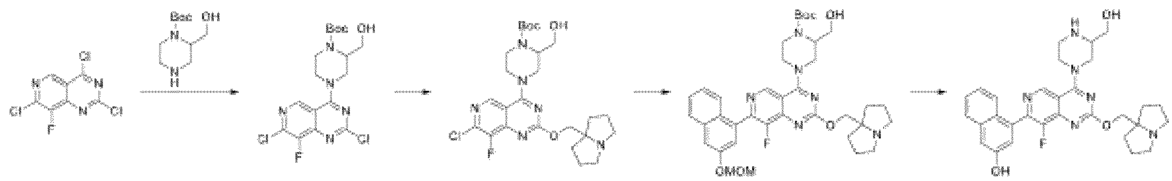

Step 1: Synthesis of tert-butyl 4-(2,7-dichloro-8-fluoropyrido[4,3-d]pyrimidin-4-yl)-2-(hydroxymethyl)piperazin-1-carboxylate

To a reaction flask, 2,4,7-trichloro-8-fluoropyrido[4,3-d]pyrimidine (250 mg, 1.0 mmol), DIPEA (387 mg, 3.0 mmol), and 10 mL of dichloromethane were added, and tert-butyl 2-(hydroxymethyl)piperazin-1-carboxylate (216 mg, 1.0 mmol) was added at -40°C. The reaction system was stirred at -40°C for 1 h, then poured into 50 mL of water, and extracted with ethyl acetate. The organic phase was dried, evaporated under reduced pressure and purified by silica gel column chromatography (PE/EA=1/1) to obtain 234 mg of yellow solid, with a yield of 54%.

Step 2: Synthesis of tert-butyl 4-(7-chloro-8-fluoro-2-((tetrahydro-1H-pyrrolin-7a(5H)-yl)methoxy)pyrido[4,3-d]pyrimidin-4-yl )-2-(hydroxymethyl)piperazin-1-carboxylate

To a reaction flask, (tetrahydro-1H-pyrrolizin-7a(5H)-yl) methanol (85 mg, 0.60 mmol) and 2 mL of tetrahydrofuran were added, and NaH (60%, 28 mg, 0.70 mmoL) was added at 0°C. The mixture was stirred at room temperature for 1 h. The reaction solution was added with tert-butyl 4-(2,7-dichloro-8-fluoropyrido[4,3-d]pyrimidin-4-yl)-2-(hydroxymethyl) piperazin-1-carboxylate (216 mg, 0.50 mmol), and then stirred at room temperature for 2 h. The reaction solution was poured into 10 mL of water, and extracted with ethyl acetate. The organic phase was dried, evaporated under reduced pressure and purified by Prep-TLC (DCM/MeOH=20/1) to obtain 170 mg of grey solid, with a yield of 63%.

Step 3: Synthesis of tert-butyl 4-(8-fluoro-7-(3-(methoxymethyl)naphthalen-1yl)-2-((tetrahydro-1H-pyrrolin-7a(5H)-yl) methox y)pyrido[4,3-d]pyrimidin-4-yl)-2- (hydroxymethyl)piperazin-1-carboxylate

To a reaction flask, tert-butyl 4-(7-chloro-8-fluoro-2-((tetrahydro-1H-pyrrolin-7a(5H)-yl)methoxy)pyrido[4,3-d] pyrimidin-4-yl )-2-(hydroxymethyl)piperazin-1-carboxylate (107 mg, 0.20 mmol), 2-(3-(methoxymethoxy)naphthalen-1-yl)-4,4,5,5-tetramethyl-1,3,2-dioxaborolane (62 mg, 0.20 mmol), potassium carbonate (55 mg, 0.40 mmol) and dioxane/-water (2 mL/0.2 mL) were added. After replacement with nitrogen, $Pd(dppf)Cl_2$ (7 mg, 0.01 mmol) was added. Under the protection of nitrogen, the reaction mixture was heated to 100°C and stirred for 6 h. The reaction system was cooled down to room temperature, poured into 10 mL of water, and extracted with ethyl acetate. The organic phase was dried, evaporated under reduced pressure and purified by Prep-TLC (DCM/MeOH=20/1) to obtain 42 mg of yellow solid, with a yield of 30%.

Step 4: Synthesis of 4-(8-fluoro-4-(3-(hydroxymethyl)piperazin-1-yl)-2-((tetrahydro-1H-pyrrolizin-7a(5H)-yl)methox y) pyrido[4,3-d]pyrimidin-7-yl)naphthalen-2-ol

Tert-butyl 4-(8-fluoro-7-(3-(methoxymethyl)naphthalen-1-yl)-2-((tetrahydro-1H-pyrrolin-7a(5H)-yl)methox y) pyrido[4,3-d]pyrimidin-4-yl)-2-(hydroxymethyl)piperazin-1-carboxylate (40 mg, 0.058 mmol) was dissolved in 2 mL of ethyl acetate, added with HCl/EA (4 M, 0.5 mL) and stirred for 10 min. The reaction system was poured into 10 mL of water, added with sodium bicarbonate to adjust the pH to be weak alkaline, and extracted with ethyl acetate. The organic phase was dried, evaporated under reduced pressure and purified by Prep-TLC (DCM/MeOH=5/1) to obtain 19 mg of grey solid, with a yield of 59%.

LC/MS: m/z 545.3 $[M+H]^+$.

**Example 21**

1-(8-Fluoro-7-(3-hydroxynaphthalen-1-yl)-2-((tetrahydro-1H-pyrrolizin-7a(5H)-yl)meth oxy)pyrido[4,3-d]pyrimidin-4-yl)-4-hydroxypiperidin-4-carboxamide

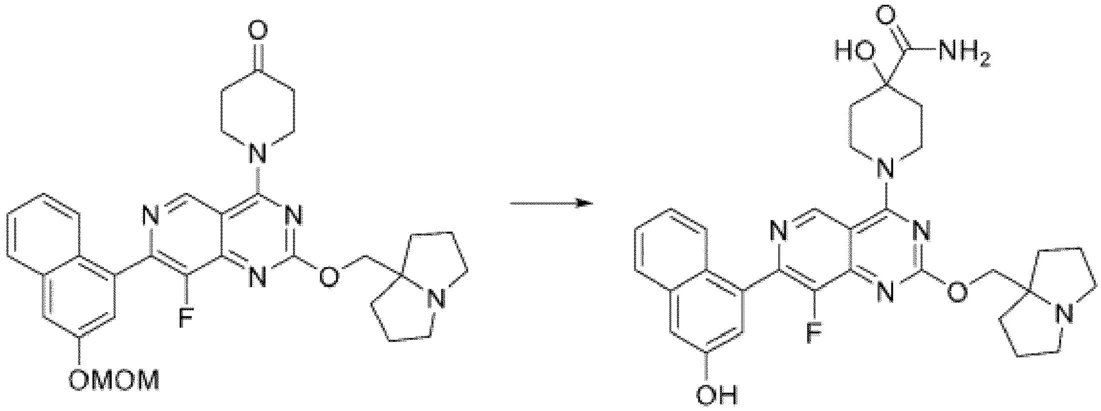

To a solution of 1-(8-fluoro-7-(3-(methoxymethoxy)naphthalen-1-yl)-2-((tetrahydro-1H-pyrrolizin-7a(5H)-yl)met hyl)pyrido[4,3-d]pyrimidin-4-yl)piperidin-4-one (30 mg, 0.05 mmol) in 5 mL of DCM, trimethylsilyl cyanide (15 mg, 0.15 mmol) was added. The reaction mixture was stirred under the protection of nitrogen at room temperature for 3 h. HCl/EA solution (1 mL) was added to the reaction solution and the mixture was stirred again at room temperature for 4 h. The reaction solution was concentrated to dryness at low temperature, then added with 10 mL of water, adjusted to alkaline pH with sodium carbonate solution, and extracted with ethyl acetate. The organic phase was dried, evaporated under reduced pressure and purified by pre-TLC (DCM/MeOH=12/1) to obtain 3 mg of yellow solid, with a yield of 10%.

LC/MS: m/z 573.3 $[M+H]^+$.

$^1H$ NMR (400 MHz, $CDCl_3$) δ 9.00 (s, 1H), 7.61 (t, J = 7.8 Hz, 2H), 7.38-7.29 (m, 2H), 7.23-7.18 (m, 2H), 4.58-4.45 (m, 3H), 4.30 (dd, J = 3.5, 10.5 Hz, 1H), 4.08 (t, J = 10.1 Hz, 1H), 3.61 (brs, 4H), 3.36 (s, 1H), 2.94 (t, J = 5.0 Hz, 1H), 2.89-2.86 (m, 1H), 2.48-2.44 (m, 2H), 1.87-1.80 (m, 2H), 1.72-1.59 (m, 5H), 1.39-1.29 (m, 3H), 0.90-0.83 (m, 1H).

**Example 22**

4-(4-(3-Amino-1-oxa-8-azaspiro[4.5]deca-8-yl)-8-fluoro-2-((tetrahydro-1H-pyrrolizin-7 a(5H)-yl)methoxy)pyrido[4,3-d] pyrimidin-7-yl)naphthalen-2-ol

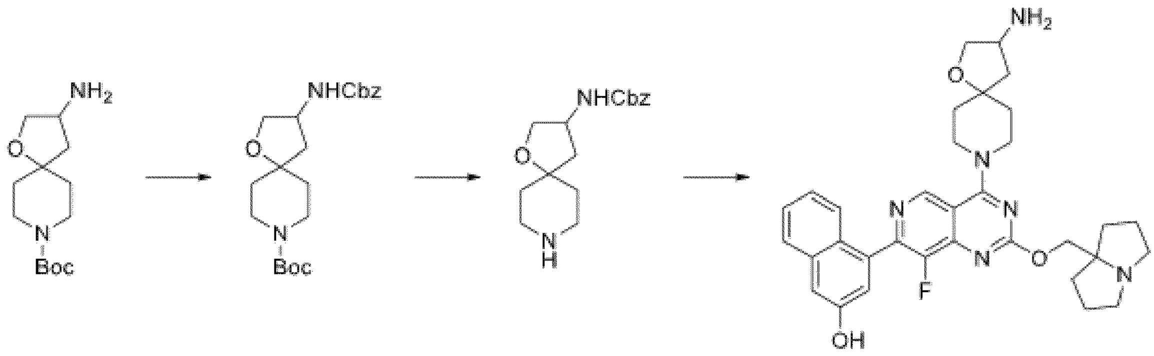

Step 1: Synthesis of tert-butyl 3-(((benzyloxy)carbonyl)amino)-1-oxa-8-azaspiro[4.5]decan-8-carboxylate

To a solution of tert-butyl 3-amino-1-oxa-8-azaspiro[4.5]decan-8-carboxylate (512 mg, 2.0 mmol), and DIPEA (774 mg, 6.0 mmol) in 10 mL of THF, benzyl chloroformate (510 mg, 3.0 mmol) was added under an ice bath. Afterwards, this reaction mixture was stirred at room temperature for 2 h. The reaction solution was poured into 50 mL of water, and extracted with ethyl acetate. The organic phase was dried, and evaporated under reduced pressure to obtain approximately 700 mg of off-white solid, with a yield of 90%.

Step 2: Synthesis of benzyl (1-oxa-8-azaspiro[4.5]deca-3-yl)carbamate

To a single-necked flask containing tert-butyl 3-(((benzyloxy)carbonyl)amino)-1-oxa-8-azaspiro[4.5]decan-8-carboxylate (700 mg, 1.79 mmol), 10 mL of HCl/EA solution was added, and then the reaction mixture was stirred at room temperature for 2 h. The reaction solution was evaporated under reduced pressure to obtain approximately 590 mg of a crude product as white solid, with a yield of 100%.

Step 3: Synthesis of 4-(4-(3-amino-1-oxa-8-azaspiro[4.5]deca-8-yl)-8-fluoro-2-((tetrahydro-1H-pyrrolizin-7a(5H)-yl) methoxy)pyrido[4,3 -d]pyrimidin-7-yl)naphthalen-2-ol

Using the product obtained in the previous step and the corresponding intermediates as raw materials, the target product was synthesized by a method similar to that of Example 16.

LC/MS: m/z 585.3 $[M+H]^+$.

**Example 23**

3-(4-((1R,5S)-3,8-diazabicyclo[3.2.1]octa-3-yl)-8-fluoro-2-((tetrahydro-1H-pyrrolizin-7 a(5H)-yl)methoxy)pyrido[4,3-d] pyrimidin-7-yl)-4-phenoxyphenol

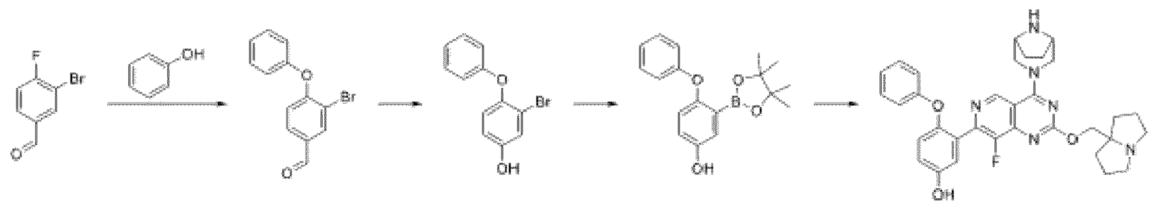

Step 1: Synthesis of 3-bromo-4-phenoxybenzaldehyde

A mixture of 3-bromo-4-fluorobenzaldehyde (203 mg, 1.0 mmol), phenol (113 mg, 1.2 mmol), and potassium carbonate (414 mg, 3.0 mmol) in 10 mL acetonitrile was stirred at 50°C for 2 h. After reaction, the mixture was evaporated under reduced pressure, and purified by column chromatography (PE/EA=20/1) to obtain 210 mg of light yellow solid, with a yield of 76%.

Step 2: Synthesis of 3-bromo-4-phenoxyphenol

To a solution of 3-bromo-4-phenoxybenzaldehyde (210 mg, 0.76 mmol) in 80 mL DCM, meta-chloroperoxybenzoic acid (262 mg, 1.52 mmol) was added. Afterwards, the reaction mixture was stirred under the protection of nitrogen at room temperature for 5 h. The reaction solution was added with 30 ml of water and extracted three times with DCM. The organic phase was dried, concentrated, and purified by pre-TLC to ontain 70 mg of off-white solid, with a yield of 35%.

Step 3: Synthesis of 4-phenoxy-3-(4,4,5,5-tetramethyl-1,3,2-dioxaborolan-2-yl)phenol

To a solution of 3-bromo-4-phenoxyphenol (70 mg, 0.26 mmol), potassium acetate (76 mg, 0.78 mmol), and bis(pinacolato)diboron (132 mg, 0.52 mmol) in 3 mL of 1,4-dioxane, [1,1'-bis(diphenylphosphino)ferrocene]dichloropalladium (II) (15 mg, 0.02 mmol) was added. The reaction mixture was stirred under the protection of nitrogen at 90°C for 2 h. The reaction solution was poured into 10 mL of water, and extracted with ethyl acetate. The organic phase was dried, evaporated under reduced pressure and purified by pre-TLC (PE/EA=5/1) to obtain 35 mg of light yellow solid, with a yield of 43%.

Step 4: Synthesis of 3-(4-((1R,5S)-3,8-diazabicyclo[3.2.1]octa-3-yl)-8-fluoro-2-((tetrahydro-1H-pyrrolizin-7a(5H)-yl)methoxy)pyrido[4,3-d]pyrimidin-7-yl)-4-phenoxyphenol

Using the product obtained in the previous step and the corresponding intermediates as raw materials, the target product was synthesized by a method similar to that of Example 1.

LC/MS: m/z 583.3 $[M+H]^+$.

## Example 24

2-(4-((1R,5S)-3,8-diazabicyclo[3.2.1]octa-3-yl)-8-fluoro-2-((tetrahydro-1H-pyrrolizin-7 a(5H)-yl)methoxy)pyrido[4,3-d]pyrimidin-7-yl)-[1,1'-biphenyl]-4-ol

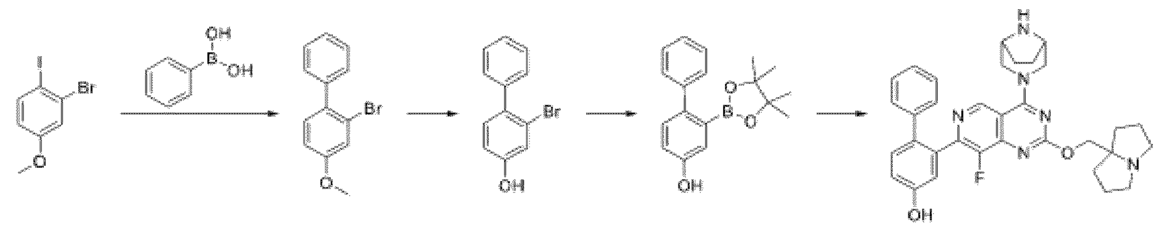

Step 1: Synthesis of 2-bromo-4-methoxy-1,1'-biphenyl

To a solution of 2-bromo-1-iodo-4-methoxybenzene (626 mg, 2.0 mmol), phenylboronic acid (366 mg, 3.0 mmol), and potassium carbonate (828 mg, 6.0 mmol) in 10 mL of 1,4-dioxane/water, [1,1'-bis(diphenylphosphino)ferrocene] dichloropalladium (II) (125 mg, 0.17 mmol) was added. Afterwards, under the protection of nitrogen, this reaction mixture was heated to 80°C and stirred for 2 h. The reaction solution was poured into 50 mL of water, and extracted with ethyl acetate. The organic phase was dried, evaporated under reduced pressure and purified by column chromatography (PE/EA=15/1) to obtain 350 mg of off-white solid, with a yield of 67%.

Step 2: Synthesis of 2-bromo-[1,1'-biphenyl]-4-ol

2-Bromo-4-methoxy-1,1'-biphenyl (350 mg, 1.33 mmol) was dissolved in 5 mL of HBr/acetic acid solution and the reaction mixture was stirred at 100°C overnight. The reaction solution was poured into 50 mL of water, and extracted with ethyl acetate. The organic phase was dried, evaporated under reduced pressure and purified by column chromatography (PE/EA=4/1) to obtain 200 mg of off-white solid, with a yield of 60%.

Step 3: Synthesis of 2-(4,4,5,5-tetramethyl-1,3,2-dioxaborolan-2-yl)-[1,1'-biphenyl]-4-ol

To a solution of 2-bromo-[1,1'-biphenyl]-4-ol (50 mg, 0.20 mmol), potassium acetate (49 mg, 0.50 mmol), and bis(pinacolato)diboron (51 mg, 0.20 mmol) in 3 mL of 1,4-dioxane, [1,1'-bis(diphenylphosphino)ferrocene]dichloropalladium (II) (7 mg, 0.01 mmol) was added. The reaction mixture was stirred under the protection of nitrogen at 90°C for 2 h. The reaction solution was poured into 10 mL of water, and extracted with ethyl acetate. The organic phase was dried, evaporated under reduced pressure and purified by Prep-TLC (PE/EA=5/1) to obtain 40 mg of light yellow solid, with a yield of 68%.

Step 4: Synthesis of 2-(4-((1R,5S)-3,8-diazabicyclo[3.2.1]octa-3-yl)-8-fluoro-2-((tetrahydro-1H-pyrrolizin-7a(5H)-yl) methoxy)pyrid o[4,3-d]pyrimidin-7-yl)-[1,1'-biphenyl]-4-ol

Using the product obtained in the previous step and the corresponding intermediates as raw materials, the target product was synthesized in a method similar to that of Example 1.

LC/MS: m/z 567.3 $[M+H]^+$.

**Example 25**

4-(4-(3,3-difluorooctahydro-5H-pyrrolo[3,2-c]pyridin-5-yl)-8-fluoro-2-((tetrahydro-1H-pyrrolizin-7a(5H)-yl)methoxy)pyrido[4,3-d]pyrimidin-7-yl)naphthalen-2-ol

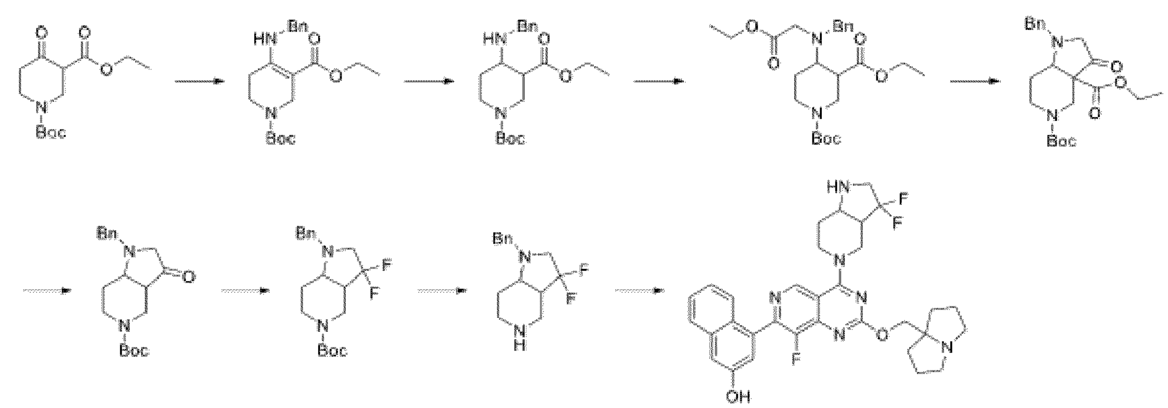

Step 1: Synthesis of 1-(tert-butyl)3-ethyl 4-(benzylamino)-5,6-dihydropyridin-1,3(2H)-dicarboxylate

1-(Tert-butyl)3-ethyl 4-oxopiperidin-1,3-dicarboxylate (1 g, 3.64 mmol) was dissolved in 10 mL of toluene, and benzylamine (390 mg, 3.64 mmol) was added. The reaction system was heated to reflux overnight. The reaction solution was evaporated under reduced pressure and the resulting crude product was used directly for next step.

Step 2: Synthesis of 1-(tert-butyl)3-ethyl 4-(benzylamino) piperidin-1,3-dicarboxylate

The crude product obtained in the previous step was dissolved in 10 mL of methanol, Pd/C (10%, 20 mg) was added. The reaction solution was stirred under hydrogen atmosphere at room temperature for 2 h, and subjected to suction filteration. The filtrate was evaporated under reduced pressure and the crude product was used directly for next step.

Step 3: Synthesis of 1-(tert-butyl)3-ethyl 4-(benzyl(2-ethoxy-2-oxoethyl)amino)piperidin-1,3-dicarboxylate

The crude product obtained in the previous step was dissolved in 5 mL of acetonitrile, and potassium carbonate (138 mg, 1 mmol) and ethyl bromoacetate (84 mg, 0.5 mmol) were added. Afterwards, the reaction solution was stirred at room temperature overnight. The system was poured into 20 mL of water, and extracted with ethyl acetate. The organic phase was dried, evaporated under reduced pressure and purified by column chromatography (PE/EA=1/1) to obtain 150 mg of yellow solid, with a three-step yield of 9%.

Step 4: Synthesis of 5-(tert-butyl)3a-ethyl 1-benzyl-3-oxohexahydro-5H-pyrrolo[3,2-c]pyridin-3a,5(4H)-dicarboxylate

1-(Tert-butyl)3-ethyl 4-(benzyl(2-ethoxy-2-oxoethyl)amino)piperidin-1,3-dicarboxylate (150 mg, 0.33 mmol) was dissolved in 5 mL of toluene. Potassium tert-butoxide (98 mg, 1.00 mmol) was added at 0°C, and stirred at room temperature for 1 h. Then the reaction solution was poured into 20 mL of water and extracted with ethyl acetate. The organic phase was dried, evaporated under reduced pressure and purified by Prep-TLC (PE/EA=2/1) to obtain 65 mg of yellow solid, with a yield of 48%.

Step 5: Synthesis of tert-butyl 1-benzyl-3-oxooctahydro-5H-pyrrolo[3,2-c]pyridin-5-carboxylate

5-(Tert-butyl)3a-ethyl 1-benzyl-3-oxohexahydro-5H-pyrrolo[3,2-c]pyridin-3a,5(4H)-dicarboxylate (65 mg, 0.16 mmol) was dissolved in 5 mL of dioxane, and added with 1 mL of 5 M aqueous sodium hydroxide. The reaction solution was subjected to reflux with stirring at overnight. The system was poured into 20 mL of water, and extracted with ethyl acetate. The organic phase was dried and evaporated under reduced pressure, and the resulting crude product was used directly for next step.

Step 6: Synthesis of tert-butyl 1-benzyl-3,3-difluorooctahydro-5H-pyrrolo[3,2-c]pyridin-5-carboxylate

The crude product obtained in the previous step was dissolved in 3 mL of dichloromethane, and DAST (40 mg, 0.24 mmol) was added at -78°C. Afterwards, the reaction solution was stirred at room temperature for 1 h. The system was poured into 20 mL of water, and extracted with ethyl acetate. The organic phase was dried, evaporated under reduced pressure and purified by Prep-TLC (PE/EA=1/1) to obtain 35 mg of yellow solid, with a two-step yield of 61%.

Step 7: Synthesis of 1-benzyl-3,3-difluorooctahydro-1H-pyrrolo[3,2-c]pyridine

Tert-butyl 1-benzyl-3,3-difluorooctahydro-5H-pyrrolo[3,2-c]pyridin-5-carboxylate (35 mg, 0.1 mmol) was dissolved in 2 mL of dichloromethane, and 0.2 mL of trifluoroacetic acid was added. The reaction solution was stirred at room temperature for 1 h. The system was evaporated under reduced pressure and the crude product was used directly for next step.

Step 8: Synthesis of 4-(4-(3,3-difluorooctahydro-5H-pyrrolo[3,2-c]pyridin-5-yl)-8-fluoro-2-((tetrahydro-1H-pyrrolizi n-7a(5H)-yl)methoxy)pyrido[4,3-d]pyrimidin-7-yl)naphthalen-2-ol

Using the product of the previous step and the corresponding intermediates as raw materials, the target product was synthesized by a method similar to that of Example 16.

LC/MS: m/z 591.3 $[M+H]^+$.

**Example 26**

4-(4-((1R,5S)-8,8-difluoro-3-azabicyclo[3.2.1]octane-3-yl)-8-fluoro-2-((1-((3-fluoropyr rolidin-1)-yl)methyl)cyclopropyl) methoxy)pyrido[4,3-d]pyrimidin-7-yl)naphthalen-2-ol

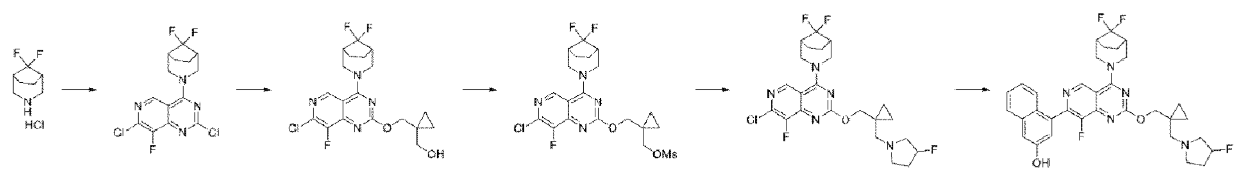

Step 1: Synthesis of 2,7-dichloro-4-((1R,5S)-8,8-difluoro-3-azabicyclo[3.2.1]octane-3-yl)-8-fluoropyrido[4,3-d]pyrim idine

2,4,7-Trichloro-8-fluoropyrido[4,3-d]pyrimidine (250 mg, 1.0 mmol), DIPEA (184 mg, 3.0 mmol) and 10 mL dichloromethane were added to a reaction flask, and then 8,8-difluoro-3-azabicyclo[3.2.1]octane hydrochloride (184 mg, 1.0 mmol) was added at -40°C. After stirring at -40°C for 1 h, the mixture was poured into 50 mL of water, and extracted with ethyl acetate. The organic phase was dried, evaporated under reduced pressure, and purified by silica gel column

chromatography (PE/EA=1/1) to obtain 210 mg of yellow solid with a yield of 58%.

Step 2: Synthesis of (1-(((7-chloro-4-((1R,5S)-8,8-difluoro-3-azabicyclo[3.2.1]octane-3-yl)-8-fluoropyrido[4,3-d]pyri midin-2-yl)oxy)methyl)cyclopropyl)methanol

Sodium hydride (100 mg, 2.5 mmol) was added to 5 mL of a solution of cyclopropan-1,1-diyldimethanol (102 mg, 1.0 mmol) in THF under the protection of nitrogen gas and ice bath. Afterwards, the reaction mixture was stirred at room temperature for 1 h under the protection of nitrogen gas, and then 2,7-dichloro-4-((1R,5S)-8,8-difluoro-3-azabicyclo[3.2.1]octan-3-yl)-8-fluoropyrido[4,3-d]pyrimi dine (181 mg, 0.5 mmol) was added to the reaction system under ice bath, followed by stirring at room temperature for 2 h. The reaction solution was poured into 50 mL of aqueous ammonium chloride solution, and extracted with ethyl acetate. The organic phase was dried, evaporated under reduced pressure, and purified by column chromatography (PE/EA=3/1) to obtain 100 mg off-white solid with a yield of 47%.

Step 3: Synthesis of (1-(((7-chloro-4-((1R,5S)-8,8-difluoro-3-azabicyclo[3.2.1]octan-3-yl)-8-fluoropyrido[4,3-d]pyri midin-2-yl)oxy)methyl)cyclopropyl)methanesulfonate

Methanesulfonyl chloride (53 mg, 0.46 mmol) was added to (1-(((7-chloro-4-((1R,5S)-8,8-difluoro-3-azabicyclo[3.2.1]octan-3-yl)-8-fluoropyrido[4,3-d]pyri midin-2-yl)oxy)methyl)cyclopropyl)methanol (100 mg, 0.23 mmol) and triethylamine (100 mg, 0.92 mmol) in 5 mL DCM. The reaction mixture was stirred at room temperature for 3 h. The reaction solution was poured into 30mL of water, and extracted with DCM. The organic phase was dried and evaporated under reduced pressure to obtain 90 mg of light yellow oil with a yield of 77%.

Step 4: Synthesis of 7-chloro-4-((1R,5S)-8,8-difluoro-3-azabicyclo[3.2.1]octan-3-yl)-8-fluoro-2-((1-((3-fluoropyrrolid in-1-yl)methyl)cyclopropyl)methoxy)pyrido[4,3-d]pyrimidine

A mixture of (1-(((7-chloro-4-((1R,5S)-8,8-difluoro-3-azabicyclo[3.2.1]octan-3-yl)-8-fluoropyrido[4,3-d]pyri midin-2-yl)oxy)methyl)cyclopropyl)methanesulfonate (90 mg, 0.18 mmol), potassium carbonate (99 mg, 0.72 mmol) and 3-fluoropyrrolidine hydrochloride (45 mg, 0.36 mmol) in 4 mL of acetonitrile was heated to 70°C and stirred for 5 h. The reaction solution was poured into 20 mL of water, and extracted with ethyl acetate. The organic phase was dried, evaporated under reduced pressure and purified by pre-TLC (DCM/MeOH=30/1) to obtain 20 mg of off-white solid with a yield of 22%.

Step 5: Synthesis of 4-(4-((1R,5S)-8,8-difluoro-3-azabicyclo[3.2.1]octan-3-yl)-8-fluoro-2-((1-((3-fluoropyrrolidin-1)-yl) methyl)cyclopropyl)methoxy)pyrido[4,3-d]pyrimidin-7-yl)naphthalen-2-ol

Using the product of the previous step as raw materials, the target product was synthesized by a method similar to that of Example 1.

LC/MS: m/z=608.3 $[M+H]^+$.

$^1H$ NMR (400 MHz, $CDCl_3$) δ 8.94 (s, 1H), 7.66-7.61 (m, 2H), 7.36 (t, J = 7.5 Hz, 1H), 7.24-7.17 (m, 3H), 4.61 (d, J = 13.0 Hz, 2H), 4.40 (dd, J = 10.9, 47.7 Hz, 2H), 3.74 (d, J = 13.1 Hz, 2H), 2.88-2.82 (m, 2H), 2.68 (d, J = 11.4 Hz, 1H), 2.554-2.48 (m, 2H), 2.39-2.32 (m, 2H), 2.08-1.97 (m, 2H), 1.89-1.84 (m, 2H), 1.68-1.58 (m, 2H), 1.16-1.01 (m, 2H), 0.69 (s, 2H), 0.51 (s, 2H).

**Example 27**

4-(4-((1R,5S,8s)-8-(3,3-difluoroazetidin-1-yl)-3-azabicyclo[3.2.1]octan-3-yl)-8-fluoro-2 -((tetrahydro-1H-pyrrolidin-7a(5H)-yl)methoxy)pyrido[4,3-d]pyrimidin-7-yl)naphthalen-2-ol

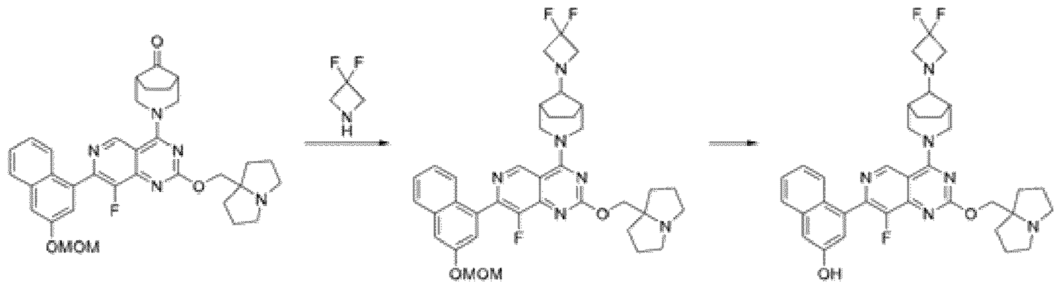

Step 1: Synthesis of 4-((1R,5S,8s)-8-(3,3-difluoroazetidin-1-yl)-3-azabicyclo[3.2.1]octan-3-yl)-8-fluoro-7-(3-(methox ymethoxy)naphthalen-1-yl)-2-((tetrahydro-1H-pyrrolidin-7a(5H)-yl)methoxy)pyrido[4,3-d]pyrim idine

3,3-Difluoro-azetidine (9 mg, 0.10 mmol) was added to a solution of (1R,5S)-3-(8-fluoro-7-(3-(methoxymethoxy) naphthalen-1-yl)-2-((tetrahydro-1H-pyrrolizin-7a(5H )-yl)methyl)pyrido[4,3-d]pyrimidin-4-yl)-3-azabicyclo[3.2.1]octan-8-one (30 mg, 0.05 mmol, synthesized by a method similar to Example 9) in 2 mL THF. After the reaction mixture was stirred at room temperature for 0.5 h, 10 mg of sodium triacetoxyborohydride was added to the reaction solution, and then stirred at room temperature for 2 h. The reaction solution was added with 10 mL of water, and extracted with ethyl acetate. The organic phase was dried, evaporated under reduced pressure and purified by pre-TLC (DCM/MeOH=10/1) to obtain 22 mg of yellow solid with a yield of 65%.

Step 2: Synthesis of 4-(4-((1R,5S,8s)-8-(3,3-difluoroazetidin-1-yl)-3-azabicyclo[3.2.1]octan-3-yl)-8-fluoro-2-((tetrahy dro-1H-pyrrolidin-7a(5H)-yl)methoxy)pyrido[4,3-d]pyrimidin-7-yl)naphthalen-2-ol

4-((1R,5S,8s)-8-(3,3-difluoroazetidin-1-yl)-3-azabicyclo[3.2.1]octan-3-yl)-8-fluoro-7-(3 -(methoxymethoxy) naphthalen-1-yl)-2-((tetrahydro-1H-pyrrolidin-7a(5H)-yl)methoxy)pyrido[4,3 -d]pyrimidine (20 mg, 0.030 mmol) was dissolved in 2 mL of ethyl acetate. HCl/EA (4 M, 0.5 mL) was added and stirred for 10 min. The reaction system was poured into 10 mL of water, adjusted to slightly alkaline pH with sodium bicarbonate, and extracted with ethyl acetate. The organic phase was dried, evaporated under reduced pressure and purified by Prep-TLC (DCM/MeOH=10/1) to obtain 8 mg of grey solid with a yield of 42%.

LC/MS: m/z=631.3 [M+H]$^+$.

**Example 28**

4-(4-((1R,5S)-3,8-diazabicyclo[3.2.1]octan-3-yl)-8-fluoro-2-(((2R,7aS)-2-fluorotetrahy dro-1H-pyrrolizin-7a(5H)-yl) methoxy)pyrido[4,3-d]pyrimidin-7-yl)phenanthren-2-ol

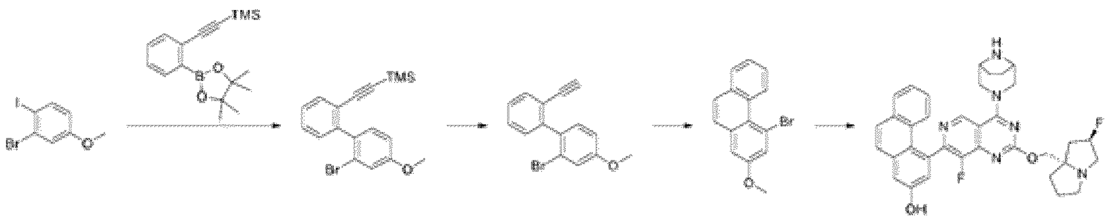

Step 1: Synthesis of ((2'-bromo-4'-methoxy-[1,1'-dibiphenyl]-2-yl)ethynyl)trimethylsilane

[1,1'-Bis(diphenylphosphino)ferrocene]dichloropalladium (II) (37 mg, 0.05 mmol) was added to a 5 mL dioxane/-water (10/1) solution of 2-bromo-1-iodoanisole (311 mg, 1.0 mmol), sodium carbonate (216 mg, 2.0 mmol) and ((2-(4,4,5,5-tetramethyl-1,3,2-dioxaborolan-2-yl)phenyl)ethynyl)trimethylsilane (300 mg, 1.0 mmol). The reaction mixture was stirred under nitrogen gas protection at 90°C for 2 h. The reaction solution was poured into 10 mL of water and extracted with ethyl acetate. The organic phase was dried, evaporated under reduced pressure and purified by Prep-TLC (PE/EA=100/1) to obtain 280 mg of white solid with a yield of 78%.

Step 2: Synthesis of 2-bromo-2'-ethynyl-4-methoxy-1,1'-biphenyl

((2'-Bromo-4'-methoxy-[1,1'-dibiphenyl]-2-yl)ethynyl)trimethylsilane (280 mg, 0.78 mmol) was dissolved in 5 mL of methanol, and 1 g of potassium carbonate was added. The reaction mixture was stirred at room temperature for 2 h. After filtration, the filtrate was evaporated under reduced pressure to obtain 230 mg of yellow solid with a yield of 100%.

Step 3: Synthesis of 4-bromo-2-methoxyphenanthrene

2-Bromo-2'-ethynyl-4-methoxy-1,1'-biphenyl (230 mg, 0.80 mmol) was dissolved in 5 mL of 1,2-dichloroethane, and 20 mg of platinum dichloride was added. The reaction mixture was stirred at 90°C overnight. The reaction solution was evaporated under reduced pressure and then purified by Prep-TLC (PE/EA = 100/1) to obtain 180 mg of white solid with a yield of 78%.

Step 4: Synthesis of 4-(4-((1R,5S)-3,8-diazabicyclo[3.2.1]octan-3-yl)-8-fluoro-2-(((2R,7aS)-2-fluorotetrahydro-1H-py rrolizin-7a(5H)-yl)methoxy)pyrido[4,3-d]pyrimidin-7-yl)phenanthren-2-ol

Using the product obtained in the previous step and the corresponding intermediate as raw materials, the target product was synthesized by a method similar to that in Example 1.

LC/MS: m/z=609.3 [M+H]$^+$.

**Example 29**

(4-(4-((1R,5S)-3,8-diazabicyclo[3.2.1]octan-3-yl)-8-fluoro-2-((tetrahydro-1H-pyrrolizin -7a(5H)-yl)methoxy)pyrido[4,3-d]pyrimidin-7-yl)naphthalen-2-yl)(methyl) phosphite

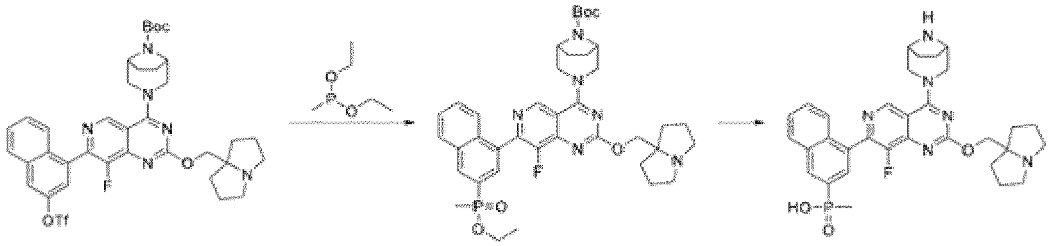

Step 1: Synthesis of tert-butyl (1R,5S)-3-(7-(3-(ethoxy(methyl)phosphinyl)naphthalen-1-yl)8-fluoro-2-((tetrahydro-1H-pyrrolizi n-7a(5H)-yl)methoxy)-pyrido[4,3-d]pyrimidin-4-yl)-3,8-diazabicyclo[3.2.1]octan-8-carboxylate

5 mg of $Pd_2(dba)_3$ was added to a 3 mL dioxane solution of tert-butyl (1R,5S)-3-(8-fluoro-2-((tetrahydro-1H-pyrrolizin-7a(5H)-yl)methoxy)-7-(3-(((trifluoromethyl)sul fonyl)oxy)naphthalen-1-yl)pyrido[4,3-d]pyrimidin-4-yl)-3,8-diazabicyclo[3.2.1]octan-8-carboxyl ate (30 mg, 0.039 mmol), diethyl methylphosphite (14 mg, 0.1 mmol), triethylamine (20 mg, 0.2 mmol), and 5 mg of Xantphos. The reaction mixture was stirred under nitrogen gas protection at 100°C for 3 h. The reaction solution was poured into 10 mL of water and extracted with ethyl acetate. The organic phase was dried, evaporated under reduced pressure, and purified by Prep-TLC (DCM/MeOH=20/1) to obtain 10 mg of yellow solid with a yield of 36%.

Step 2: Synthesis of (4-(4-((1R,5S)-3,8-diazabicyclo[3.2.1]octan-3-yl)-8-fluoro-2-((tetrahydro-1H-pyrrolizin-7a(5H)-yl) methoxy)pyrido[4,3-d]pyrimidin-7-yl)naphthalen-2-yl)(methyl) phosphite

Tert-butyl (1R,5S)-3-(7-(3-(ethoxy(methyl)phosphinyl)naphthalen-1-yl)8-fluoro-2-((tetrahydro-1H-pyrrolizi n-7a(5H)-yl)methoxy)-pyrido[4,3-d]pyrimidin-4-yl)-3,8-diazabicyclo[3.2.1]octan-8-carboxylate (10 mg, 0.014 mmol) was dissolved in 2 mL of dioxane, and 0.5 mL of 1 M aqueous sodium hydroxide solution was added and stirred at room temperature for 6 h. 1 mL of 4 M HCl dioxane solution was added and stirred for 0.5 h. The reaction solution was poured into 10 mL of water, adjusted to weakly alkaline with sodium carbonate, and extracted with ethyl acetate. The organic phase was dried, evaporated under reduced pressure, and purified by Prep-TLC (DCM/MeOH=10/1) to obtain 1 mg of grey solid with a yield of 12%.

LC/MS: m/z=603.3 [M+H]$^+$.

**Example 30**

4-(4-((1R,5S)-3,8-diazabicyclo[3.2.1]octan-3-yl)-6-ethynyl-8-fluoro-2-(((2R,7aS)-2-flu orotetrahydro-1H-pyrrolizin-7a(5H)-yl)methoxy)quinazolin-7-yl)-5,6-difluoronaphthalen-2-ol

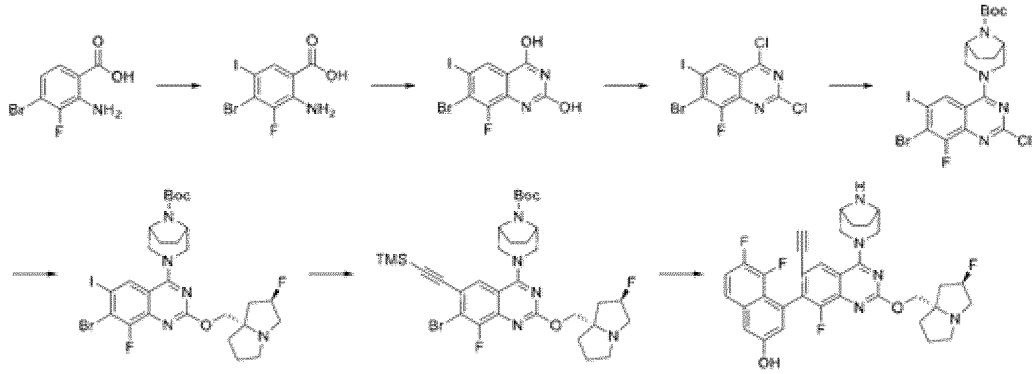

Step 1: Synthesis of 2-amino-4-bromo-3-fluoro-5-iodobenzoic acid

2-Amino-4-bromo-3-fluorobenzoic acid (2.34 g, 10.0 mmol), $Ag_2SO_4$ (3.08 g, 10.0 mmol), and 30 mL of absolute ethanol were added to a reaction flask, and an ethanol solution of iodine (2.53 g, 10.0 mmol) was slowly added dropwise. The reaction mixture was stirred at room temperature for 4 h. After filtration, the filtrate was concentrated and extracted with water and ethyl acetate. The organic phase was dried, evaporated under reduced pressure and purified by column chromatography (PE/EA = 1/1) to obtain 1.96 g of the target product with a yield of 54%.

Step 2: Synthesis of 7-bromo-8-fluoro-6-iodoquinazolin-2,4-diol

2-Amino-4-bromo-3-fluoro-5-iodobenzoic acid (1.9 g, 5.28 mmol) and urea (3.5 g, 50.00 mmol) were added to a reaction flask. The reaction system was heated to 170°C and stirred for 4 h. The reaction system was cooled to room temperature and extracted 3 times with water and ethyl acetate. The organic phases were combined, washed with saturated brine, dried over anhydrous sodium sulfate, and filtered. The filtrate was concentrated under reduced pressure, and purified by column chromatography (PE/EA = 5/1) to obtain 1.2 g of the target product with a yield of 60%.

Step 3: Synthesis of 7-bromo-2,4-dichloro-8-fluoro-6-iodoquinazoline

7-Bromo-8-fluoro-6-iodoquinazolin-2,4-diol (1.2 g, 3.12 mmol) and phosphorus oxychloride (2.3 g, 15.00 mmol) were added to a reaction flask, and the reaction system was heated to 90°C and stirred for 3 h. The reaction system was then cooled to room temperature, concentrated under reduced pressure to obtain a crude product, which was directly used in the next step.

Step 4: Synthesis of tert-butyl (1R,5S)-3-(7-bromo-2-chloro-8-fluoro-6-iodoquinazolin-4-yl)-3,8-diazacyclo[3.2.1]octan-8-carb oxylate

7-Bromo-2,4-dichloro-8-fluoro-6-iodoquinazoline (the crude product of the previous step), DIEA (1.29 g, 10.00 mmol), tert-butyl (1R,5S)-3,8-diazabicyclo[3.2.1]octan-8-carboxylate (678 mg, 3.20 mmol), and 20 mL of absolute ethanol were added to a reaction flask. The reaction system was stirred at room temperature for 4 h and extracted 3 times with water and ethyl acetate. The organic phases were combined, washed with saturated brine, dried over anhydrous sodium sulfate, and filtered. The filtrate was concentrated under reduced pressure, and purified by column chromatography (PE/EA = 4/1) to obtain 410 mg of the target product, with a two-step yield of 22%.

Step 5: Synthesis of tert-butyl (1R,5S)-3-(7-bromo-8-fluoro-2-((2R,7aS)-2-fluorotetrahydro-1H-pyrrolizin-7a(5H)-ylmethoxy)-6-iodoquinazolin-4-yl)-3,8-diazacyclo[3.2.1]octan-8-carboxylate

Tert-butyl (1R,5S)-3-(7-bromo-2-chloro-8-fluoro-6-iodoquinazolin-4-yl)-3,8-diazacyclo[3.2.1]octan-8-carb oxylate (120 mg, 0.20 mmol), ((2R,7aS)-2-fluorotetrahydro-1H-pyrrolizin-7a(5H)-yl)methanol (48 mg, 0.30 mmol), cesium

carbonate (130 mg, 0.40 mmol), and 1,4-diazabicyclo[2.2.2]octane (22 mg, 0.20 mmol) were added to a reaction flask. Under nitrogen gas protection, anhydrous THF (1 mL) and DMF (1 mL) were added. After replacement with nitrogen again, the reaction mixture was stirred at 60°C for 4 h, then quenched by adding water, and extracted with ethyl acetate. The organic phase was washed with saturated brine, dried, filtered, and concentrated under reduced pressure. The crude product was separated and purified by Prep-TLC (PE/EA=1/1) to obtain 85 mg of yellow solid, with a yield of 59%.

Step 6: Synthesis of tert-butyl (1R,5S)-3-(7-bromo-8-fluoro-2-((2R,7aS)-2-fluorotetrahydro-1H-pyrrolizin-7a(5H)-ylmethoxy)-6-((trimethylsilyl)ethynyl)quinazolin-4-yl)-3,8-diazacyclo[3.2.1]octan-8-carboxylate

Tert-butyl (1R,5S)-3-(7-bromo-8-fluoro-2-((2R,7aS)-2-fluorotetrahydro-1H-pyrrolizin-7a(5H)-ylmethoxy)-6-iodoquinazolin-4-yl)-3,8-diazacyclo[3.2.1]octan-8-carboxylate (85 mg, 0.12 mmol), trimethylsilylacetylene (15 mg, 0.15 mmol), triethylamine (30 mg, 0.30 mmol), 5 mg of cuprous iodide, and 2 mL of DMF were added to a reaction flask. After replacement with nitrogen, 5 mg of $Pd(PPh_3)Cl_2$ was added. The reaction system was heated to 80°C under nitrogen protection and stirred for 3 h. The reaction mixture was cooled to room temperature, poured into water, extracted with ethyl acetate, washed with saturated brine, dried over anhydrous sodium sulfate, and filtered. The filtrate was concentrated under reduced pressure and purified by Prep-TLC (DCM/MeOH = 20/1) to obtain 28 mg of the target product with a yield of 35%.

Step 7: Synthesis of 4-(4-((1R,5S)-3,8-diazabicyclo[3.2.1]octan-3-yl)-6-ethynyl-8-fluoro-2-(((2R,7aS)-2-fluorotetrahydro-1H-pyrrolizin-7a(5H)-yl)methoxy)quinazolin-7-yl)-5,6-difluoronaphthalen-2-ol

Using the product obtained in the previous step and the corresponding intermediate as raw materials, the target product was synthesized by a method similar to that in Example 2.

LC/MS: m/z=618.3 $[M+H]^+$.

**Example 31**

4-(4-((1R,5S)-3,8-diazabicyclo[3.2.1]octan-3-yl)-8-fluoro-2-((5-(3-fluoropropyl)-2-oxa-5-azabicyclo[2.2.1]heptan-6-yl)methoxy)pyrido[4,3-d]pyrimidin-7-yl)-5,6-difluoronaphthalen-2-ol

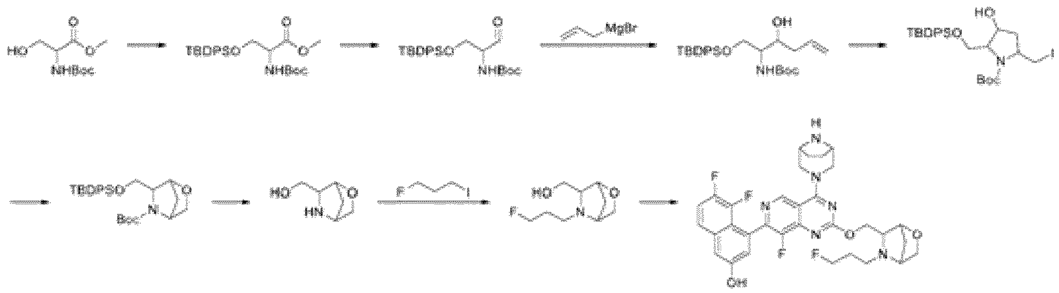

Step 1: Synthesis of N-(tert-butoxycarbonyl)-O-(tert-butyldiphenylsilyl)serine methyl ester

(Tert-butoxycarbonyl) serine methyl ester (3.0 g, 13.7 mmol), imidazole (2.8 g, 0.41 mmol), and 30 mL of dichloromethane were added to a reaction flask. TBDPSCl (3.7 g, 13.7 mmol) was added and stirred at room temperature for 3 h. The reaction solution was poured into water, extracted with ethyl acetate, washed with saturated brine, dried over anhydrous sodium sulfate, and filtered. The filtrate was concentrated under reduced pressure and purified by column chromatography (PE/EA=10/1) to obtain 6.3 g of the target product, with a yield of 100%.

Step 2: Synthesis of tert-buty(1-((tert-butyldiphenylsilyl)oxy)-3-oxopropan-2-yl)carbamate

N-(tert-butoxycarbonyl)-O-(tert-butyldiphenylsilyl)serine methyl ester (6.3 g, 13.7 mmol) was dissolved in 100 mL of toluene. DIBALH (1 M, 14 mL, 14 mmol) was added dropwise at -78°C and stirred at -78°C for 1 h. The reaction solution was poured into water, extracted with ethyl acetate, washed with saturated brine, dried over anhydrous sodium sulfate, and filtered. The filtrate was concentrated under reduced pressure to obtain a crude product.

Step 3: Synthesis of tert-butyl(1-((tert-butyldiphenylsilyl)oxy)-3-hydroxyhex-5-en-2-yl)carbamate

The crude product from the previous step was dissolved in 100 mL of tetrahydrofuran, and allylmagnesium bromide (1 M, 14 mL, 14 mmol) was added dropwise at -78°C. Afterwards, the reaction solution was warmed to room temperature and stirred for 1 h. The reaction solution was poured into water, extracted with ethyl acetate, washed with saturated brine, dried over anhydrous sodium sulfate, and filtered. The filtrate was concentrated under reduced pressure and purified by column chromatography (PE/EA=5/1) to obtain 2.6 g of the target product, with a two-step yield of 40%.

Step 4: Synthesis of tert-butyl 2-((((tert-butyldiphenylsilyl)oxy)methyl)-3-hydroxy-5-(iodomethyl)pyrrolidin-1-carboxylate

Tert-butyl(1-((tert-butyldiphenylsilyl)oxy)-3-hydroxyhex-5-en-2-yl)carbamate (1.5 g, 3.2 mmol), 10 mL of aqueous sodium bicarbonate solution (1 M), and 10 mL of ethyl acetate were added to a reaction flask, and then elemental iodine (0.8 g, 3.1 mmol) was added and stirred at room temperature for 6 h. The reaction solution was poured into water, extracted with ethyl acetate, washed with saturated brine, dried over anhydrous sodium sulfate, and filtered. The filtrate was concentrated under reduced pressure and purified by column chromatography (PE/EA=5/1) to obtain 2.0 g of the target product, with a yield of 53%.

Step 5: Synthesis of tert-butyl 6-((((tert-butyldiphenylsilyl)oxy)methyl)-2-oxa-5-azabicyclo[2.2.1]heptan-5-carboxylate

Tert-butyl 2-((((tert-butyldiphenylsilyl)oxy)methyl)-3-hydroxy-5-(iodomethyl)pyrrolidin-1-carboxylate (1.0 g, 1.7 mmol) was dissolved in 10 mL of tetrahydrofuran, and potassium tert-butoxide (196 mg, 2.0 mmol) was added at 0°C. The system was stirred at room temperature overnight. The reaction solution was poured into water, extracted with ethyl acetate, washed with saturated brine, dried over anhydrous sodium sulfate, and filtered. The filtrate was concentrated under reduced pressure and purified by column chromatography (PE/EA=10/1) to obtain 350 mg of the target product, with a yield of 44%.

Step 6: Synthesis of (2-oxa-5-azabicyclo[2.2.1]heptan-6-yl)methanol

Tert-butyl 6-((((tert-butyldiphenylsilyl)oxy)methyl)-2-oxa-5-azabicyclo[2.2.1]heptan-5-carboxylate (350 mg, 0.75 mmol) was dissolved in 10 mL of tetrahydrofuran, and 200 mg of TBAF was added and stirred at room temperature overnight. 0.5 mL of 6 M dilute hydrochloric acid was added to the reaction solution and stirred at room temperature for 0.5 h. The reaction system was then directly evaporated under reduced pressure to obtain a crude product.

Step 7: Synthesis of (5-(3-fluoropropyl)-2-oxa-5-azabicyclo[2.2.1]heptan-6-yl)methanol

The crude product from the previous step was dissolved in 5 mL of acetonitrile, and 1-fluoro-3-iodopropane (150 mg, 0.8 mmol) and potassium carbonate (276 mg, 2.0 mmol) were added. The resulting mixture was stirred at 80°C for 6 h. After filtration, the filtrate was concentrated under reduced pressure and purified by column chromatography (DCM/MeOH=10/1) to obtain 25 mg of the target product, with a two-step yield of 18%.

Step 8: Synthesis of 4-(4-((1R,5S)-3,8-diazabicyclo[3.2.1]octan-3-yl)-8-fluoro-2-((5-(3-fluoropropyl)-2-oxa-5-azabic yclo[2.2.1]heptan-6-yl)methoxy)pyrido[4,3-d]pyrimidin-7-yl)-5,6-difluoronaphthalen-2-ol

Using the product obtained in the previous step and the corresponding intermediate as raw materials, the target product was synthesized by a method similar to that in Example 6.

LC/MS: m/z=625.3 $[M+H]^+$.

**Example 32**

4-(4-((1R,5S)-3,8-diazabicyclo[3.2.1]octan-3-yl)-8-fluoro-2-(((2R,7aS)-2-fluorotetrahy dro-1H-pyrrolizin-7a(5H)-yl) methoxy)pyrido[4,3-d]pyrimidin-7-yl)-5,6,8-trifluoronaphthalen-2-ol

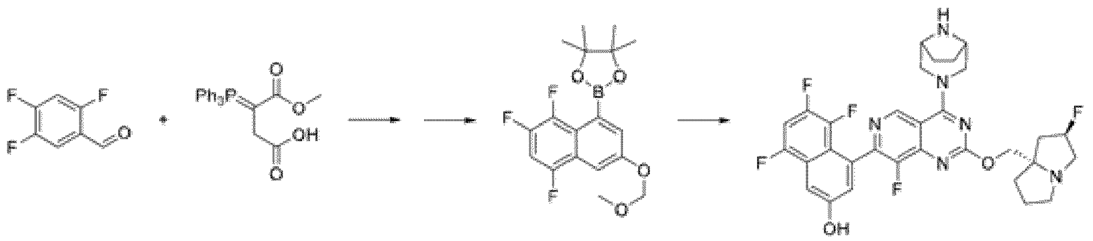

Step 1: Synthesis of 4,4,5,5-tetramethyl-2-(5,7,8-trifluoro-3-(methoxymethoxy)naphthalen-1-yl)-1,3,2-dioxaborolane

Using 2,4,5-trifluorobenzaldehyde and 4-methoxy-4-oxo-3-(triphenyl-phosphinylidene)butanoic acid as raw materials, the target product was synthesized by a method similar to that of intermediate 5.

Step 2: Synthesis of 4-(4-((1R,5S)-3,8-diazabicyclo[3.2.1]octan-3-yl)-8-fluoro-2-(((2R,7aS)-2-fluorotetrahydro-1H-py rrolizin-7a(5H)-yl)methoxy)pyrido[4,3-d]pyrimidin-7-yl)-5,6,8-trifluoronaphthalen-2-ol

Using the product obtained in the previous step and the corresponding intermediate as raw materials, the target product was synthesized by a method similar to that in Example 6.

LC/MS: m/z=613.2 $[M+H]^+$.

**Example 33**

3-(((4-((1R,5S)-3,8-diazabicyclo[3.2.1]octan-3-yl)-7-(7,8-difluoro-3-hydroxynaphthalen -1-yl)-8-fluoropyrido[4,3-d]pyrimidin-yl)oxy)methyl)-N-(2,2,2-trifluoroethyl)-2-azabicyclo[2.2.1 ]heptan-2-carboxamide

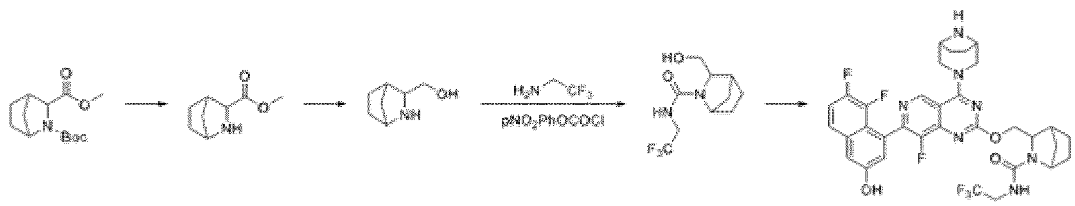


Step 1: Synthesis of methyl 2-azabicyclo[2.2.1]heptan-3-carboxylate

Methyl 2-(tert-butoxycarbonyl)-2-azabicyclo[2.2.1]heptan-3-carboxylate (255 mg, 1.0 mmol) was dissolved in 5 mL of dichloromethane, and 1 mL of trifluoroacetic acid was added and stirred at room temperature for 0.5 h. The reaction mixture was evaporated under reduced pressure to obtain a crude product, which was directly used for the next step.

Step 2: Synthesis of (2-azabicyclo[2.2.1]heptan-3-yl)-methanol

Methyl 2-azabicyclo[2.2.1]heptan-3-carboxylate (crude product) was dissolved in 5 mL of tetrahydrofuran. Lithium aluminum hydride (76 mg, 2.0 mmol) was added at 0°C and stirred for 1 h. The reaction system was added with 20 mL of water, extracted with ethyl acetate, washed with saturated brine, dried over anhydrous sodium sulfate and filtered. The filtrate was concentrated under reduced pressure and purified by column chromatography (DCM/MeOH=20/1) to obtain 40 mg of the target product, with a two-step yield of 31%.

Step 3: Synthesis of 3-(hydroxymethyl)-N-(2,2,2-trifluoro ethyl)-2-azabicyclo[2.2.1]heptan-2-carboxamide

2,2,2-Trifluoroethylamine (50 mg, 0.5 mmol) and 0.2 mL of triethylamine were dissolved in 2 mL of dichloromethane, and p-nitrophenyl chloroformate (100 mg, 0.5 mmol) was added at 0°C and stirred at room temperature for 1 h. Then, (2-azabicyclo[2.2.1]heptan-3-yl)-methanol (40 mg, 0.3 mmol) was added. The reaction mixture was heated to 60 °C and stirred overnight. The reaction system was added with 20 mL of water, extracted with ethyl acetate, washed with saturated brine, dried over anhydrous sodium sulfate, and filtered. The filtrate was concentrated under reduced pressure and purified by column chromatography (DCM/MeOH = 20/1) to obtain 35 mg of the target product, with a yield of 44%.

Step 4: Synthesis of 3-(((4-((1R,5S)-3,8-diazabicyclo[3.2.1]octan-3-yl)-7-(7,8-difluoro-3-hydroxynaphthalen-1-yl)-8-f luoropyrido[4,3-d]pyrimidin-yl)oxy)methyl)-N-(2,2,2-trifluoroethyl)-2-azabicyclo[2.2.1]heptan-2 -carboxamide

Using the product obtained in the previous step and the corresponding intermediate as raw materials, the target product was synthesized by a method similar to that in Example 6.
LC/MS: m/z=688.2 [M+H]+.

**Example 34**

4-(4-((1R,5S)-3,8-diazabicyclo[3.2.1]octan-3-yl)-8-fluoro-2-((3-(4-methoxyphenyl)tetra hydro-1H-pyrrolidin-7a(5H)-yl) methoxy)pyrido[4,3-d]pyrimidin-7-yl)-5,6-difluoronaphthalen-2-ol

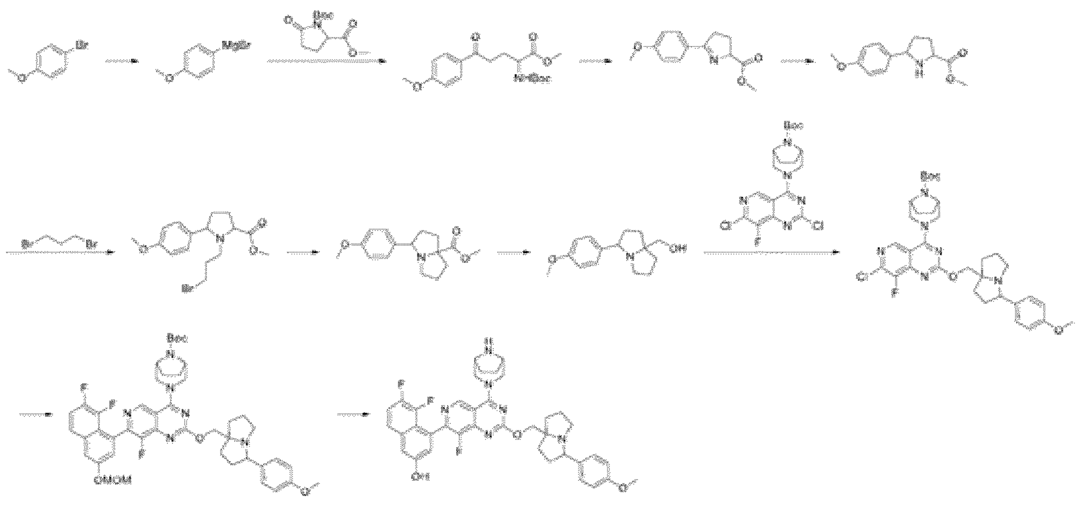

Step 1: Synthesis of p-methoxyphenylmagnesium bromide

Magnesium chips (4.8 g, 200 mmol) were weighed and added into a flask. Methoxybromobenzene (1 g, 5.3 mmol), 0.2 g of elemental iodine, and 5 mL of tetrahydrofuran were added. After heating to initiate the reaction, a 150 mL tetrahydrofuran solution of methoxybromobenzene (17.7 g, 94.7 mmol) was added dropwise, and the reflux state of the reaction system was maintained. Afterwards, reflux was continued for 0.5 h. The system was directly used for the next step of reaction.

Step 2: Synthesis of methyl 2-((tert-butoxycarbonyl)amino)-5-(4-methoxyphenyl)5-oxopentanoate

1-(Tert-butyl)-2-methyl-5-oxopyrrolidin-1,2-dicarboxylate (24.3 g, 100 mmol) and 250 mL of tetrahydrofuran were added to a reaction flask. The reaction mixture was cooled to -70°C under a dry ice-ethanol bath and nitrogen gas protection. Then, p-methoxyphenylmagnesium bromide (the reaction liquid obtained in the previous step) was added. The reaction was allowed to proceed at this temperature for 3 h. A saturated ammonium chloride solution (500 mL) was added, and the mixture was extracted with ethyl acetate three times. The organic phase was dried, evaporated under reduced pressure, and purified by column chromatography (PE/EA=1/1) to obtain 18.0 g of the target product with a yield of 51%.

Step 3: Synthesis of methyl 5-(4-methoxyphenyl)-3,4-dihydro-2H-pyrrole-2-carboxylate

Methyl 2-((tert-butoxycarbonyl)amino)-5-(4-methoxyphenyl)5-oxopentanoate (18 g, 51.3 mmol) was added to a reaction flask. HCl/MeOH (4 M, 100 mL) was added at room temperature, and stirred overnight. The reaction system was directly evaporated under reduced pressure to obtain a crude product, which was not purified.

Step 4: Synthesis of methyl 5-(4-methoxyphenyl)pyrrole-2-carboxylate

The crude product obtained in the previous step was dissolved in 200 mL of methanol, and sodium borohydride (3.8 g, 100 mmol) was added at 0°C and stirred for 1 h. The reaction system was evaporated under reduced pressure,

added with 5 mL of cold water, and extracted with ethyl acetate. The organic phase was dried and evaporated under reduced pressure to obtain a crude product, which was not purified.

Step 5: Synthesis of methyl 1-(3-bromopropyl)-5-(4-methoxyphenyl)pyrrolidin-2-carboxylate

The crude product obtained in the previous step was dissolved in 200 mL of acetonitrile, and potassium carbonate (13.8 g, 100 mmol) and 1,3-dibromopropane (10.1 g, 50 mmol) were added. The reaction mixture was stirred at 60°C for 6 h. The mixture was suction-filtered, and the filtrate was evaporated under reduced pressure and then purified by column chromatography (DCM/MeOH=20/1) to obtain 7.9 g of the target product, with a three-step yield of 44%.

Step 6: Synthesis of methyl 3-(4-methoxyphenyl)tetrahydro-1H-pyrrolizin-7a(5H)-carboxylate

Methyl 1-(3-bromopropyl)-5-(4-methoxyphenyl)pyrrolidin-2-carboxylate (7.9 g, 22.2 mmol) was dissolved in 100 mL of tetrahydrofuran, and LDA (1 M, 25 mL, 25 mmol) was added dropwise at 0°C. Afterwards, the mixture was stirred at 0°C for 1 h and then at room temperature for 1 h. The reaction mixture was poured into 300 mL of water, extracted with ethyl acetate, washed with saturated brine, dried over anhydrous sodium sulfateand filtered. The filtrate was concentrated under reduced pressure and purified by column chromatography (DCM/MeOH=20/1) to obtain 3.6 g of the target product, with a yield of 59%.

Step 7: Synthesis of (3-(4-methoxyphenyl)tetrahydro-1H-pyrrolizin-7a(5H)-yl)methanol

Methyl 3-(4-methoxyphenyl)tetrahydro-1H-pyrrolizin-7a(5H)-formate (2.7 g, 10 mmol) was dissolved in 30 mL of tetrahydrofuran, and lithium aluminum hydride (760 mg, 20 mmol) was added at 0°C and stirred for 1 h. 100 mL of water was added to the reaction system, and the mixture was extracted with ethyl acetate, washed with saturated brine, dried over anhydrous sodium sulfateand filtered. The filtrate was concentrated under reduced pressure and purified by column chromatography (DCM/MeOH=20/1) to obtain 2.2 g of the target product, with a yield of 89%.

Step 8: Synthesis of tert-butyl (1R,5S)-3-(7-chloro-8-fluoro-2-((3-(4-methoxyphenyl)tetrahydro)-1H-pyrrolizin-7a(5H)-yl)meth oxy)pyrido[4,3-d]pyrimidin-4-yl)-3,8-diazabicyclo[3.2.1]octan-8-carboxylate

(3-(4-Methoxyphenyl)tetrahydro-1H-pyrrolidin-7a(5H)-yl)methanol (130 mg, 0.53 mmol) and 5 mL of tetrahydrofuran were added to a reaction flask, and NaH (60%, 64 mg, 1.6 mmol) was added at 0°C. The mixture was stirred at room temperature for 1 h. After adding tert-butyl (1R,5S)-3-(2,7-dichloro-8-fluoropyrido[4,3-d]pyrimidin-4-yl)-3,8-diazabicyclo[3.2.1]octan-8-car boxylate (173 mg, 0.40 mmol), the reaction solution was further stirred at room temperature for 2 h. The reaction solution was poured into 20 mL of water and extracted with ethyl acetate. The organic phase was dried, evaporated under reduced pressure, and purified by Prep-TLC (DCM/MeOH=15/1) to obtain 75 mg of yellow solid, with a yield of 29%.

Step 9: Synthesis of tert-butyl (1R,5S)-3-(7-(7,8-difluoro-3-(methoxymethoxy)naphthalen-1-yl)-8-fluoro-2-((3-(4-methoxyphen yl)tetrahydro-1H-pyrrolidin-7a(5H)-yl)methoxy)pyrido[4,3-d]pyrimidin-4-yl)-3,8-diazabicyclo[3. 2.1]octan-8-carboxylate

Tert-butyl (1R,5S)-3-(7-chloro-8-fluoro-2-((3-(4-methoxyphenyl)tetrahydro)-1H-pyrrolizin-7a(5H)-yl)meth oxy) pyrido[4,3-d]pyrimidin-4-yl)-3,8-diazabicyclo[3.2.1]octan-8-carboxylate (50 mg, 0.078 mmol), 2-(7,8-difluoro-3-(methoxymethoxy)naphthalen-1-yl)-4,4,5,5-tetramethyl-1,3,2-dioxaborolane (27 mg, 0.078 mmol), potassium carbonate (21 mg, 0.15 mmol), and dioxane/water (2 mL/0.2 mL) were added to a reaction flask. After replacement with nitrogen, 5 mg of $Pd(dppf)Cl_2$ was added. The reaction mixture was heated to 100°C under nitrogen protection and stirred for 6 h. After the reaction system was cooled to room temperature, it was poured into 10 mL of water and extracted with ethyl acetate. The organic phase was dried, evaporated under reduced pressure, and purified by Prep-TLC (DCM/MeOH=15/1) to obtain 38 mg of grey solid, with a yield of 58%.

Step 10: Synthesis of 4-(4-((1R,5S)-3,8-diazabicyclo[3.2.1]octan-3-yl)-8-fluoro-2-((3-(4-methoxyphenyl)tetrahydro-1 H-pyrrolidin-7a(5H)-yl)methoxy)pyrido[4,3-d]pyrimidin-7-yl)-5,6-difluoronaphthalen-2-ol

Tert-butyl (1R,5S)-3-(7-(7,8-difluoro-3-(methoxymethoxy)naphthalen-1-yl)-8-fluoro-2-((3-(4-methoxyphen yl) tetrahydro-1H-pyrrolidin-7a(5H)-yl)methoxy)pyrido[4,3-d]pyrimidin-4-yl)-3,8-diazabicyclo[3. 2.1]octan-8-carboxylate (38 mg, 0.046 mmol) was dissolved in 2 mL of ethyl acetate, and HCl/EA (4 M, 0.5 mL) was added and stirred for 10 min. The reaction system was poured into 10 mL of water, adjusted to weakly alkaline with sodium carbonate, and

extracted with ethyl acetate. The organic phase was dried, evaporated under reduced pressure, and purified by Prep-TLC to obtain 22 mg of grey solid, with a yield of 71%.

LC/MS: m/z=684.3 [M+H]+.

$^1$H NMR (400 MHz, $CDCl_3$) δ 8.89 (s, 1H), 7.45-7.39 (m, 1H), 7.27-7.17 (m, 4H), 6.78-6.74 (m, 2H), 4.67-4.60 (m, 1H), 4.47-4.40 (m, 1H), 4.36-4.21 (m, 2H), 3.71-3.58 (m, 7H), 2.91-2.83 (m, 1H), 2.72-2.58 (m, 1H), 2.35-2.25 (m, 1H), 2.13-2.05 (m, 1H), 1.94-1.53 (m, 8H).

**Example 35**

3-(4-(7a-(((4-((1R,5S)-3,8-diazabicyclo[3.2.1]octan-3-yl)-8-fluoro-7-(3-hydroxynaphth alen-1-yl)pyrido[4,3-d]pyrimidin-2-yl)oxy)methyl)hexahydro-1H-pyrrolidin-3-yl)phenoxy)propa n-1,2-diol

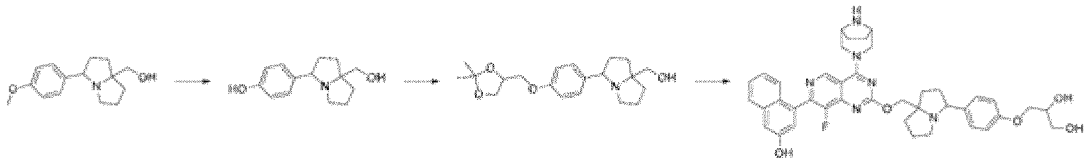

Step 1: Synthesis of 4-(7a-(hydroxymethyl)hexahydro-1H-pyrrolizin-3-yl)phenol

(3-(4-Methoxyphenyl)tetrahydro-1H-pyrrolizin-7a(5H)-yl)methanol (247 mg, 1.0 mmol) was dissolved in 5 mL of dichloromethane, and 0.2 mL of boron tribromide was added at 0°C. After stirring for 1 h at 0°C, the reaction system was directly evaporated under reduced pressure, and the crude product was directly used for the next step of reaction.

Step 2: Synthesis of (3-(4-((2,2-dimethyl-1,3-dioxolane-4-yl)methoxy)phenyl)tetrahydro-1H-pyrrolizin-7a(5H)-yl)me thanol

The crude product from the previous step was dissolved in 5 mL of acetonitrile, and potassium carbonate (276 mg, 2.0 mmol) and 4-(bromomethyl)-2,2-dimethyl-1,3-dioxolane (195 mg, 1.0 mmol) were added. The mixture was stirred at 80°C for 6 h and then subjected to suction filtration. The filtrate was evaporated under reduced pressure. The crude product was purified by column chromatography to obtain 110 mg of the target product, with a two-step yield of 32%.

Step 3: Synthesis of 3-(4-(7a-(((4-((1R,5S)-3,8-diazabicyclo[3.2.1]octan-3-yl)-8-fluoro-7-(3-hydroxynaphthalen-1-yl) pyrido[4,3-d]pyrimidin-2-yl)oxy)methyl)hexahydro-1H-pyrrolidin-3-yl)phenoxy)propan-1,2-diol

Using the product obtained in the previous step and the corresponding intermediate as raw materials, the target product was synthesized by a method similar to that in Example 34.

LC/MS: m/z=707.3 [M+H]+.

**Example 36**

4-(4-((1R,5S)-3,8-diazabicyclo[3.2.1]octan-3-yl)-8-fluoro-2-((5-(4-fluorophenyl)-1-(3-f luoropropyl)pyrrolidin-2-yl)methoxy)pyrido[4,3-d]pyrimidin-7-yl)-5,6-difluoronaphthalen-2-ol

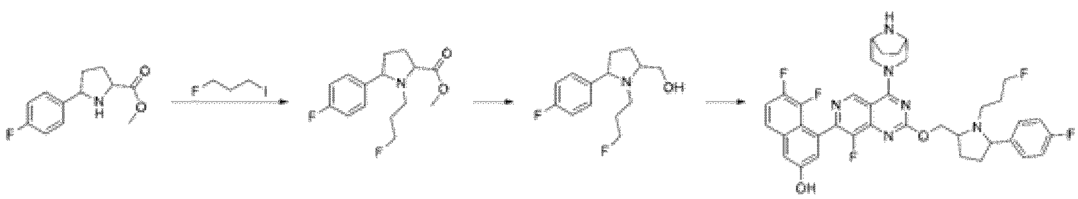

Step 1: Synthesis of methyl 5-(4-fluorophenyl)-1-(3-fluoropropyl)pyrrolidin-2-formate

Methyl 5-(4-fluorophenyl)pyrrolidin-2-carboxylate (223 mg, 1.0 mmol), 1-fluoro-3-iodopropane (188 mg, 1.0 mmol), potassium carbonate (414 mg, 3.0 mmol), and 5 mL of acetonitrile were added to a reaction flask. The mixture was heated to 80°C and stirred for 6 h. The reaction system was cooled to room temperature and subjected to suction filtration. The filtrate was evaporated under reduced pressure to obtain a crude product, which was directly used for the next step of reaction.

Step 2: Synthesis of (5-(4-fluorophenyl)-1-(3-fluoropropyl)pyrrolidin-2-yl)-methanol

Methyl 5-(4-fluorophenyl)-1-(3-fluoropropyl)pyrrolidin-2-formate (crude product) was dissolved in 5 mL of tetrahydrofuran, and lithium aluminum hydride (76 mg, 2.0 mmol) was added at 0°C and stirred for 1 h. The reaction system was added with 20 mL of water, extracted with ethyl acetate, washed with saturated brine, dried over anhydrous sodium sulfateand filtered. The filtrate was concentrated under reduced pressure and purified by Prep-TLC (DCM/MeOH=20/1) to obtain 95 mg of the target product, with a two-step yield of 37%.

Step 3: Synthesis of 4-(4-((1R,5S)-3,8-diazabicyclo[3.2.1]octan-3-yl)-8-fluoro-2-((5-(4-fluorophenyl)-1-(3-fluoroprop yl) pyrrolidin-2-yl)methoxy)pyrido[4,3-d]pyrimidin-7-yl)-5,6-difluoronaphthalen-2-ol

Using the product obtained in the previous step and the corresponding intermediate as raw materials, the target product was synthesized by a method similar to that in Example 34.

LC/MS: m/z=691.3 $[M+H]^+$.

**Example 37**

4-(4-((1R,5S)-3,8-diazabicyclo[3.2.1]octan-3-yl)-8-fluoro-2-(((2R,7aS)-2-fluorotetrahy dro-1H-pyrrolizin-7a(5H)-yl) methoxy)pyrido[4,3-d]pyrimidin-7-yl)-8-cyclopropyl-5,6-difluoron aphthalen-2-ol

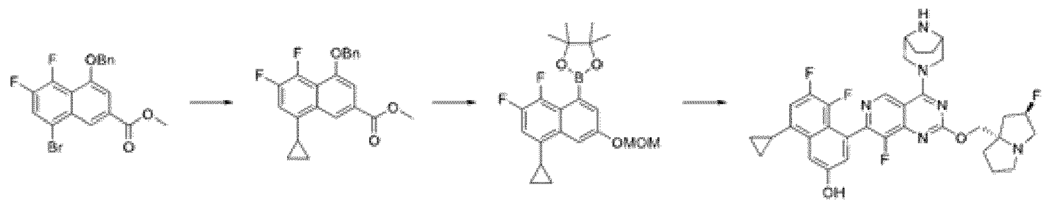

Step 1: Synthesis of methyl 4-(benzyloxy)-8-cyclopropyl-5,6-difluoro-2-naphthoate

Methyl 4-(benzyloxy)-8-bromo-5,6-difluoro-2-naphthoate (407 mg, 1.0 mmol), cyclopropylboronic acid (172 mg, 2.0 mmol), potassium carbonate (414 mg, 3.0 mmol), and dioxane/water (5 mL/0.5 mL) were added to a reaction flask. After replacement of nitrogen, $Pd(dppf)Cl_2$ (37 mg, 0.05 mmol) was added. The reaction mixture was heated to 100°C under nitrogen protection and stirred for 3 h. After cooling to room temperature, the reaction mixture was poured into 30 mL of water and extracted with ethyl acetate. The organic phase was dried, evaporated under reduced pressure, and purified by Prep-TLC (PE/EA=10/1) to obtain 295 mg of off-white solid, with a yield of 80%.

Step 2: Synthesis of 2-(5-cyclopropyl-7,8-trifluoro-3-(methoxymethoxy)naphthalen-1-yl)-4,4,5,5-tetramethyl-1,3,2-di oxaborolane

Using the product obtained in the previous step as raw materials, the target product was synthesized by a method similar to that of intermediate 5.

Step 3: Synthesis of 4-(4-((1R,5S)-3,8-diazabicyclo[3.2.1]octan-3-yl)-8-fluoro-2-(((2R,7aS)-2-fluorotetrahydro-1H-py rrolizin-7a(5H)-yl)methoxy)pyrido[4,3-d]pyrimidin-7-yl)-8-cyclopropyl-5,6-difluoronaphthalen-1-ol

Using the product obtained in the previous step and the corresponding intermediate as raw materials, the target product was synthesized by a method similar to that in Example 6.

LC/MS: m/z=635.3 [M+H]+.

**Example 38**

(1R,5S,Z)-3-(7-(7,8-difluoro-3-hydroxynaphthalen-1-yl)-8-fluoro-2-(((2R,7aS)-2-fluoro tetrahydro-1H-pyrrolidin-7a(5H)-yl)methoxy)pyrido[4,3-d]pyrimidin-4-yl)-3-azabicyclo[3.2.1]oc tan-8-one O-methyl oxime

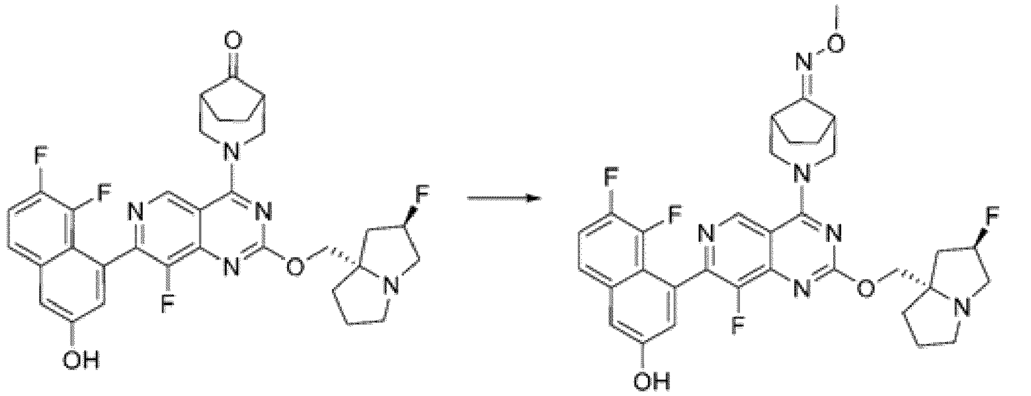

(1R,5S)-3-(8-fluoro-7-(3-(methoxymethoxy)naphthalen-1-yl)-2-((tetrahydro-1H-pyrroli zin-7a(5H)-yl)methyl) pyrido[4,3-d]pyrimidin-4-yl)-3-azabicyclo[3.2.1]octan-8-one (30 mg, 0.05 mmol) was dissolved in 2 mL of ethanol, and 0.1 mL of pyridine and methoxyammonium chloride (9 mg, 0.10 mmol) were added. The reaction mixture was stirred overnight at room temperature. The reaction solution was evaporated under reduced pressure, and then puried by pre-TLC (DCM/MeOH=10/1) to obtain 8 mg of white solid, with a yield of 25%.
LC/MS: m/z=637.3 [M+H]+.

**Example 39**

N-(4-(4-((1R,5S)-3,8-diazabicyclo[3.2.1]octan-3-yl)-8-fluoro-2-((tetrahydro-1H-pyrroli zin-7a(5H)-yl)methoxy)pyrido [4,3-d]pyrimidin-7-yl)naphthalen-2-yl) methanesulfonamide

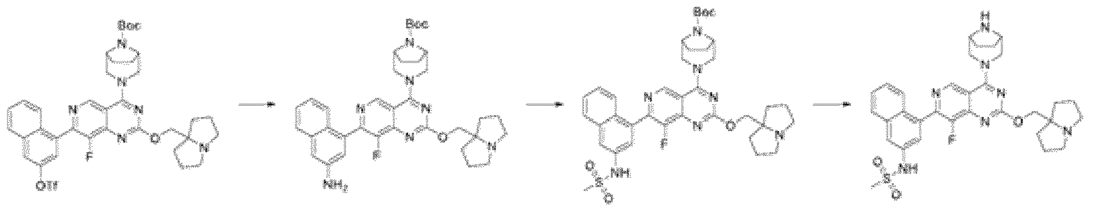

Step 1: Synthesis of tert-butyl (1R,5S)-3-(7-(3-aminonaphthalen-1-yl)8-fluoro-2-((tetrahydro-1H-pyrrolizin-7a(5H)-yl) methoxy )-pyrido[4,3-d]pyrimidin-4-yl)-3,8-diazabicyclo[3.2.1]octan-8-carboxylate

Under the protection of nitrogen, 5 mg of $Pd_2(dba)_3$ was added to a 3 mL dioxane solution of tert-butyl (1R,5S)-3-(8-fluoro-2-((tetrahydro-1H-pyrrolizin-7a(5H)-yl)methoxy)-7-(3-(((trifluoromethyl)sul fonyl)oxy)naphthalen-1-yl)pyrido[4,3-d]pyrimidin-4-yl)-3,8-diazabicyclo[3.2.1]octan-8-carboxyl ate (30 mg, 0.039 mmol), benzophenone imine (18 mg, 0.1 mmol), cesium carbonate (66 mg, 0.2 mmol), and 5 mg of RuPhos. The reaction mixture was stirred under nitrogen protection at 100°C for 3 h. The reaction solution was poured into 10 mL of water and extracted with ethyl acetate. The organic phase was dried, evaporated under reduced pressure, and purified by Prep-TLC (DCM/MeOH=20/1). The obtained product was dissolved in 5 mL of methanol, and 10 mg of Pd/C (10%) was added. The mixture was stirred overnight at room temperature under hydrogen atmosphere. After suction filtration, the filtrate was evaporated under reduced pressure to obtain15 mg of yellow solid, with a yield of 60%.

Step 2: Synthesis of tert-butyl (1R,5S)-3-(8-fluoro-7-(3-(methylsulfonamido)naphthalen-1-yl)2-((tetrahydro-1H-pyrrolizin-7a(5 H)-yl)methoxy)-pyrido[4,3-d]pyrimidin-4-yl)-3,8-diazabicyclo[3.2.1]octan-8-carboxylate

Tert-butyl (1R,5S)-3-(7-(3-aminonaphthalen-1-yl)8-fluoro-2-((tetrahydro-1H-pyrrolizin-7a(5H)-yl)methoxy )-pyrido[4,3-d]pyrimidin-4-yl)-3,8-diazabicyclo[3.2.1]octan-8-carboxylate (15 mg, 0.023 mmol), and triethylamine (10 mg, 0.1 mmol) were dissolved in 2 mL of dichloromethane, and methanesulfonyl chloride (4 mg, 0.03 mmol) was added. The reaction mixture was stirred at room temperature for 3 h. The reaction solution was poured into 10 mL of water and extracted with dichloromethane. The organic phase was dried, evaporated under reduced pressure, and purified by Prep-TLC (DCM/MeOH=20/1) to obtain 10 mg of yellow solid, with a yield of 59%.

Step 3: Synthesis of N-(4-(4-((1R,5S)-3,8-diazabicyclo[3.2.1]octan-3-yl)-8-fluoro-2-((tetrahydro-1H-pyrrolizin-7a(5H )-yl)methoxy)pyrido[4,3-d]pyrimidin-7-yl)naphthalen-2-yl)methanesulfonamide

Tert-butyl(1R,5S)-3-(8-fluoro-7-(3-(methylsulfonamido)naphthalen-1-yl)2-((tetrahydro-1H-pyrrolizin-7a(5H)-yl) methoxy)-pyrido[4,3-d]pyrimidin-4-yl)-3,8-diazabicyclo[3.2.1]octan-8-carboxylate (10 mg, 0.014 mmol) was added to a reaction flask and dissolved in EA (0.5 mL) followed by hydrochloric acid-ethyl acetate solution (1.5 mL). The reaction was allowed to proceed at room temperature for 1 h. Then the reaction system was evaporated under reduced pressure, adjusted to alkaline pH with saturated sodium bicarbonate solution, extracted with ethyl acetate, dried over anhydrous $Na_2SO_4$, filtered, and concentrated under reduced pressure. The resulting crude product was purified on a thin-layer silica gel plate (DCM/MeOH=7/1) to obtain 3 mg of the title compound as white solid, with a yield of 35%.
LC/MS: m/z=618.3 $[M+H]^+$.

**Example 40**

4-(4-((1R,5S)-3,8-diazabicyclo[3.2.1]octan-3-yl)-8-fluoro-2-((tetrahydro-1H-pyrrolizin-7a(5H)-yl)methoxy)pyrido[4,3-d] pyrimidin-7-yl)naphthalen-2-amine

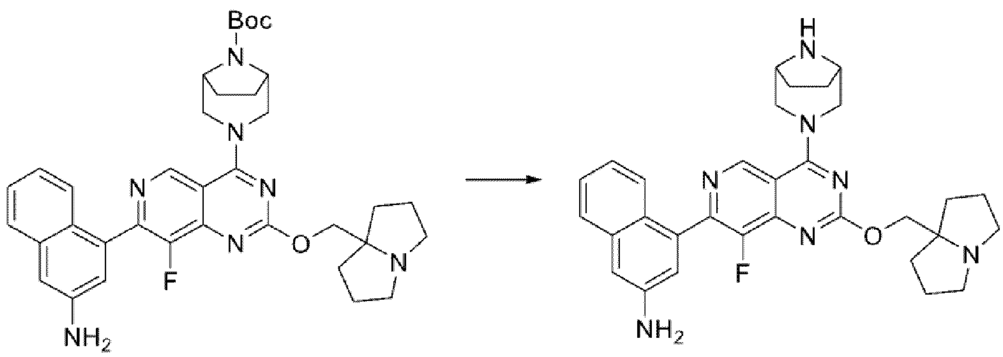

Tert-butyl (1R,5S)-3-(7-(3-aminonaphthalen-1-yl)8-fluoro-2-((tetrahydro-1H-pyrrolizin-7a(5H)-yl)methoxy )-pyrido[4,3-d]pyrimidin-4-yl)-3,8-diazabicyclo[3.2.1]octan-8-carboxylate (10 mg, 0.016 mmol) was added to a reaction flask and dissolved in EA (0.5 mL) followed by hydrochloric acid-ethyl acetate solution (1.5 mL). The reaction was allowed to proceed at room temperature for 1 h. The reaction system was evaporated under reduced pressure, adjusted to alkaline pH with saturated sodium bicarbonate solution, extracted with ethyl acetate, dried over anhydrous $Na_2SO_4$, filtered, and concentrated under reduced pressure. The resulting crude product was purified on a thin-layer silica gel plate (DCM/MeOH=5/1) to obtain 5 mg of the titled compound as grey solid, with a yield of 59%.
LC/MS: m/z=540.3 $[M+H]^+$.

**Example 41**

3-(4-((1R,5S)-3,8-diazabicyclo[3.2.1]octan-3-yl)-7-(7,8-difluoro-3-hydroxynaphthalen-1-yl)-8-fluoro-2-(((2R,7aS)-2-fluorotetrahydro-1H-pyrrolidin-7a(5H)-yl)methoxy)quinazolin-6-yl )propionitrile

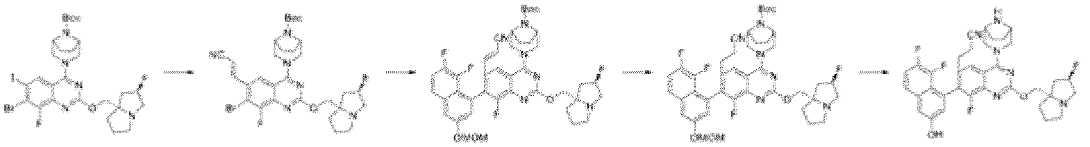

Step 1: Synthesis of tert-butyl (1R,5S)-3-(7-bromo-6-((E)-2-cyanoethenyl)-8-fluoro-2-(((2R,7aS)-2-fluorotetrahydro-1H-pyrroli din-7a(5H)-yl)methoxy)quinazolin-4-yl)-3,8-diazabicyclo[3.2.1]octan-8-carboxylate

Tert-butyl (1R,5S)-3-(7-bromo-8-fluoro-2-((2R,7aS)-2-fluorotetrahydro-1H-pyrrolizin-7a(5H)-ylmethoxy)-6-iodoquinazolin-4-yl)-3,8-diazacyclo[3.2.1]octan-8-carboxylate (166 mg, 0.23 mmol), triethylamine (50 mg, 0.50 mmol), acrylonitrile (2.8 g, 20 mmol), and 5 mL of DMF were added to a reaction flask. After replacement with nitrogen, 5 mg of tri(o-tolyl)phosphine and 5 mg of palladium acetate were added. The reaction system was heated to 80°C under the protection of nitrogen and stirred overnight. The reaction system was cooled to room temperature, and the reaction mixture was poured into water and extracted with ethyl acetate. The organic phase was concentrated under reduced pressure and purified by Prep-TLC (DCM/MeOH=20/1) to obtain 76 mg of the target product, with a yield of 51%.

Step 2: Synthesis of tert-butyl (1R,5S)-3-(6-((E)-2-cyanoethenyl)-7-(7,8-difluoro-3-(methoxymethoxy)naphthalen-1-yl)-8-fluor o-2-(((2R,7aS)-2-fluorotetrahydro-1H-pyrrolidin-7a(5H)-yl)methoxy)quinazolin-4-yl)-3,8-diazab icyclo[3.2.1]octan-8-carboxylate

Tert-butyl (1R,5S)-3-(7-bromo-6-((E)-2-cyanoethenyl)-8-fluoro-2-(((2R,7aS)-2-fluorotetrahydro-1H-pyrroli din-7a(5H)-yl)methoxy)quinazolin-4-yl)-3,8-diazabicyclo[3.2.1]octan-8-carboxylate(76 mg, 0.12 mmol), cesium carbonate (65 mg, 0.2 mmol), 2-(7,8-difluoro-3-(methoxymethoxy)naphthalen-1-yl)-4,4,5,5-tetramethyl-1,3,2-dioxaborolane (53 mg, 0.15 mmol), and 2 mL of dioxane/water (10/1) were added to a reaction flask. After replacement with nitrogen, 5 mg of Pd(dppf)$Cl_2$ was added. The reaction system was heated to 90°C under nitrogen protection and stirred for 2 h. The reaction system was cooled to room temperature, and the reaction mixture was poured into water and extracted with ethyl acetate. The organic phase was concentrated under reduced pressure and purified by Prep-TLC (DCM/MeOH=15/1) to obtain 17 mg of the target product, with a yield of 18%.

Step 3: Synthesis of tert-butyl (1R,5S)-3-(6-(2-cyanoethyl)-7-(7,8-difluoro-3-(methoxymethoxy)naphthalen-1-yl)-8-fluoro-2-((( 2R,7aS)-2-fluorotetrahydro-1H-pyrrolidin-7a(5H)-yl)methoxy)quinazolin-4-yl)-3,8-diazabicyclo[ 3.2.1]octan-8-carboxylate

At 0°C, lithium triethylborohydride (1 M in THF, 0.1 mL) was added to a tetrahydrofuran solution of tert-butyl (1R,5S)-3-(6-((E)-2-cyanoethenyl)-7-(7,8-difluoro-3-(methoxymethoxy)naphthalen-1-yl)-8-fluor o-2-(((2R,7aS)-2-fluorotetrahydro-1H-pyrrolidin-7a(5H)-yl)methoxy)quinazolin-4-yl)-3,8-diazab icyclo[3.2.1]octan-8-carboxylate (17 mg, 0.021 mmol). The mixture was stirred for 10 min at 0°C. The reaction mixture was poured into water and extracted with ethyl acetate. The organic phase was concentrated under reduced pressure to obtain a crude product.

Step 4: Synthesis of 3-(4-((1R,5S)-3,8-diazabicyclo[3.2.1]octan-3-yl)-7-(7,8-difluoro-3-hydroxynaphthalen-1-yl)-8-fl uoro-2-(((2R,7aS)-2-fluorotetrahydro-1H-pyrrolidin-7a(5H)-yl)methoxy)quinazolin-6-yl)propioni trile

The crude product obtained in the previous step was dissolved in 2 mL of ethyl acetate, and HCl/EA (4 M, 0.5 mL) was added and stirred for 10 min. The reaction system was poured into 10 mL of water, adjusted to weakly alkaline with sodium carbonate, and extracted with ethyl acetate. The organic phase was dried, evaporated under reduced pressure, and purified by Prep-TLC to obtain 3 mg of grey solid with a two-step yield of 21%.

LC/MS: m/z=647.3 [M+H]$^+$.

## Example 42

4-(4-((1R,5S)-3,8-diazabicyclo[3.2.1]octan-3-yl)-8-fluoro-2-(((2R,7aS)-2-fluorotetrahy dro-1H-pyrrolidin-7a(5H)-yl) methoxy)-1,6-naphthyridin-7-yl)-5,6-difluoronaphthalen-2-ol

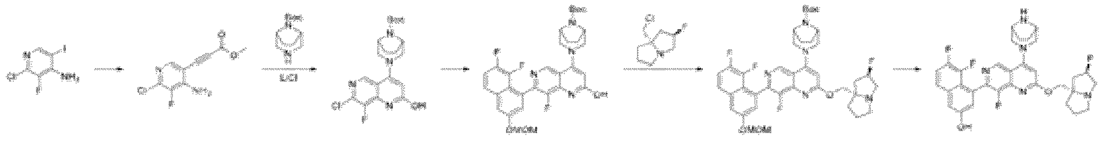

Step 1: Synthesis of methyl 3-(4-amino-6-chloro-5-fluoropyridine-3-yl)propiolate

2-Chloro-3-fluoro-5-iodopyridin-4-amine (272 mg, 1.0 mmol), potassium carbonate (276 mg, 2.0 mmol), methyl propiolate (168 mg, 0.2 mmol), cuprous iodide (19 mg, 0.1 mmol), and 2 mL of tetrahydrofuran were added to a reaction flask. After replacement with nitrogen, $Pd(PPh_3)Cl_2$ (35 mg, 0.05 mmol) was added. The reaction system was heated to 70°C under nitrogen protection and stirred for 4 h. The reaction system was cooled to room temperature, and the reaction solution was poured into water and extracted with ethyl acetate. The organic phase was concentrated under reduced pressure and purified by Prep-TLC (DCM/MeOH=20/1) to obtain 40 mg of the target product, with a yield of 18%.

Step 2: Synthesis of tert-butyl (1R,5S)-3-(7-chloro-8-fluoro-2-hydroxy-1,6-naphthyridin-4-yl)-3,8-diazabicyclo[3.2.1]octan-8-c arboxylate

Methyl 3-(4-amino-6-chloro-5-fluoropyridine-3-yl) propiolate (40 mg, 0.18 mmol), 100 mg of lithium chloride, tert-butyl (1R,5S)-3,8-diazabicyclo[3.2.1]octan-8-carboxylate (42 mg, 0.20 mmol), and 2 mL of tert-butanol were added to a reaction flask. The reaction mixture was heated to 120°C and stirred for 2 days. After cooling the reaction system to room temperature, the reaction solution was poured into water and extracted with ethyl acetate. The organic phase was concentrated under reduced pressure and purified by Prep-TLC (DCM/MeOH=20/1) to obtain 20 mg of the target product, with a yield of 28%.

Step 3: Synthesis of tert-butyl (1R,5S)-3-(7-(7,8-difluoro-3-(methoxymethoxy)naphthalen-1-yl)-8-fluoro-2-hydroxy-1,6-naphth yridin-4-yl)-3,8-diazabicyclo[3.2.1]octan-8-carboxylate

Tert-butyl(1R,5S)-3-(7-chloro-8-fluoro-2-hydroxy-1,6-naphthyridin-4-yl)-3,8-diazabicy clo[3.2.1]octan-8-carboxylate (20 mg, 0.049 mmol), cesium carbonate (33 mg, 0.2 mmol), 2-(7,8-difluoro-3-(methoxymethoxy)naphthalen-1-yl)-4,4,5,5-tetramethyl-1,3,2-dioxaborolane (18 mg, 0.05 mmol), and 2 mL of dioxane/water (10/1) were added to a reaction flask. After replacement with nitrogen, 3 mg of $Pd(dppf)Cl_2$ was added. The reaction system was heated to 90°C under nitrogen protection and stirred for 2 h. The reaction system was cooled to room temperature, and the reaction solution was poured into water and extracted with ethyl acetate. The organic phase was concentrated under reduced pressure and purified by Prep-TLC (DCM/MeOH=15/1) to obtain 10 mg of the target product, with a yield of 34%.

Step 4: Synthesis of tert-butyl (1R,5S)-3-(7-(7,8-difluoro-3-(methoxymethoxy)naphthalen-1-yl)-8-fluoro-2-(((2R,7aS)-2-fluorot etrahydro-1H-pyrrolidin-7a(5H)-yl)methoxy)-1,6-naphthyridin-4-yl)-3,8-diazabicyclo[3.2.1]octa n-8-carboxylate

Tert-butyl (1R,5S)-3-(7-(7,8-difluoro-3-(methoxymethoxy)naphthalen-1-yl)-8-fluoro-2-hydroxy-1,6-naphth yridin-4-yl)-3,8-diazabicyclo[3.2.1]octan-8-carboxylate (10 mg, 0.017 mmol), silver carbonate (28 mg, 0.1 mmol), (2R,7aS)-7a-(chloromethyl)-2-fluorohexahydro-1H-pyrrolizine (4 mg, 0.02 mmol), and 2 mL of DMF were added to a reaction flask. The reaction system was heated to 80°C under nitrogen protection and stirred for 4 h. The reaction system was cooled to room temperature, and the reaction solution was poured into water and extracted with ethyl acetate. The organic phase was concentrated under reduced pressure to obtain a crude product.

Step 5: Synthesis of 4-(4-((1R,5S)-3,8-diazabicyclo[3.2.1]octan-3-yl)-8-fluoro-2-(((2R,7aS)-2-fluorotetrahydro-1H-py rrolidin-7a(5H)-yl)methoxy)-1,6-naphthyridin-7-yl)-5,6-difluoronaphthalen-2-ol

The crude product obtained from the previous step was dissolved in 2 mL of ethyl acetate, and HCl/EA (4 M, 0.5 mL) was added and stirred for 10 min. The reaction system was poured into 10 mL of water, adjusted to weakly alkaline with sodium carbonate, and extracted with ethyl acetate. The organic phase was dried, evaporated under reduced pressure and purified by Prep-TLC to obtain 0.5 mg of grey solid, with a two-step yield of 5%.

LC/MS: m/z=594.2 $[M+H]^+$.

**Example 43**

4-(4-((1R,5S)-3,8-diazabicyclo[3.2.1]octan-3-yl)-2-((3-(1,3-dihydroisobenzofuran-5-yl) tetrahydro-1H-pyrrolidin-7a(5H)-yl)methoxy)-8-fluoropyrido[4,3-d]pyrimidin-7-yl)naphthalen-2-ol

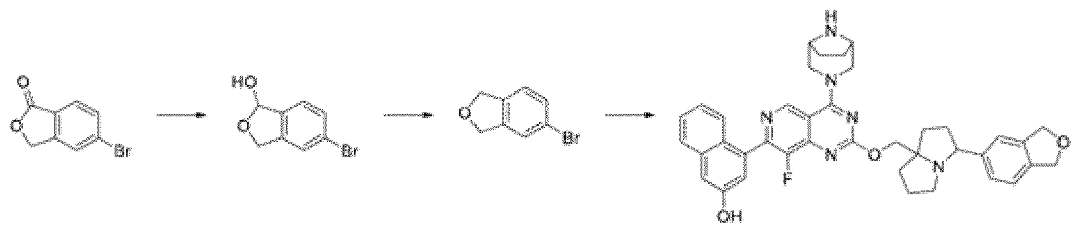

Step 1: Synthesis of 5-bromo-1,3-dihydroisobenzofuran-1-ol

5-Bromoisobenzofuran-1(3H)-one (213 mg, 1.0 mmol) was dissolved in 5 mL of tetrahydrofuran, and DIBALH (1 M, 1 mL, 1 mmol) was added dropwise at -70°C. Afterwards, the mixture was stirred for 2 h at -70°C. The reaction mixture was poured into 10 mL of water and extracted with dichloromethane. The organic phase was dried and evaporated under reduced pressure. The crude product was directly used for the next step of reaction.

Step 2: Synthesis of 5-bromo-1,3-dihydroisobenzofuran-1-ol

The crude product obtained in the previous step was dissolved in 5 mL of dichloromethane, and 0.2 mL of trifluoroacetic acid and 0.2 mL of triethylsilane were added. The reaction mixture was stirred at room temperature overnight. After the reaction system was cooled to room temperature, it was poured into 20 mL of water and extracted with ethyl acetate. The organic phase was dried, evaporated under reduced pressure and purified by Prep-TLC (PE/EA=2/1) to obtain 110 mg of the target product, with a two-step yield of 55%.

Step 3: Synthesis of 4-(4-((1R,5S)-3,8-diazabicyclo[3.2.1]octan-3-yl)-2-((3-(1,3-dihydroisobenzofuran-5-yl)tetrahydro-1H-pyrrolidin-7a(5H)-yl)methoxy)-8-fluoropyrido[4,3-d]pyrimidin-7-yl)naphthalen-2-ol

Using the product obtained in the previous step and the corresponding intermediate as raw materials, the target product was synthesized by the same method as in Example 34.

LC/MS: m/z=659.3 $[M+H]^+$.

**Example 44**

4-(7a-(((4-((1R,5S)-3,8-diazabicyclo[3.2.1]octan-3-yl)-8-fluoro-7-(3-hydroxynaphthale n-1-yl)pyrido [4,3-d]pyrimidin-2-yl)oxy)methyl)hexahydro-1H-pyrrolizin-3-yl)benzonitrile

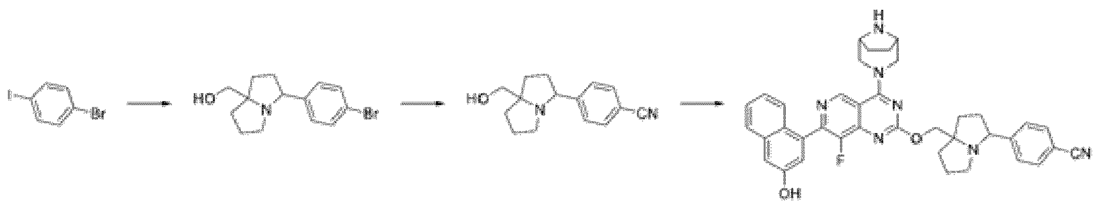

Step 1: Synthesis of (3-(4-bromophenyl)tetrahydro-1H-pyrrolizin-7a(5H)-yl)methanol

Using p-bromoiodobenzene and the corresponding reagent as raw materials, the product was synthesized by the same method as in Example 34.

Step 2: Synthesis of 4-(7a-(hydroxymethyl)hexahydro-1H-pyrrolizin-3-yl)-benzonitrile

(3-(4-Bromophenyl)tetrahydro-1H-pyrrolizin-7a(5H)-yl)methanol (295 mg, 1.0 mmol), cuprous cyanide (445 mg, 5.0 mmol), 0.5 mL of pyridine, and 5 mL of DMSO were added to a reaction flask. The reaction mixture was heated to 130°C under nitrogen protection and stirred for 1 h. After the reaction system cooled to room temperature, it was poured into 20 mL of water and extracted with ethyl acetate. The organic phase was dried, evaporated under reduced pressure and purified by Prep-TLC (PE/EA=2/1) to obtain 160 mg of white solid with a yield of 66%.

Step 3: Synthesis of 4-(7a-(((4-((1R,5S)-3,8-diazabicyclo[3.2.1]octan-3-yl)-8-fluoro-7-(3-hydroxynaphthalen-1-yl)pyr ido[4,3-d]pyrimidin-2-yl)oxy)methyl)hexahydro-1H-pyrrolizin-3-yl)benzonitrile

Using the product obtained in the previous step and the corresponding intermediate as raw materials, the target product was synthesized by the same method as in Example 34.

LC/MS: m/z=642.3 $[M+H]^+$.

**Example 45**

4-(4-((1R,5S)-3,8-diazabicyclo[3.2.1]octan-3-yl)-2-((3-(4-(3,6-dihydro-2H-pyran-4-yl)p henyl)tetrahydro-1H-pyrrolidin-7a(5H)-yl)methoxy)-8-fluoropyrido[4,3-d]pyrimidin-7-yl)naphth alen-2-ol

**Example 46**

4-(4-((1R,5S)-3,8-diazabicyclo[3.2.1]octan-3-yl)-8-fluoro-2-((3-(4-(tetrahydro-2H-pyra n-4-yl)phenyl)tetrahydro-1H-pyrrolidin-7a(5H)-yl)methoxy)pyrido[4,3-d]pyrimidin-7-yl)naphtha len-2-ol

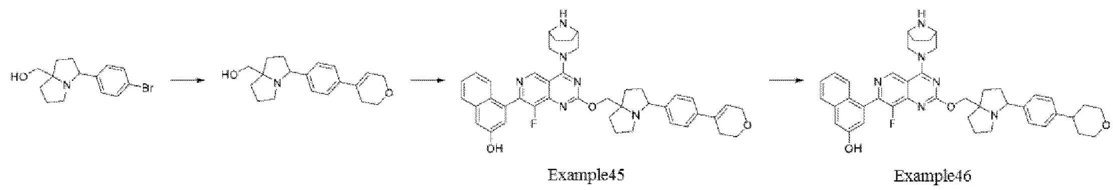

Step 1: Synthesis of (3-(4-(3,6-dihydro-2H-pyran-4-yl)-phenyl)tetrahydro-1H-pyrrolizin-7a(5H)-yl)methanol

(3-(4-Bromophenyl)tetrahydro-1H-pyrrolizin-7a(5H)-yl)methanol (296 mg, 1.0 mmol), 2-(3,6-dihydro-2H-pyran-4-yl)-4,4,5,5-tetramethyl-1,3,2-dioxaborolane (210 mg, 1.0 mmol), potassium carbonate (276 mg, 2.0 mmol), and dioxane/water (5 mL/0.5 mL) were added to a reaction flask. After replacement with nitrogen, $Pd(dppf)Cl_2$ (36 mg, 0.05 mmol) was added. The reaction mixture was heated to 100°C under nitrogen protection and stirred for 6 h. After the reaction system cooled to room temperature, it was poured into 20 mL of water and extracted with ethyl acetate. The organic phase was dried, evaporated under reduced pressure and purified by Prep-TLC (DCM/MeOH=20/1) to obtain 230 mg of yellow solid, with a yield of 77%.

Step 2: Synthesis of 4-(4-((1R,5S)-3,8-diazabicyclo[3.2.1]octan-3-yl)-2-((3-(4-(3,6-dihydro-2H-pyran-4-yl)phenyl)tet rahydro-1H-pyrrolidin-7a(5H)-yl)methoxy)-8-fluoropyrido[4,3-d]pyrimidin-7-yl)naphthalen-2-ol

Using the product obtained in the previous step and the corresponding intermediate as raw materials, the target product was synthesized by the same method as in Example 34.

LC/MS: m/z=699.3 $[M+H]^+$.

$^1H$ NMR (400 MHz, $CDCl_3$) δ 8.92 (s, 1H), 7.64-7.58 (m, 1H), 7.48-7.42 (m, 1H), 7.25-7.04 (m, 8H), 5.95 (s, 1H), 4.55-4.42 (m, 2H), 4.30-4.12 (m, 4H), 3.81-3.56 (m, 5H), 3.22 (s, 2H), 2.86-2.76 (m, 1H), 2.65-2.54 (m, 1H), 2.37 (s, 2H), 2.28-2.21 (m, 1H), 2.21-2.04 (m, 1H), 1.96-1.54 (m, 10H).

Step 3: Synthesis of 4-(4-((1R,5S)-3,8-diazabicyclo[3.2.1]octan-3-yl)-8-fluoro-2-((3-(4-(tetrahydro-2H-pyran-4-yl)ph enyl)tetrahydro-1H-pyrrolidin-7a(5H)-yl)methoxy)pyrido[4,3-d]pyrimidin-7-yl)naphthalen-2-ol

4-(4-((1R,5S)-3,8-diazabicyclo[3.2.1]octan-3-yl)-2-((3-(4-(3,6-dihydro-2H-pyran-4-yl)p henyl)tetrahydro-1H-pyrrolidin-7a(5H)-yl)methoxy)-8-fluoropyrido[4,3-d]pyrimidin-7-yl)naphth alen-2-ol (10 mg, 0.014 mmol) was dissolved in 3 mL of methanol, and 5 mg of Pd/C (10%) was added. The reaction mixture was stirred under hydrogen atmosphere at room temperature for 6 h. After suction filtration, the filtrate was evaporated under reduced pressure to obtain 8 mg of grey solid, with a yield of 80%.

LC/MS: m/z=701.3 $[M+H]^+$.

**Example 47**

4-(4-((1R,5S)-3,8-diazabicyclo[3.2.1]octan-3-yl)-8-fluoro-2-((3-(4-methoxyphenyl)tetra hydro-1H-pyrrolidin-7a(5H)-yl) methoxy-d2)pyrido[4,3-d]pyrimidin-7-yl)naphthalen-2-ol

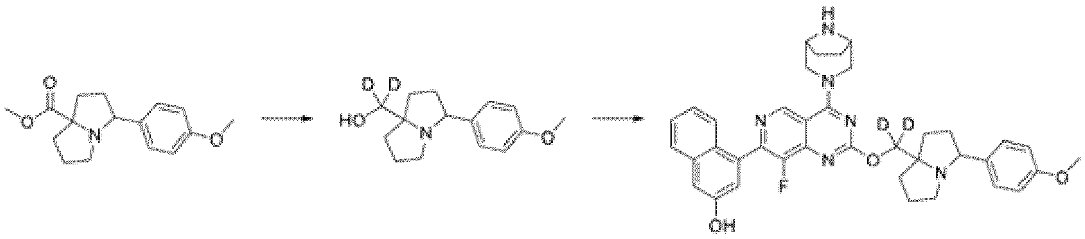

Step 1: Synthesis of (3-(4-methoxyphenyl)tetrahydro-1H-pyrrolizin-7a(5H)-yl)methan-$d_2$-ol

Methyl methyl 3-(4-methoxyphenyl)tetrahydro-1H-pyrrolizin-7a(5H)-carboxylate (138 mg, 0.5 mmol) was dissolved in 2 mL of tetrahydrofuran, and lithium aluminum deuteride (42 mg, 1.0 mmol) was added at 0°C and stirred for 1 h. 10 mL of water was added to the reaction system, and the reaction system was extracted 3 times with ethyl acetate. After the organic phase was dried and evaporated under reduced pressure, it was purified by column chromatography (PE/EA = 5/1) to obtain 75 mg of the target product, with a yield of 60%.

Step 2: Synthesis of 4-(4-((1R,5S)-3,8-diazabicyclo[3.2.1]octan-3-yl)-8-fluoro-2-(((2R)-2-fluoro-5-(4-methoxyphenyl ) tetrahydro-1H-pyrrolidin-7a(5H)-yl)methoxy)pyrido[4,3-d]pyrimidin-7-yl)naphthalen-2-ol

Using the product obtained in the previous step and the corresponding intermediate as raw materials, the target product was synthesized by a method similar to that in Example 34.

LC/MS: m/z=649.3 $[M+H]^+$.

$^1H$ NMR (400 MHz, $CDCl_3$) δ 8.96 (s, 1H), 7.64 (d, J = 8.2 Hz, 1H), 7.49 (d, J = 8.0 Hz, 1H), 7.34-7.30 (m, 1H), 7.21-7.12 (m, 5H), 6.72 (d, J = 8.4 Hz, 2H), 4.54 (d, J = 12.0 Hz, 2H), 3.67-3.55 (m, 5H), 3.26 (s, 3H), 2.95-2.85 (m, 1H), 2.77-2.67 (m, 1H), 2.35-2.25 (m, 1H), 2.13-2.05 (m, 1H), 1.99-1.70 (m, 10H).

**Example 48**

4-(4-((1R,5S)-3,8-diazabicyclo[3.2.1]octan-3-yl)-8-fluoro-2-(((2R)-2-fluoro-5-(4-metho xyphenyl)tetrahydro-1H-pyrrolidin-7a(5H)-yl)methoxy)pyrido[4,3-d]pyrimidin-7-yl)naphthalen-2-ol

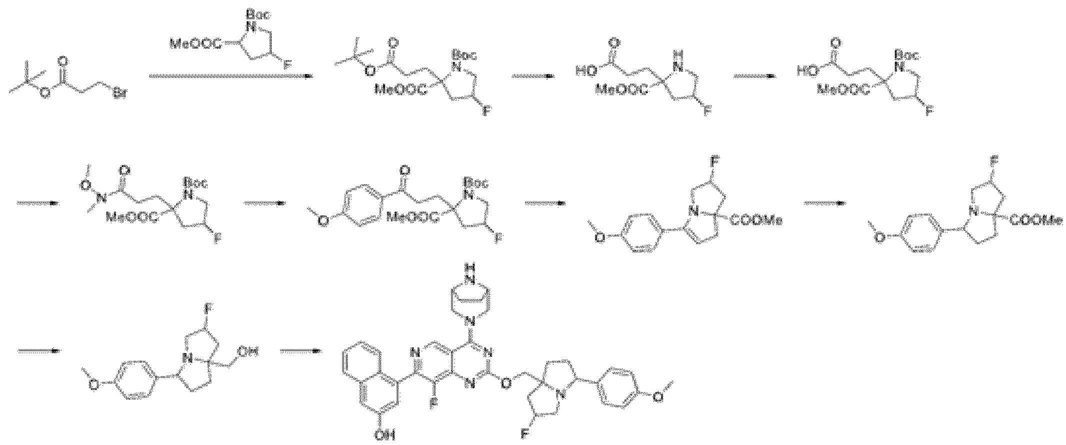

Step 1: Synthesis of 1-(tert-butyl)2-methyl(4R)-2-(3-(tert-butoxy)-3-oxopropyl)-4-fluoropyrrolidin-1,2-dicarboxylate

1-(Tert-butyl)2-methyl(4R)-4-fluoropyrrolidin-1,2-dicarboxylate (3 g, 12.15 mmol), HMPA (2.8 g, 15.80 mmol), and 30 mL of tetrahydrofuran were added to a reaction flask. The reaction mixture was cooled to -70°C under a dry ice-ethanol bath and nitrogen protection. Then, LiHMDS (1 M, 15.8 mL) was added and reacted at this temperature for 30 min. Afterwards, a tetrahydrofuran solution (3 mL) of tert-butyl 3-bromopropionate (2.5 g, 12.15 mmol) was added, and stirred at -70°C for 4 h. A saturated ammonium chloride solution (20 mL) was added, and then the mixture was extracted 3 times with ethyl acetate. The organic phase was dried, evaporated under reduced pressure, and purified by column chromatography (PE/EA=5/1) to obtain 3.29 g of the target product with a yield of 72%.

Step 2: Synthesis of 3-((4R)-4-fluoro-2-(methoxycarbonyl)pyrrolidin-2-yl)propionic acid

1-(Tert-butyl)2-methyl(4R)-2-(3-(tert-butoxy)-3-oxopropyl)-4-fluoropyrrolidin-1,2-dica rboxylate (3.29 g, 8.75 mmol) and 25 mL of dichloromethane were added to a reaction flask. Trifluoroacetic acid (25 mL) was then added, and the mixture was stirred at room temperature overnight. The system was directly evaporated under reduced pressure to obtain a crude product of trifluoroacetate of 3-((4R)-4-fluoro-2-(methoxycarbonyl)pyrrolidin-2-yl)propionic acid (4.05 g), which was not purified.

Step 3: Synthesis of 3-((4R)-1-(tert-butoxycarbonyl)-4-fluoro-2-(methoxycarbonyl)pyrrolidin-2-yl)propionic acid

The crude product of trifluoroacetate of 3-((4R)-4-fluoro-2-(methoxycarbonyl)pyrrolidin-2-yl)propionic acid (4.05 g, 12.15 mmol), triethylamine (6.14 g, 60.75 mmol), and 40 mL of methanol were added to a reaction flask. When the pH value tested with a pH paper was greater than 7, $(Boc)_2O$ (3.45 g, 15.80 mmol) was added, and the mixture was stirred at room temperature for 1 h. The system was directly evaporated under reduced pressure to obtain a crude product (3.88 g), which was not purified.

Step 4: Synthesis of 1-(tert-butyl)2-methyl(4R)-4-fluoro-2-(3-(methoxy(methyl)amino)-3-oxopropyl)pyrrolidin-1,2-di carboxylate

The crude product of 3-((4R)-1-(tert-butoxycarbonyl)-4-fluoro-2-(methoxycarbonyl)pyrrolidin-2-yl)propionic acid (3.88 g, 12.15 mmol), N,O-dimethylhydroxyamine hydrochloride, HATU (6 g, 15.80 mmol), triethylamine (6.14 g, 60.75 mmol), and 40 mL of N,N-dimethylformamide were added to a reaction flask and stirred at room temperature overnight. The system was directly poured into 400 mL of water and extracted 3 times with ethyl acetate. The organic phase was dried, evaporated under reduced pressure, and purified by column chromatography (PE/EA = 5/1) to obtain 291 mg of the target product, with a total yield of 6.6% for four steps.

Step 5: Synthesis of 1-(tert-butyl)2-methyl(4R)-4-fluoro-2-(3-(4-methoxyphenyl)-3-oxopropyl)pyrrolidin-1,2-dicarbo xylate

1-(Tert-butyl)2-methyl(4R)-4-fluoro-2-(3-(methoxy(methyl)amino)-3-oxopropyl)pyrroli din-1,2-dicarboxylate (291 mg, 0.8 mmol) and 4 mL of tetrahydrofuran were added to a reaction flask. The reaction mixture was cooled to -70°C under a dry ice-ethanol bath and nitrogen protection. Then, (4-methoxyphenyl)magnesium bromide (1 M, 1.6 mL)

was added and the reaction was allowed to proceed at this temperature for 3 h. A saturated ammonium chloride solution (4 mL) was added, and the mixture was extracted 3 times with ethyl acetate. The organic phase was dried, evaporated under reduced pressure, and purified by Prep-TLC (PE/EA = 1/1) to obtain 235 mg of the target product with a yield of 72%.

Step 6: Synthesis of methyl (6R)-6-fluoro-3-(4-methoxyphenyl)-6,7-dihydro-1H-pyrrolizin-7a(5H)-carboxylate

1-(Tert-butyl)2-methyl(4R)-4-fluoro-2-(3-(4-methoxyphenyl)-3-oxopropyl)pyrrolidin-1, 2-dicarboxylate (235 mg, 0.57 mmol) was added to a reaction flask. HCl/MeOH (4 M, 20 mL) was then added at room temperature, and the mixture was stirred overnight. The reaction system was directly evaporated under reduced pressure to obtain a crude product (167 mg), which was not purified.

Step 7: Synthesis of methyl (2R)-2-fluoro-5-(4-methoxyphenyl)tetrahydro-1H-pyrrolizin-7a(5H)-carboxylate

The crude product of methyl (6R)-6-fluoro-3-(4-methoxyphenyl)-6,7-dihydro-1H-pyrrolizin-7a(5H)-carboxylate (167 mg, 0.57 mmol) was dissolved in 5 mL of methanol, and sodium borohydride (32 mg, 0.86 mmol) was added at 0°C and stirred for 1 h. The reaction system was evaporated under reduced pressure, and 5 mL of cold water was added. The mixture was extracted with ethyl acetate. The organic phase was dried, evaporated under reduced pressure, and purified by Prep-TLC (PE/EA = 1/1) to obtain 120 mg of a white liquid with a yield of 72%.

Step 8: Synthesis of ((2R)-2-fluoro-5-(4-methoxyphenyl)tetrahydro-1H-pyrrolizin-7a(5H)-yl)methanol

Methyl (2R)-2-fluoro-5-(4-methoxyphenyl)tetrahydro-1H-pyrrolizine-7a(5H)-carboxylate (120 mg, 0.41 mmol) was dissolved in 1 mL of tetrahydrofuran. Lithium aluminum hydride (24 mg, 0.62 mmol) was added at 0°C and stirred for 1 h. To the reaction system, 24 mg of water, 24 mg of 15% sodium hydroxide solution, and 72 mg of water were added. A small amount of anhydrous sodium sulfate was then added for drying, and the mixture was filtered and evaporated under reduced pressure to obtain approximately 110 mg of a crude target product, which was not purified.

Step 9: Synthesis of 4-(4-((1R,5S)-3,8-diazabicyclo[3.2.1]octan-3-yl)-8-fluoro-2-(((2R)-2-fluoro--5-(4-methoxypheny 1) tetrahydro-1H-pyrrolidin-7a(5H)-yl)methoxy)pyrido[4,3-d]pyrimidin-7-yl)naphthalen-2-ol

Using the product obtained in the previous step and the corresponding intermediate as raw materials, the target product was synthesized by a method similar to that in Example 34.

LC/MS: m/z=665.3 $[M+H]^+$.

$^1H$ NMR (400 MHz, $CDCl_3$) δ 9.07 (s, 1H), 7.72-7.59 (m, 2H), 7.42-7.28 (m, 6H), 6.82 (d, J = 8.2 Hz, 2H), 5.41 (d, J = 53.6 Hz, 1H), 4.66-4.52 (m, 2H), 4.45-4.33 (m, 2H), 3.77 (s, 3H), 3.74-3.58 (m, 4H), 3.39-3.31 (m, 1H), 2.94-2.80 (m, 1H), 2.70-2.61 (m, 1H), 2.45-2.39 (m, 1H), 2.26-2.20 (m, 1H), 2.04-1.51 (m, 8H).

**Example 49**

8-(4-((1R,5S)-3,8-diazacyclo[3.2.1]octyl-3-yl)-6,8-difluoro-2-((2R,7aS)-2-fluorotetrahy dro-1H-pyrrolidin-7a(5H)-ylmethoxy)quinazolin-7-yl)quinolin-6-ol

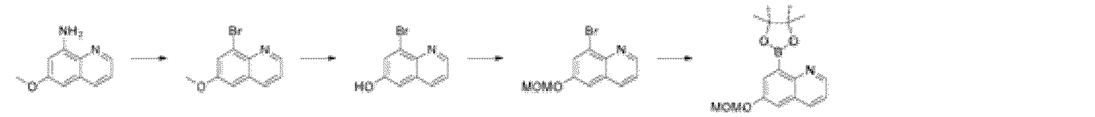

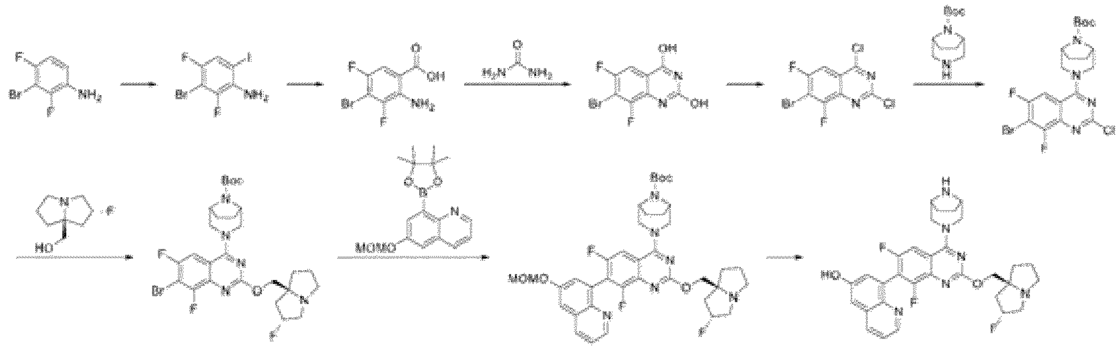

Step 1: Synthesis of 8-bromo-6-methoxyquinoline

Compound 6-methoxyquinolin-8-amine (5.0 g, 28.70 mmol) was added to a reaction flask and dissolved in 40% HBr (50 mL). The reaction system was cooled to°C. An aqueous solution (10 mL) of sodium nitrite (2.57 g, 37.31 mmol) was prepared and then added dropwise to the reaction system. The reaction was allowed to proceed for 30 min at 0°C. In another reaction flask, cuprous bromide (4.94 g, 34.44 mmol) and 40% HBr (50 mL) were added. The above reaction system solution was added dropwise to this reaction flask, and the reaction was allowed to proceed for 1 h at room temperature. The reaction system was quenched by adding a saturated aqueous solution of sodium bicarbonate, and then extracted with ethyl acetate. The organic phase was washed with saturated brine, dried over anhydrous $Na_2SO_4$, filtered, and concentrated under reduced pressure. The crude product was separated and purified by Prep-TLC (PE/EA=7/1) to obtain 3.56 g of the title compound as white solid, with a yield of 52%.

Step 2: Synthesis of 8-bromoquinolin-6-ol

Compound 8-bromo-6-methoxyquinoline (3.0 g, 12.60 mmol) was added to a three-necked flask. Under nitrogen protection, the reaction system was then cooled to -78°C, and 2.0 M boron tribromide (25.2 mL, 50.4 mmol) was added dropwise. The reaction was allowed to proceed at -78°C for 1 h, and then proceed at room temperature for 2 h. The reaction system was quenched by adding a saturated aqueous solution of sodium bicarbonate, and then extracted with ethyl acetate. The organic phase was washed with saturated brine, dried over anhydrous $Na_2SO_4$, filtered, and concentrated under reduced pressure. The crude product was separated and purified by Prep-TLC (PE/EA=3/1) to obtain 2.40 g of the title compound as white solid, with a yield of 85%.

Step 3: Synthesis of 8-bromo-6-(methoxymethoxy)quinolone

8-Bromoquinolin-6-ol (2.25 g, 10.04 mmol) was added to a three-necked flask. Under nitrogen protection, dry tetrahydrofuran (30 mL) was then added. The reaction system was cooled in an ice-water bath, and sodium hydride (803 mg, 20.08 mmol) was added in batches, allowing the reaction to proceed for 30 min. Then, chloromethyl methyl ether (970 mg, 12.05 mmol) was added dropwise, and the reaction was allowed to proceed for 1 h at room temperature. The reaction system was quenched by adding a saturated aqueous solution of ammonium chloride, and then extracted with ethyl acetate. The organic phase was washed with saturated brine, dried over anhydrous $Na_2SO_4$, filtered, and concentrated under reduced pressure. The crude product was separated and purified by Prep-TLC (PE/EA=2/1) to obtain 2.15 g of the title compound as white solid, with a yield of 80%.

Step 4: Synthesis of 6-(methoxymethoxy)-8-(4,4,5,5-tetramethyl-1,3,2-dioxaborolan-2-yl)quinolone

8-Bromo-6-(methoxymethoxy)quinoline (300 mg, 1.12 mmol), bis(pinacolato)diboron (569 mg, 2.24 mmol), potassium acetate (220 mg, 2.24 mmol), and Pd(dppf)$Cl_2$ (81 mg, 0.11 mmol) were added to a three-necked flask. Under nitrogen protection, 1,4-dioxane (3 mL) and water (0.3 mL) were then added. After replacement with nitrogen again, the mixture was stirred at 80°C for 4 h. The reaction system was quenched by adding water and extracted with ethyl acetate. The organic phase was washed with saturated brine, dried over anhydrous $Na_2SO_4$, filtered, and concentrated under

reduced pressure. The crude product was separated and purified by Prep-TLC (PE/EA=1/1) to obtain 170 mg of the title compound as white solid, with a yield of 48%.

Step 5: Synthesis of 3-bromo-2,4-difluoro-6-iodoaniline

3-Bromo-2,4-difluoroaniline (3.3 g, 15.87 mmol), $Ag_2SO_4$ (4.9 g, 15.87 mmol), and 30 mL of anhydrous ethanol were added to a reaction flask. An ethanol solution of iodine (4.03 g, 15.87 mmol) was slowly added dropwise, and the reaction mixture was stirred at room temperature for 4 h. After filtration, the filtrate was concentrated, and extracted with water and ethyl acetate. The organic phase was dried, evaporated under reduced pressure, and then purified by column chromatography (PE/EA = 50/1) to obtain 2.7 g of the target product, with a yield of 52%.

Step 6: Synthesis of 2-amino-4-bromo-3,5-difluorobenzoic acid

3-Bromo-2,4-difluoro-6-iodoaniline (2.7 g, 8.08 mmol), $K_2CO_3$ (2.8 g, 20 mmol), DMF (30 mL), and 30 mL of water were added to a reaction flask, and Pd(dppf)$Cl_2$ (293 mg, 0.4 mmol) was added. Under carbon monoxide protection, the reaction system was heated to 90°C and stirred overnight. After the reaction system was cooled to room temperature, the reaction mixture was poured into water and extracted with ethyl acetate. The aqueous phase was collected, adjusted to weakly acidic pH with dilute hydrochloric acid, and then extracted with ethyl acetate. The organic phase was collected, washed with saturated brine, dried over anhydrous sodium sulfate, and filtered. The filtrate was concentrated under reduced pressure and then purified by column chromatography (DCM/MeOH = 20/1) to obtain 1.0 g of the target product, with a yield of 49%.

Step 7: Synthesis of 7-bromo-6,8-difluoroquinazolin-2,4-diol

2-Amino-4-bromo-3,5-difluorobenzoic acid (1.0 g, 3.98 mmol) and urea (2.4 g, 39.84 mmol) were added to a reaction flask, and the reaction system was heated to 170°C and stirred for 4 h. The reaction system was cooled to room temperature and extracted 3 times with water and ethyl acetate. The organic phases was combined, then washed with saturated brine, dried over anhydrous sodium sulfate, and filtered. The filtrate was concentrated under reduced pressure and then purified by column chromatography (PE/EA = 5/1) to obtain 650 mg of the target product, with a yield of 59%.

Step 8: Synthesis of 7-bromo-2,4-dichloro-6,8-difluoroquinazoline

7-Bromo-6,8-difluoroquinazolin-2,4-diol (300 mg, 1.09 mmol), phosphorus oxychloride (1.67 g, 10.86 mmol), and DIEA (630 mg, 4.91 mmol) were added to a reaction flask. The reaction system was heated to 90°C and stirred for 3 h. The reaction system was cooled to room temperature and concentrated under reduced pressure to obtain a crude product, which was directly used for the next step.

Step 9: Synthesis of tert-butyl (1R,5S)-3-(7-bromo-2-chloro-6,8-difluoroquinazolin-4-yl)-3,8-diazacyclo[3.2.1]octan-8-carboxyl ate

7-Bromo-2,4-dichloro-6,8-difluoroquinazoline (300 mg, 0.95 mmol), DIEA (630 mg, 4.91 mmol), tert-butyl (1R,5S)-3,8-diazacyclo[3.2.1]octan-8-carboxylate (201 mg, 0.95 mmol), and 30 mL of anhydrous ethanol were added to a reaction flask. The reaction system was heated to 80°C and stirred for 4 h. The reaction system was cooled to room temperature and extracted 3 times with water and ethyl acetate. The organic phases were combined, washed with saturated brine, dried over anhydrous sodium sulfate, and filtered. The filtrate was concentrated under reduced pressure and then purified by Prep-TLC (PE/EA = 8/1) to obtain 35 mg of the target product, with a two-step yield of 6%.

Step 10: Synthesis of tert-butyl (1R,5 S)-3-(7-bromo-6,8-difluoro-2-((2R,7aS)-2-fluorotetrahydro-1H-pyrrolizin-7a(5H)-ylmethox y)quinazolin-4-yl)-3,8-diazacyclo[3.2.1]octan-8-carboxylate

Tert-butyl (1R,5S)-3-(7-bromo-2-chloro-6,8-difluoroquinazolin-4-yl)-3,8-diazacyclo[3.2.1]octan-8-carboxyl ate (50 mg, 0.10 mmol), (2R,7aS)-2-fluorotetrahydro-1H-pyrrolizin-7a(5H)-yl) methanol (24 mg, 0.15 mmol), cesium carbonate (65 mg, 0.20 mmol), and 1,4-diazabicyclo[2.2.2]octane (11 mg, 0.10 mmol) were added to a reaction flask. Under nitrogen protection, anhydrous THF (1 mL) and DMF (1 mL) were added. After replacement with nitrogen, the mixture was stirred at 60°C for 4 h. The reaction system was quenched by adding water and then extracted with ethyl acetate. The organic phase was washed with saturated brine, dried over anhydrous $Na_2SO_4$, filtered, and concentrated under reduced pressure. The crude product was separated and purified by Prep-TLC (PE/EA = 2/1) to obtain 22 mg of the title compound as yellow solid, with a yield of 36%.

Step 11: Synthesis of tert-butyl (1R,5S)-3-(6,8-difluoro-2-(((2R,7aS)-2-fluorotetrahydro-1H-pyrrolizin-7a(5H)-yl)methoxy)-7-(6-(methoxymethoxy)quinolin-8-yl)quinazolin-4-yl)-3,8-diazabicyclo[3.2.1]octan-8-carboxylate

Tert-butyl (1R,5S)-3-(7-bromo-6,8-difluoro-2-((2R,7aS)-2-fluorotetrahydro-1H-pyrrolizin-7a(5H)-ylmethox y)quinazolin-4-yl)-3,8-diazacyclo[3.2.1]octan-8-carboxylate (20 mg, 0.033 mmol), 6-(methoxymethoxy)-8-(4,4,5,5-tetramethyl-1,3,2-dioxaborolan-2-yl)quinoline (16 mg, 0.050 mmol), cesium carbonate (22 mg, 0.066 mmol), and Pd(dppf)$Cl_2$ (3 mg, 0.0033 mmol) were added to a reaction flask. Under nitrogen protection, 1,4-dioxane (1 mL) and water (0.1 mL) were added. After replacement with nitrogen, the mixture was stirred at 100°C for 2 h. The reaction system was quenched by adding water and extracted with ethyl acetate. The organic phase was washed with saturated brine, dried over anhydrous $Na_2SO_4$, filtered, and concentrated under reduced pressure. The crude product was separated and purified by Prep-TLC (PE/EA = 1/1) to obtain 10 mg of the title compound as yellow solid, with a yield of 43%.

Step 12: Synthesis of 8-(4-((1R,5S)-3,8-diazacyclo[3.2.1]octanyl-3-yl)-6,8-difluoro-2-((2R,7aS)-2-fluorotetrahydro-1H -pyrrolidin-7a(5H)-ylmethoxy)quinazolin-7-yl)quinolin-6-ol

Tert-butyl (1R,5S)-3-(6,8-difluoro-2-(((2R,7aS)-2-fluorotetrahydro-1H-pyrrolizin-7a(5H)-yl)methoxy)-7-(6-(methoxymethoxy)quinolin-8-yl)quinazolin-4-yl)-3,8-diazabicyclo[3.2.1]octan-8-carboxylate (10 mg, 0.014 mmol) was added to a reaction flask and dissolved in EA (0.5 mL) followed by hydrochloric acid-ethyl acetate solution (1.5 mL). The reaction was allowed to proceed at room temperature for 1 h. The reaction system was evaporated under reduced pressure, adjusted to alkaline pH with saturated sodium bicarbonate solution, then extracted with ethyl acetate, dried over anhydrous $Na_2SO_4$, filtered, and concentrated under reduced pressure. The obtained crude product was purified on a thin-layer silica gel plate (DCM/MeOH = 10/1) to obtain 5 mg of the title compound as white solid, with a yield of 62%.

LC/MS: m/z=577.3 $[M+H]^+$.

$^1H$ NMR (400 MHz, $CDCl_3$) δ 8.62-8.59 (m, 1H), 7.99-7.95 (m, 1H), 7.55 (dd, J = 2.1, 43.2 Hz, 1H), 7.31-7.29 (m, 2H), 7.17-7.15 (m, 1H), 5.22 (dd, J = 20.4, 54.4 Hz, 1H), 4.50-4.41 (m, 1H), 4.24-4.15 (m, 3H), 3.59-3.53 (m, 3H), 3.41-2.93 (m, 5H), 2.44-1.63 (m, 10H).

**Example 50**

4-(4-((1R,5S)-3,8-diazabicyclo[3.2.1]octan-3-yl)-2-(((2R,7aS)-2-fluorotetrahydro-1H-p yrrolizin-7a(5H))-yl)methoxy) pyrido[2,3-d]pyrimidin-7-yl)naphthalen-2-ol

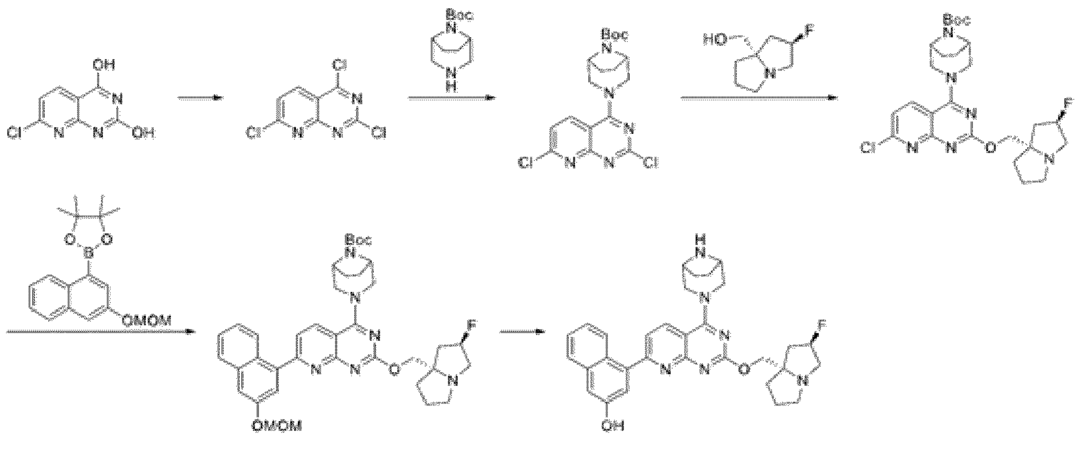

Step 1: Synthesis of 2,4,7-trichloropyrido[2,3-d]pyrimidine

7-Chloropyrido[2,3-d]pyrimidin-2,4-diol (200 mg, 1.01 mmol), DIPEA (774 mg, 6.00 mmol), and 3 mL of toluene were added to a reaction flask, and phosphorus oxychloride (459 mg, 3.00 mmol) was added. After stirring at 110°C for 4 h, the mixture was evaporated under reduced pressure, and the crude product was directly used for the next step.

Step 2: Synthesis of tert-butyl (1R,5S)-3-(2,7-dichloropyrido[2,3-d]pyrimidin-4-yl)-3,8-diazabicyclo[3.2.1]octan-8-carboxylate

Tert-butyl (1R,5S)-3,8-diazabicyclo[3.2.1]octan-8-carboxylate (106 mg, 0.50 mmol), DIPEA (774 mg, 6.00 mmol), and 5 mL of DCM were added to a reaction flask. The crude product from the previous step was added at 0°C. After stirring for 1 h, the mixture was poured into 20 mL of water and extracted with ethyl acetate. The organic phase was dried, evaporated under reduced pressure, and then purified by Prep-TLC (EA/PE = 2/1) to obtain 205 mg of white solid, with a two-step yield of 49%.

Step 3: Synthesis of tert-butyl (1R,5S)-3-(7-chloro-2-(((2R,7aS)-2-fluorotetrahydro-1H-pyrrolidin-7a(5H)-yl)methoxy) pyrido[2, 3-d]pyrimidin-4-yl)-3,8-diazabicyclo[3.2.1]octan-8-carboxylate

((2R,7aS)-2-fluorotetrahydro-1H-pyrrolizin-7a(5H)-yl)methanol (80 mg, 0.50 mmol) and 2 mL of tetrahydrofuran were added to a reaction flask. NaH (60%, 20 mg, 0.50 mmol) was added at 0°C, and the mixture was stirred for 1 h at room temperature. Tert-butyl (1R,5S)-3-(2,7-dichloropyrido[2,3-d]pyrimidin-4-yl)-3,8-diazabicyclo[3.2.1]octan-8-carboxylate (200 mg, 0.49 mmol) was added, and then the reaction solution was stirred at room temperature for 2 h. The reaction solution was poured into 10 mL of water and extracted with ethyl acetate. The organic phase was dried, evaporated under reduced pressure, and then purified by Prep-TLC (DCM/MeOH = 20/1) to obtain 120 mg of white solid, with a yield of 46%.

Step 4: Synthesis of tert-butyl (1R,5S)-3-(2-(((2R,7aS)-2-fluorotetrahydro-1H-pyrrolizin-7a(5H)-yl)methoxy)-7-(3-(methoxyme thoxy)naphthalen-1-yl)pyrido[2,3-d]pyrimidin-4-yl)-3,8-diazabicyclo[3.2.1]octan-8-carboxylate

Tert-butyl (1R,5S)-3-(7-chloro-2-(((2R,7aS)-2-fluorotetrahydro-1H-pyrrolidin-7a(5H)-yl)methoxy)pyrido[2, 3-d] pyrimidin-4-yl)-3,8-diazabicyclo[3.2.1]octan-8-carboxylate (53 mg, 0.10 mmol), 2-(3-(methoxymethoxy)naphthalen-1-yl)-4,4,5,5-tetramethyl-1,3,2-dioxaborolane (31 mg, 0.10 mmol), potassium carbonate (28 mg, 0.20 mmol), and dioxane/-water (2 mL/0.2 mL) were added to a reaction flask. After replacement with nitrogent, Pd(dppf)$Cl_2$ (7 mg, 0.01 mmol) was added. The reaction mixture was heated to 100°C under nitrogen protection and stirred for 6 h. The reaction system was cooled to room temperature, poured into 10 mL of water and extracted with ethyl acetate. The organic phase was dried, evaporated under reduced pressure, and then purified by Prep-TLC (DCM/MeOH = 20/1) to obtain 23 mg of yellow solid, with a yield of 34%.

Step 5: Synthesis of 4-(4-((1R,5S)-3,8-diazabicyclo[3.2.1]octan-3-yl)-2-(((2R,7aS)-2-fluorotetrahydro-1H-pyrrolizin-7a(5H))-yl)methoxy)pyrido[2,3-d]pyrimidin-7-yl)naphthalen-2-ol

Tert-butyl (1R,5S)-3-(2-(((2R,7aS)-2-fluorotetrahydro-1H-pyrrolizin-7a(5H)-yl)methoxy)-7-(3-(methoxyme thoxy)naphthalen-1-yl)pyrido[2,3-d]pyrimidin-4-yl)-3,8-diazabicyclo[3.2.1]octan-8-carboxylate (23 mg, 0.034 mmol) was dissolved in 2 mL of ethyl acetate. HCl/EA (4 M, 0.5 mL) was added, and the mixture was stirred for 10 min. The reaction system was poured into 10 mL of water, adjusted to weakly alkaline with sodium carbonate, and extracted with ethyl acetate. The organic phase was dried, evaporated under reduced pressure, and then purified by Prep-TLC (DCM/MeOH=5/1) to obtain 13 mg of light yellow solid, with a two-step yield of 72%.

LC/MS: m/z=541.3 $[M+H]^+$.

$^1H$ NMR (400 MHz, $CDCl_3$) $\delta$ 8.30 (d, J = 8.5 Hz, 1H), 8.01 (d, J = 9.0 Hz, 1H), 7.59 (d, J = 8.2 Hz, 1H), 7.54 (s, 1H), 7.33-7.29 (m, 1H), 7.17-7.14 (m, 2H), 6.73 (d, J = 8.8 Hz, 1H), 5.16 (d, J = 53.9 Hz, 1H), 4.29-4.18 (m, 4H), 3.52-3.43 (m, 4H), 3.23-2.88 (m, 4H), 2.10-1.66 (m, 10H).

**Example 51**

1-((1R,5S)-3,8-diazabicyclo[3.2.1]octan-3-yl)-3-(((2R,7aS)-2-fluorotetrahydro-1H-pyrr olidin-7a(5H)-yl)methoxy)-6-(3-hydroxynaphthalen-1-yl)isoquinolin-4-carbonitrile

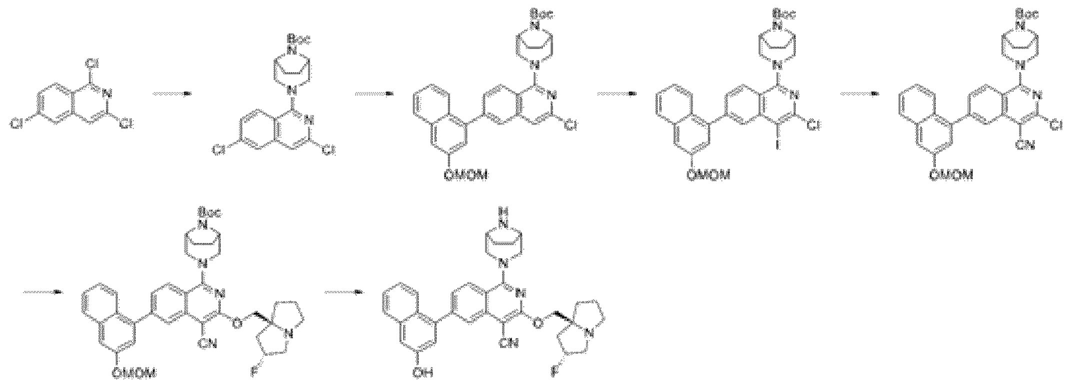

Step 1: Synthesis of tert-butyl (1R,5S)-3-(3,6-dichloroisoquinolin-1-yl)-3,8-diazabicyclo[3.2.1]octan-8-carboxylate

1,3,6-Trichloroisoquinoline (100 mg, 0.43 mmol), DIPEA (129 mg, 1.00 mmol), tert-butyl (1R,5S)-3,8-diazabicyclo[3.2.1]octan-8-carboxylate (106 mg, 0.50 mmol), and 3 mL of NMP were added to a reaction flask. After stirring at 100°C for 4 h, the mixture was poured into 20 mL of water, resulting in the precipitation of solids. The mixture was subjected to suction filtration and the filter cake was evaporated under reduced pressure to obtain 150 mg of white solid with a yield of 85%.

Step 2: Synthesis of tert-butyl (1R,5S)-3-(3-chloro-6-(3-(methoxymethoxy)naphthalen-1-yl)isoquinolin-1-yl)-3,8-diazabicyclo[ 3.2.1]octan-8 -carboxylate

Tert-butyl (1R,5S)-3-(3,6-dichloroisoquinolin-1-yl)-3,8-diazabicyclo[3.2.1]octan-8-carboxylate (150 mg, 0.37 mmol), 2-(3-(methoxymethoxy)naphthalen-1-yl)-4,4,5,5-tetramethyl-1,3,2-dioxaborolane (126 mg, 0.40 mmol), potassium carbonate (138 mg, 1.0 mmol), and dioxane/water (4 mL/0.4 mL) were added to a reaction flask. After replacement with nitrogen, Pd(dppf)$Cl_2$ (15 mg, 0.02 mmol) was added. The reaction mixture was heated to 100°C under nitrogen protection and stirred for 6 h. The reaction system was cooled to room temperature, poured into 10 mL of water and extracted with ethyl acetate. The organic phase was dried, evaporated under reduced pressure, and then purified by Prep-TLC (DCM/MeOH = 20/1) to obtain 135 mg of white solid, with a yield of 66%.

Step 3: Synthesis of tert-butyl (1R,5S)-3-(3-chloro-4-iodo-6-(3-(methoxymethoxy)naphthalen-1-yl)isoquinolin-1-yl)-3,8-diazab icyclo[3.2.1]octan-8-carboxylate

Tert-butyl (1R,5S)-3-(3-chloro-6-(3-(methoxymethoxy)naphthalen-1-yl)isoquinolin-1-yl)-3,8-diazabicyclo [ 3.2.1]octan-8 -carboxylate (112 mg, 0.2 mmol) was dissolved in 3 mL of acetonitrile, and NIS (54 mg, 0.24 mmol) was added at 0°C. The mixture was stirred at room temperature for 2 h. The reaction solution was evaporated under reduced pressure and purified by Prep-TLC (PE/EA = 2/1) to obtain 130 mg of white solid, with a yield of 95%.

Step 4: Synthesis of tert-butyl (1R,5S)-3-(3-chloro-4-cyano-6-(3-(methoxymethoxy)naphthalen-1-yl)isoquinolin-1-yl)-3,8-diaza bicyclo[3.2.1]octan-8-carboxylate

Tert-butyl (1R,5S)-3-(3-chloro-4-iodo-6-(3-(methoxymethoxy)naphthalen-1-yl)isoquinolin-1-yl)-3,8-diazab icyclo[3.2.1]octan-8-carboxylate (130 mg, 0.19 mmol), cuprous cyanide (43 mg, 0.48 mmol), 0.1 mL of pyridine, and 1 mL of DMSO were added to a reaction flask. The reaction mixture was heated to 130°C under nitrogen protection and stirred for 1 h. The reaction system was cooled to room temperature, poured into 10 mL of water and extracted with ethyl acetate. The organic phase was dried, evaporated under reduced pressure, and then purified by Prep-TLC (PE/EA = 2/1) to obtain 77 mg of white solid, with a yield of 69%.

Step 5: Synthesis of tert-butyl (1R,5S)-3-(4-cyano-3-(((2R,7aS)-2-fluorotetrahydro-1H-pyrrolidin-7a(5H)-yl)methoxy)-6-(3-(m ethoxymethoxy)naphthalen-1-yl)isoquinolin-1-yl)-3,8-diazabicyclo[3.2.1]octan-8-carboxylate

((2R,7aS)-2-fluorotetrahydro-1H-pyrrolizin-7a(5H)-yl)methanol (13 mg, 0.082 mmol) and 2 mL of tetrahydrofuran were added to a reaction flask. At 0°C, NaH (60%, 4 mg, 0.10 mmol) was added, and the mixture was stirred at room temperature for 1 h. Tert-butyl (1R,5S)-3-(3-chloro-4-cyano-6-(3-(methoxymethoxy)naphthalen-1-yl)isoquinolin-1-yl)-3,8-diaza bicyclo[3.2.1]octan-8-carboxylate (40 mg, 0.068 mmol) was added, and the reaction solution was stirred

at room temperature for 2 h. The reaction solution was poured into 10 mL of water and extracted with ethyl acetate. The organic phase was dried, evaporated under reduced pressure, and then purified by Prep-TLC (DCM/MeOH = 20/1) to obtain 18 mg of white solid with a yield of 37%.

Step 6: Synthesis of 1-((1R,5S)-3,8-diazabicyclo[3.2.1]octan-3-yl)-3-(((2R,7aS)-2-fluorotetrahydro-1H-pyrrolidin-7a( 5H)-yl)methoxy)-6-(3-hydroxynaphthalen-1-yl)isoquinolin-4-carbonitrile

Tert-butyl (1R,5S)-3-(4-cyano-3-(((2R,7aS)-2-fluorotetrahydro-1H-pyrrolidin-7a(5H)-yl)methoxy)-6-(3-(methoxymethoxy)naphthalen-1-yl)isoquinolin-1-yl)-3,8-diazabicyclo[3.2.1]octan-8-carboxylate (18 mg, 0.025 mmol) was dissolved in 2 mL of ethyl acetate, and HCl/EA (4 M, 0.5 mL) was added. The mixture was stirred for 10 min. The reaction system was poured into 10 mL of water, adjusted to weakly alkaline with sodium carbonate, and extracted with ethyl acetate. The organic phase was dried, evaporated under reduced pressure, and then purified by Prep-TLC (DCM/MeOH = 5/1) to obtain 12 mg of grey solid, with a yield of 84%.

LC/MS: m/z=564.3 $[M+H]^+$.

$^1H$ NMR (400 MHz, $CDCl_3$) δ 7.87-7.83 (m, 2H), 7.61 (d, J = 7.8 Hz, 1H), 7.55 (d, J = 8.2 Hz, 1H), 7.39 (d, J = 7.7 Hz, 1H), 7.31-7.27 (m, 1H), 7.14-7.07 (m, 2H), 7.00 (s, 1H), 5.36 (d, J = 51.8 Hz, 1H), 4.44-4.31 (m, 2H), 4.22-4.15 (m, 2H), 3.78-3.31 (m, 6H), 3.22-3.05 (m, 2H), 2.53-1.92 (m, 10H).

**Example 52**

4-(4-((1R,5S)-3,8-diazabicyclo[3.2.1]octan-3-yl)-8-fluoro-2-((3-(4-methoxyphenyl)tetra hydro-1H-pyrrolidin-7a(5H)-yl) methoxy)pyrido[4,3-d]pyrimidin-7-yl)-5-ethynyl-6-fluoronaphtha len-2-ol

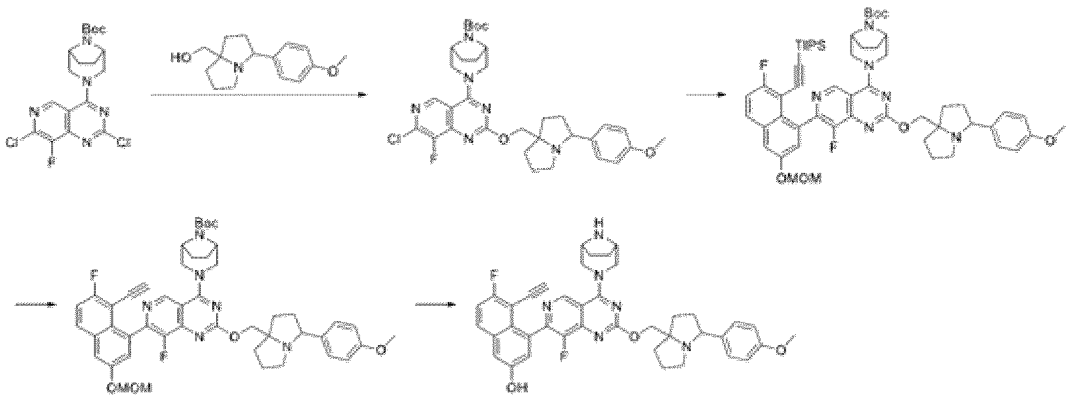

Step 1: Synthesis of tert-butyl (1R,5S)-3-(7-chloro-8-fluoro-2-((3-(4-methoxyphenyl)tetrahydro)-1H-pyrrolizin-7a(5H)-yl)meth oxy)pyrido[4,3-d]pyrimidin-4-yl)-3,8-diazabicyclo[3.2.1]octan-8-carboxylate

(3-(4-Methoxyphenyl)tetrahydro-1H-pyrrolidin-7a(5H)-yl)methanol (130 mg, 0.53 mmol) and 5 mL of tetrahydrofuran were added to a reaction flask, and NaH (60%, 64 mg, 1.6 mmol) was added at 0°C. The mixture was stirred at room temperature for 1 h. Tert-butyl (1R,5S)-3-(2,7-dichloro-8-fluoropyrido[4,3-d]pyrimidin-4-yl)-3,8-diazabicyclo[3.2.1]octan-8-car boxylate (173 mg, 0.40 mmol) was added, and the reaction solution was stirred at room temperature for 2 h. The reaction solution was poured into 20 mL of water and extracted with ethyl acetate. The organic phase was dried, evaporated under reduced pressure, and then purified by Prep-TLC (DCM/MeOH=15/1) to obtain 75 mg of yellow solid, with a yield of 29%.

Step 2: Synthesis of tert-butyl (1R,5S)-3-(8-fluoro-7-(7-fluoro-3-(methoxymethoxy)-8-((triisopropylsilyl)ethynyl) naphthalen-1-yl)-2-((3-(4-methoxyphenyl)tetrahydro-1H-pyrrolidin-7a(5H)-yl)methoxy)pyrido[4,3-d]pyrimidi n-4-yl)-3,8-diazabicyclo[3.2.1]octan-8-carboxylate

Tert-butyl (1R,5S)-3-(7-chloro-8-fluoro-2-((3-(4-methoxyphenyl)tetrahydro)-1H-pyrrolizin-7a(5H)-yl)meth oxy) pyrido[4,3-d]pyrimidin-4-yl)-3,8-diazabicyclo[3.2.1]octan-8-carboxylate (50 mg, 0.078 mmol), ((2-fluoro-6-(methoxymethoxy)-(4,4,5,5-tetramethyl-1,3,2-dioxaborolan-2-yl)naphthalen-1-yl)eth ynyl)triisopropylsilane (40 mg, 0.078 mmol), potassium carbonate (21 mg, 0.15 mmol), and dioxane/water (2 mL/0.2 mL) were added to a reaction flask. After

replacement with nitrogen, 5 mg of $Pd(dppf)Cl_2$ was added. The reaction mixture was heated to 100°C under nitrogen protection and stirred for 6 h. The reaction system was cooled to room temperature, poured into 10 mL of water and extracted with ethyl acetate. The organic phase was dried, evaporated under reduced pressure, and then purified by Prep-TLC (DCM/MeOH=15/1) to obtain 31 mg of grey solid, with a yield of 40%.

Step 3: Synthesis of tert-butyl (1R,5S)-3-(7-(8-ethynyl-7-fluoro-3-(methoxymethoxy)naphthalen-1-yl)-8-fluoro-2-((3-(4-methox yphenyl))tetrahydro-1H-pyrrolidin-7a(5H)-yl)methoxy)pyrido[4,3-d]pyrimidin-4-yl)-3,8-diazabic yclo[3.2.1]octan-8-carboxylate

Tert-butyl (1R,5S)-3-(8-fluoro-7-(7-fluoro-3-(methoxymethoxy)-8-((triisopropylsilyl)ethynyl)naphthalen-1-yl)-2-((3-(4-methoxyphenyl)tetrahydro-1H-pyrrolidin-7a(5H)-yl)methoxy)pyrido[4,3-d]pyrimidi n-4-yl)-3,8-diazabicyclo [3.2.1]octan-8-carboxylate (30 mg, 0.030 mmol) was dissolved in 1 mL of DMF, and CsF (10 mg) was added at room temperature and stirred overnight. The reaction system was poured into 10 mL of water and extracted with ethyl acetate. The organic phase was dried and evaporated under reduced pressure. The crude product was directly used for the next step of reaction.

Step 4: Synthesis of 4-(4-((1R,5S)-3,8-diazabicyclo[3.2.1]octan-3-yl)-8-fluoro-2-((3-(4-methoxyphenyl)tetrahydro-1 H-pyrrolidin-7a(5H)-yl)methoxy)pyrido[4,3-d]pyrimidin-7-yl)-5-ethynyl-6-fluoronaphthalen-2-ol

The crude product obtained from the previous step was dissolved in 2 mL of ethyl acetate, and HCl/EA (4 M, 0.5 mL) was added and stirred for 10 min. The reaction system was poured into 10 mL of water, adjusted to weakly alkaline pH with sodium carbonate, and extracted with ethyl acetate. The organic phase was dried, evaporated under reduced pressure and purified by Prep-TLC to obtain 12 mg of grey solid, with a two-step yield of 38%.

LC/MS: m/z=689.3 $[M+H]^+$.

$^1H$ NMR (400 MHz, $CDCl_3$) δ 8.85 (s, 1H), 7.71-7.62 (m, 1H), 7.29-7.10 (m, 4H), 6.78-6.69 (m, 2H), 4.59-4.43 (m, 2H), 4.31-4.20 (m, 2H), 3.56-3.35 (m, 5H), 3.25 (s, 3H), 3.23-3.19 (m, 1H), 2.95-2.84 (m, 1H), 2.79-2.71 (m, 1H), 2.35-2.24 (m, 1H), 2.11-2.02 (m, 1H), 1.99-1.64 (m, 10H).

The following compounds of Examples 53 to 140 were prepared by using commercially available corresponding reagents as raw materials according to the operations similar to those described in the above examples.

Table 1

| Exam ple | Structure | MS (ES1) m/z | $^1H$ NMR | Examp le of re ference for syn thesis |
|---|---|---|---|---|
| 53 | 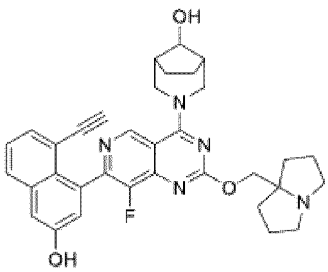 | 580.3 | | 2 |
| 54 | 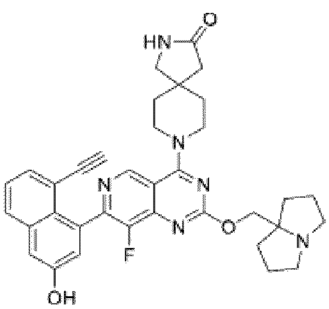 | 607.3 | | 2 |

(continued)

| Example | Structure | MS (ES1) m/z | 1H NMR | Example of reference for synthesis |
|---|---|---|---|---|
| 55 | 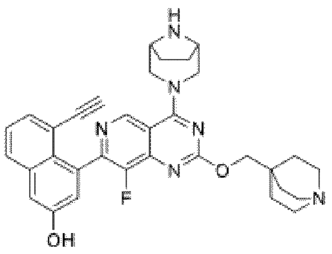 | 565.3 | | 2 |
| 56 | 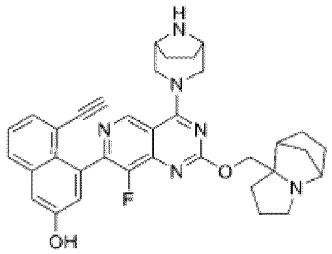 | 591.3 | | 2, 6 |
| 57 | 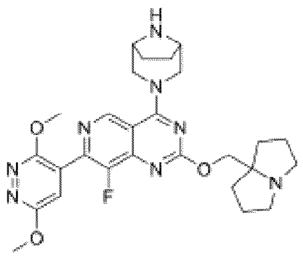 | 537.3 | | 1 |
| 58 | 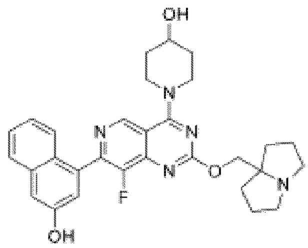 | 530.2 | | 9 |
| 59 | 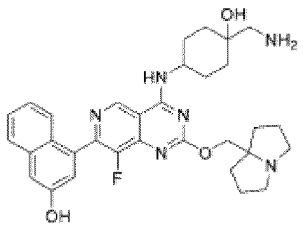 | 573.3 | $^1$H NMR (400 MHz, (d6-DMSO) δ 9.46 (1H, s), 8.68-8.70 (1H, m), 7.79 (1H, d, J = 8.4 Hz), 7.41-7.47 (2H, m), 7.28 (1H, s), 7.21-7.24 (1H, m), 7.18-7.19 (1H, m), 4.18-4.22 (1H, m), 4.07 (211, s), 3.58-3.60 (1H, m),3.49-3.51 (1H, m), 2.90-3.01 (2H, m), 2.50-2.52 (2H, m), 1.75-1.90 (8H, m), 1.52-1.70 (4H, m), 1.25-1.50 (4H, m). | 10, 11 |
| 60 | 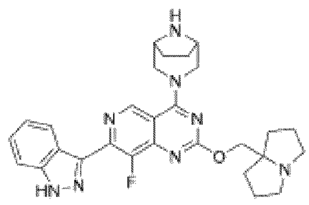 | 515.3 | | I |

(continued)

| Exam ple | Structure | MS (ES1) m/z | 1H NMR | Examp le of re ference for syn thesis |
|---|---|---|---|---|
| 61 | 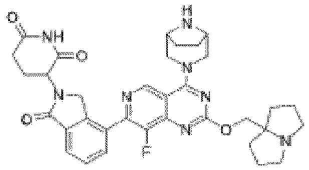 | 641.3 | | 1 |
| 62 | 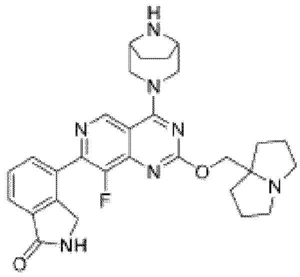 | 530.3 | 1H NMR (400 MHz, d6-DMSO) δ 10.71 (1H, brs), 9.93-9.95 (1H, m), 9.63-9.65 (1H, m), 9.22 (1H, s), 8.74 (1M, s), 8.03 (1H, d. J = 7.2 Hz), 7.84 (IH., d, J = 7.2 Hz), 7.68-7.72 (1H, m), 4.61-4.67 (4H, m), 4.17 (2H, s), 3.97-4.00 (211, m), 3.49)-3.53 (2H, m), 3.19-3.22 (2H, m), 1.87-2,22 (12H, m). | 1 |
| 63 | 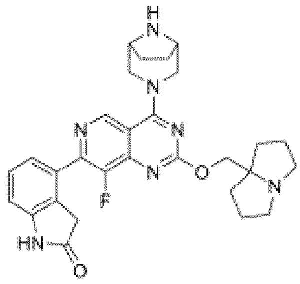 | 530.3 | 1H NMR (400 MHz, d6-DMSO) δ 10.79 (1H, s). 10.61 (1H, s), 10.00-10.02 (1H, m), 9.70-9.75 (1H, m), 9.20 (1H, s), 7.26-7.39 (2H, m), 6.98 (1H, d, J = 7.6 Hz), 4.61-4.66 (4H, m), 4,16 (2H, s), 3.97-4.07 (2H, m), 3.49-3.53 (2H, m), 3.19-3.27 (2H, m),1.87-2.32 (12H, m). | I |
| 64 | 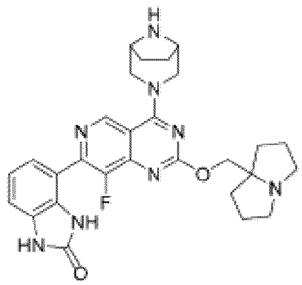 | 531.3 | 1H NMR (400 MHz, d6-DMSO) δ 10.91 (IH, s), 10.63-10.69) (2H, m), 9.92-9.98 (1H, m), 9.60-9.65 (1H, m), 9.23 (1H, s), 7.50 (1H, d, J = 7.6 Hz), 7.08-7.14 (2H, m), 4.61-4.65 (4H, m),4.19 (2H, s), 3.97-4.07 (2H, m), 3.48-3.54 (2H, m), 3.19-3.25 (2H, m), 1.88-2,22 (12H, m). | 1 |
| 65 | 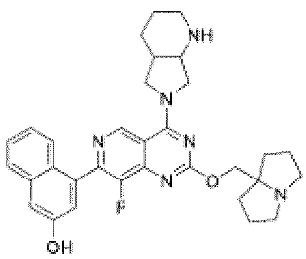 | 555.3 | | 20 |

(continued)

| Example | Structure | MS (ES1) m/z | 1H NMR | Example of reference for synthesis |
|---|---|---|---|---|
| 66 | 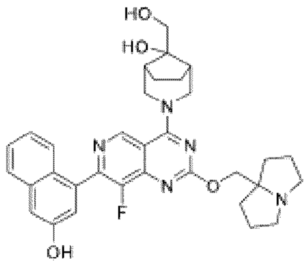 | 586.3 | | 10 |
| 67 | 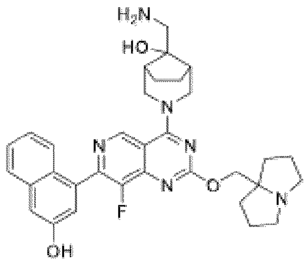 | 585.3 | | 10, 11 |
| 68 | 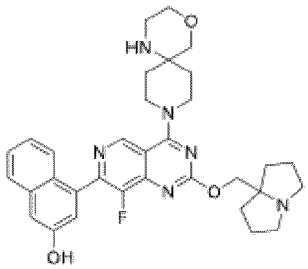 | 585.3 | | 20 |
| 69 | 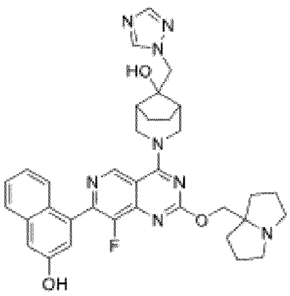 | 637.3 | | 11 |
| 70 | 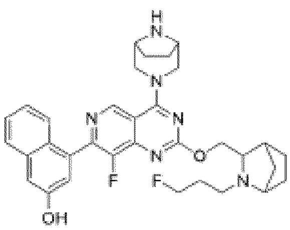 | 587.3 | | 12 |

(continued)

| Exam ple | Structure | MS (ES1) m/z | 1H NMR | Examp le of re ference for syn thesis |
|---|---|---|---|---|
| 71 | 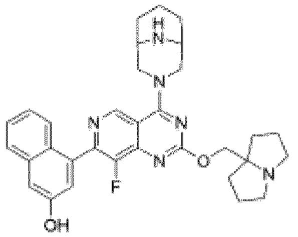 | 555.3 | $^1$H NMR (400 MHz, $CDCl_3$) δ 9.07 (s, 1H), 7.68 (d, J = 7,8 Hz, 1H), 7.57-7.52 (m, 1H), 7.38-7.33 (m, 1H), 7.21-7.15 (m, 3H), 4.72 (d, J = 16,0 Hz, 2H), 4.52 (s, 2H), 3.85 (d, J = 15.3 Hz, 2H), 3.63-3.55 (m, 2H), 3.24-3.14 (m, 2H), 2.97-2.88 (m, 2H), 2.29-2.18 (m, 3H), 2.10-1.85 (m, 11H). | 20 |
| 72 | 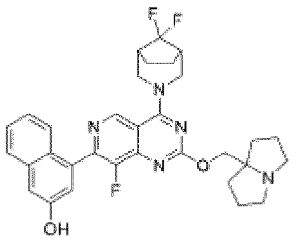 | 576.3 | $^1$H NMR (400 MHz, $CDCl_3$) δ 8.93 (s, 1H), 7.64 (d, J = 8.2 Hz, 1H), 7.57 (d, J = 7.9 Hz, 1H), 7.35 (t, J = 7.1 Hz, 1H), 7.23-7.17 (m, 3H, 4.60 (d, J = 12.6 Hz, 2H), 4.31 (s, 2H), 3.70 (d, J = 12.6 Hz, 2H), 3.26-3.23 (m, 2H), 2.73-2.67 (m, 2H), 2.29 (brs, 2H), 2.16-2.09 (m, 2H), 1.94-1.88 (m, 4H), 1.75-1.68 (m, 4H), 1.46-1.41 (m, 2H), | 26 |
| 73 | 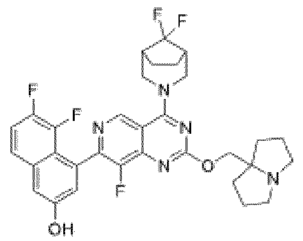 | 612.2 | | 26 |
| 74 | 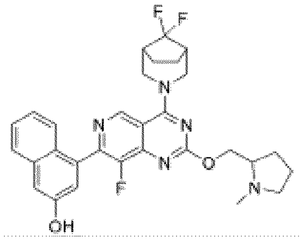 | 550.2 | $^1$H NMR (400 MHz, $CDCl_3$) δ 8.98 (s, 1H), 7.64 (d, J = 8,1 Hz, 1H), 7.52-7.48 (m, 1H), 7.36-7.24 (m, 3H), 7.16 (t, J = 7.5 Hz, 1H), 4.73 (d, J = 13.3 Hz, 1H), 4.61 (dd, J = 6.5, 10.2 Hz, 1H), 4.46 (d. J = 12.2 Hz, 1H), 4.37 (dd, J =4.4, 11.2 Hz, 1H), 3.82 (d, J = 12.6 Hz, 1H), 3.6l (d, J = 12.5 Hz, 1H), 3.30-3.24 (m, 1H), 2.97-2.90 (m, 1H), 2.70 (s, 3H), 2.46-2.40 (m, 1H), 2.31-2.20 (m, 2H), 2.14-1.94 (m, 2H), 1.90-1.81 (m, 2H), 1.65-1.57 (m, 1H), 1.51-1.41 (m, 1H), 1.29-1.17 (m, 2H). | 26 |
| 75 | 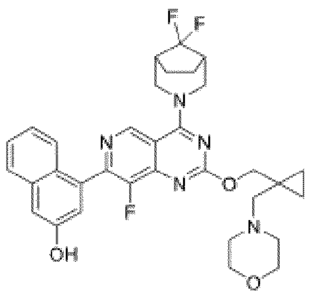 | 606.3 | $^1$H NMR (400 MHz, $CDCl_3$) δ 8.96 (s, 1H), 7.67-7.61 (m, 2H), 7.37 (t, J = 7.3 Hz, 1H), 7.25-7.18 (m, 3H), 4.62 (d, J = 12.7 Hz, 2H), 4.42 (s, 2H), 3.76 (d, J = 13.1 Hz, 2H), 3.67 (s, 4H), 2.50-2.37 (m, 8H), 1.93-1.85 (m, 2H), 1.68-1.57 (m, 2H), 0.70 (s, 2H), 0.49 (s, 2H), | 26 |

(continued)

| Exam ple | Structure | MS (ES1) m/z | $^1$H NMR | Examp le of re ference for syn thesis |
|---|---|---|---|---|
| 76 | 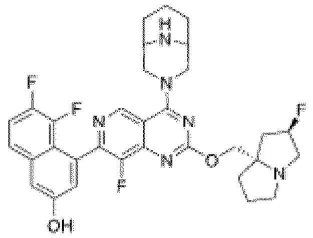 | 609.3 | $^1$H NMR (400 MHz, $CDCl_3$) δ 9.02 (s, 1H), 7.42-7.37 (m, 2H), 7.22-7.16 (m, 2H), 4.75-4.58 (m, 2H), 4.26 (s, 2H), 3.85-3,71 (m, 2H), 3.38-3.28 (m, 4H), 3.24-3.15 (m, 2H), 3.02-2.96 (m, 1H), 2.36-1.60 (m, 12H). | 20 |
| 77 | 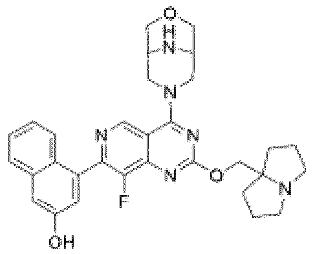 | 557.3 | | 20 |
| 78 | 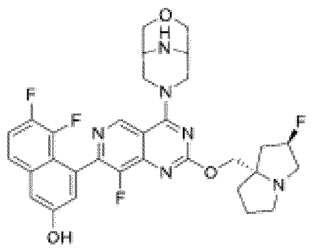 | 611.2 | $^1$H NMR (400 MHz, $CDCl_3$) δ 8.99 (d. J = 10.8 Hz, 1H), 7.42-7.38 (m, 1H), 7.23-7.16 (m, 3H), 5.31 (d, J = 55.7 Hz, 1H), 4.96-4.90 (m, 1H), 4.83-4.76 (m, 1H), 4.39-4.30 (m, 2H), 4.00-3.73 (m, 6H), 3.52-3.39 (m, 2H), 3.32-3,24 (m, 1H), 3.02 (s, 2H), 2.36-1.86 (m. 7H). | 20 |
| 79 | 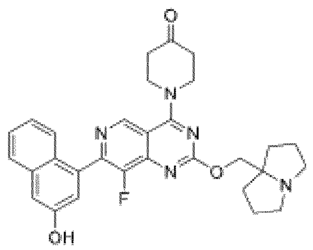 | 528.2 | | 9 |
| 80 | 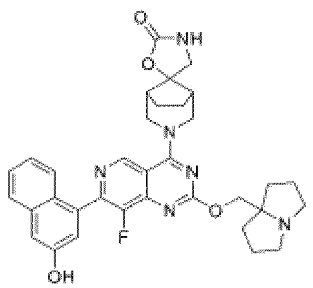 | 611.3 | | 10, 11,18 |
| | | | | |

(continued)

| Exam ple | Structure | MS (ES1) m/z | $^1$H NMR | Examp le of re ference for syn thesis |
|---|---|---|---|---|
| 81 | 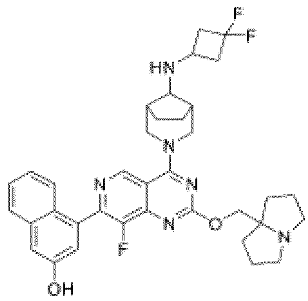 | 645.3 | $^1$H NMR (400 MHz, $CDCl_3$) δ 9.03 (s, 1H), 7.64 (t, J = 9.4 Hz, 2H), 7.38-7.31 (m, 2H), 7.23-7.19 (m, 2H), 4.32 (d, J 12.1 Hz, 2H), 4.25 (s, 2H), 3.83 (d, J = 12.3 Hz, 2H), 3.29-3.17 (m, 3H), 2.90-2.81 (m, 3H, 2.70-2.64 (m, 2H), 2.38-2.28 (m, 2H), 2.15-2.08 (m, 2H), 2.07-2.01 (m, 2H), 1.91-1.85 (m, 4H), 1.72-1.65 (m, 4H), 1,52-1,47 (m, 2H). | 27 |
| 82 | 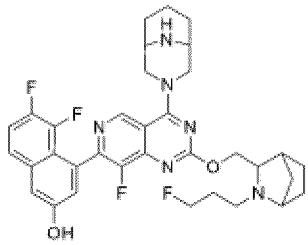 | 637.3 | $^1$H NMR (400 MHz, $CDCl_3$) δ 9.07 (s, 1H), 7.46-7.40 (m, 2H), 7.24-7.16 (m, 2H), 5.35 (t, J = 5.0 Hz, 1H), 4.77 (d, J = 13.1 Hz, 1H), 4.65-4.53 (m, 2H), 4.45-4.35 (m, 2H), 4.13-4.08 (m, 1H), 3.87-3.74 (m, 2H), 3,38-3.31, (m, 3H), 2.85-2.68 (m, 3H), 2.49-2.43 (m, 2H), 2,04-1.60 (m, 12H), | 12 |
| 83 | 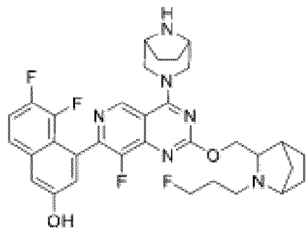 | 623.3 | | 12 |
| 84 | 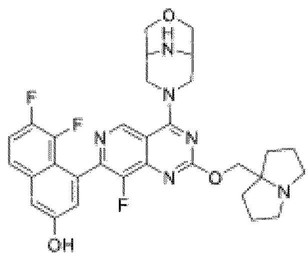 | 593.3 | | 20 |
| 85 | 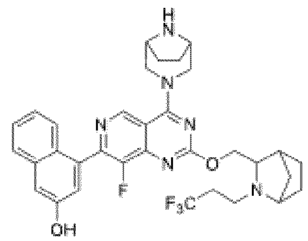 | 623.3 | $^1$H NMR (400 MHz, $CDCl_3$) δ 9.02 (s, 1H), 7.62 (d, J = 8.2 Hz, 2H), 7,37 (t, J = 7.4 Hz, 1H), 7.29-7.18 (m, 3H), 4.27 (d, J = 4.3, 10.8 Hz, 1H), 4.08-4.03 (m, 1H), 3.65-3.56 (m, 4H), 3.28 (s, 1H), 2.91-2.74 (m, 2H), 2.49-2.22 (m, 8H), 1.85-1.60 (m,8H), | 12 |
| 86 | 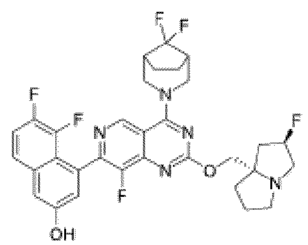 | 630.2 | | 26 |

(continued)

| Example | Structure | MS (ES1) m/z | $^1H$ NMR | Example of reference for synthesis |
|---|---|---|---|---|
| 87 | 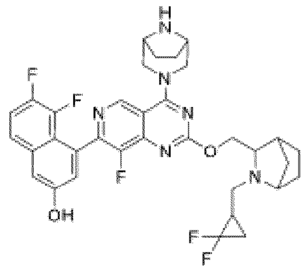 | 653.3 | $^1H$ NMR. (400 MHz, $CDCl_3$) δ 8.98 (s, 1H), 7,42-7.35 (m, 1H), 7.24-7.06 (m, 3H), 4.70-,4.60 (m, 1H), 4.47-4.39 (m, 1H), 4.34-4.27 (m, 1H), 4.15-4.05 (m, 1H), 3.84-3.61 (m, 4H), 3.40) (s, 2H), 2.90-2.80 (m, 1H), 2.68-2.57 (m, 2H), 2.42 (s, 2H), 2.01-1.73 (m, 8H), 1.66-1.57 (m, 2H), 0.90-0.83 (m, 2H), | 12 |
| 88 | 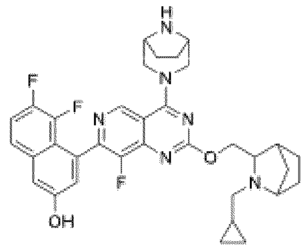 | 617.3 | | 12 |
| 89 | 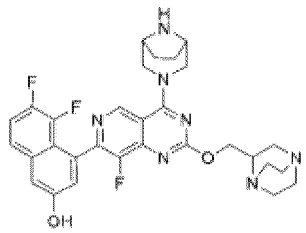 | 578.2 | | 6 |
| 90 | 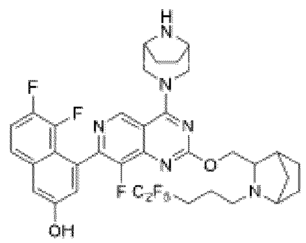 | 773.3 | | 12 |
| 91 | 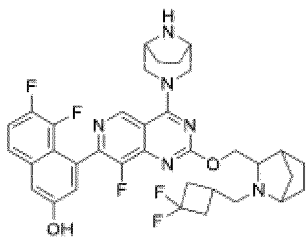 | 667.3 | | 12 |
| 92 | 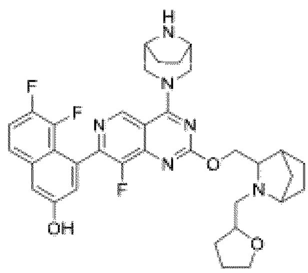 | 647.3 | | 12 |

(continued)

| Exam ple | Structure | MS (ES1) m/z | $^1$H NMR | Examp le of re ference for syn thesis |
|---|---|---|---|---|
| 93 | 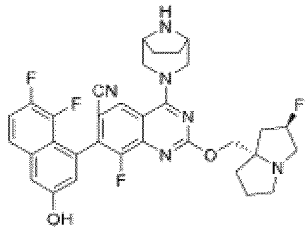 | 619.2 | | 30 |
| 94 | 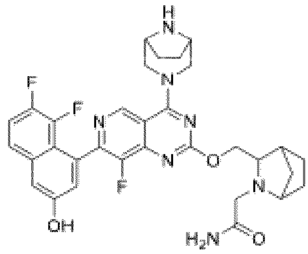 | 620.3 | | 12 |
| 95 | 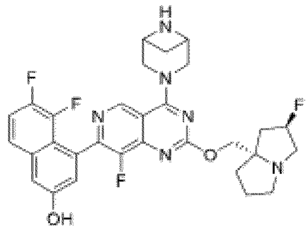 | 581.2 | | 20 |
| 96 | 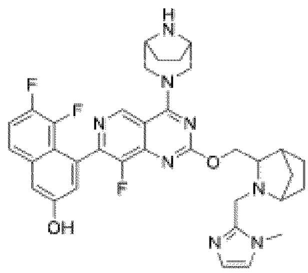 | 657.3 | | 12 |
| 97 | 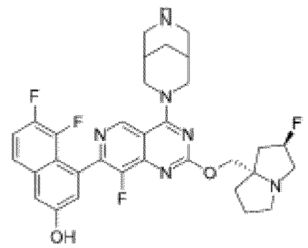 | 609.3 | | 20 |
| 98 | 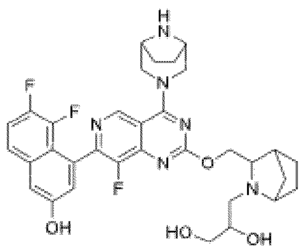 | 637.3 | | 12 |

(continued)

| Example | Structure | MS (ES1) m/z | $^1$H NMR | Example of reference for synthesis |
|---|---|---|---|---|
| 99 | 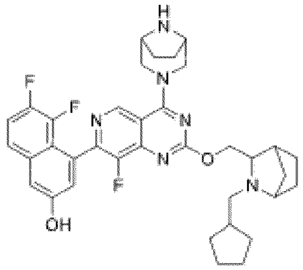 | 645.3 | | 12 |
| 100 | 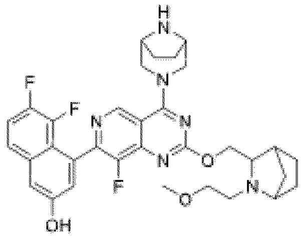 | 521.3 | | 12 |
| 101 | 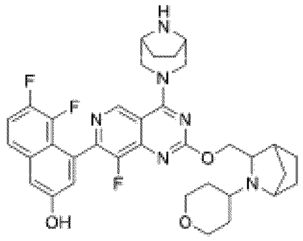 | 647.3 | | 12 |
| 102 | 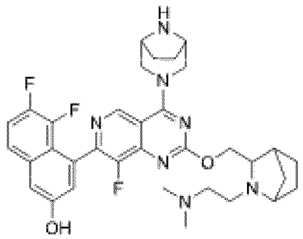 | 634.3 | | 12 |
| 103 | 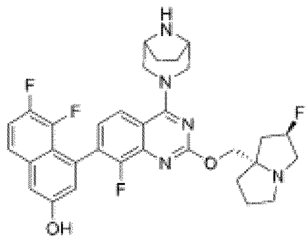 | 594.3 | | 6 |
| 104 | 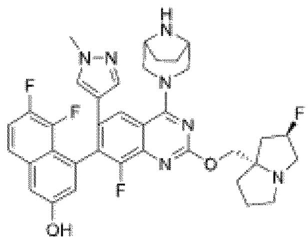 | 674.3 | | 30 |

(continued)

| Example | Structure | MS (ES1) m/z | 1H NMR | Example of reference for synthesis |
|---|---|---|---|---|
| 105 | 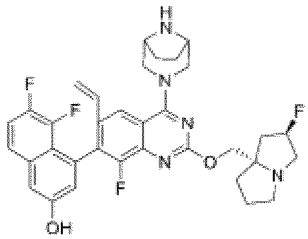 | 620.3 | | 30 |
| 106 | 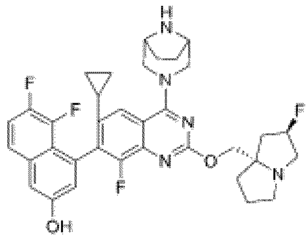 | 634.3 | | 30 |
| 107 | 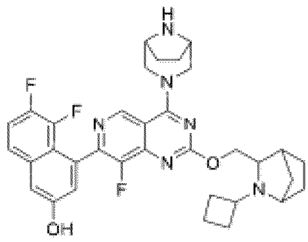 | 617.3 | | 12 |
| 108 | 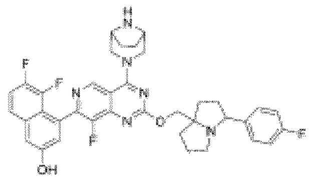 | 671.3 | | 34 |
| 109 | 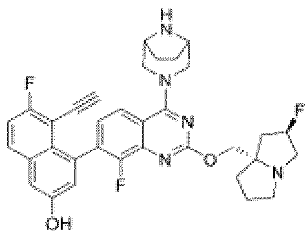 | 600.3 | | 2 |
| 110 | 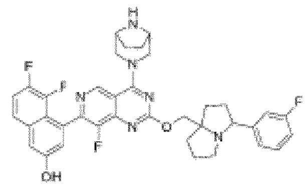 | 671.3 | | 34, |

(continued)

| Example | Structure | MS (ES1) m/z | 1H NMR | Example of reference for synthesis |
|---|---|---|---|---|
| 111 | 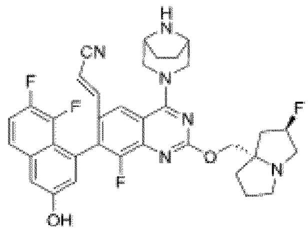 | 645.3 | | 30 |
| 112 | 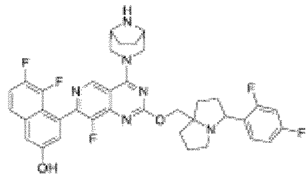 | 689.3 | | 34 |
| 113 | 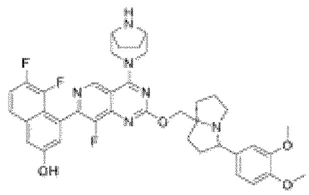 | 713.3 | | 34 |
| 114 | 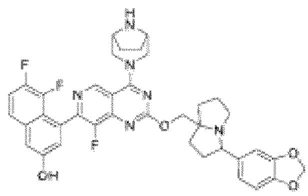 | 697.3 | $^1$H NMR (400 MHz, $CDCl_3$) δ 8.87 (s, 1H), 7.42-7.34 (m, 1H), 7.22-7.07 (m, 3H), 6.83-6.51 (m, 3H), 5.78 (s, 2H), 4.68-4.58 (m, 1H), 4.47-4.25 (m, 3H), 3.77-3.62 (m, 5H), 3.23 (s, 2H), 2.98-2.69 (m, 2H), 2.37-2.22 (m, 1H), 2.07-1.69 (m, 9H). | 34 |
| 115 | 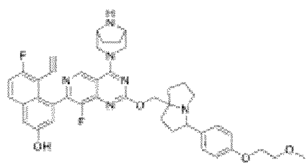 | 733.3 | | 35,52 |
| 116 | 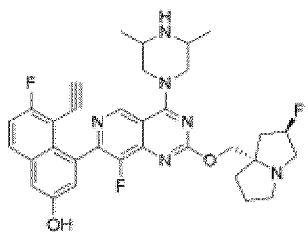 | 603.3 | | 20 |
| 117 | 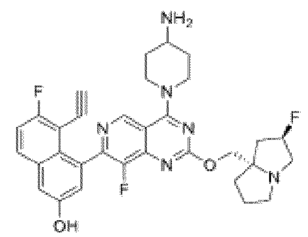 | 589.3 | | 20 |

(continued)

| Example | Structure | MS (ES1) m/z | $^1$H NMR | Example of reference for syn thesis |
|---|---|---|---|---|
| 118 | 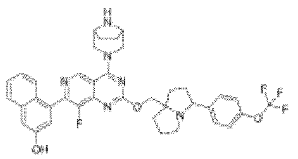 | 701.3 | $^1$H NMR (400 MHz, $CDCl_3$) δ 9.04 (s, 1H), 7.65-7.58 (m, 2H), 7.40 (d, J = 8.6 Hz, 2H), 7.36-7.31 (m, 2H), 7,24-7.16 (m, 2H), 7.10 (d, J = 8.1 Hz, 2H), 4.54-4.48 (m, 211), 4.29 (q, J = 10.4 Hz, 2H), 3.74 (dd, J = 5.3, 10.8 Hz, 1H), 3.62 (brs, 4H), 2.93-2.87 (m, 1H), 2.63-2.58 (m, 1H), 2.34 (dd, J = (6.4, 12.4 Hz, 1H), 2.20-2.14 (m, 1H), 2.04-1.98 (m, 1H), 1.96-1.87 (m, 3H), 1.80-1.65 (m, 6H). | 34 |
| 119 | 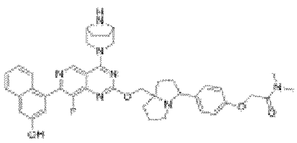 | 718.3 | $^1$H NMR (400 MHz, $CDCl_3$) δ 9.08 (s, 1H), 7.71 (d, J = 7.8 Hz, 1H), 7.65 (d, J = 9.0 Hz, 1H), 7.44-7.36 (m, 2H), 7.28-7.21 (m, 2H), 7,12 (d, J = 8.4 Hz, 2H), 6.68 (d, J = 8.5 Hz, 2H), 4.67-4.65 (m, 1H), 4.62 (s, 2H), 4.55 (d. J = 11.1 Hz, 1H), 4.50-4.45 (m, 1H), 4.39-4.34 (m, 1H), 3.73-330 (m, 5H), 3.04, (s, 3H), 2.98 (s, 3H), 2.87-2.80 (m, 1H), 2.64-2.56 (m, 1H), 2.04-1.76 (m, 12H). | 35 |
| 120 | 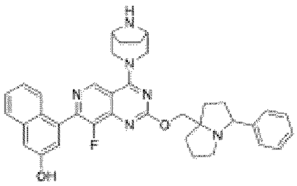 | 617.3 | | 34 |
| 121 | 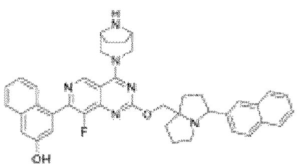 | 667.3 | | 34 |
| 122 | 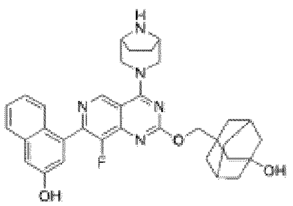 | 582.3 | | 6 |
| 123 | 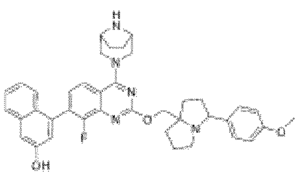 | 646.3 | | 34 |

(continued)

| Exam ple | Structure | MS (ES1) m/z | $^1$H NMR | Examp le of re ference for syn thesis |
|---|---|---|---|---|
| 124 | 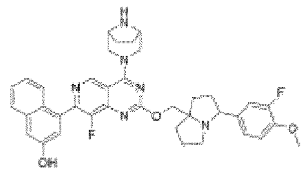 | 665.3 | $^1$H NMR (400 MHz, $CDCl_3$) δ 8.99 (s, 1H), 7.69-7.65 (m, 1H), 7.52-7.48 (m, 1H), 7.38-7.31 (m, 1H), 7.24-7.03 (m, 5H). 6.86-6.80 (m, 1H), 4.66-4.61 (m, 211), 4.50-4.38 (m, 2H), 4.02-3.94 (m, 4H), 3.85-3.75 (m, 5H), 3.10-3.01 (m, 1H), 2.92-2.81 (m, 1H), 2.42-2.33 (m, 1H), 2.19-1.82 (m, 10H). | 34 |
| 125 | 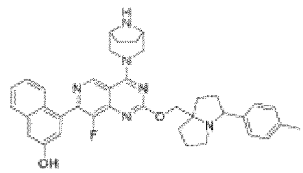 | 631.3 | $^1$HNMP, (400 MHz, $CDCl_3$) δ 9.00 (s, 1H), 7.62-7.53 (m, 2H), 7.40-7.25 (m, 4H), 7.21-7.13 (m, 2H), 7,06 (d, J = 7.6 Hz, 211), 4.55-4.43 (m, 211), 4.29 (dd, J = 10.6, 27.1 Hz, 2H), 3.71-3.53 (m, 5H), 2.93-2.87 (m, 1H), 2.67-2.61 (m, 1H), 2.35.2.27 (m, 4H), 2.17-2.11 (m, 1H), 2.05-1.64 (m, 10H). | 34 |
| 126 | 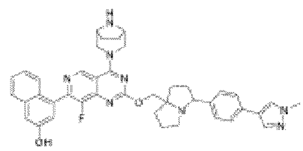 | 697.3 | | 44 |
| 127 | 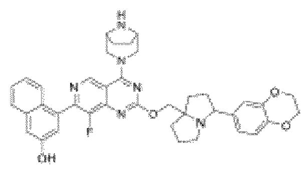 | 675.3 | $^1$H NMR (400 MHz, $CDCl_3$) δ 9.03 (s, 1H), 7.64-7.57 (m, 2H), 7.37-7.30 (m, 2H), 7.23-7.15 (m, 2H), 6.88-6.73 (m, 3H), 4.52. (d, J = 10.6 Hz, 2H), 4.29 (dd, J = 10.7, 24.7 Hz, 211), 4.16 (s, 4H), 3.68-3.54 (m, 5H), 2.92-2.87 (m, 1H), 2.65-2.60 (m, 1H), 2.34-2.29 (m, 4H), 2 14-2.08 (m, 1H), 1.96-1.61 (m, 10H). | 34 |
| 128 | 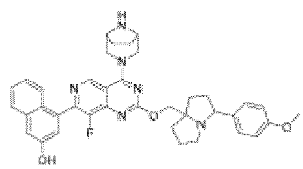 | 647.3 | $^1$H NMR (400 MHz, $CDCl_3$) δ 9.05 (s, 1H), 7.68-7.61 (m, 2H), 7.40-7.35 (m, 1H), 7.30 (d, J = 8.6 Hz, 3H), 7.24-7.19 (m, 2H), 6.80 (d, J = 8,4 Hz, 2H), 4.59-4.51 (m, 211), 4.36-4.27 (m, 211), 3.75 (s, 3H), 3.71-3.59 (m, 4H), 2.93-2.87 (m, 1H), 2.68-2.63 (m, 1H), 2.37-2.32 (m, 1H), 2.17-2.10 (m, 1H), 2.04-1.88 (m, 5H), 1.77-1.61 (m, 6H). | 34 |
| 129 | 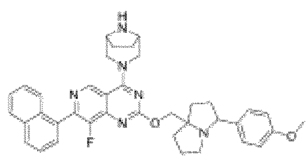 | 631.3 | $^1$H NMR (400 MHz, $CDCl_3$) δ 9.11 (s, 1H), 7.96 (dd, J = 7.9, 18.2 Hz, 2H), 7.83 (d, J = 8.0 Hz, 1H), 7.65 (dd, J = 7.6, 31.0 Hz, 2H), 7.53-7.,44 (m, 2H), 7.32 (d, J = 8.6 Hz, 2H), 6.83 (d, J = 8.7 Hz, 2H), 4.61 (t, J = 13.3 Hz, 2H), 4.35 (dd, J = 10.2, 24.3 Hz, 2H), 3.78 (s, 3H), 3.75-3.69 (m, 5H), 2.95-2,89 (m, 1H), 2.38 (dd, J = 6.2, 12.4 Hz, 1H), 2.19-2.13 (m, 1H), 2.08-1.67 (m, 10H). | 34 |

(continued)

| Exam ple | Structure | MS (ES1) m/z | $^{1}$H NMR | Examp le of re ference for syn thesis |
|---|---|---|---|---|
| 130 | 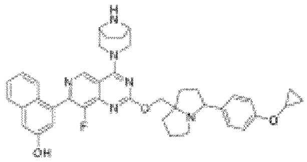 | 673.3 | | 35 |
| 131 | 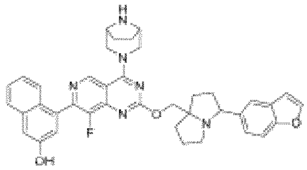 | 657.3 | | 34 |
| 132 | 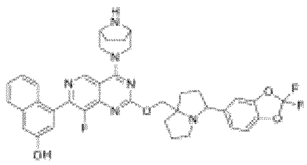 | 697.3 | $^{1}$H NMR (400 MHz, $CDCl_3$) δ 9.05 (s, 1H), 7,67-7-57 (m, 2H), 7.40-7.28 (m, 3H), 7.23-7.14 (m, 211), 6.96 (dd, J = 8.2, 42.5 Hz, 2H), 4.61-4.46, (m, 2H), 4.30 (s, 211), 3.82-3.56 (m, 5H), 2.92-2.85 (m, 1H), 2.62-2.55 (m, 1H), 2.37-2.28 (m, 1H), 2.18-2.10 (m, 1H), 2.01-1.62 (m, 10H). | 34 |
| 133 | 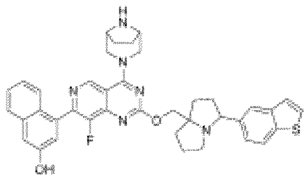 | 673.3 | | 34 |
| 134 | 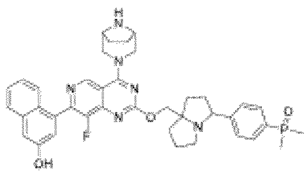 | 693.3 | | 44 |
| 135 | 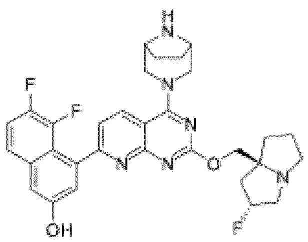 | 577.3 | | 50 |
| 136 | 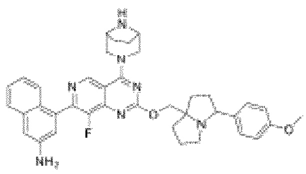 | 646.3 | $^{1}$H NMR (400 MHz, $CDCl_3$) δ 909 (s, 1H), 7.67 (d, J = 8.3 Hz, 1H), 7.63 (d, J = 9.5 Hz, 1H), 7.39 (t, J = 7.0 Hz, 1H), 7.32 (d, J = 8.6 Hz, 2H), 7.22-7.13 (m, 3H), 6.83 (d, J = 8.7 Hz, 211), 4.61 (t, J = 13.0 Hz, 211), 4.42-4.32 (m, 2H), 3.97-3.88 (m, 1H), 3.81-3.70 (m, 7H), 2.99-2.91 (m, 1H), 2.72-2.65 (m, 1H), 2.42-2.34 (m, 1H), 2.20-2-14 (m, 1H), 2.06-1.60 (m, 10H). | 39, 40 |

(continued)

| Example | Structure | MS (ES1) m/z | $^1$H NMR | Example of reference for synthesis |
|---|---|---|---|---|
| 137 | 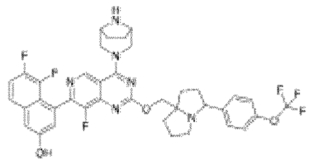 | 737.3 | | 34 |

**Example 138**

4-(4-((1R,5S)-3,8-diazabicyclo[3.2.1]octan-3-yl)-8-fluoro-2-(((3S,7aR)-3-(4-fluorophen yl)tetrahydro-1H-pyrrolizin-7a(5H)-yl)methoxy)pyrido[4,3-d]pyrimidin-7-yl)-5,6-difluoronaphth alen-2-ol

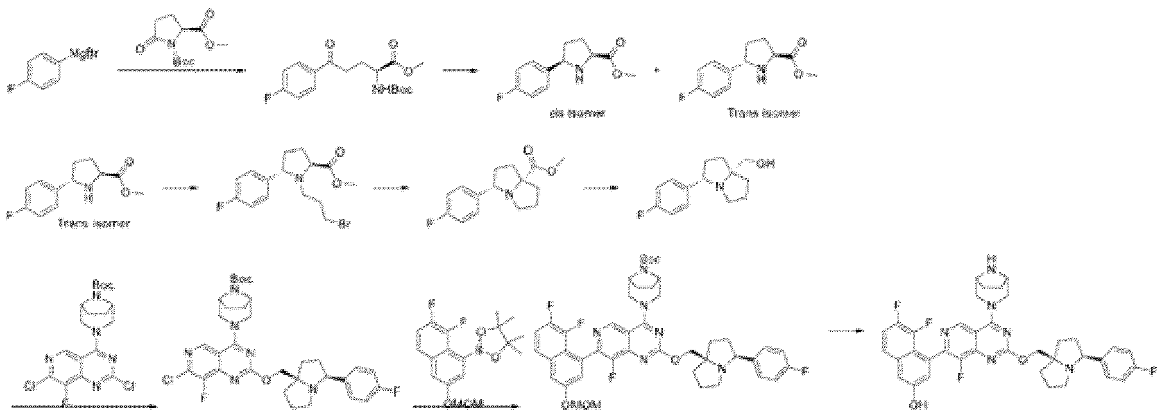

Using the starting materials shown in the synthetic route as raw materials, the desired isomers were separated, and the target product was synthesized by a method similar to that in Example 34.

LC/MS: m/z=671.3 [M+H]$^+$.

$^1$H NMR (400 MHz, $CDCl_3$) δ 8.92 (s, 1H), 7.34-7.28 (m, 3H), 7.21-7.16 (m, 2H), 7.06 (brs, 1H), 7.02-6.87 (m, 2H), 4.65-4.56 (m, 1H), 4.41-4.35 (m, 1H), 4.30-4.21 (m, 2H), 3.76-3.37 (m, 6H), 2.85-2.81 (m, 1H) , 2.61-2.59 (m, 1H), 2.30-2.29 (m, 1H), 2.13-2.11 (m, 1H), 1.98-1.81 (m, 4H), 1.76-1.61 (m, 5H).

**Example 139**

4-(4-((1R,5S)-3,8-diazabicyclo[3.2.1]octan-3-yl)-8-fluoro-2-(((3R,7aS)-3-(4-fluorophen yl)tetrahydro-1H-pyrrolizin-7a(5H)-yl)methoxy)pyrido[4,3-d]pyrimidin-7-yl)-5,6-difluoronaphth alen-2-ol

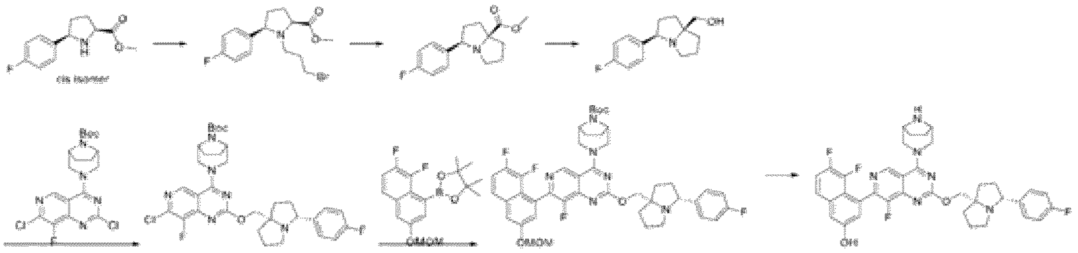

Using the starting materials shown in the synthetic route as raw materials, the target product was synthesized by a method similar to that in Example 34.

LC/MS: m/z=671.3 $[M+H]^+$.

$^1H$ NMR (400 MHz, $CDCl_3$) δ 8.92 (s, 1H), 7.33-7.31 (m, 3H), 7.20-7.16 (m, 2H), 7.05 (brs, 1H), 6.95-6.87 (m, 2H), 4.39-4.08 (m, 5H), 3.66-3.52 (m, 5H), 2.90-2.82 (m, 1H), 2.62-2.54 (m, 1H), 2.34-2.29 (m, 1H), 2.17-2.11 (m, 1H), 1.93-1.81 (m, 4H), 1.75-1.56 (m, 5H).

**Example 140**

4-(4-((1R,5S)-3,8-diazabicyclo[3.2.1]octan-3-yl)-2-((3-(2,2-difluorobenzo[d][1,3]dioxy -5-yl)tetrahydro-1H-pyrrolizin-7a(5H)-yl)methoxy)-8-fluoropyridin[4,3d]pyrimidin-7-yl)-5,6-difl uoronaphthalen-2-ol (Trans racemate)

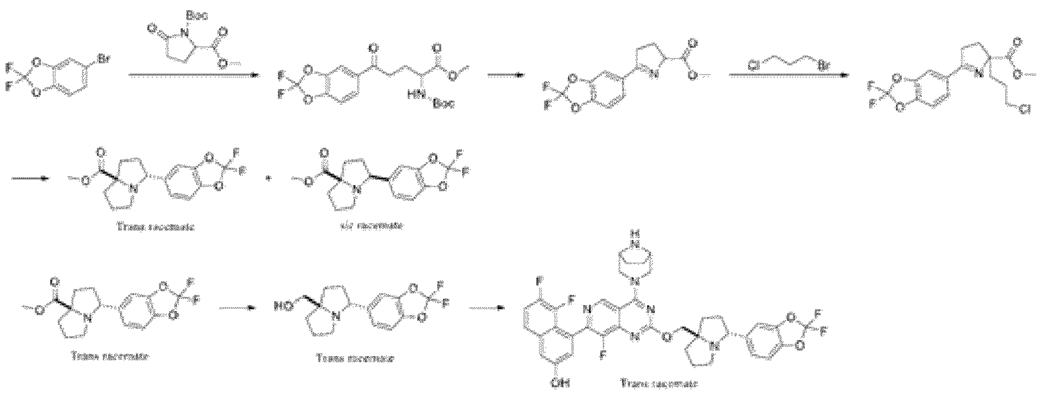


Steps 1 and 2: Using the starting materials shown in the synthetic route as raw materials, the target product was synthesized by a method similar to that in Example 34.

Step 3: Synthesis of methyl 2-(3-chloropropyl)-5-(2,2-difluorobenzo[d][1,3]dioxy-5-yl)-3,4-dihydro-2H-pyrrol-2-carboxylate

Methyl 5-(2,2-difluorobenzo[d][1,3]dioxy-5-yl)-3,4-dihydro-2H-pyrrole-2-carboxylate (5.0 g, 17.66 mmol) and 1-bromo-3-chloropropane (5.56 g, 35.32 mmol) was dissolved in 10 mL of DMF and 40 mL of THF. Sodium hydride (1.06 g, 26.49 mmol) was added in batches in an ice bath, and the mixture was stirred at room temperature for 1-2 h. The reaction system was poured into 100 mL of saturated ammonium chloride aqueous solution, and extracted with ethyl acetate. The organic phase was dried and evaporated under reduced pressure. The crude product was purified by column chromatography (PE\EA=8\1) to obtain 4.1 g of off-white solid, with a total yield of 64.6%.

Step 4: Synthesis of methyl 3-(2,2-difluorobenzo[d][1,3]dioxy-5-yl)tetrahydro-1H-pyrrolizin-7a(5H)-carboxylate (Trans racemate)

The product (4.1 g, 11.40 mmol) obtained from the previous step was dissolved in 40 mL of methanol, and acetic acid (1.03 g, 17.1 mL) was added. Then, $NaBH_3CN$ (1.07 g, 17.1 mL) was added in batches under an ice bath, and the mixture was stirred at room temperature for 3 h. The reaction system was concentrated to dryness, added with 100 mL of water, adjusted to weakly alkaline with sodium carbonate, and extracted with ethyl acetate. The organic phase was dried and evaporated under reduced pressure. The crude product was purified and eluted to obtain 1.8 g of light yellow oil of a cis intermediate by column chromatography (PE\EA=5\1) and 1.5 g of off-white solid of a trans intermediate by column chromatography (PE\EA=2\1), with a total yield of 88.9%.

Using the trans intermediate obtained in the previous step as raw materials, the target product was synthesized by a method similar to that in Example 34.

LC/MS: m/z=733.2 $[M+H]^+$.

$^1H$ NMR (400 MHz, $CDCl_3$) δ 10.29 (brs, 1H), 9.12 (s, 1H), 7.76-7.73 (m, 1H), 7.61-7.54 (m, 1H), 7.40 (s, 1H), 7.35 (s, 1H), 7.30 (d, 1H, $J$ = 8.0 Hz), 7.25 (s, 1H), 7.17 (d, 1H, $J$ = 8.4 Hz), 4.50-4.40 (m, 2H), 4.30-4.27 (m, 1H), 4.23-4.18 (m, 2H), 3.65-3.56 (m, 4H), 2.32-2.30 (m, 1H), 2.23-2.17 (m, 1H), 2.15-2.05 (m, 2H), 1.96-1.93 (m, 1H), 1.88-1.85 (m, 2H), 1.65-1.51 (m, 7H).

**Example 141 (Trans isomer 1), Example 142 (Trans isomer 2)**

4-(4-((1R,5S)-3,8-diazabicyclo[3.2.1]octan-3-yl)-2-((3-(2,2-difluorobenzo[d][1,3]dioxy -5-yl)tetrahydro-1H-pyrrolizin-7a(5H)-yl)methoxy)-8-fluoropyrido[4,3d]pyrimidin-7-yl)-5,6-difl uoronaphthalen-2-ol

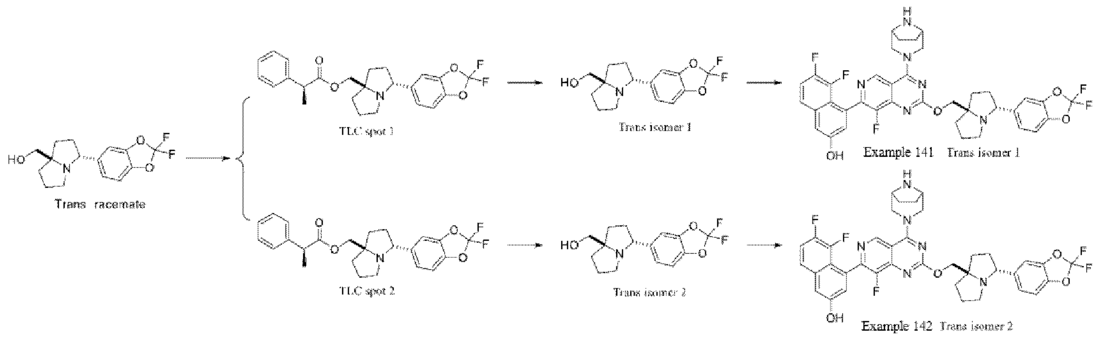


Step 1: Synthesis of (3-(2,2-difluorobenzo[d][1,3]dioxy-5-yl)tetrahydro-1H-pyrrolizin-7a(5H)-yl)methyl(S)-2-phenyl propionate

(3-(2,2-Difluorobenzo[d][1,3]dioxol-5-yl)tetrahydro-1H-pyrrolizin-7a(5H)-yl)methanol (trans-racemate, 297 mg, 1.0 mmol), (S)-2-phenylpropionic acid (180 mg, 1.2 mmol), DMAP (12 mg, 0.1 mmol), and 5 mL of dichloromethane were added to a reaction flask. EDCI (288 mg, 1.5 mmol) was added in batches at room temperature, and the mixture was stirred overnight at room temperature. The reaction solution was poured into 20 mL of water and extracted with dichloromethane. The organic phase was dried, evaporated under reduced pressure, and then purified by column chromatography (DCM/MeOH = 50/1) to obtain 120 mg of an upper spot (TLC spot 1) as light yellow solid, and 130 mg of a lower spot (TLC spot 2) as light yellow solid, with a total yield of 58%.

Step 2: Synthesis of (3-(2,2-difluorobenzo[d][1,3]dioxol-5-yl)tetrahydro-1H-pyrrolizin-7a(5H)-yl)methanol

(3-(2,2-Difluorobenzo[d][1,3]dioxy-5-yl)tetrahydro-1H-pyrrolizin-7a(5H)-yl)methyl(S) -2-phenyl propionate (TLC spot 1, 120 mg, 0.28 mmol) was dissolved in MeOH/$H_2O$ (3 mL/0.3 mL), and NaOH (40 mg, 1.0 mmol) was added. The mixture was stirred at room temperature for 1 h. The reaction system was poured into 10 mL of water and extracted with ethyl acetate. The organic phase was backwashed once with 1 M NaOH aqueous solution, dried, and evaporated under reduced pressure to obtain 85 mg of the target product (Trans isomer 1), with a yield of 100%.

Step 3: Synthesis of 4-(4-((1R,5S)-3,8-diazabicyclo[3.2.1]octan-3-yl)-2-((3-(2,2-difluorobenzo[d][1,3]dioxy-5-yl)tetra hydro-1H-pyrrolizin-7a(5H)-yl)methoxy)-8-fluoropyridine[4,3d]pyrimidin-7-yl)-5,6-difluoronap hthalen-2-ol

Using the product (Trans isomer 1) obtained in the previous step as raw materials, the target product was synthesized by a method similar to that in Example 34.
LC/MS: m/z=733.3 $[M+H]^+$.
$^1H$ NMR (400 MHz, $CDCl_3$) δ 10.29 (brs, 1H), 9.12 (s, 1H), 7.76-7.73 (m, 1H), 7.61-7.54 (m, 1H), 7.40 (s, 1H), 7.35 (s, 1H), 7.30 (d, 1H, *J* = 8.0 Hz), 7.25 (s, 1H), 7.17 (d, 1H, *J* = 8.4 Hz), 4.50-4.40 (m, 2H), 4.30-4.27 (m, 1H), 4.23-4.18 (m, 2H), 3.65-3.56 (m, 4H), 2.32-2.30 (m, 1H), 2.23-2.17 (m, 1H), 2.15-2.05 (m, 2H), 1.96-1.93 (m, 1H), 1.88-1.85 (m, 2H), 1.65-1.51 (m, 7H).
Trans isomer 2 of Example 142 can be obtained by the same method with the product of step 1 (TLC spot 2) as raw materials.
LC/MS: m/z=733.3 $[M+H]^+$.
$^1H$ NMR (400 MHz, $CDCl_3$) δ 10.29 (brs, 1H), 9.12 (s, 1H), 7.76-7.73 (m, 1H), 7.61-7.54 (m, 1H), 7.40 (s, 1H), 7.35 (s, 1H), 7.30 (d, 1H, *J* = 8.0 Hz), 7.25 (s, 1H), 7.17 (d, 1H, *J* = 8.4 Hz), 4.50-4.40 (m, 2H), 4.30-4.27 (m, 1H), 4.23-4.18 (m, 2H), 3.65-3.56 (m, 4H), 2.32-2.30 (m, 1H), 2.23-2.17 (m, 1H), 2.15-2.05 (m, 2H), 1.96-1.93 (m, 1H), 1.88-1.85 (m, 2H), 1.65-1.51 (m, 7H).

**Example 143**

4-(4-((1R,5S)-3,8-diazabicyclo[3.2.1]octan-3-yl)-8-fluoro-2-((5'-(4-(trifluoromethoxy)p henyl)dihydro-1'H,3'H-spiro[cyclopropan-1,2'-pyrrolizin]-7a'(5'H)-yl)methoxy]pyrido[4,3d]pyr imidin-7-yl)naphthalen-2-ol

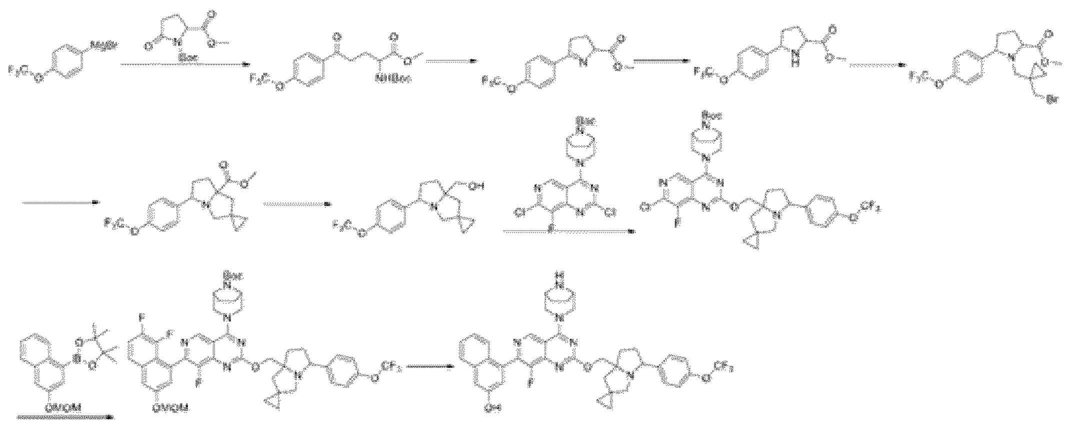

Using the starting materials shown in the synthetic route as raw materials and 1,1-bis(bromomethyl) cyclopropane as a nitrogen alkylating reagent in step 4, the target product was synthesized by a method similar to that in Example 34.
LC/MS: m/z=727.3 $[M+H]^+$.

**Example 144**

4-(4-((1R,5S)-3,8-diazabicyclo[3.2.1]octan-3-yl)-8-fluoro-2-((2-fluoro-3-(4-(trifluorom ethoxy)phenyl)tetrahydro-1H-pyrrolizin-7a(5H)-yl)methoxyyl)pyrido[4,3-d]pyrimidin-7-yl)napht halen-2-ol

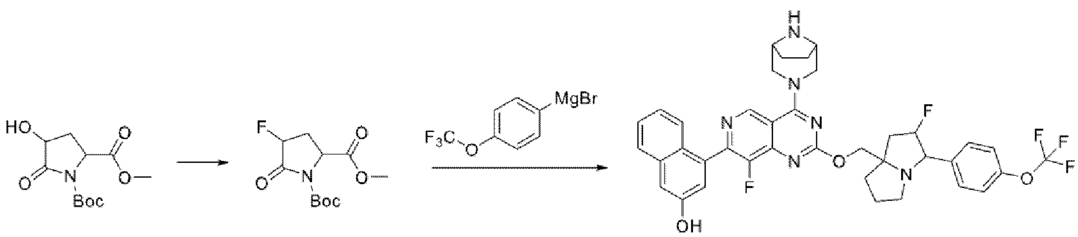

Step 1: Synthesis of 1-(tert-butyl)2-methyl4-fluoro-5-oxopyrrolidin-1,2-dicarboxylate

1-(Tert-butyl)2-methyl-4-hydroxy-5-oxopyrrolidin-1,2-dicarboxylate (2.59 g, 10.0 mmol) and 25 mL of dichloromethane were added to a reaction flask. DAST (2.4 g, 15.0 mmol) was then added at 0°C. The mixture was stirred at room temperature for 4 h. The reaction solution was poured into 100 mL of water and extracted with dichloromethane. The organic phase was dried and evaporated under reduced pressure. The crude product was purified by column chromatography (PE\EA=5\1) to obtain 1.7 g of colorless oil, with a yield of 65%.

Step 2: Synthesis of 4-(4-((1R,5S)-3,8-diazabicyclo[3.2.1]octan-3-yl)-8-fluoro-2-((2-fluoro-3-(4-(trifluoromethoxy)ph enyl)tetrahydro-1H-pyrrolizin-7a(5H)-yl)methoxyyl)pyrido[4,3-d]pyrimidin-7-yl)naphthalen-2-ol

Using the product obtained in the previous step as raw materials, the target product was synthesized by a method similar to that in Example 34.
LC/MS: m/z=719.3 $[M+H]^+$.
$^1H$ NMR (400 MHz, $CDCl_3$) δ 9.01 (s, 1H), 7.65-7.55 (m, 2H), 7.47 (d, 2H, *J* = 8.4 Hz), 7.37-7.30 (m, 4H), 7.23-7.15 (m, 4H), 5.19 (s, 0.5H), 5.07 (s, 0.5H), 4.47-4.32 (m, 4H), 3.98 (s, 0.5H), 3.91 (s, 0.5H), 3.65-3.47 (m, 4H), 3.04-2.98 (m,

1H), 2.78-2.70 (m, 1H), 2.56-2.50 (m, 1H), 2.17-2.12 (m, 1H), 1.99-1.87 (m, 3H), 1.76-1.60 (m, 5H).

**Example 145**

4-(4-((1R,5S)-3,8-diazabicyclo[3.2.1]octan-3-yl)-2-((3-(4,4-difluorophenethylamin-6-yl )tetrahydro-1H-pyrrolizin-7a(5H)-yl)methoxy)-8-fluoropyridin[4,3-d]pyrimidin-7-yl)-5,6-difluor onaphthalen-2-ol

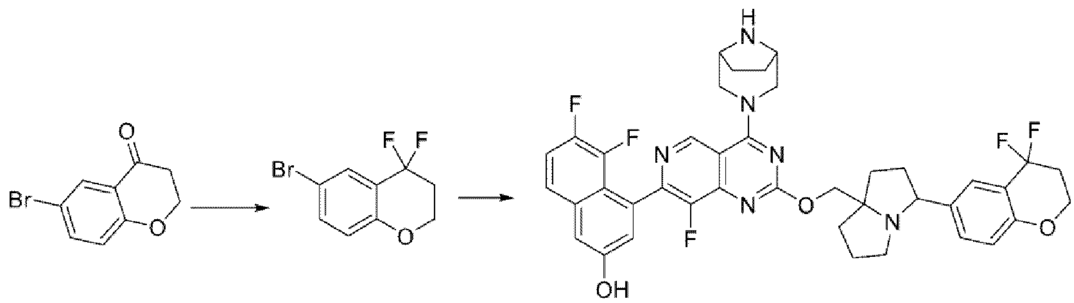

Step 1: Synthesis of 7-bromo-1,1-difluorochromane

6-Bromo-4-dihydrochromanone (5.0 g, 22.03 mmol) and 20 mL of BAST were added to a reaction flask, and the mixture was stirred overnight at 70°C. The reaction solution was poured into 200 mL of ice water and extracted with ethyl acetate. The organic phase was dried and evaporated under reduced pressure. The crude product was purified by column chromatography (PE\EA=8\1) to obtain 2.1 g of light yellow oil, with a yield of 38.2%.

Using the above intermediate as raw materials, the target product was synthesized by a method similar to that in Example 34.

LC/MS: m/z=745.3 $[M+H]^+$.

**Example 146**

4-(4-((1R,5S)-3,8-diazabicyclo[3.2.1]octan-3-yl)-2-((3-(1,1-dimethyl-1,3-dihydroisoben zofuran-5-yl)tetrahydro-1H-pyrrolizin-7a(5H)-yl)methoxy)-8-fluoropyrido[4,3-d]pyrimidin-7-yl) -5,6-difluoronaphthalen-2-ol

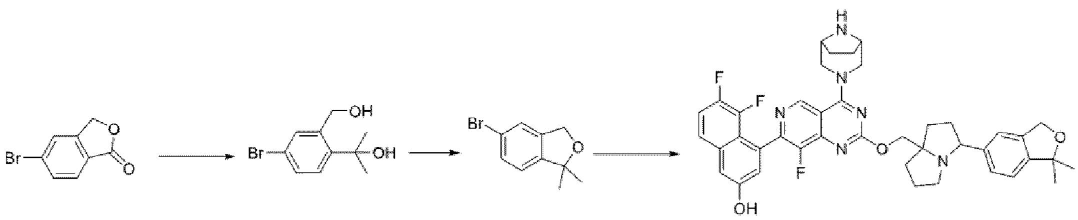

Step 1: Synthesis of 2-(4-bromo-2-(hydroxymethyl)phenyl)propan-2-ol

5-Bromobenzofuran-1(3H)-one (6.4 g, 30.00 mmol) and 50 mL of tetrahydrofuran were added to a three-necked reaction flask. Methylmagnesium bromide (60 mL, 120.00 mmol) was then added at 0°C. The mixture was heated to reflux and reacted overnight. The reaction solution was poured into 200 mL of saturated ammonium chloride, and extracted with ethyl acetate. The organic phase was dried and evaporated under reduced pressure. The crude product was purified by column chromatography (PE\EA=2\1) to obtain 2.5 g of light yellow solid, with a yield of 34%.

Step 2: Synthesis of 5-bromo-1,1-dimethyl-1,3-dihydroisobenzofuran

2-(4-Bromo-2-(hydroxymethyl)phenyl)propan-2-ol (2.5 g, 10.20 mmol) and 20 mL of toluene were added to a reaction flask. Phosphoric acid (1.5 g, 15.30 mmol) was then added at room temperature, and the reaction mixture was heated to 80°C and stirred for 2 h. The reaction system was cooled to room temperature, poured into 50 mL of water and

extracted with ethyl acetate. The organic phase was dried and evaporated under reduced pressure. The crude product was purified by column chromatography (PE\EA=10\1) to obtain 1.5 g of off-white solid, with a yield of 65.2%.

Using the above intermediate as raw materials, the target product was synthesized by a method similar to that in Example 34.

LC/MS: m/z=723.3 [M+H]+.

### Example 147

4-(4-((1R,5S)-3,8-diazabicyclo[3.2.1]octan-3-yl)-2-((3-(4-(difluoro(tetrahydro-2H-pyra n-4-yl)methyl)phenyl)tetrahydro-1H-pyrrolizin-7a(5H)-yl)methoxy)-8-fluoropyrido[4,3-d]pyrimi din-7-yl)-5,6-difluoronaphthalen-2-ol

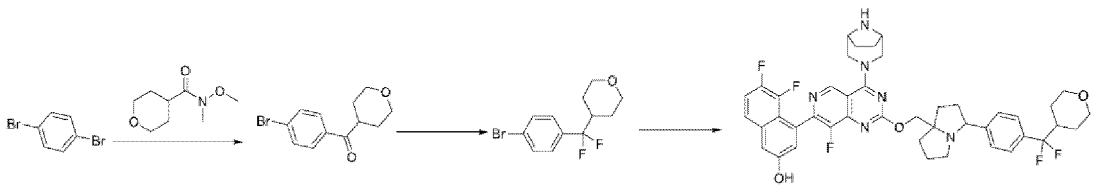

Step 1: Synthesis of (4-bromophenyl)(tetrahydro-2H-pyran-4-yl)methanone

1,4-Dibromobenzene (5.0 g, 21.2 mmol) and 30 mL of tetrahydrofuran were added to a reaction flask. At -78°C, n-BuLi (2.5 M, 8.5 mL, 21.2 mmol) was added and stirred for 1 h. Then, N-methoxy-N-methyltetrahydro-2H-pyran-4-carboxamide (4.41 g, 25.44 mmol) was added and stirred for 1 h at -78°C. The reaction solution was poured into 100 mL of saturated ammonium chloride aqueous solution, and extracted with ethyl acetate. The organic phase was dried and evaporated under reduced pressure. The crude product was purified by column chromatography (PE\EA=3\1) to obtain 4.5 g of off-white solid, with a yield of 79.8%.

Step 2: Synthesis of 4-((4-bromophenyl)difluoromethyl)tetrahydro-2H-pyran

(4-Bromophenyl)(tetrahydro-2H-pyran-4-yl)methanone (4.5 g, 16.72 mmol) and 20 mL of BAST were added to a reaction flask, and the mixture was stirred overnight at 70°C. The reaction solution was poured into 200 mL of ice water and extracted with ethyl acetate. The organic phase was dried and evaporated under reduced pressure. The crude product was purified by column chromatography (PE\EA=8\1) to obtain 3.1 g of light yellow oil, with a yield of 63.7%.

Using the above intermediate as raw materials, the target product was synthesized by a method similar to that in Example 34.

LC/MS: m/z=787.3 [M+H]+.

### Example 148

4-(4-((1R,5S)-3,8-diazabicyclo[3.2.1]octan-3-yl)-2-((3-(1,3-dihydroisobenzofuran-5-yl-1,1,3,3-d4)tetrahydro-1H-pyrrolizin-7a(5H)-yl)methoxy)-8-fluoropyridin[4,3-d]pyrimidin-7-yl)n aphthalen-2-ol

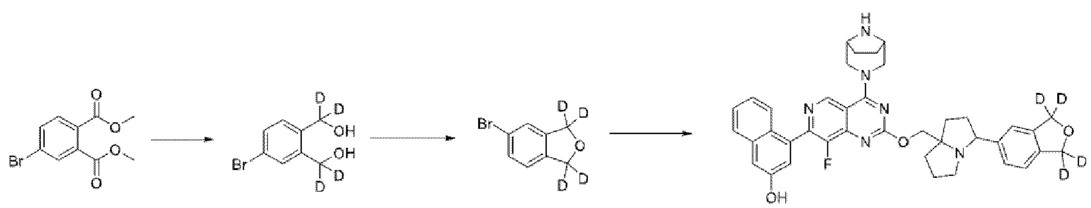

Step 1: Synthesis of (4-bromo-1,2-phenylene)bis(methan-d2-ol)

Dimethyl 4-bromophthalate (5.0 g, 18.32 mmol) and 50 mL of tetrahydrofuran were added to a reaction flask, and $LiAlD_4$ (1.04 g, 27.47 mmol) was added at 0°C. The mixture was stirred at 0°C for 1 h. After the reaction was completed,

under an ice bath condition, 1 mL of water and 1 mL of 15% sodium hydroxide aqueous solution were added dropwise to the system, stirred for 5 min, and then 3 mL of water was added. An appropriate amount of anhydrous magnesium sulfate was added, and the mixture was stirred at room temperature for 15 min. The system was subjected to suction filtration, and the filtrate was concentrated to obtain 3.5 g of off-white solid, with a yield of 86%.

Step 2: Synthesis of 5-bromo-1,3-dihydroisobenzofuran-1,1,3,3-d4

(4-Bromo-1,2-phenylene)bis(methane-d2-ol) (3.5 g, 15.84 mmol) and 30 mL of toluene were added to a reaction flask, and phosphoric acid (2.33 g, 23.76 mmol) was added at room temperature. The reaction was heated to 80°C and stirred for 2 h. The reaction system was cooled to room temperature, poured into 80 mL of water and extracted with ethyl acetate. The organic phase was dried and evaporated under reduced pressure. The crude product was purified by column chromatography (PE\EA=10\1) to obtain 2.3 g of off-white solid, with a yield of 72%.

Using the above intermediate as raw materials, the target product was synthesized by a method similar to that in Example 34.

LC/MS: m/z=663.3 $[M+H]^+$.

**Example 149**

4-(4-((1R,5S)-3,8-diazabicyclo[3.2.1]octan-3-yl)-8-fluoro-2-((3-(1,1,3,3-tetramethyl-1,3 -dihydroisobenzofuran-5-yl)tetrahydro-1H-pyrrolizin-7a(5H)-yl)methoxy)pyrido[4,3d]pyrimidin-7-yl)naphthalen-2-ol

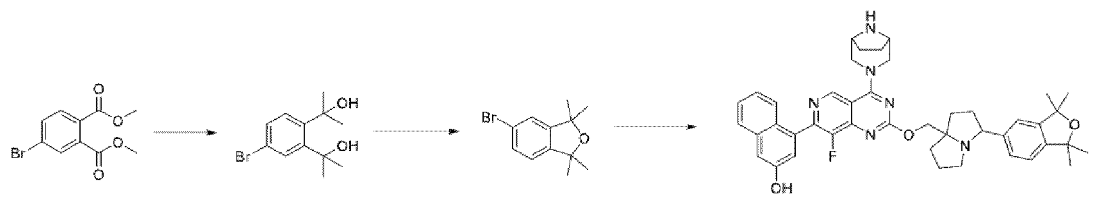

Using the starting materials shown in the synthetic route as raw materials, the target product was synthesized by a method similar to that in Example 34 and Example 146.

LC/MS: m/z=715.4 $[M+H]^+$.

**Example 150**

4-(4-((1R,5S)-3,8-diazabicyclo[3.2.1]octan-3-yl)-8-fluoro-2-((3-(2-methoxy-2,3-dihydr o-1H-inden-5-yl)tetrahydro-1H-pyrrolizin-7a(5H)-yl)methoxy)pyrido[4,3-d]pyrimidin-7-yl)napht halen-2-ol

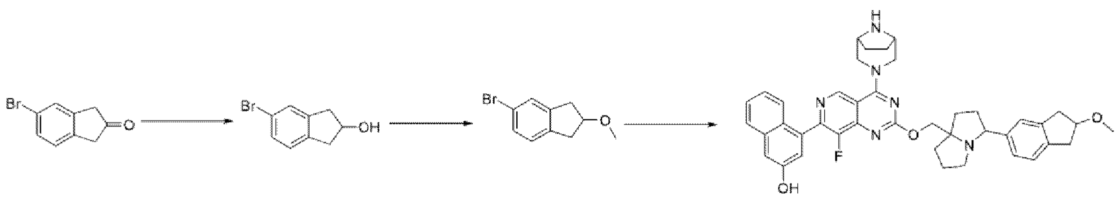

Step 1: Synthesis of 5-bromo-2,3-dihydro-1H-inden-2-ol

5-Bromo-1,3-dihydro-2H-inden-2-one (5.0 g, 23.70 mmol) and 30 mL of methanol were added to a reaction flask. NaBH4 (1.08 g, 28.44 mmol) was added at 0°C, and the mixture was stirred at room temperature for 2 h. The system was quenched by adding diluted hydrochloric acid, concentrated to remove methanol, and then extracted with water and ethyl acetate. The organic phase was concentrated to dryness to obtain 4.6 g of off-white solid with a yield of 92%.

Step 2: Synthesis of 5-bromo-2-methoxy-2,3-dihydro-1H-indene

5-Bromo-2,3-dihydro-1H-inden-2-ol (4.6 g, 21.60 mmol), potassium carbonate (8.94 g, 64.79 mmol), and DMF (50 mL) were added to a reaction flask, and iodomethane (6.13 g, 43.20 mmol) was added at room temperature. The reaction mixture was heated to 80°C under nitrogen protection and stirred for 16 h. The reaction system was cooled to room temperature, poured into 10 mL of water and extracted with ethyl acetate. The organic phase was dried and evaporated under reduced pressure. The crude product was purified by column chromatography (PE\EA=5\1) to obtain 2.8 g of colorless oil, with a yield of 57.1%.
Using the above intermediate as raw materials, the target product was synthesized by a method similar to that in Example 34.
LC/MS: m/z=687.3 $[M+H]^+$.

**Example 151**

4-(4-((1R,5S)-3,8-diazabicyclo[3.2.1]octan-3-yl)-2-((3-(1,3-dihydroisobenzofuran-5-yl-1,1-d2)tetrahydro-1H-pyrrolizin-7a(5H)-yl)methoxy)-8-fluoropyrido[4,3-d]pyrimidin-7-yl)-5,6-di fluoronaphthalen-2-ol

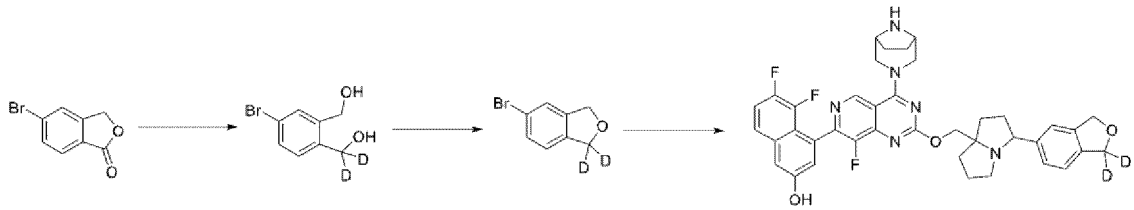

Using the starting material shown in the synthetic route as raw materials, the target product was synthesized by a method similar to that in Example 34 and Example 148.
LC/MS: m/z=697.3 $[M+H]^+$.
$^1$H NMR (400 MHz, $CDCl_3$) δ 9.13 (s, 1H), 8.03 (s, 1H), 8.00-7.97 (m, 1H), 7.79-7.73 (m, 1H), 7.57 (d, 1H, *J* = 6.0 Hz), 7.35-7.32 (m, 2H), 7.05-6.97 (m, 2H), 4.44-4.4 (m, 2H), 4.32-4.18 (m, 2H), 3.76-3.45 (m, 12H), 2.76-2.74 (m, 1H), 2.17-2.11 (m, 2H), 1.88-1.57 (m, 6H).

**Example 152**

4-(4-((1R,5S)-3,8-diazabicyclo[3.2.1]octan-3-yl)-8-fluoro-2-((3-(2-fluoro-2,3-dihydro-1 H-inden-5-yl)tetrahydro-1H-pyrrolizin-7a(5H)-yl)methoxy)pyrido[4,3-d]pyrimidin-7-yl)naphthal en-2-ol

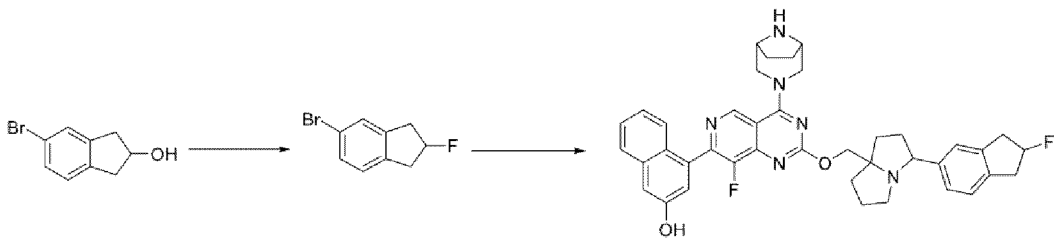

Step 1: Synthesis of 5-bromo-2-fluoro-2,3-dihydro-1H-indene

5-Bromo-2,3-dihydro-1H-inden-2-ol (3.0 g, 14.08 mmol) and 25 mL of dichloromethane were added to a reaction flask. DAST (3.4 g, 21.13 mmol) was added at 0°C, and the mixture was stirred at room temperature for 4 h. The reaction solution was poured into 100 mL of water and extracted with dichloromethane. The organic phase was dried and evaporated under reduced pressure. The crude product was purified by column chromatography (PE\EA=5\1) to obtain 1.2 g of colorless oil, with a yield of 39.6%.
Using the above intermediate as raw materials, the target product was synthesized by a method similar to that in

Example 34.

LC/MS: m/z=675.3 [M+H]+.

**Example 153**

4-(4-((1R,5S)-3,8-diazabicyclo[3.2.1]octan-3-yl)-8-fluoro-2-((3-(1,2,3,4-tetrahydro-1,4-epoxynaphthalen-6-yl)tetrahydro-1H-pyrrolizin-7a(5H)-yl)methoxy)pyrido[4,3d]pyrimidin-7-yl)-5,6-difluoronaphthalen-2-ol

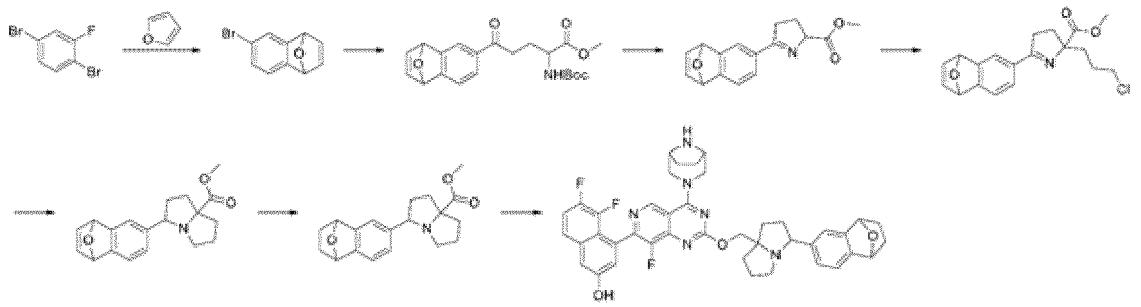

Step 1: Synthesis of 6-bromo-1,4-dihydro-1,4-epoxynaphthalene

1,4-Dibromo-2-fluorobenzene (50 g, 0.20 mol) and furan (40 g, 0.59 mol) were dissolved in toluene (150 mL). After replacement with nitrogen for three times, the reaction system was cooled to -30°C, and then n-butyllithium (80 mL, 2.5M) was slowly added dropwise. Afterwards, the mixture was warmed to room temperature and stirred for 0.5 h. The reaction mixture was poured into a saturated aqueous solution of ammonium chloride and extracted twice with ethyl acetate. The organic phases were combined, washed once with saturated brine and dried over anhydrous sodium sulfate. After filtration, the solution was concentrated under reduced pressure to dryness and purified by column chromatography (PE/EA = 10:1) to obtain 30 g of colorless oil, with a yield of 68%.

Step 2: Synthesis of methyl 2-((tert-butoxycarbonyl)amino)-5-(1,4-dihydro-1,4-epoxynaphthalen-6-yl)-5-oxopentanoate

6-Bromo-1,4-dihydro-1,4-epoxynaphthalene (30 g, 0.13 mol) was dissolved in tetrahydrofuran (150 mL). After replacement with nitrogen for three times, the reaction system was cooled to -78°C, and n-butyllithium (54 mL, 2.5M) was slowly added dropwise. Afterwards, the mixture was stirred at -78°C for 0.5 h and kept for future use.

Compound Boc-L-pyroglutamic acid methyl ester (33 g, 0.13 mol) was dissolved in tetrahydrofuran (330 mL). After replacement with nitrogen for three times, the reaction system was cooled to -78°C, and then the above reaction mixture was slowly added while maintaining the temperature below -55°C. Afterwards, the mixture was stirred at -78°C for 0.5 h. The reaction mixture was poured into a saturated aqueous solution of ammonium chloride and extracted twice with ethyl acetate. The organic phases were combined, washed once with saturated brine, dried over anhydrous sodium sulfate, filtered, and concentrated to dryness to obtain 60 g of a crude product as light yellow oil.

Step 3: Synthesis of methyl 5-(1,4-dihydro-1,4-epoxynaphthalen-6-yl)-3,4-dihydro-2H-pyrrole-2-carboxylate

The crude product obtained from the previous step was dissolved in dichloromethane (60 mL), and trifluoroacetic acid (60 mL) was slowly added under an ice-water bath. Afterwards, the mixture was warmed to room temperature and stirred for 1 h. It was then concentrated under reduced pressure to dryness. The crude product was adjusted to pH 7-8 with an aqueous solution of sodium bicarbonate and extracted with ethyl acetate three times. The organic phases were combined, washed once with saturated brine, dried over anhydrous sodium sulfate, filtered, concentrated to dryness, and purified by column chromatography (PE/EA = 3:1) to obtain 11 g of light yellow oil, with a yield of 30% (two steps).

Step 4: Synthesis of methyl 2-(3-chloropropyl)-5-(1,4-dihydro-1,4-epoxynaphthalen-6-yl)-3,4-dihydro-2H-pyrrole-2-carboxyl ate

Methyl 5-(1,4-dihydro-1,4-epoxynaphthalen-6-yl)-3,4-dihydro-2H-pyrrole-2-carboxylate (4 g, 14.9 mmol) and 1-bromo-3-chloropropane (4 g, 22.3 mmol) were dissolved in THF/DMF (36 mL/3.6 mL). After replacement with nitrogen for

three times, sodium hydride (892 mg, 22.3 mmol) was slowly added. Afterwards, the mixture was stirred at room temperature for 1 h. The reaction system was then poured into a saturated aqueous solution of ammonium chloride and extracted twice with ethyl acetate. The organic phases were combined, washed once with saturated brine , dried over anhydrous sodium sulfate, filtered, concentrated to dryness, and purified by column chromatography (PE/EA = 5:1) to obtain 3.7 g of colorless oil, with a yield of 73%.

Step 5: Synthesis of methyl 3-(1,4-dihydro-1,4-epoxynaphthalen-6-yl)tetrahydro-1H-pyrrolizin-7a(5H)-carboxylate

Methyl 2-(3-chloropropyl)-5-(1,4-dihydro-1,4-epoxynaphthalen-6-yl)-3,4-dihydro-2H-pyrrole-2-carboxyl ate (3.7 g, 10.7 mmol) was dissolved in methanol (37 mL). Ice acetic acid (965 mg, 16.0 mmol) and sodium cyanoborohydride (1.0 g, 16.0 mmol) were added sequentially, and the mixture was stirred at room temperature for 1 h. The reaction mixture was quenched with an aqueous solution of sodium bicarbonate. Most of the methanol was removed by concentration under reduced pressure. The crude product was extracted three times with water and ethyl acetate. The organic phases were combined, washed once with saturated brine , dried over anhydrous sodium sulfate, filtered, concentrated to dryness, and purified by column chromatography (PE/EA = 1:1) to collect a lower spot to obtain 1.6 g of colorless oil, with a yield of 50%.

Step 6: Synthesis of methyl 3-(1,2,3,4-tetrahydro-1,4-epoxynaphthalen-6-yl)tetrahydro-1H-pyrrolizin-7a(5H)-carboxylate

Methyl 3-(1,4-dihydro-1,4-epoxynaphthalen-6-yl)tetrahydro-1H-pyrrolizin-7a(5H)-carboxylate (3.5 g, 11.2 mmol) was dissolved in ice acetic acid (18 mL). After replacement with nitrogen for three times, palladium carbon 10% (1.0 g) was added. After replacement with hydrogen for three times, the mixture was stirred at room temperature for 1 h with a hydrogen balloon. The reaction mixture was filtered through a pad of celite, concentrated to dryness, adjusted to alkaline pH with an aqueous solution of sodium bicarbonate, and extracted three times with ethyl acetate. The organic phases were combined, washed once with saturated brine, dried over anhydrous sodium sulfate, filtered, concentrated to dryness, and separated by reverse phase separation to obtain 1.6 g of light yellow oil, with a yield of 46%.

Step 4: Synthesis of 4-(4-((1R,5S)-3,8-diazabicyclo[3.2.1]octan-3-yl)-8-fluoro-2-((3-(1,2,3,4-tetrahydro-1,4-epoxynap hthalen-6-yl)tetrahydro-1H-pyrrolizin-7a(5H)-yl)methoxy)pyrido[4,3d]pyrimidin-7-yl)-5,6-difluo ronaphthalen-2-ol

Using the above intermediate as raw materials, the target product was synthesized by a method similar to that in Example 34.

LC/MS: m/z=721.3 $[M+H]^+$.

$^1H$ NMR (400 MHz, $CDCl_3$) δ 10.27 (s, 1H), 9.13 (s, 1H), 7.77-7.73 (m, 1H), 7.61-7.55 (m, 1H), 7.40 (s, 1H), 7.28-7.21 (m, 2H), 7.11-7.04 (m, 2H), 5.32-5.22 (m, 2H), 4.45-4.18 (m, 5H), 3.74-3.71 (m, 1H), 3.65-3.56 (m, 4H), 2.77-2.74 (m, 1H), 2.18-2.08 (m, 2H), 1.91-1.66 (m, 14H).

**Example 154**

4-(4-((1R,5S)-3,8-diazabicyclo[3.2.1]octan-3-yl)-8-fluoro-2-((3-(spiro[cyclopropan-1,4' -isochromane]-6'-yl)tetrahydro-1H-pyrrolizin-7a(5H)-yl)methoxy)pyrido[4,3d]pyrimidin-7-yl)-5, 6-difluoronaphthalen-2-ol

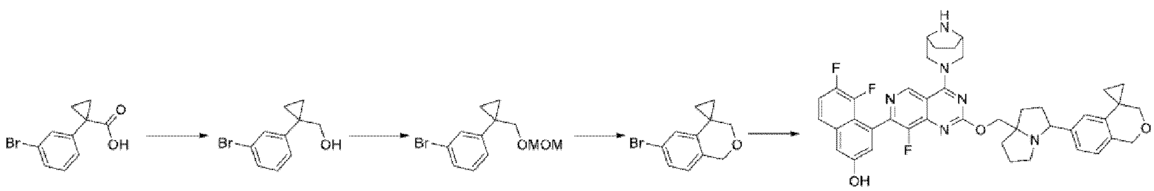

Step 1: Synthesis of 1-(3-bromophenyl)cyclopropyl)methanol

1-(3-Bromophenyl)cyclopropan-1-formic acid (4.82 g, 20.00 mmol) and 20 mL of tetrahydrofuran were added to a reaction flask, and $BH_3$-THF (1 M, 30 mL) was added at 0°C. The mixture was stirred at room temperature for 4 h. Methanol was added to quench the reaction. The system was concentrated to dryness to obtain a crude product, which was directly used for the next step of reaction.

Step 2: Synthesis of 1-bromo-3-(1-((methoxymethoxy)methyl)cyclopropyl)benzene

The crude product mentioned above was dissolved in 30 mL of dichloromethane. TEA (6.06 g, 60.00 mmol) was added at room temperature, and MOMOCl (2.42 g, 30.00 mmol) was added at 0°C. The reaction mixture was stirred for 8 h at room temperature under nitrogen protection. The reaction system was cooled to room temperature, poured into 100 mL of water and extracted with dichloromethane. The organic phase was backwashed twice with dilute hydrochloric acid, dried, and evaporated under reduced pressure to obtain a crude product, which was directly used for the next step of reaction.

Step 3: Synthesis of 6'-bromospiro[cyclopropan-1,4'-isochromane]

The crude product obtained in step 2 was dissolved in 30 mL of DCM, and TMSOTf (13.33 g, 60.00 mmol) was added at 0°C and stirred for 2 h. The reaction mixture was directly evaporated under reduced pressure, and the crude product was purified by column chromatography (PE/EA = 5/1) to obtain 1.8 g of light yellow oil, with a yield of 37.5% (3 steps).

Using the above intermediate as raw materials, the target product was synthesized by a method similar to that in Example 34.

LC/MS: m/z=735.3 $[M+H]^+$.

**Example 155**

5-(7a-(((4-((1R,5S)-3,8-diazabicyclo[3.2.1]octan-3-yl)-7-(7,8-difluoro-3-hydroxynaphth alen-1-yl)-8-fluoropyridin[4,3-d]pyrimidin-2-yl)oxy)methyl)hexahydro-1H-pyrrolizin-3-yl)isobe nzofuran-1(3H)-one

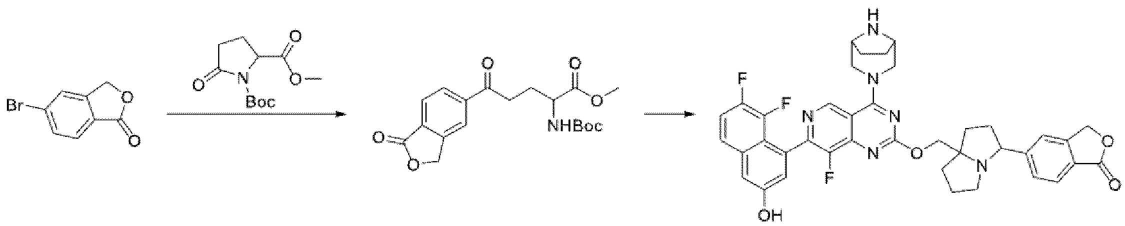

Step 1: Synthesis of methyl 2-((tert-butoxycarbonyl)amino)-5-oxo-5-(1-oxo-1,3-dihydroisobenzofuran-5-yl)pentanoate

5-Bromobenzofuran-1(3H)-one (2.1 g, 10.0 mmol), 1-(tert-butyl) 2-methyl 5-oxopyrrolidin-1,2-dicarboxylate (2.4 g, 10.0 mmol), and 20 mL of super-dry tetrahydrofuran were added to a reaction flask. N-butyllithium (2.5 M, 4.0 mL, 10.0 mmol) was added dropwise at 0°C, and then stirred at 0°C for 1 h. The reaction mixture was poured into 100 mL of saturated aqueous solution of ammonium chloride, and extracted with ethyl acetate. The organic phase was dried, evaporated under reduced pressure, and purified by column chromatography (PE/EA = 5/1) to obtain 160 mg of yellow solid, with a yield of 4%.

Step 2: Synthesis of 5-(7a-(((4-((1R,5S)-3,8-diazabicyclo[3.2.1]octan-3-yl)-7-(7,8-difluoro-3-hydroxynaphthalen-1-yl )-8-fluoropyridine[4,3-d]pyrimidin-2-yl)oxy)methyl)hexahydro-1H-pyrrolizin-3-yl)isobenzofuran -1(3H)-one

Using the product obtained from the previous step as raw materials, the target product was synthesized by a method similar to that in Example 34.

LC/MS: m/z=709.3 $[M+H]^+$.

**Example 156**

4-(4-((1R,5S)-3,8-diazabicyclo[3.2.1]octan-3-yl)-2-((3-(3,3-dimethyl-1,3-dihydroisoben zofuran-5-yl)tetrahydro-1H-pyrrolizin-7a(5H)-yl)methoxy)-8-fluoropyridin[4,3-d]pyrimidin-7-yl) -5,6-difluoronaphthalen-2-ol

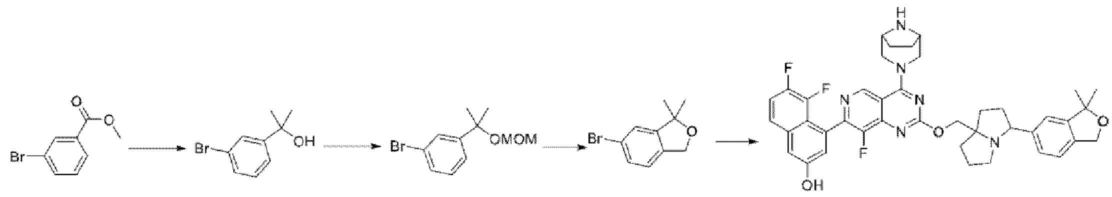

Using the starting material shown in the synthetic route as raw materials, the target product was synthesized by a method similar to that in Example 34, Example 146 and Example 154.
LC/MS: m/z=723.3 [M+H]$^+$.
$^1$H NMR (400 MHz, $CDCl_3$) δ 10.28 (brs, 1H), 9.12 (s, 1H), 7.77-7.73 (m, 1H), 7.61-7.55 (m, 1H), 7.30 (s, 1H), 7.25-7.21 (m, 2H), 7.16 (1H, d, $J$ = 2.8 Hz), 7.07-7.04 (m, 1H), 4.88 (s, 2H), 4.49-4.31 (m, 3H), 4.25-4.18 (m, 1H), 3.79-3.74 (m, 1H), 3.65-3.55 (m, 4H), 2.78 (brs, 1H), 2.56-2.50 (m, 1H), 2.18-2.10 (m, 2H), 1.92-1.80 (m, 4H), 1.79-1.63 (m, 6H), 1.34 (s, 6H).

**Example 157**

4-(4-((1R,5S)-3,8-diazabicyclo[3.2.1]octan-3-yl)-8-fluoro-2-((3-(1,3,4,5-tetrahydrobenz o[c]oxetan-7-yl)tetrahydro-1H-pyrrolizin-7a(5H)-yl)methoxy)pyrido[4,3-d]pyrimidin-7-yl]-5,6-d ifluoronaphthalen-2-ol

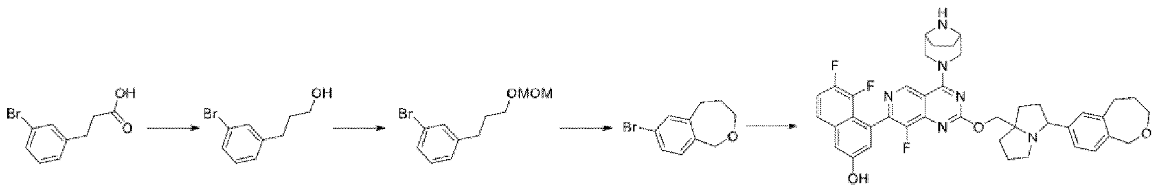

Using the starting material shown in the synthetic route as raw materials, the target product was synthesized by a method similar to that in Example 34 and Example 154.
LC/MS: m/z=723.3 [M+H]$^+$.

**Example 158**

4-(4-((1R,5S)-3,8-diazabicyclo[3.2.1]octan-3-yl)-8-fluoro-2-((3-(4-fluorophenyl)tetrahy dro-1H-pyrrolizin-7a(5H)-yl-3d) methoxy)pyrido[4,3-d]pyrimidin-7-yl)-5,6-difluoronaphthalen-2 -ol

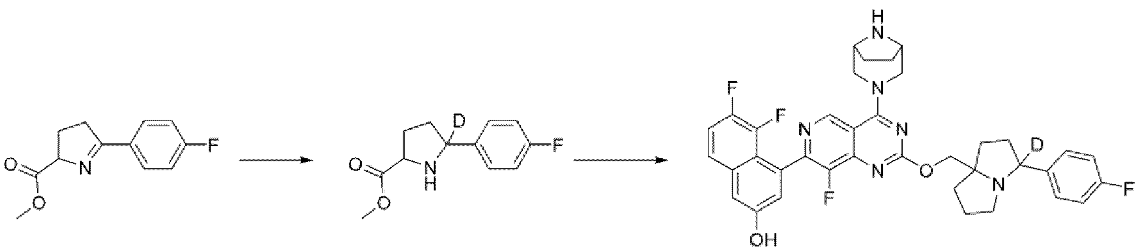

Step 1: Synthesis of methyl 5-(4-fluorophenyl)pyrrolidin-2-carboxylate-5-d

Methyl 5-(4-fluorophenyl)-3,4-dihydro-2H-pyrrole-2-carboxylate (2.21 g, 10.00 mmol) and 20 mL of methanol were added to a reaction flask. At 0°C, acetic acid (0.9 g, 15.00 mmol) and $NaBD_3CN$ (0.99 g, 15.00 mmol) were added. The mixture was stirred at room temperature for 2 h. The reaction mixture was poured into 100 mL of water adjusted to about pH 9 with sodium carbonate, and extracted with ethyl acetate. The organic phase was dried and evaporated under reduced pressure to obtain a crude product, which was directly used for the next step of the reaction.
Using the above intermediate as raw materials, the target product was synthesized by a method similar to that in

Example 34.

LC/MS: m/z=672.3 $[M+H]^+$.

**Example 159**

4-(4-((1R,5S)-3,8-diazabicyclo[3.2.1]octan-3-yl)-8-fluoro-2-((3-(4-fluorophenyl)-3-met hyltetrahydro-1H-pyrrolizin-7a(5H)-yl)methoxy)pyrido[4,3d]pyrimidin-7-yl)-5,6-difluoronaphth alen-2-ol (Trans racemate)

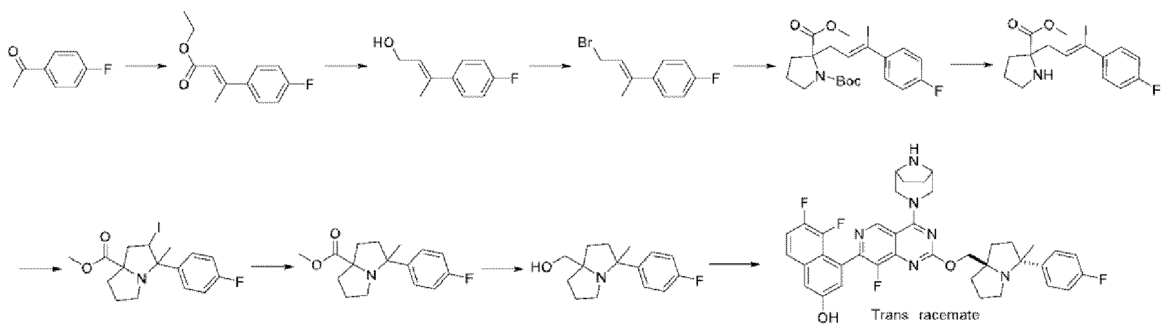


Step 1: Synthesis of (E)-3-(4-fluorophenyl)but-2-enoic acid ethyl ester

Ethyl 2-(dimethoxyphosphoryl) acetate (14.7 g, 75.0 mmol) and 50 mL of tetrahydrofuran were added to a reaction flask. NaH (60%, 3.2 g, 80 mmol) was added in batches at 0°C, and the mixture was stirred at room temperature for 1 h. 4-Fluoroacetophenone (7.0 g, 50.7 mmol) was added, and the reaction mixture was stirred at room temperature for 16 h. The reaction mixture was poured into 200 mL of saturated aqueous solution of ammonium chloride, and extracted with ethyl acetate. The organic phase was dried and evaporated under reduced pressure to obtain a crude product, which was directly used for the next step of reaction.

Step 2: Synthesis of (E)-3-(4-fluorophenyl)but-2-en-1-ol

The crude product mentioned above was dissolved in 50 mL of tetrahydrofuran, and $LiAlH_4$ (1.91 g, 50.0 mmol) was added under an ice bath. The reaction mixture was stirred at room temperature for 2 h. After the reaction was complete, 1.9 mL of water and 1.9 mL of 15% aqueous solution of sodium hydroxide were added dropwise to the system under an ice bath, stirred for 5 min, and then 5.7 mL of water was added. An appropriate amount of anhydrous magnesium sulfate was added, and the mixture was stirred at room temperature for 15 min. The system was subjected to suction filtration, and the filtrate was concentrated to dryness. The crude product was purified by column chromatography (PE \EA=2\1) to obtain 3.5 g of off-white solid with a yield of 42.2% (2 steps).

Step 3: Synthesis of (E)-1-(4-bromobut-2-en-2-yl)-4-fluorobenzene

(E)-3-(4-fluorophenyl)but-2-en-1-ol (3.5 g, 21.06 mmol) was dissolved in 30 mL of diethyl ether. Under nitrogen protection, phosphorus tribromide (6.84 g, 25.27 mmol) was added under an ice bath, and the reaction mixture was stirred at room temperature for 15 min. The reaction system was poured into 100 mL of ice water and extracted with petroleum ether. The organic phase was dried and evaporated under reduced pressure. The crude product was directly used for the next step of reaction.

Step 4: Synthesis of 1-(tert-butyl)2-methyl(E)-2-(3-(4-fluorophenyl)but-2-en-1-yl)pyrrolidin-1,2-dicarboxylate

Boc-L-proline methyl ester (4.13 g, 18.00 mmol) was dissolved in 40 mL of dry tetrahydrofuran, and LDA (2M, 13.5 mL, 27.00 mmol) was added dropwise under nitrogen protection at -78°C. The mixture was stirred for 30 min. The crude product obtained in step 3 was dissolved in 10 mL of tetrahydrofuran, and then it was added dropwise to the reaction system at -78°C. The reaction was stirred for 2 h. The reaction system was directly poured into a saturated aqueous solution of ammonium chloride, and extracted with ethyl acetate. The organic phase was dried and concentrated to obtain a crude product, which was directly used for the next step of reaction.

Step 5: Synthesis of (E)-methyl -2-(3-(4-fluorophenyl)but-2-en-1-yl)pyrrolidin-2-carboxylate

The crude product obtained in step 4 was dissolved in 40 mL of hydrochloric acid ethyl acetate solution and stirred at room temperature for 1 h. The reaction system was directly concentrated to dryness and then water was added. The aqueous phase was first extracted twice with ethyl acetate, then adjusted to alkaline with sodium carbonate, and then extracted with ethyl acetate again. After adjusting the pH to alkaline, the organic phase was extracted, dried and concentrated to obtain 1.5 g of pure product, with a yield of 30% (2 steps).

Step 6: Synthesis of methyl 3-(4-fluorophenyl)-2-iodo-3-methyltetrahydro-1H-pyrrolizin-7a(5H)-carboxylate

(E)-methyl-2-(3-(4-fluorophenyl)but-2-en-1-yl)pyrrolidin-2-carboxylate (1.5 g, 5.41 mmol) was dissolved in 20 mL of ethyl acetate. Elemental iodine (1.98 g, 8.11 mmol) was added at 0°C, and then the reaction mixture was stirred at room temperature for 2 h. The reaction system was directly poured into water and extracted with ethyl acetate. The organic phase was washed twice with an appropriate amount of sodium hydrogen sulfite aqueous solution, dried and concentrated to obtain a crude product, which was directly used for the next step of reaction.

Step 7: Synthesis of methyl 3-(4-fluorophenyl)-3-methyltetrahydro-1H-pyrrolizin-7a(5H)-carboxylate

The crude product obtained in step 6 was dissolved in 20 mL of toluene, and tributyltin hydride (3.15 g, 10.82 mmol) and AIBN (88 mg, 0.54 mmol) were added at room temperature. The reaction mixture was heated to 80°C and stirred for 2 h. The reaction system was directly poured into water and extracted with ethyl acetate. The organic phase was dried and concentrated to obtain a crude product. It was then purified and eluted to obtain about 0.6 g of a cis-intermediate as a light yellow oil by column chromatography (PE\EA=8\1) and about 0.5 g of a trans-intermediate as light yellow solid by column chromatography (PE\EA=2\1), with a total yield of 73.3% (2 steps).

Using the above-mentioned trans intermediate as raw materials, the target product was synthesized by a method similar to that in Example 140.

LC/MS: m/z=685.3 $[M+H]^+$.

$^1$H NMR (400 MHz, $CDCl_3$) δ 8.93 (s, 1H), 7.48-7.44 (m, 2H), 7.37-7.33 (m, 1H), 7.25-7.17 (m, 3H), 7.04-6.99 (m, 2H), 4.68-4.41 (m, 5H), 3.74-3.52 (m, 4H), 2.88-2.82 (m, 1H), 2.37-2.23 (m, 4H), 2.11-1.93 (m, 4H), 1.87-1.59 (m, 7H).

**Example 160**

4-((3R,7aR)-7a-(((4-((1R,5S)-3,8-diazabicyclo[3.2.1]octan-3-yl)-7-(7,8-difluoro-3-hydr oxynaphthalen-1-yl)-8-fluoropyridin[4,3-d]pyrimidin-2-yl)oxy)methyl)hexahydro-1H-pyrrolizin-3-yl)phenyldimethylcarbamate (Trans racemate)

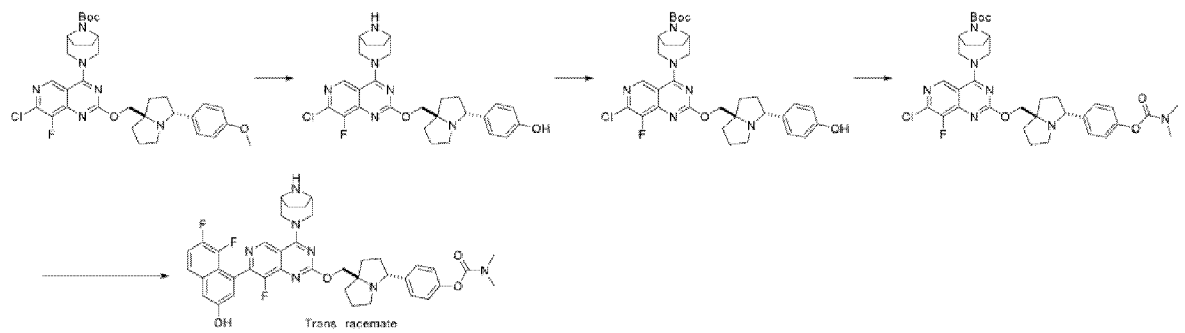

Step 1: Synthesis of 4-((3R,7aR)-7a-(((4-((1R,5S)-3,8-diazabicyclo[3.2.1]octan-3-yl)-7-chloro-8-fluoropyrido[4,3-d]p yrimidin-2-yl)oxy)methyl)hexahydro-1H-pyrrolizin-3-yl)phenol

Tert-butyl (1R,5S)-3-(7-chloro-8-fluoro-2-((3R,7aR)-3-(4-methoxyphenyl)tetrahydro-1H-pyrrolizin-7a(5H)-yl) methoxy)pyrido[4,3-d]pyrimidin-4-yl)-3,8-diazabicyclo[3.2.1]octan-8-carboxylate (300 mg, 0.47 mmol) and 5 mL of dichloromethane were added to a reaction flask. $BBr_3$ (1M, 1.5 mL, 1.5 mmol) was added at 0°C, and the mixture was stirred at room temperature for 1 h. The reaction solution was poured into 20 mL of water and extracted with dichloromethane. The organic phase was dried and evaporated under reduced pressure to obtain a crude product, which was directly used for the next step of reaction.

Step 2: Synthesis of tert-butyl (1R,5S)-3-(7-chloro-8-fluoro-2-(((3R,7aR)-3-(4-hydroxyphenyl)tetrahydro-1H-pyrrolizin-7a(5H) -yl)methoxy)pyrido[4,3-d]pyrimidin-4-yl)-3,8-diazabicyclo[3.2.1]octan-8-carboxylate

The crude product mentioned above was dissolved in 5 mL of dichloromethane, and TEA (152 mg, 1.5 mmol) and $Boc_2O$ (110 mg, 0.5 mmol) were added at room temperature. The reaction mixture was stirred at room temperature for 1 h. The reaction system was directly concentrated to dryness and then purified by Prep-TLC (DCM/MeOH = 30/1) to obtain 120 mg of grey solid, with a yield of 40.8% (2 steps).

Step 3: Synthesis of tert-butyl (1R,5S)-3-(7-chloro-2-(((3R,7aR)-3-(4-((dimethylcarbamoyl)oxy)phenyl)tetrahydro-1H-pyrrolizi n-7a(5H)-yl)methoxy)-8-fluoropyridine[4,3-d]pyrimidin-4-yl)-3,8-diazabicyclo[3.2.1]octan-8-car boxylate

Tert-butyl (1R,5S)-3-(7-chloro-8-fluoro-2-(((3R,7aR)-3-(4-hydroxyphenyl)tetrahydro-1H-pyrrolizin-7a(5H) -yl) methoxy)pyrido[4,3-d]pyrimidin-4-yl)-3,8-diazabicyclo[3.2.1]octan-8-carboxylate (120 mg, 0.19 mmol) was dissolved in 3 mL of dichloromethane. DIEA (74 mg, 0.57 mmol) and dimethylcarbamoyl chloride (41 mg, 0.38 mmol) were added at room temperature, and the mixture was stirred overnight. The reaction system was directly concentrated to dryness and then purified by Prep-TLC (DCM/MeOH = 40/1) to obtain 60 mg of grey solid, with a yield of 45.1%.

Using the above intermediate as raw materials, the target product was synthesized by a method similar to that in Example 34.

LC/MS: m/z=740.3 $[M+H]^+$.

**Example 161**

3-(4-((1R,5S)-3,8-diazabicyclo[3.2.1]octan-3-yl)-2-((3-(2,2-difluorobenzo[d][1,3]dioxy -5-yl)tetrahydro-1H-pyrrolizin-7a(5H)-yl)methoxy)-8-fluoropyridin[4,3-d]pyrimidin-7-yl)-5-chlo ro-4-(trifluoromethyl) aniline (Trans racemate)

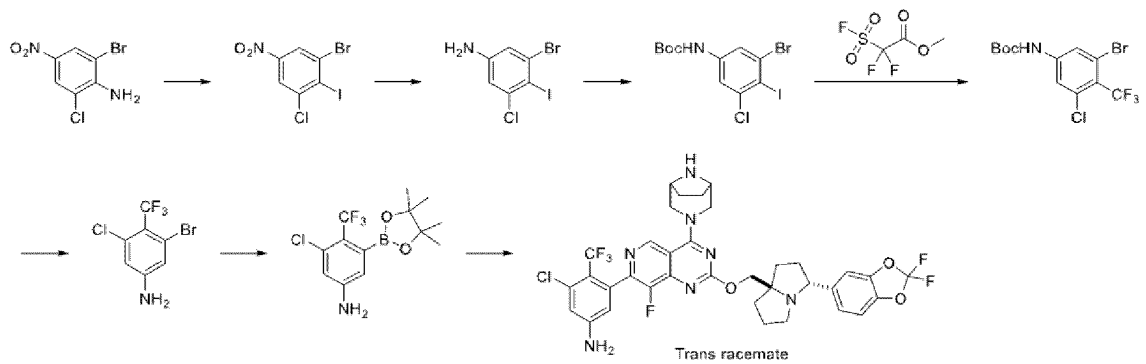


Step 1: Synthesis of 1-bromo-3-chloro-2-iodo-5-nitrobenzene

2-Bromo-6-chloro-4-nitroaniline (10 g, 40.0 mmol), potassium iodide (13.3 g, 80.1 mmol), cuprous iodide (15.2 g, 80.0 mmol), and 100 mL of acetonitrile were added to a reaction flask. Tert-butyl nitrite (5.5 g, 53.4 mmol) was added at room temperature, and the mixture was stirred at 80°C overnight. The reaction solution was poured into 500 mL of water and extracted with ethyl acetate. The organic phase was dried and evaporated under reduced pressure to obtain a crude product, which was directly used for the next step of reaction.

Step 2: Synthesis of 3-bromo-5-chloro-4-iodoaniline

1-Bromo-3-chloro-2-iodo-5-nitrobenzene (the crude product obtained in the previous step), 10 mL of concentrated hydrochloric acid, and 100 mL of ethanol were added to a reaction flask. Iron powder (6.7 g, 119.6 mmol) was added at room temperature, and the mixture was stirred at 80°C for 2 h. The reaction solution was poured into 500 mL of water, adjusted to alkaline pH with sodium hydroxide, and then extracted with ethyl acetate. The organic phase was dried and evaporated under reduced pressure to obtain a crude product, which was directly used for the next step of reaction.

Step 3: Synthesis of tert-butyl (3-bromo-5-chloro-4-iodophenyl) carbamate

3-Bromo-5-chloro-4-iodoaniline (the crude product obtained in the previous step), triethylamine (8.1 g, 80.2 mmol), DMAP (1.2 g, 9.8 mmol), and 100 mL of dichloromethane were added to a reaction flask. $Boc_2O$ (17.4 g, 80.0 mmol) was added at room temperature, and the mixture was stirred at 50°C overnight. The reaction solution was poured into 500 mL of water and extracted with ethyl acetate. The organic phase was dried, evaporated under reduced pressure, and then purified by column chromatography (DCM/MeOH = 50/1) to obtain 3.5 g of light yellow solid, with a three-step yield of 20%.

Step 4: Synthesis of tert-butyl (3-bromo-5-chloro-4-(trifluoromethyl)phenyl) carbamate

Tert-butyl (3-bromo-5-chloro-4-iodophenyl) carbamate (3.5 g, 8.1 mmol), methyl 2,2-difluoro-2-(fluorosulfonyl) acetate (3.1 g, 16.2 mmol), cuprous iodide (1 g, 5.3 mmol), and 50 mL of DMF were added to a reaction flask. The mixture was stirred at 90°C under nitrogen protection for 4 h. The reaction solution was poured into 200 mL of water and extracted with ethyl acetate. The organic phase was dried, evaporated under reduced pressure, and then purified by column chromatography (DCM/MeOH = 50/1) to obtain 1.6 g of light yellow solid, with a yield of 53%.

Step 5: Synthesis of 3-bromo-5-chloro-4-(trifluoromethyl) aniline

Tert-butyl (3-bromo-5-chloro-4-(trifluoromethyl)phenyl) carbamate (1.6 g, 4.3 mmol) and 20 mL of dichloromethane were added to a reaction flask. At room temperature, 2 mL of trifluoroacetic acid was added and stirred for 4 h. The reaction solution was poured into 10 mL of water, adjusted to alkaline pH with sodium hydroxide, and then extracted with ethyl acetate. The organic phase was dried and evaporated under reduced pressure to obtain 1.2 g of yellow solid, with a yield of 100%.

Step 6: Synthesis of 3-chloro-5-(4,4,5,5-tetramethyl-1,3,2-dioxaborolane-2-yl)-4-(trifluoromethyl) aniline

3-Bromo-5-chloro-4-(trifluoromethyl) aniline (1.2 g, 4.38 mmol), bis(pinacolato)diboron (1.3 g, 5.1 mmol), potassium acetate (1.0 g, 10.2 mmol), and 10 mL of dioxane were added to a reaction flask. After replacement with nitrogen, $Pd(dppf)Cl_2$ (220 mg, 0.3 mmol) was added. The reaction mixture was heated to 80°C under nitrogen protection and stirred overnight. The reaction system was cooled to room temperature, poured into 50 mL of water and extracted with ethyl acetate. The organic phase was dried, evaporated under reduced pressure, and then purified by silica gel column chromatography (PE/EA = 10/1) to obtain 830 mg of white solid, with a yield of 59%.

Step 7: Synthesis of 3-(4-((1R,5S)-3,8-diazabicyclo[3.2.1]octan-3-yl)-2-((3-(2,2-difluorobenzo[d][1,3]dioxy-5-yl)tetra hydro-1H-pyrrolizin-7a(5H)-yl)methoxy)-8-fluoropyridin[4,3-d]pyrimidin-7-yl)-5-chloro-4-(trifl uoromethyl) aniline (Trans racemate)

Using borate shown in the synthetic route as raw materials, the target product was synthesized by a method similar to that in Example 34.

LC/MS: m/z=748.2 $[M+H]^+$.

**Example 162**

3-(4-((1R,5S)-3,8-diazabicyclo[3.2.1]octan-3-yl)-2-((3-(2,2-difluorobenzo[d][1,3]dioxy -5-yl)tetrahydro-1H-pyrrolizin-7a(5H)-yl)methoxy)-8-fluoropyridin[4,3-d]pyrimidin-7-yl)-4-((1, 1,1,3,3,3-hexafluoropropan-2-yl)oxy) aniline (Trans racemate)

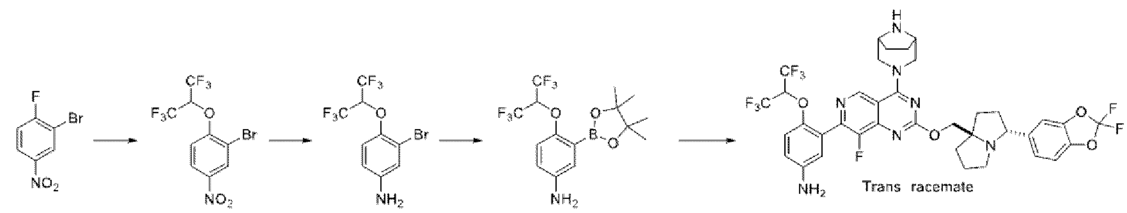

Step 1: Synthesis of 2-bromo-1-((1,1,1,3,3-hexafluoropropan-2-yl)oxy)-4-nitrobenzene

3-Bromo-4-fluoronitrobenzene (2.2 g, 10.0 mmol), hexafluoroisopropanol (2.52 g, 15.0 mmol), and 15 mL of tetrahydrofuran were added to a reaction flask. Potassium tert-butoxide (2.24 g, 20.0 mmol) was added at 0°C, and the mixture was heated to 50°C and stirred for 6 h. The reaction solution was poured into 50 mL of water and extracted with ethyl acetate. The organic phase was dried and evaporated under reduced pressure to obtain a crude product, which was directly used for the next step of reaction.

Step 2: Synthesis of 3-bromo-4-((1,1,1,3,3,3-hexafluoropropan-2-yl)oxy)aniline

The crude product was dissolved in 20 mL of ethanol. 4 mL of water, iron powder (2.79 g, 50.0 mmol), and ammonium chloride (2.68 g, 50.0 mmol) were added at room temperature. The reaction mixture was stirred at 80°C for 4 h. The reaction system was diluted with ethyl acetate, suction-filtered, and the filtrate was concentrated to obtain a crude product. The crude product was purified by column chromatography (PE/EA = 5/1) to obtain 300 mg of grey solid, with a yield of 8.9% (2 steps).

Using the above intermediate as raw materials, the target product was synthesized by a method similar to that in Example 161.

LC/MS: m/z=812.3 $[M+H]^+$.

## Example 163

3-(4-((1R,5S)-3,8-diazabicyclo[3.2.1]octan-3-yl)-2-((3-(2,2-difluorobenzo[d][1,3]dioxy -5-yl)tetrahydro-1H-pyrrolizin-7a(5H)-yl)methoxy)-8-fluoropyridin[4,3d]pyrimidin-7-yl)-4-(trifl uoromethoxy) (Trans racemate)

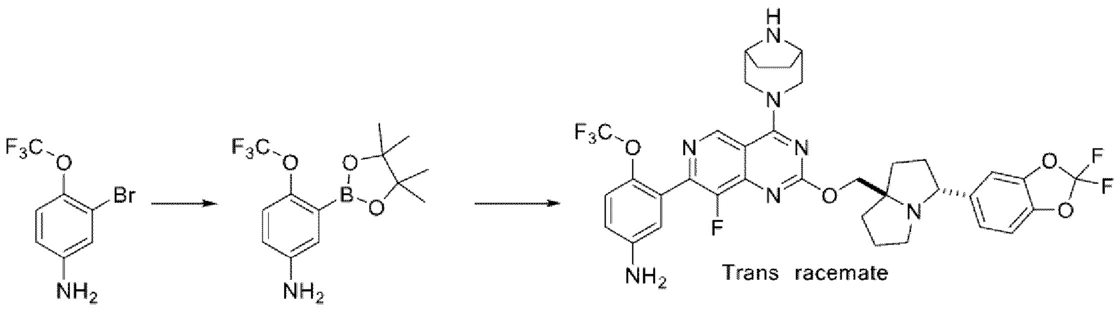


Using the starting material shown in the synthetic route as raw materials, the target product was synthesized by a method similar to that in Example 161.

LC/MS: m/z=730.3 $[M+H]^+$.

$^1H$ NMR (400 MHz, $CDCl_3$) δ 9.02 (s, 1H), 7.24-7.15 (m, 3H), 7.03-7.00 (m, 1H), 6.88-6.86 (m, 1H), 6.78-6.75 (m, 1H), 4.62-4.31 (m, 5H), 3.81 (brs, 2H), 3.73-3.57 (s, 4H), 2.62-2.52 (m, 1H), 2.38-2.24 (m, 2H), 2.17-1.99 (m, 4H), 1.76-1.56 (m, 7H).

## Example 164

3-(4-((1R,5S)-3,8-diazabicyclo[3.2.1]octan-3-yl)-2-((3-(2,2-difluorobenzo[d][1,3]dioxy -5-yl)tetrahydro-1H-pyrrolizin-7a(5H)-yl)methoxy)-8-fluoropyridin[4,3d]pyrimidin-7-yl)-5-fluor o-4-(trifluoromethoxy) (Trans racemate)

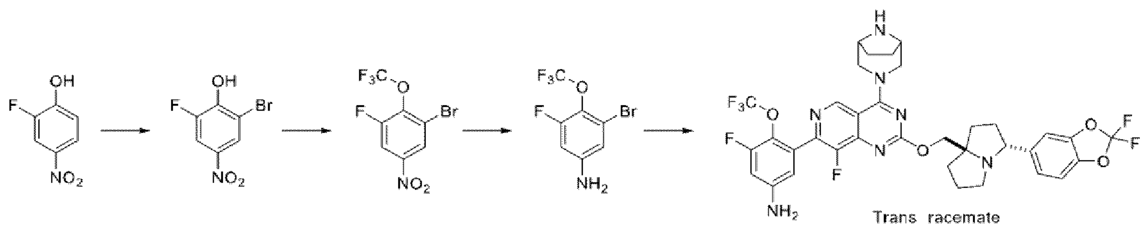

Step 1: Synthesis of 2-bromo-6-fluoro-4-nitrophenol

2-Fluoro-4-nitrophenol (2.0 g, 12.74 mmol) and 20 mL of acetonitrile were added to a reaction flask. NBS (2.72 g, 15.29 mmol) was added at 0°C, and the mixture was stirred at room temperature for 1 h. The reaction solution was poured into 80 mL of water and extracted with ethyl acetate. The organic phase was washed twice with an appropriate amount of sodium hydrogen sulfite aqueous solution, dried, evaporated under reduced pressure and purified by flash column chromatography (PE/EA = 5/1) to obtain 2.2 g of yellow solid, with a yield of 73.3%.

Step 2: Synthesis of 1-bromo-3-fluoro-5-nitro-2-(trifluoromethoxy) benzene

2-Bromo-6-fluoro-4-nitrophenol (2.2 g, 7.33 mmol), silver trifluoromethanesulfonate (5.65 g, 21.99 mmol), N-fluorobisbenzenesulfonamide (6.93 g, 21.99 mmol), Selectfluor (7.79 g, 21.99 mmol), cesium fluoride (3.34 g, 21.99 mmol), 2,6-di-tert-butylphenol (6.05 g, 29.32 mmol), and 100 mL of toluene were added to a reaction flask. Then, 2-fluoropyridine (2.85 g, 29.32 mmol) and (trifluoromethyl)trimethylsilane (5.21 g, 36.65 mmol) were added under stirring at room temperature. The reaction mixture was heated to 70°C under nitrogen protection and stirred for 6 h. The reaction system was cooled to room temperature and then filtered directly. The filtrate was poured into 200 mL of water and extracted with ethyl acetate. The organic phase was dried and evaporated under reduced pressure. The crude product was purified by column chromatography (PE/EA = 50/1) to obtain 0.4 g of yellow solid, with a yield of 14.1%.

Using the above intermediate as raw materials, the target product was synthesized by a method similar to that in Example 161.

LC/MS: m/z=748.2 $[M+H]^+$.

$^1H$ NMR (400 MHz, $d_6$-DMSO) δ 9.11 (s, 1H), 7.35-7.33 (m, 2H), 7.18-7.16 (m, 1H), 6.66-6.57 (m, 2H), 5.88 (brs, 2H), 4.41-4.38 (m, 2H), 4.29-4.17 (m, 3H), 3.61-3.55 (m, 4H), 2.30-2.10 (m, 4H), 1.96-1.85 (m, 3H), 1.61-1.53 (m, 7H).

## Example 165

5-(4-((1R,5S)-3,8-diazabicyclo[3.2.1]octan-3-yl)-2-((3-(2,2-difluorobenzo[d][1,3]dioxy -5-yl)tetrahydro-1H-pyrrolizin-7a(5H)-yl)methoxy)-8-fluoropyridin[4,3-d]pyrimidin-7-yl)-6-(trifl uoromethyl)-[1,1'-biphenyl]-3-amine (Trans racemate)

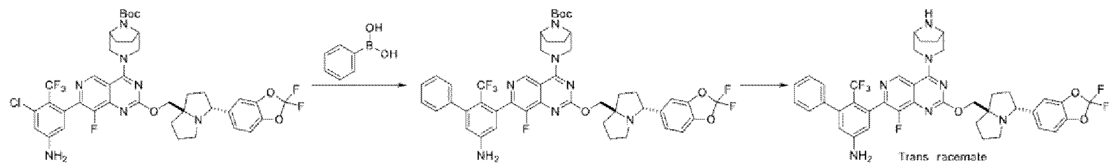


Step 1: Synthesis of tert-butyl (1R,5S)-3-(7-(5-amino-2-(trifluoromethyl)-[1,1'-biphenyl]-3-yl)-2-((3-(2,2-difluorobenzo [d][1,3] dioxy-5-yl)tetrahydro-1H-pyrrolizin-7a(5H)-yl)methoxy)-8-fluoropyrido[4,3-d]pyrimidin-4-yl)-3 ,8-diazabicyclo [3.2.1]octan-8-carboxylate (Trans racemate)

Tert-butyl (1R,5S)-3-(7-(5-amino-3-chloro-2-(trifluoromethyl)phenyl)-2-((3-(2,2-difluorobenzo[d][1,3]diox y-5-yl)tetrahydro-1H-pyrrolizin-7a(5H)-yl)methoxy)-8-fluoropyrido[4,3-d]pyrimidin-4-yl)-3,8-di azabicyclo[3.2.1]octan-8-carboxylate (the intermediate of Example 161, trans-racemate, 85 mg, 0.1 mmol), phenylboronic acid (24 mg, 0.2 mmol), potassium carbonate (41 mg, 0.3 mmol), and dioxane/water (2 mL/0.2 mL) were added to a reaction flask. After replacement with nitrogen, 1 mg of $Pd(dppf)Cl_2$ was added. The reaction mixture was heated to 100°C under nitrogen protection and stirred for 6 h. The reaction system was cooled to room temperature, poured into 5 mL of water and extracted with ethyl acetate. The organic phase was dried, evaporated under reduced pressure, and then purified by Prep-TLC (DCM/MeOH = 10/1) to obtain 60 mg of grey solid, with a yield of 67%.

Step 2: Synthesis of 5-(4-((1R,5S)-3,8-diazabicyclo[3.2.1]octan-3-yl)-2-((3-(2,2-difluorobenzo[d][1,3]dioxy-5-yl)tetra hydro-1H-pyrrolizin-7a(5H)-yl)methoxy)-8-fluoropyridine[4,3-d]pyrimidin-7-yl)-6-(trifluoromet hyl)-[1,1'-biphenyl]-3-amine (Trans racemate)

Tert-butyl (1R,5S)-3-(7-(5-amino-2-(trifluoromethyl)-[1,1'-biphenyl]-3-yl)-2-((3-(2,2-difluorobenzo[d][1,3] dioxy-5-yl)tetrahydro-1H-pyrrolizin-7a(5H)-yl)methoxy)-8-fluoropyrido[4,3-d]pyrimidin-4-yl)-3 ,8-diazabicyclo[3.2.1]octan-8-carboxylate (60 mg, 0.067 mmol) was dissolved in 2 mL of ethyl acetate, and HCl/EA (4 M, 0.5 mL) was added. The mixture was stirred for 10 min. The reaction system was poured into 10 mL of water, adjusted to weakly alkaline with sodium carbonate, and extracted with ethyl acetate. The organic phase was dried, evaporated under reduced pressure, and then purified by Prep-TLC to obtain 45 mg of grey solid, with a yield of 85%.

LC/MS: m/z=790.3 $[M+H]^+$.

**Example 166**

3-(4-((1R,5S)-3,8-diazabicyclo[3.2.1]octan-3-yl)-2-((3-(2,2-difluorobenzo[d][1,3]dioxy -5-yl)tetrahydro-1H-pyrrolizin-7a(5H)-yl)methoxy)-8-fluoropyridin[4,3d]pyrimidin-7-yl)-4-ethyn ylaniline (Trans racemate)

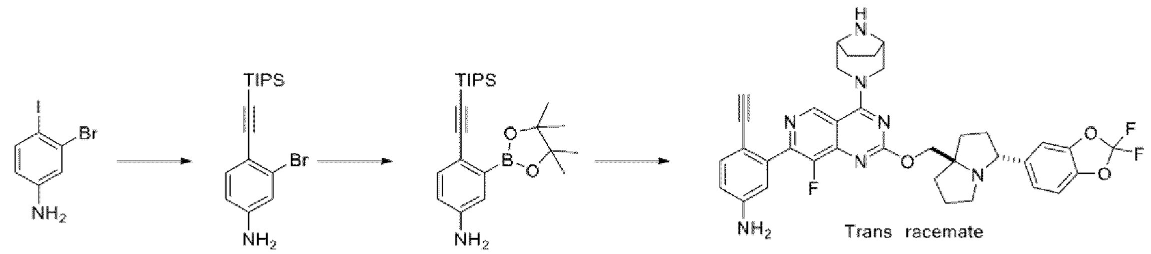


Step 1: Synthesis of 3-bromo-4-((triisopropylsilaneyl)ethynyl)aniline

3-Bromo-4-iodoaniline (1.0 g, 3.36 mmol) and 10 mL of DMF were added to a reaction flask. At room temperature, triisopropylsilylethyne (1.23 g, 6.72 mmol), TEA (1.36 g, 13.44 mmol), cuprous iodide (65 mg, 0.34 mmol), and $Pd(PPh_3)_2Cl_2$ (239 mg, 0.34 mmol) were added. The mixture was stirred at 70°C for 4 h. The reaction solution was poured into 20 mL of water and extracted with ethyl acetate. The organic phase was dried and evaporated under reduced pressure. The crude product was purified by column chromatography (PE/EA = 10/1) to obtain 0.6 g of light yellow solid, with a yield of 50.9%.

Step 2: Synthesis of 3-(4,4,5,5-tetramethyl-1,3,2-dioxaboran-2-yl)-4-((triisopropylsilyl)ethynyl)aniline

3-Bromo-4-((triisopropylsilaneyl)ethynyl)aniline (0.6 g, 1.70 mmol), bis(pinacolato)diboron (0.5 g, 1.97 mmol), potassium acetate (0.4 g, 4.08 mmol), and 50 mL of dioxane were added to a reaction flask. After replacement with nitrogen, $Pd(dppf)Cl_2$ (73 mg, 0.1 mmol) was added. The reaction mixture was heated to 80°C under nitrogen protection and stirred overnight. The reaction system was cooled to room temperature, poured into 20 mL of water and extracted with ethyl acetate. The organic phase was dried, evaporated under reduced pressure, and then purified by silica gel column chromatography (PE/EA = 20/1) to obtain 445 mg of white solid, with a yield of 65.4%.

Using the above intermediate as raw materials, the target product was synthesized by a method similar to that in Example 52.

LC/MS: m/z=670.3 $[M+H]^+$.

**Example 167**

(Isobutyryloxy)methyl(1R,5S)-3-(7-(7,8-difluoro-3-hydroxynaphthalen-1-yl)-8-fluoro-2 -((3-(4-fluorophenyl)tetrahydro-1H-pyrrolizin-7a(5H)-yl)methoxy)pyrido[4,3-d]pyrimidin-4-yl)-3,8-diazabicyclo[3.2.1]octan-8-carboxylate

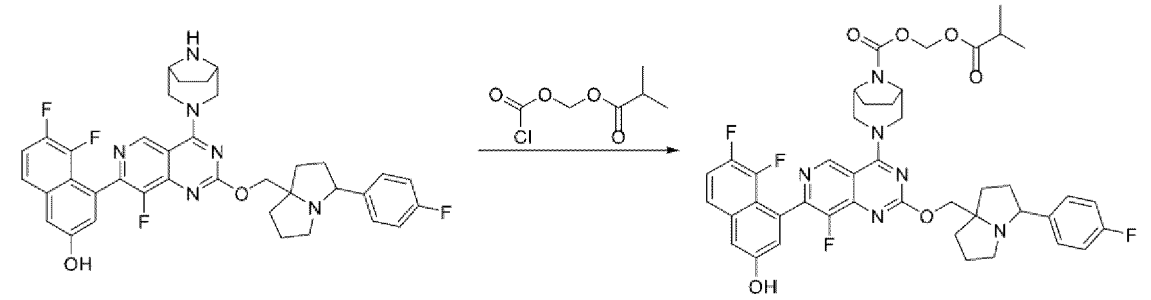

Step 1: Synthesis of (isobutyryloxy)methyl(1R,5S)-3-(7-(7,8-difluoro-3-hydroxynaphthalen-1-yl)-8-fluoro-2-((3-(4-fl uorophenyl)tetrahydro-1H-pyrrolizin-7a(5H)-yl)methoxy)pyrido[4,3-d]pyrimidin-4-yl)-3,8-diaza bicyclo[3.2.1]octan-8-carboxylate

4-(4-((1R,5S)-3,8-diazabicyclo[3.2.1]octan-3-yl)-8-fluoro-2-((3-(4-fluorophenyl)tetrahy dro-1H-pyrrolizin-7a(5H)-yl)methoxy)pyrido[4,3-d]pyrimidin-7-yl)-5,6-difluoronaphthalen-2-ol (150 mg, 0.22 mmol), TEA (89 mg, 0.88 mmol), and 5 mL of dichloromethane were added to a reaction flask. Methyl ((chlorocarbonyl)oxy) isobutyrate (43 mg, 0.24 mmol) was added at 0°C, and the mixture was stirred at room temperature for 1 h. The reaction system was directly evaporated under reduced pressure and then purified by Prep-TLC (DCM/MeOH = 35/1) to obtain 105 mg of off-white solid, with a yield of 58.7%.

LC/MS: m/z=815.3 [M+H]+.

**Example 168**

(Isobutyryloxy)methyl(1R,5S)-3-(7-(3-acetoxy-7,8-difluoronaphthalen-1-yl)-8-fluoro-2-((3-(4-fluorophenyl)tetrahydro-1H-pyrrolizin-7a(5H)-yl)methoxy)pyrido[4,3-d]pyrimidin-4-yl)-3 ,8-diazabicyclo[3.2.1]octan-8-carboxylate

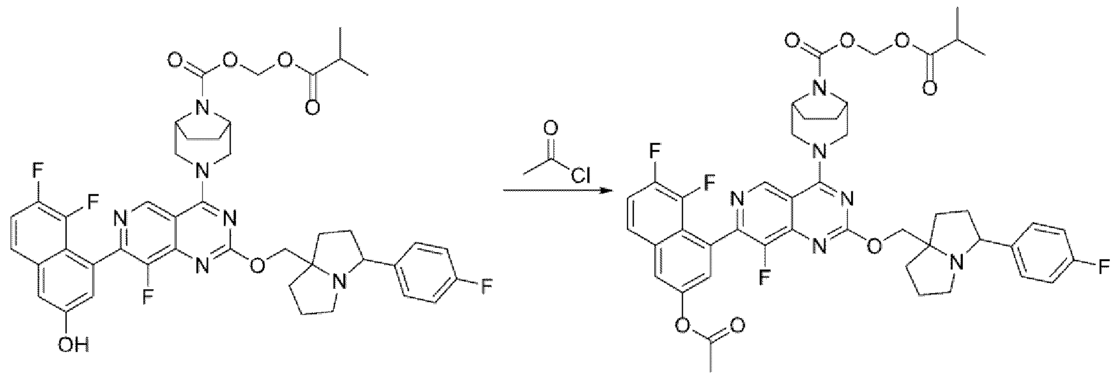

Step 1: Synthesis of ((isobutyryloxy)methyl(1R,5S)-3-(7-(3-acetoxy-7,8-difluoronaphthalen-1-yl)-8-fluoro-2-((3-(4-fl uorophenyl)tetrahydro-1H-pyrrolizin-7a(5H)-yl)methoxy)pyrido[4,3-d]pyrimidin-4-yl)-3,8-diaza bicyclo[3.2.1]octan-8-carboxylate

(Isobutyryloxy)methyl(1R,5S)-3-(7-(7,8-difluoro-3-hydroxynaphthalen-1-yl)-8-fluoro-2 -((3-(4-fluorophenyl)tetrahydro-1H-pyrrolizin-7a(5H)-yl)methoxy)pyrido[4,3-d]pyrimidin-4-yl)-3,8-diazabicyclo[3.2.1]octan-8-carboxylate (50 mg, 0.06 mmol), TEA (31 mg, 0.36 mmol), and 5 mL of dichloromethane were added to a reaction flask. Acetyl chloride (14 mg, 0.18 mmol) was added at 0°C, and the mixture was stirred at room temperature for 1 h. The reaction system was directly evaporated under reduced pressure and then purified by Prep-TLC (DCM/MeOH = 50/1) to obtain 35 mg of off-white solid, with a yield of 66.1%.

LC/MS: m/z=857.3 [M+H]+.

**Example 169**

4-(4-((1R,5S)-3,8-diazabicyclo[3.2.1]octan-3-yl)-8-fluoro-2-((3-(4-fluorophenyl)tetrahy dro-1H-pyrrolizin-7a(5H)-yl) methoxy)pyrido[4,3-d]pyrimidin-7-yl)-5,6-difluoronaphthalen-2-yld imethylcarbamate

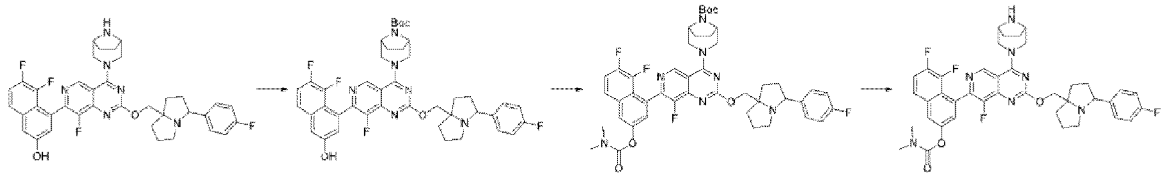

Step 1: Synthesis of tert-butyl (1R,5S)-3-(7-(7,8-difluoro-3-hydroxynaphthalen-1-yl)-8-fluoro-2-((3-(4-fluorophenyl)tetrahydro-1H-pyrrolizin-7a(5H)-yl)methoxy)pyrido[4,3-d]pyrimidin-4-yl)-3,8-diazabicyclo[3.2.1]octan-8-c arboxylate

4-(4-((1R,5S)-3,8-diazabicyclo[3.2.1]octan-3-yl)-8-fluoro-2-((3-(4-fluorophenyl)tetrahy dro-1H-pyrrolizin-7a(5H)-yl)methoxy)pyrido[4,3-d]pyrimidin-7-yl)-5,6-difluoronaphthalen-2-ol (670 mg, 1.0 mmol), triethylamine (303 mg, 3.0 mmol), and 5 mL of dichloromethane were added to a reaction flask. $Boc_2O$ (218 mg, 1.0 mmol) was added at 0°C, and the mixture was stirred at room temperature for 1 h. The reaction solution was directly used for the next step.

Step 2: Synthesis of tert-butyl (1R,5S)-3-(7-(3-((dimethylcarbamoyl)oxy)-7,8-difluoronaphthalen-1-yl)-8-fluoro-2-((3-(4-fluoro phenyl)tetrahydro-1H-pyrrolizin-7a(5H)-yl)methoxy)pyrido[4,3-d]pyrimidin-4-yl)-3,8-diazabicy clo[3.2.1]octan-8-carboxylate

At 0°C, dimethylcarbamoyl chloride (107 mg, 1.0 mmol) was added to the reaction solution obtained from the previous step and stirred overnight. The reaction system was directly concentrated to dryness and then purified by column chromatography (DCM/MeOH = 40/1) to obtain 230 mg of a grey solid, with a two-step yield of 27%.

Step 3: Synthesis of 4-(4-((1R,5S)-3,8-diazabicyclo[3.2.1]octan-3-yl)-8-fluoro-2-((3-(4-fluorophenyl)tetrahydro-1H-p yrrolizin-7a(5H)-yl)methoxy)pyrido[4,3-d]pyrimidin-7-yl)-5,6-difluoronaphthalen-2-yldimethylc arbamate

Tert-butyl (1R,5S)-3-(7-(3-((dimethylcarbamoyl)oxy)-7,8-difluoronaphthalen-1-yl)-8-fluoro-2-((3-(4-fluoro phenyl)tetrahydro-1H-pyrrolizin-7a(5H)-yl)methoxy)pyrido[4,3-d]pyrimidin-4-yl)-3,8-diazabicy clo[3.2.1]octan-8-carboxylate (230 mg, 0.27 mmol) was dissolved in 5 mL of dichloromethane. 1.5 mL of trifluoroacetic acid was added at room temperature, and the mixture was stirred for 0.5 h at room temperature. The reaction system was poured into 10 mL of water, adjusted to weakly alkaline with sodium carbonate, and extracted with ethyl acetate. The organic phase was dried, evaporated under reduced pressure, and then purified by Prep-TLC to obtain 150 mg of grey solid, with a yield of 74%.

LC/MS: m/z=742.3 $[M+H]^+$.

The following compounds of Examples 170 to 246 were prepared by using commercially available corresponding reagents as raw materials according to the operations similar to those described in the above examples.

Table 2

| Exam ple | Structure | MS (ESI) m/z | $^1$H NMR | Examp le of re ference for syn thesis |
|---|---|---|---|---|
| 170 | 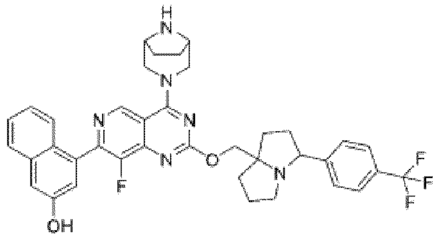 | 685.3 | | 34 |

(continued)

| Example | Structure | MS (ESI) m/z | 1H NMR | Example of reference for synthesis |
|---|---|---|---|---|
| 171 | 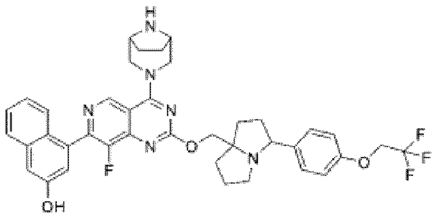 | 714.3 | | 35 |
| 172 | 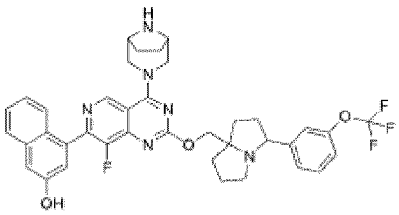 | 701.3 | | 34 |
| 173 | 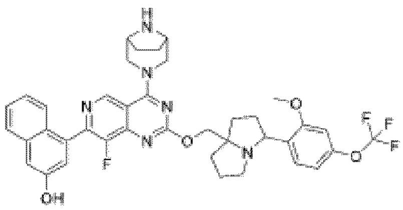 | 731.3 | | 34 |
| 174 | 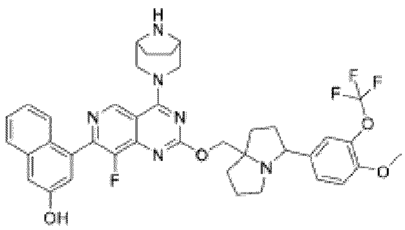 | 731.3 | | 34 |
| 175 | 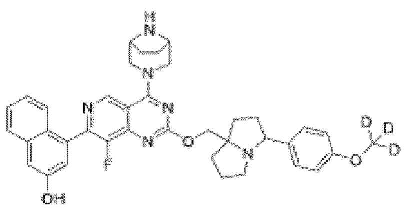 | 650.3 | | 34 |
| 176 | 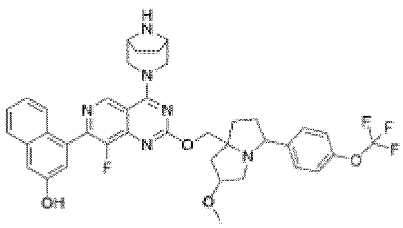 | 731.3 | | 143 |

(continued)

| Example | Structure | MS (ESI) m/z | 1H NMR | Example of reference for synthesis |
|---|---|---|---|---|
| 177 | 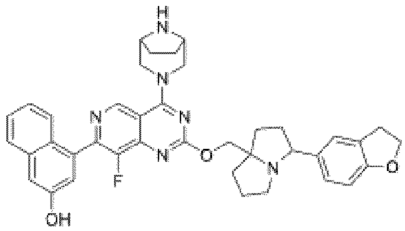 | 659.3 | | 34 |
| 178 | 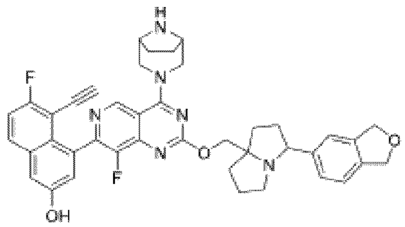 | 701.3 | | 43, 52 |
| 179 | 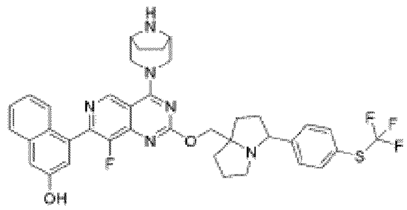 | 717.3 | | 34 |
| 180 | 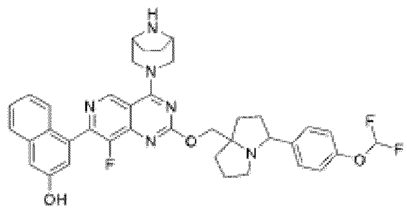 | 683.3 | | 34 |
| 181 | 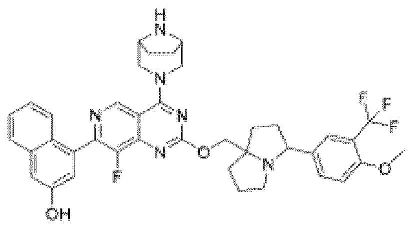 | 715.3 | | 34 |
| 182 | 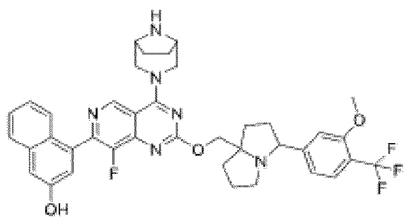 | 715.3 | | 34 |

(continued)

| Example | Structure | MS (ESI) m/z | 1H NMR | Example of reference for synthesis |
|---|---|---|---|---|
| 183 | 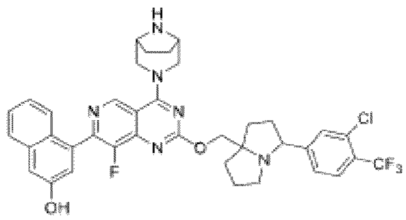 | 719.2 | | 34 |
| 184 | 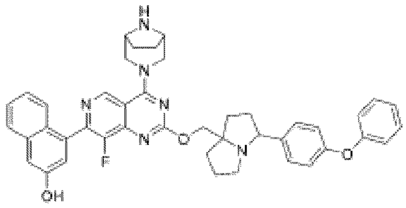 | 709.3 | 1H NMR (400 MHz, $CDCl_3$) δ 9.06 (brs, 1H), 7.72-7.52 (m, 211), 7.40-7.28 (m, 8H), 7.08-6.91 (m, 5H). 4.65-4.48 (m, 211), 4.38-4.26 (m, 211), 3.77-3.59 (m, 4H), 2.98-2.87 (m, 1H), 2.70-2.63 (m, 1H), 2.40)-2.32 (m, 1H), 2.21-1.88 (m, 5H), 1.85-1.61 (m, 6H). | 34 |
| 185 | 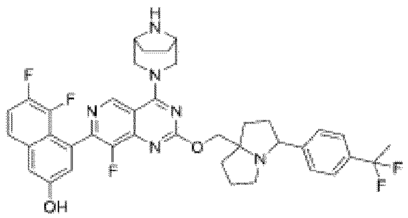 | 717.3 | | 34 |
| 186 | 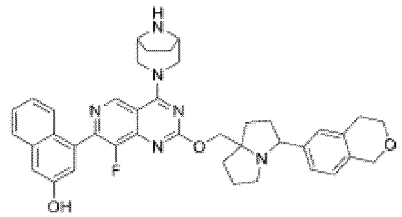 | 673.3 | | 34 |
| 187 | 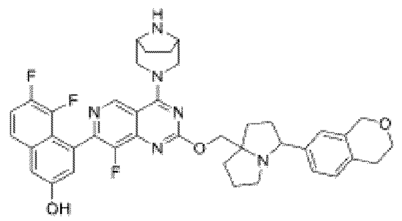 | 709.3 | 1H NMR (400 MHz, $CDCl_3$) δ 8.98 (s, 1H), 7.30-7.27 (m, 1H), 7.23-7.11 (m, 3H), 7.06-6.96 (m, 3H), 4.72-4.64 (m, 3H), 4.44-4-23 (m, 3H), 3.92 (brs, 2H), 3.74-3.54 (m, 4H), 2.93-2.89 (m, 1H), 2.80-2.79 (m, 1H), 2.65-2.63 (m, 1H), 2.33-2.29 (m, 1H), 2.16-2.1.3 (m, 1H), 2.00-1.68 (m, 10H). | 34 |
| 188 | 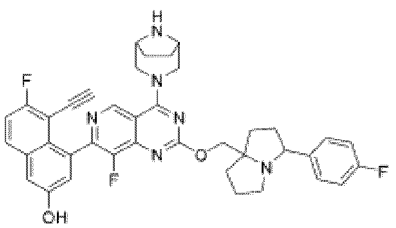 | 677.3 | | 34, 52 |

(continued)

| Example | Structure | MS (ESI) m/z | $^1$H NMR | Example of reference for synthesis |
|---|---|---|---|---|
| 189 | 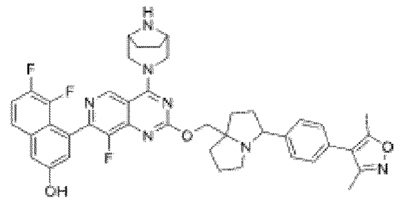 | 748.3 | | 45 |
| 190 | 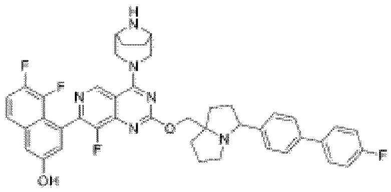 | 747.3 | | 34 |
| 191 | 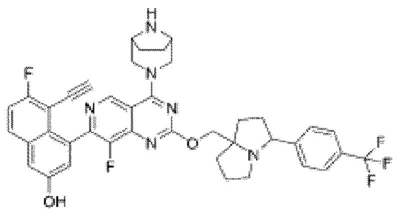 | 727.3 | | 34, 52 |
| 192 | 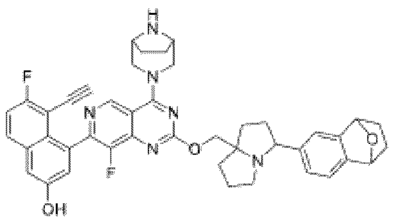 | 727.3 | 1H NMR (400 MHz, $d_6$-DMSO) $\delta$ 10.28 (brs, 1H), 9.13 (s, 1H), 7.77-7.33 (m, 1H), 7.61-7.55 (m, 1H), 7.42-7.38 (mm, 1H). 7.28-7.21 (m, 2H), 7.11-7.04 (m, 2H,), 5.32-5.21 (m, 2H), 4.46-4.18 (m, 5H), 3.74-3.56 (m, 5H), 2.78-2.75 (m, 1H), 2.13-2.10 (m, 2H), 1.91-1.85 (m, 7H), 1.73-1.55 (m, 7H). | 153 |
| 193 | 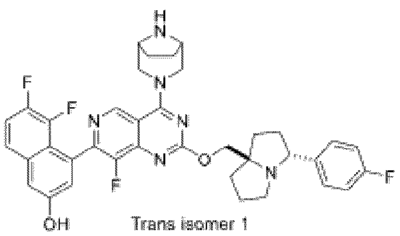 Trans isomer 1 | 671.3 | 1H NMR (400 MHz, $d_6$-DMSO) $\delta$ 10.27 (brs, 1H), 9.12 (s, 1H), 7.74 (dd, J = 8.4 Hz, 5.2 Hz, 1H), 7.57 (dd, J = 17.2 Hz, 9.6 Hz, 1H), 7.40-7.37 (m, 3H), 7.25 (s, 1H), 7.13 (t, J = 8.8 Hz, 2H), 4.48-4.40 (m, 2H), 4.30-4.28 (m, 1H), 4.21-4.18 (m, 2H), 3.65-3.53 (m, 4H), 2.29-2.26 (m, 1H), 2.22-2.16 (m, 1H), 2.12-2.06 (m, 2H) , 1.95-1.80 (m, 3H), 1.69-1.52 (m, 7H). | 141 |

(continued)

| Example | Structure | MS (ESI) m/z | $^1$H NMR | Example of reference for synthesis |
|---|---|---|---|---|
| 194 | 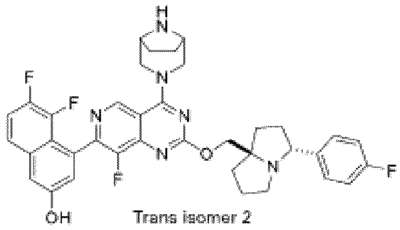 Trans isomer 2 | 671,3 | 1H NMR (400 MHz, $d_6$-DMSO) δ 10.29 (brs, 1H), 9.12 (s, 1H), 7.74 (dd, J = 8.4 Hz, 5.2 Hz, 1H), 7.57 (dd, J = 17.2 Hz, 9.6 Hz, 1H), 7.39-7.34 (m, 3H), 7.24 (s, 1H), 7.13 (t, J = 8.8 Hz, 2H), 4.49-4.40 (m, 2H), 4.29-4.26 (m, 1H), 4.20-4.17 (m, 2H), 3.64-3..55 (m, 4H), 2.29-2.25 (m, 1H), 2.20-2.16 (m, 1H), 2.12-2.04 (m, 2H) , 1.94-1.90 (m, 1H), 11.88-1.82 (m, 2H), 1.66-1.51 (m, 7H). | 142 |
| 195 | 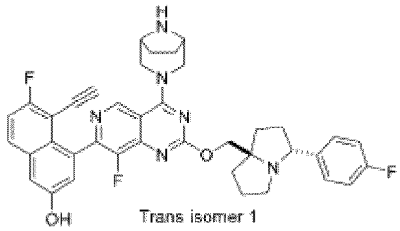 Trans isomer 1 | 677.3 | 1H NMR (400 MHz, $CDCl_3$) δ 8.90 (s, 1H), 7.60-7.52 (m, 1H), 7.40-7.35 (m, 2H), 7.29-7.28 (m, 1H), 7.12-7.03 (m, 2H), 6.99-6.92) (m, 2H), 4.69-4.30 (m, 4H), 3.73-3.41 (m, 4H), 2.92-2.59 (m, 3H), 2.38-2.30 (m, 2H), 2.16-1.92 (m, 4H), 1.89-1.52 (m, 8H). | 141 |
| 196 | 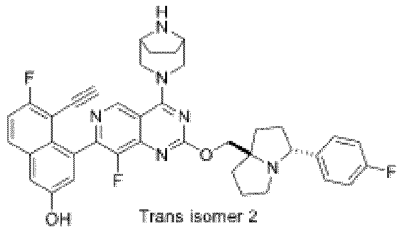 Trans isomer 2 | 677.3 | 1H NMR (400 MHz, $CDCl_3$) δ 8.91 (s, 1H), 7.60-7.54 (m, 1H), 7.40-7.35 (m,2H), 7.29-7.28 (m, 1H), 7.10-7.03 (m, 2H), 6.99-6.92 (m, 2H), 4.69-4.30 (m, 4H), 3.71-3.44 (m, 4H), 2.92-2.59 (m, 3H), 2.38-2.30 (m, 2H), 2.16-1.92 (m, 4H), 1.89-1.55 (m, 8H). | 142 |
| 197 | 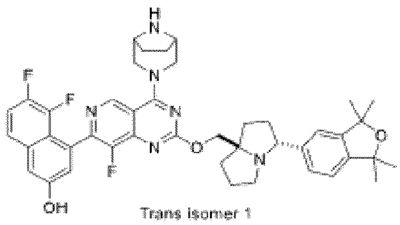 Trans isomer 1 | 751.4 | | 141 |
| 198 | 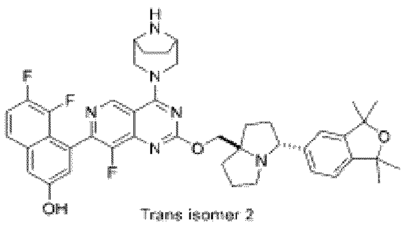 Trans isomer 2 | 751.4 | 1H NMR (400 MHz, $d_6$-DMSO) δ 10.30 (brs, 1H), 9.13 (s, 1H), 7.75 (dd, J = 8.0 Hz. 4.4 Hz, 1H), 7.61 -7.54 (m, 1H), 7.41-7.38 (m, 1H), 7.25-7.23 (m, 2H) , 7.17-7.12 (m, 2H), 4.48-4.43 (m, 2H), 4.35-4.20 (m, 3H), 3.68-3.55 (m, 4H), 2.33-2.28 (m, 1H), 2.25-2.05 (m, 3H), 1.99-1.83 (m, 3H) , 1.65-1.55 (m, 7H) , 140 (s, 12H). | 142 |

(continued)

| Example | Structure | MS (ESI) m/z | $^1$H NMR | Example of reference for synthesis |
|---|---|---|---|---|
| 199 | 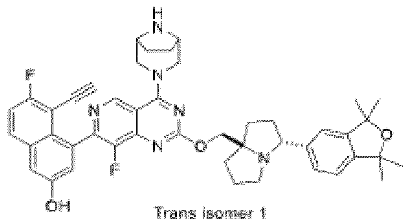 Trans isomer 1 | 757.4 | | 141 |
| 200 | 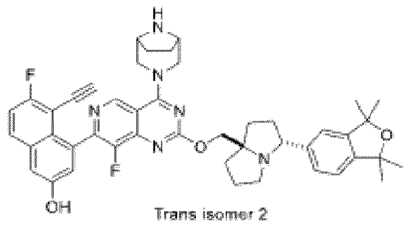 Trans isomer 2 | 757.4 | 1H NMR (400 MHz, $CDCl_3$) δ 8.88 (s, 1H), 7.55 (brs, 1H), 7.36-7.32 (m, 1H), 7.24-6.97 (m, 5H), 4.73-4.33 (m, 5H), 3.70-3.42 (m, 5H) , 2.96-2.63 (m, 2H), 2.42-2.11 (m, 5H), 2.05-1.88 (m, 2H), 1.78-1.60 (m, 5H), 1.48 (s, 12H). | 142 |
| 201 | 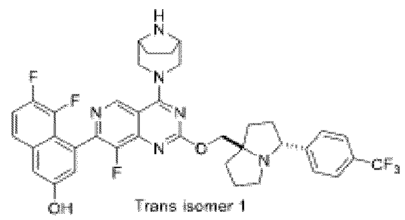 Trans isomer 1 | 721.3 | 1H NMR (400 MHz, $d_6$-DMSO) δ 10.32 (brs, 1H), 9.12 (s, 1H), 7.76-7.73 (m, 1H), 7.69-7.67 (m, 2H), 7.58-7.53 (m, 3H), 7.39 (brs, 1H), 7.25 (brs, 1H), 4.48-4.41 (m, 2H), 4.35-4.18 (m, 3H), 3.65-3.51 (m, 4H), 2.35-2.30 (m, 1H), 2.18-2.05 (m, 3H), 2.03-1.95 (m, 2H), 1.91-1.86 (m, 2H), 1.69-1.50 (m, 7H). | 141 |
| 202 | 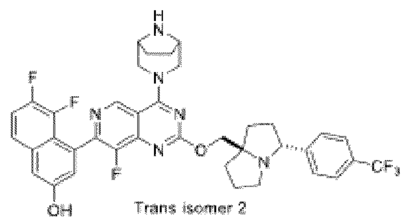 Trans isomer 2 | 721.3 | 1H NMR (400 MHz, $d_6$-DMSO) δ 10.29 (brs, 1H), 9.12 (s, 1H), 7.76-7.72 (m, 1H), 7.71-7.64 (m, 2H), 7.62-7.52 (m, 3H), 7.39 (brs, 1H) , 7.25 (brs, 1H), 4.47-4.40 (m, 2H), 4.34-4.17 (m, 3H), 3.64-3.50 (m, 4H), 2.36-2.31 (m, 1H), 2.20-2.06 (m, 3H), 2.02-1.96 (m, 2H), 1.92-1.87 (m, 2H) , 1.71-1.50 (m, 7H). | 142 |
| 203 | 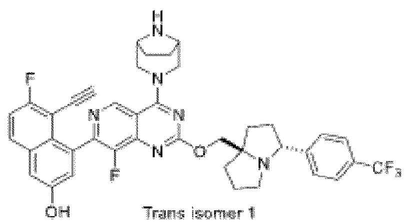 Trans isomer 1 | 727.3 | 1H NMR (400 MHz, $d_6$-DMSO) δ 10.18 (brs, 1H), 9.06 (s, 1H), 7.96-7.80 (m, 1H), 7.71-7.68 (m, 2H), 7.60-7.57 (m, 2H), 7.49-7.45 (m, 1H) , 7.40-7.39 (m, 1H), 7.19-7.17 (m, 1H), 4,55-4.45 (m, 1H), 4.34-4.27 (m, 2H), 4.20-4.18 (m, 1H), 3.95-3.94 (m, 1H), 3.67-3.64 (m, 1H), 3.56-3.52 (m, 2H) , 2.38-2.29 (m, 1H) , 2.19-2.11 (m, 3H), 2.06-1.95 (m, 2H), 1.93-1.85 (m, 2H) , 1.71-1.56 (m, 8H), | 141 |

(continued)

| Example | Structure | MS (ESI) m/z | $^{1}$H NMR | Example of reference for synthesis |
|---|---|---|---|---|
| 204 | 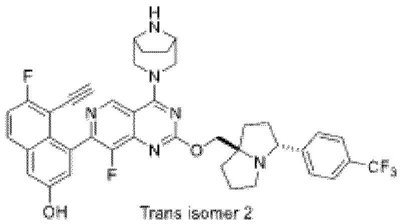 Trans isomer 2 | 727.3 | | 142 |
| 205 | 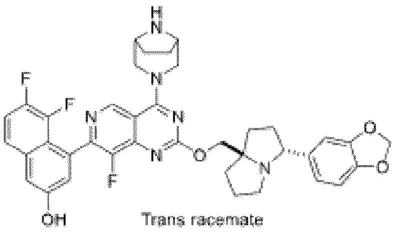 Trans racemate | 697.3 | | 140 |
| 206 | 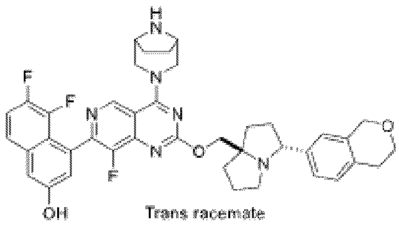 Trans racemate | 709.3 | | 140 |
| 207 | 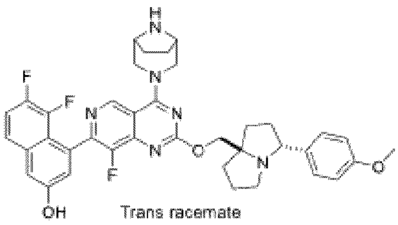 Trans racemate | 683.3 | 1H NMR (400 MHz, $CDCl_3$) δ 8.87-8.84 (m, 1H), 7.46-7.28 (m, 3H), 7.19-7.07 (m, 2H), 6.91-6.71 (m, 3H), 4.55-4.23 (m, 5H), 3.80-3.78 (m, 3H) , 3.60-3.40 (m, 5H), 2.65-2.50 (m, 1H), 2.43.2.33 (m, 1H), 2.28-2.07 (m, 4H), 1.91-1.62 (m, 7H). | 140 |
| 208 | 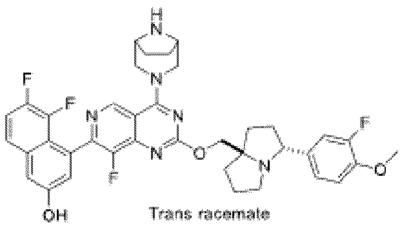 Trans racemate | 701.3 | | 140 |
| 209 | 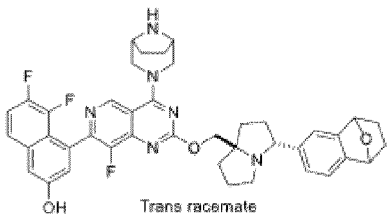 Trans racemate | 721.3 | | 140 |

(continued)

| Example | Structure | MS (ESI) m/z | $^1$H NMR | Example of reference for synthesis |
|---|---|---|---|---|
| 210 | 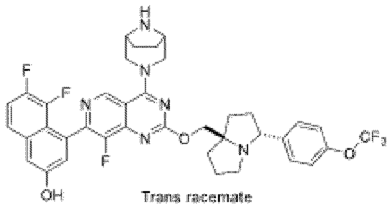 Trans racemate | 737.3 | | 140 |
| 211 | 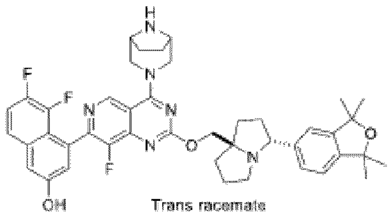 Trans racemate | 751.4 | | 140 |
| 212 | 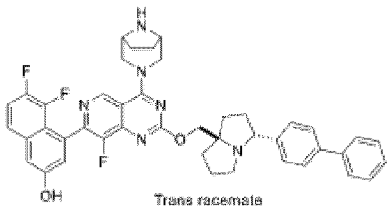 Trans racemate | 729.3 | 1H NMR (400 MHz, $CDCl_3$) δ 8.87-8.86 (m, 1H), 7.59-7.27 (m, 11H), 7.19-7.08 (m, 2H), 4.78-4.15 (m, 6H), 3.62-3.22 (m, 4H), 2.74-2.55 (m, 1H), 2.42-2.32 (m, 1H), 2.30-1.99 (m, 4H), 1.78-1.52 (m, 7H). | 140 |
| 213 | 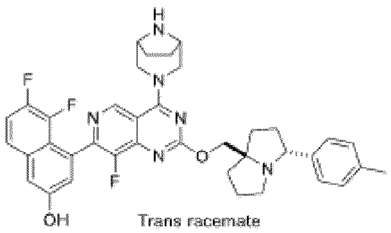 Trans racemate | 667.3 | 1H NMR (400 MHz, $CDCl_3$) δ 8.90-8.87 (m, 1H), 7.35-7.30 (m, 3H), 7.25-7.21 (m, 1H), 7.18-7.12 (m, 2H), 7.05-7.03 (m, 1H), 4.64-4.28 (m, 5H) , 3.65-3.39 (m, 4H), 2.71-2.55 (m, 1H), 2.42-2.20 (m, 5H), 2.18-1.90 (m, 4H), 1.86-1.41 (m, 7H). | 140 |
| 214 | 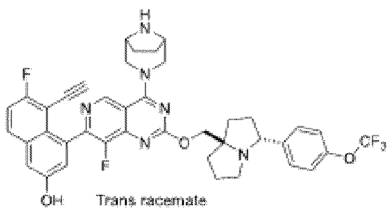 Trans racemate | 743,3 | 1H NMR (400 MHz, $CDCl_3$ δ 8.89-8.87 (m, 1H), 7.54-7.52 (m, 1H), 7.49-7..47 (m, 1H), 7.39-7.37 (m, 1H), 7.29-7.28 (m, 1H), 7.20-7.07 (m, 4H), 4.66-4.25 (m, 5H), 3.73-3.40 (m, 4H), 2.86-2.76 (m, 1H), 2.65-2.50 (m, 1H), 2.37-1.90 (m, 8H), 1.74-1.66 (m, 4H). | 140 |
| 215 | 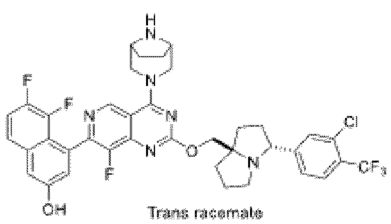 Trans racemate | 755.2 | 1H NMR (400 MHz, $CDCl_3$) δ 8.92. (s, 1H), 7.65-7.55 (m, 2H), 7.35-7-27 (m, 3H), 7.22-7.12 (m, 2H), 4.71-4.68 (m, 1H), 4.59-4.50 (m, 1H), 4.44-4.41 (m, 1H), 4.36-4.30 (m, 2H), 3.75-3.67 (m, 3H), 3.57-3.53 (m,1H), 2.34-2.11 (m, 5H), 2.09-1.85 (m, 4H), 1.85-1.67 (m, 5H). | 140 |

(continued)

| Example | Structure | MS (ESI) m/z | $^1$H NMR | Example of reference for synthesis |
|---|---|---|---|---|
| 216 | 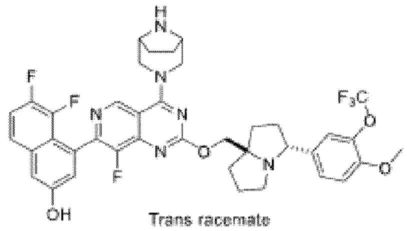 Trans racemate | 767.3 | | 140 |
| 217 | 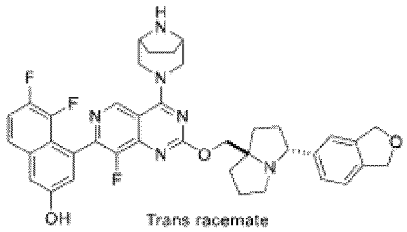 Trans racemate | 695.3 | 1H NMR (400 MHz, $CDCl_3$) δ 8-86-8.81 (m, 1H), 7.38-7.27 (m, 2H), 7.25-7.17 (m, 2H), 7.15-7.02 (m, 3H), 4.57-4.23 (m, 6H), 3.73-3.33 (m, SH), 2.64-2.44 (m, 1H), 2.37-1.94 (m, 6H), 1.83-1.54 (m, 4H). | 140 |
| 218 | 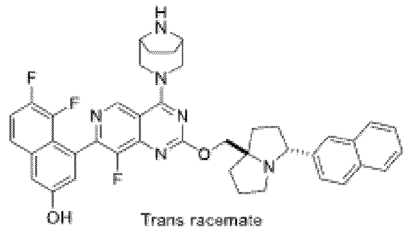 Trans racemate | 703.3 | 1H NMR (400 MHz, $CDCl_3$) δ 8.86-8.82 (m, 1H), 7.31-7.59 (m, 5H), 7.45-7.38 (m, 3H), 7.15-7.06 (m, 3H), 4.80-4.26 (m, 6H), 3.67-3.37 (m, 5H) , 2.65-2.50 (m, 1H), 2.40-2.09 (m, 5H), 2.02-1.61 (m, 6H). | 140 |
| 219 | 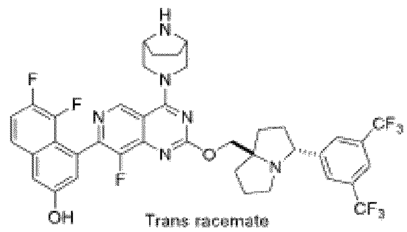 Trans racemate | 789, 3 | | 140 |
| 220 | 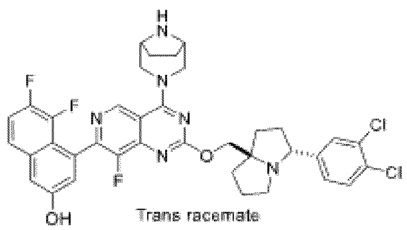 Trans racemate | 721.3 | 1H NMR (400 MHz, $CDCl_3$) δ 8.91-8.86 (m, 1H), 7.49.7.28 (m, 4H), 7.19-7.08 (m, 3H), 4.60-4.20 (m, 5H), 3.75-3.40 (m, 4H), 2.63-2.45 (m, 1H) , 2.31- 1.91 (m, 6H), 1.88-1.51 (m, 7H). | 140 |
| 221 | 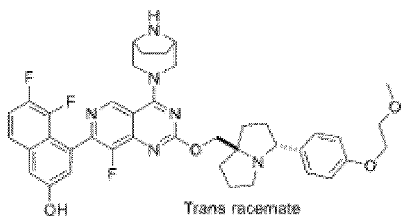 Trans racemate | 727.3 | | 140 |

(continued)

| Example | Structure | MS (ESI) m/z | 1H NMR | Example of reference for synthesis |
|---|---|---|---|---|
| 222 | 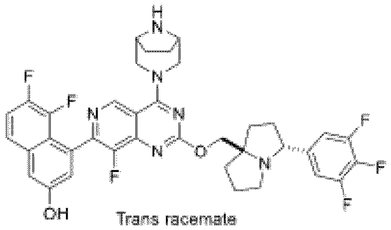 Trans racemate | 707.3 | 1H NMR (400 MHz, $d_6$-DMSO) δ 10.28 (brs, 1H), 9.12 (s, 1H), 7.73 (dd, J = 8.8 Hz, 4.8 Hz, 1H), 7.57 (dd, J = 17.6 Hz, 9,2 Hz, 1H), 7.40 (brs, 1H), 7.26-7.22 (m, 3H) , 4.48-4.41 (m, 2H), 4.30-4.27 (m, 1H), 4.19-4.17 (m, 2H), 3.66-3.55 (m, 4H), 3.36-3.34 (m, 1H), 2.23-2.08 (m, 3H), 1.93-1.85 (m, 3H) , 1.64-1.53 (m, 7H). | 140 |
| 223 | 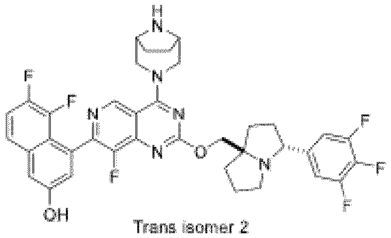 Trans isomer 2 | 707.3 | 1H NMR (400 MHz, $d_6$-DMSO) δ 10.30 (brs, 1H), 9.12 (s, 1H), 7.74 (dd. J = 8.8 Hz, 4.8 Hz, 1H), 7.57 (dd, J = 17.6 Hz, 9.2 Hz, 1H), 7.40 (brs, 1H), 7-26-7-22 (m, 3H) , 4.49-4.40 (m, 2H), 4-30-4.27 (m, 1H), 4.20-4.17 (m, 2H), 3.66-3.57 (m, 4H), 3.36.3.34 (m, 1H), 2.23-2.06 (m, 3H), 1.95-1.92 (m, 1H), 1.87-1.85 (m, 2H) , 1.68-1.52 (m, 7H). | 142 |
| 224 | 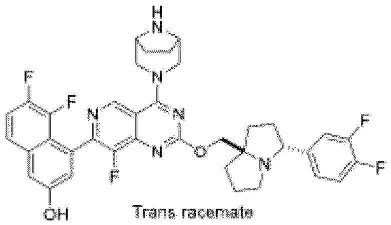 Trans racemate | 689.3 | 1H NMR (400 MHz, $CDCl_3$) δ 8.92-8.88 (m, 1H), 7.34-7.32 (m, 2H), 7.23-6.91 (m, 5H), 4.60-4.18 (m, 5H), 3.67-3.40) (m, 4H), 2.63-2.50 (m, 1H) , 2.31-1.93 (m, 6H), 1.88-1.50 (m, 7H). | 140 |
| 225 | 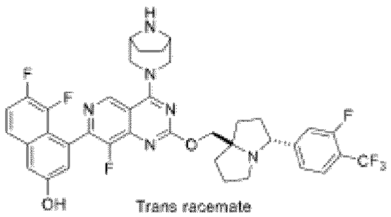 Trans racemate | 739.3 | | 140 |
| 226 | 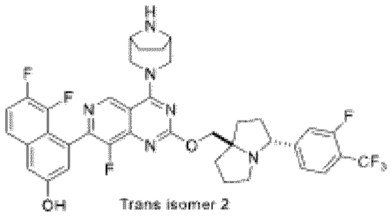 Trans isomer 2 | 739.3 | 1H NMR (400 MHz, $d_6$-DMSO) δ 10.29 (brs, 1H), 9.13 (s, 1H), 7.76-7.71 (m, 2H), 7.57 (dd, J = 17.6 Hz, 9.2 Hz, 1H), 7.44-7.37 (m, 3H), 7.26-7.22 (m, 1H), 4.49-4.41 (m, 2H), 4.33-4.20 (m, 3H), 3.63-3.57 (m, 4H), 3.39-3.37 (m, 1H), 2.20-2.00 (m, 4H), 1.91-1.88 (m, 2H), 1.64-1.56 (m, 7H). | 142 |
| 227 | 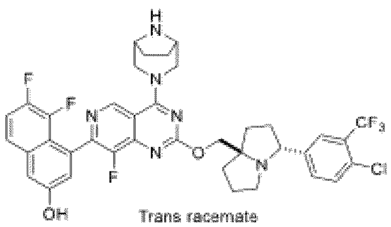 Trans racemate | 755.2 | 1H NMR (400 MHz, $CDCl_3$) δ 8.95.8.82 (m, 1H), 7.73-7.33 (m, 3H), 7.22-6.99 (m, 4H), 4.62-4.21 (m, 5H), 3.74-3.36 (m, 4H), 2.61-2.45 (m, 1H) , 2.30-1.91 (m, 6H), 1.86-1.43 (m, 7H). | 140 |

(continued)

| Example | Structure | MS (ESI) m/z | $^1$H NMR | Example of reference for synthesis |
|---|---|---|---|---|
| 228 | 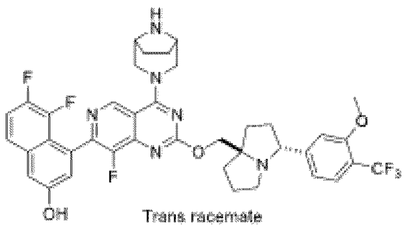 Trans racemate | 751.3 | | 140 |
| 229 | 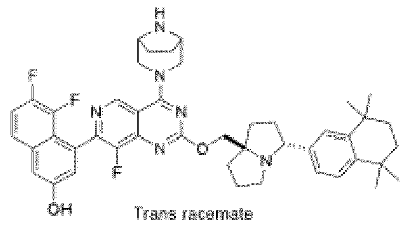 Trans racemate | 763.4 | | 140 |
| 230 | 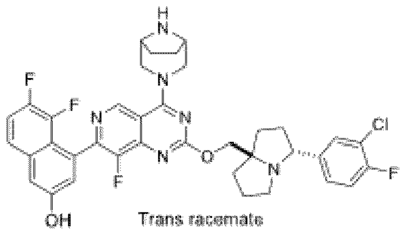 Trans racemate | 705.2 | | 140 |
| 231 | 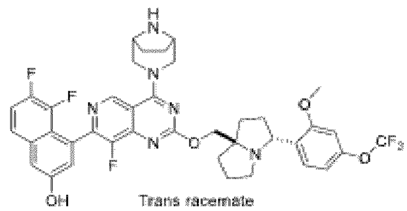 Trans racemate | 767.3 | 1H NMR (400 MHz, $d_6$-DMSO) δ 10.29 (brs, 1H), 9.13 (s, 1H), 7.77-7.70 (m, 1H), 7.63-7.53 (m, 1H), 7.51-7.48 (m, 1H), 7.36 (brs, 1H) , 7.26 (brs, 1H), 7.02-6.90 (m, 2H), 4.52-4.31 (m, 3H), 4.27-4.08 (m, 2H), 3.79 (s, 3H), 3.70-3.53 (m, 4H), 2.30-1.95 (m, 5H), 1..92-1..74 (m, 3H) , 1.69-1.45 (m, 6H). | 140 |
| 232 | 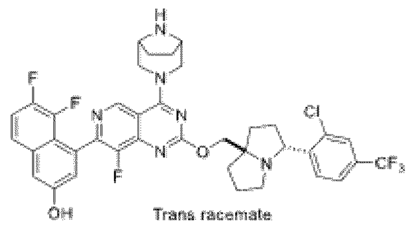 Trans racemate | 755.2 | | 140 |
| 233 | 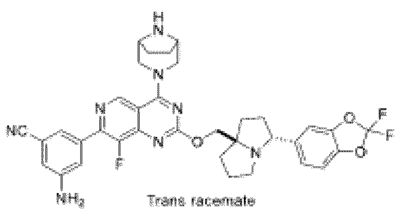 Trans racemate | 671.3 | 1H NMR (400 MHz, $CDCl_3$) δ 9.01 (s, 1H), 7.77 (s, 1H), 7.62 (s, 1H), 7.27-7.19 (m, 2H), 7.04-6.99 (m, 2H), 4.64-4.34 (m, 5H) , 4.07 (brs, 2H), 3.74-3.59 (m, 4H), 2.68-2.58 (m, 1H), 2.39-2.26 (m, 3H). 2.18-2.05 (m, 4H) , 1.83-1.73 (m, 5H), 1.68-1.59 (m, 1H). | 161 |

(continued)

| Example | Structure | MS (ESI) m/z | $^1$H NMR | Example of reference for synthesis |
|---|---|---|---|---|
| 134 | 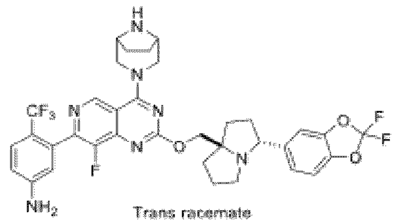 Trans racemate | 714.3 | | 161 |
| 235 | 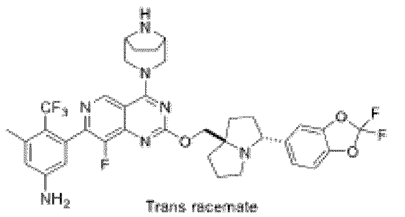 Trans racemate | 728.3 | | 161 |
| 236 | 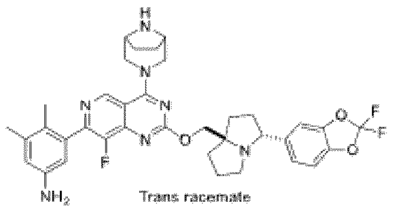 Trans racemate | 674.3 | 1H NMR (400 MHz, $CDCl_3$) δ 9.02 (s, 1H), 7.24-7.20 (m, 2H), 7.04-7.02 (m, 1H), 6.66-6.61 (m, 2H), 4.64-4.36 (m, 6H), 3.73-3.58 (m, 5H) , 2.68-2.57 (m, 1H), 2.42-2.30 (m, 3H), 2.27 (s, 3H), 2.20-2.08 (m, 4H), 2.04 (s, 3H) , 1.85-1.75 (m, 5H), 1.68-1.58 (m, 1H). | 161 |
| 237 | 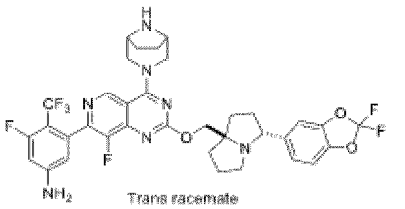 Trans racemate | 732.2 | | 161 |
| 238 | 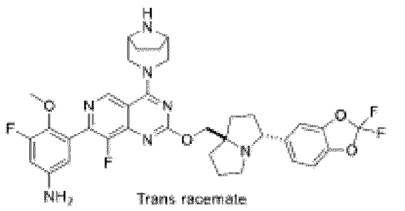 Trans racemate | 694.3 | | 162 |
| 239 | 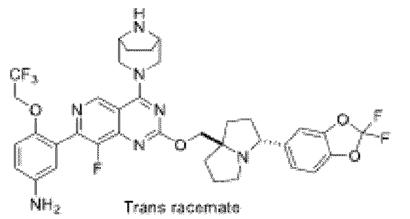 Trans racemate | 744.3 | 1H NMR (400 MHz, $d_6$-DMSO) δ 9.06 (s, 1H), 7.37-7.33 (m, 2H), 7.18-7.16 (m, 1H), 7.01-6.99 (m, 1H), 6.70-6.66 (m, 2H), 5.01 (s, 2H) , 4.53 (q, J = 8.0 Hz, 2H), 4.42-4.38 (m, 2H), 4.27-4.16 (m, 3H), 3.58-3.52 (m, 4H), 2.32-2.07 (m, 3H), 1.95-1.75 (m, 3H), 1.56-1.33, (m, 5H). | 162 |

(continued)

| Exam ple | Structure | MS (ESI) m/z | $^1$H NMR | Examp le of re ference for syn thesis |
|---|---|---|---|---|
| 240 | 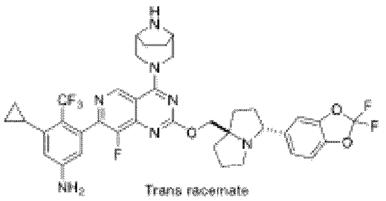 Trans racemate | 754.3 | | 165 |
| 241 | 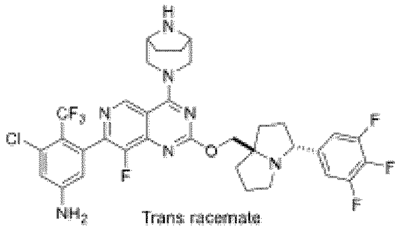 Trans racemate | 722.2 | | 161 |
| 242 | 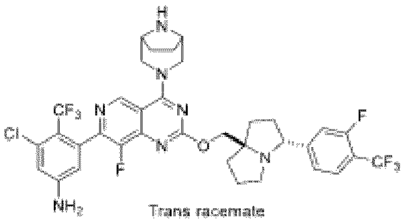 Trans racemate | 754.2 | | 161 |
| 243 | 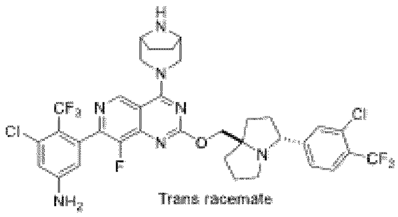 Trans racemate | 770.2 | | 161 |
| 244 | 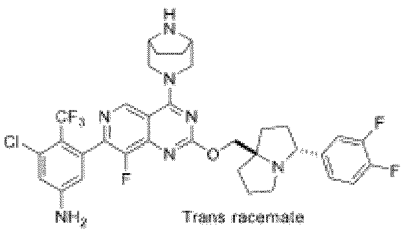 Trans racemate | 704.2 | | 161 |
| 245 | 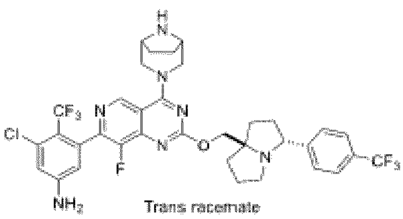 Trans racemate | 736.2 | | 161 |

(continued)

| Example | Structure | MS (ESI) m/z | $^1$H NMR | Example of reference for synthesis |
|---|---|---|---|---|
| 246 | 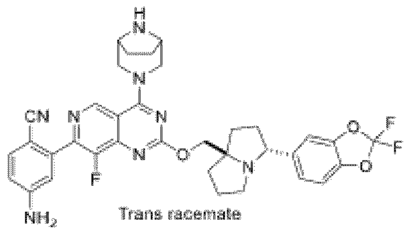 Trans racemate | 671.3 | | 161 |

**Drug efficacy test**

**Test Example 1: KRAS G12D GTP::SOS1 homogeneous time-resolved fluorescence binding experiment**

1. Test method:

Protein-protein interactions were determined by homogeneous time-resolved fluorescence technology.

In a 384-well reaction plate (Corning, CLS4514), 0.1 microliter of the compound was added. After centrifugation, a mixture of 5 microliters of Tag2-KRAS G12D (Cisbio, 63ADK000CB17PEG) protein and GTP (SIGMA, V900868) with a final concentration of 10 μM was added. Then, 5 microliters of Tag1-SOS1 (Cisbio, 63ADK000CB17PEG) protein solution was added, and the reaction was allowed to proceed at room temperature for 15 min. All protein interactions occurred in Diluent (Cisbio, 62DLBDDF) buffer solution. 10 microliters of premixed 100X Anti-Tag1-Tb and 25X Anti-Tag2-XL665 detection solutions were added, and the reaction was allowed to proceed at 4°C for 180 min. The detection reagents occurred in Detection Buffer (Cisbio, 62DB2FDG). The reaction signals were detected by a multifunctional microplate reader, and the data was analyzed by using GraphPad Prism data analysis software.

2. The inhibitory activity of the compounds of the present disclosure on KRAS G12D GTP::SOS1 binding is shown in Table 3.

Table 3: Inhibitory activity of the compounds of the present disclosure on KRAS G12D GTP::SOS binding (IC50: A<=100, 100<B<=500, 500<C<=1000, D>1000)

| Example | IC50 | Example | IC50 | Example | IC50 |
|---|---|---|---|---|---|
| 1 | D | 2 | B | 3 | D |
| 4 | B | 5 | A | 6 | A |
| 7 | D | 8 | D | 9 | D |
| 10 | D | 11 | A | 12 | A |
| 13 | D | 14 | B | 15 | D |
| 16 | B | 17 | A | 18 | C |
| 19 | A | 20 | C | 21 | D |
| 22 | D | 23 | D | 24 | D |
| 25 | D | 26 | D | 27 | D |
| 28 | B | 29 | D | 53 | A |
| 54 | B | 55 | B | 56 | A |
| 57 | D | 58 | B | 59 | D |
| 60 | D | 61 | D | 62 | D |
| 63 | D | 64 | D | 65 | D |

(continued)

| Example | IC50 | Example | IC50 | Example | IC50 |
|---|---|---|---|---|---|
| 66 | D | 67 | A | 68 | C |
| 69 | D | 70 | A | 71 | A |
| 72 | D | 73 | B | 74 | D |
| 75 | D | 76 | A | 77 | A |
| 78 | A | 79 | D | 80 | D |
| 81 | D | 82 | A | 83 | A |
| 84 | A | 85 | B | 86 | C |
| 87 | A | | | | |

Conclusion: From the above data, it can be seen that some compounds of the present disclosure can effectively block the binding of KRAS G12D protein to SOS1, and can be used to treat pancreatic ductal adenocarcinoma, colon cancer, rectal cancer, non-small cell lung cancer and the like, indicating broad application prospects.

**Test Example 2: Inhibitory activity test of compounds on KRas Gl2D-mediated ERK phosphorylation**

1. Test method

Day 1: AGS cells were seeded into 384-well cell culture plates and cultured overnight in a 37°C, 5% $CO_2$ incubator.

Day 2: The compounds were added to the plates using Echo550 and incubated for 3 h in a 37°C, 5% $CO_2$ incubator. Then cells were fixed with paraformaldehyde, permeated with methanol, and blocked with the blocking solution. Finally, a mixed solution of primary antibodies (rabbit anti pERK, mouse anti GAPDH) was added, and the mixture was incubated at 4°C overnight.

Day 3: The mixed solution of primary antibodies was discarded, and a mixed solution of secondary antibodies (goat anti rabbit 800CW, goat anti mouse 680RD) was added. The mixture was incubated at room temperature in the dark. Finally, the cell culture plate was washed with PBST, and then inverted for 1 minute of centrifugation. The fluorescence signal values were read with Odyssey CLx.

ERK phosphorylation was calculated according to the following formula:

Relative expression: (fluorescence signal ratio of compound - average value of positive control) / (average value of negative control - average value of positive control)

Negative control: DMSO

Positive control: 10 μM Reference

IC50 value was fitted and calculated by XLFit 5.0 using the parametric formula.

$$Y = Bottom + ( Top - Bottom ) / ( 1 + 10 \wedge (( Log\,IC50 - X ) \times HillSlope ))$$

X: log value of compound concentration.

Y: average value of relative expression of p-ERK between replicate wells.

2. The inhibitory activity of the compounds of the present disclosure on ERK phosphorylation is shown in Table 4.

Table 4: Inhibitory activity of the compounds of the present disclosure on ERK phosphorylation (IC50: A<=200, 200<B<=1000, 1000<C<=10000, D>10000)

| Example | IC50 | Example | IC50 | Example | IC50 |
|---|---|---|---|---|---|
| 5 | C | 6 | C | 11 | D |
| 12 | C | 14 | C | 16 | D |
| 17 | D | 18 | D | 19 | C |
| 23 | D | 25 | D | 27 | D |
| 28 | C | 29 | D | 30 | A |
| 31 | B | 32 | A | 33 | B |
| 34 | A | 35 | C | 36 | C |
| 37 | B | 38 | B | 39 | D |
| 40 | B | 41 | A | 42 | D |
| 43 | A | 44 | B | 45 | B |
| 46 | B | 47 | B | 48 | C |
| 49 | B | 50 | D | 51 | D |
| 52 | A | 56 | B | 67 | D |
| 68 | D | 70 | C | 71 | C |
| 73 | C | 76 | A | 77 | D |
| 78 | D | 81 | D | 82 | C |
| 83 | A | 84 | C | 85 | C |
| 86 | D | 87 | A | 88 | A |
| 89 | C | 90 | B | 91 | A |
| 92 | A | 93 | A | 94 | C |
| 95 | B | 96 | D | 97 | C |
| 98 | D | 99 | B | 100 | A |
| 101 | B | 102 | C | 103 | A |
| 104 | A | 105 | A | 106 | A |
| 107 | A | 108 | A | 109 | A |
| 110 | A | 111 | A | 112 | B |
| 113 | A | 114 | A | 115 | C |
| 116 | C | 117 | D | 118 | B |
| 119 | B | 120 | A | 121 | B |
| 122 | C | 123 | B | 124 | A |
| 125 | A | 126 | A | 127 | B |
| 128 | A | 129 | C | 130 | B |
| 131 | B | 132 | B | 133 | C |
| 134 | B | 135 | C | 136 | C |
| 137 | B | 138 | A | 139 | A |
| 140 | A | 141 | A | 142 | A |
| 143 | C | 144 | C | 145 | B |
| 146 | A | 147 | A | 148 | B |
| 149 | B | 150 | B | 151 | B |

(continued)

| Example | IC50 | Example | IC50 | Example | IC50 |
|---|---|---|---|---|---|
| 152 | B | 153 | A | 154 | A |
| 155 | A | 156 | A | 157 | A |
| 158 | A | 159 | A | 160 | A |
| 161 | A | 162 | A | 163 | A |
| 164 | A | 165 | C | 166 | B |
| 167 | / | 168 | / | 169 | / |
| 170 | C | 171 | B | 172 | C |
| 173 | C | 174 | B | 175 | B |
| 176 | C | 177 | B | 178 | A |
| 179 | C | 180 | D | 181 | B |
| 182 | C | 183 | C | 184 | C |
| 185 | C | 186 | B | 187 | A |
| 188 | A | 189 | A | 190 | B |
| 191 | A | 192 | A | 193 | A |
| 194 | A | 195 | A | 196 | A |
| 197 | A | 198 | A | 199 | A |
| 200 | A | 201 | A | 202 | A |
| 203 | A | 204 | A | 205 | A |
| 206 | A | 207 | A | 208 | A |
| 209 | A | 210 | A | 211 | A |
| 212 | A | 213 | A | 214 | A |
| 215 | A | 216 | A | 217 | A |
| 218 | A | 219 | A | 220 | A |
| 221 | A | 222 | A | 223 | A |
| 224 | A | 225 | A | 226 | A |
| 227 | A | 228 | A | 229 | A |
| 230 | A | 231 | A | 232 | A |
| 233 | D | 234 | B | 235 | A |
| 236 | B | 237 | A | 238 | C |
| 239 | C | 240 | B | 241 | A |
| 242 | A | 243 | B | 244 | A |
| 245 | B | 246 | C | | |

Table 5: AGS-pERK IC50 values of some compounds

| Example | AGS-pERK IC50 (nM) | Example | AGS-pERK IC50 (nM) |
|---|---|---|---|
| 34 | 27 | 198 | 0.72 |
| 43 | 41 | 200 | 0.59 |
| 52 | 6.0 | 202 | 1.8 |
| 108 | 14 | 204 | 0.78 |

(continued)

| Example | AGS-pERK IC50 (nM) | Example | AGS-pERK IC50 (nM) |
|---|---|---|---|
| 110 | 35 | 211 | 0.96 |
| 113 | 15 | 213 | 1.8 |
| 114 | 14 | 214 | 1.0 |
| 142 | 0.83 | 216 | 1.6 |
| 146 | 11 | 217 | 1.3 |
| 148 | 12 | 218 | 2.4 |
| 149 | 23 | 219 | 6.2 |
| 151 | 7.6 | 220 | 3.2 |
| 153 | 5.2 | 222 | 1.1 |
| 156 | 15 | 223 | 0.74 |
| 178 | 3.8 | 224 | 0.70 |
| 187 | 13 | 225 | 1.5 |
| 188 | 5.2 | 227 | 2.4 |
| 192 | 1.8 | 228 | 1.9 |
| 193 | 9.1 | 235 | 13 |
| 194 | 1.1 | 244 | 6.4 |
| 196 | 1.4 | MRTX1133 | 1.2 |

Conclusion: From the above data, it can be seen that the compounds of the present invention can effectively inhibit the phosphorylation of ERK, a downstream target of the KRAS pathway. The activity of some compounds was comparable to that of MRTX1133, and the activity of some compounds was superior to that of MRTX1133, indicating broad application prospects.

**Test Example 3: 3D Cell Proliferation Inhibition Test of Compounds**

The diluted compounds to be tested were added to 384-well low-adhesion cell culture plates by using a nanoliter pipetting system (LABCYTE, P-0200). Then, the cells were plated and incubated in a 37°C, 5% $CO_2$ incubator for 7 days. Afterward, CellTiter-Glo® 2D reagent was added, and the luminescent values were read by an Envision multifunctional microplate reader (the luminescent signal is proportional to the amount of ATP in the system, and the ATP content directly represents the number of viable cells in the system). Finally, the IC50 values (half-maximal inhibitory concentration) of the compounds were obtained by the XLFIT software using the nonlinear fitting formula.

1. Experimental Method

   a) The diluted compounds to be tested were added to 384-well low-adsorption cell culture plates by using a nanoliter pipetting system.

   b) Cells were seeded into the 384-well plates in a) and then centrifuged at 1000 rpm for 1 min at room temperature, with a final DMSO concentration of compound being 0.5%. The plates were then incubated in a 37°C, 5% $CO_2$ constant temperature incubator for 7 days.

   c) CellTiter-Glo® 3D was added to the 384-well cell culture plates in b), shaken in the dark, and incubated at room temperature.

   d) The luminescence values were read by using an Envision multifunctional microplate reader.

2. Data Analysis

The IC50 (half-maximal inhibitory concentration) values of the compounds were obtained by using XLFIT software according to the following nonlinear fitting formula:

Y=Bottom + (Top-Bottom)/(1+10^((LogIC50-X)× HillSlope))

X: log value of compound concentration.

Y: inhibition rate (%)

Inhibition rate (%)=100× (average value of negative control - compound)/(average value of negative control - average value of positive control)

Negative control: DMSO.

Positive control: Medium only.

3. The 3D cell proliferation inhibition activity data of some compounds of the present disclosure are shown in Table 6.

Table 6: Anti-cell proliferation activity data of some compounds

| Example | GP2d cells 3D CTG IC50 (nM) | ASPC-1 cells 3D CTG IC50 (nM) |
|---|---|---|
| 142 | 1.15 | 13.95 |
| 161 | 10.95 | 46.92 |
| 192 | 3.51 | 12.20 |
| 223 | 0.67 | 3.09 |
| 225 | 1.49 | 11.06 |
| MRTX1133 | 1.38 | 10.88 |

Conclusion: From the above data, it can be seen that some compounds of the present disclosure can effectively inhibit the proliferation of KRAS G12D mutant cells with activities comparable to that of MRTX1133.

**Test Example 4: Pharmacokinetics of Compounds in Rats**

Male SD rats were purchased from Shanghai SIPPR-Bk Lab Animal Co., Ltd. Each test compound was administered orally (3 rats per group) once to SD rats for pharmacokinetic study. The test compounds were prepared on the day of administration. The test compounds were suspended in 5% DMSO + 95% saline, and then vortexed for 2 min and ultrasonicated for 5 min to prepare the administration system. The animals were fasted for 10-14 h before oral administration, and the feeding was resumed 4 h after administration. After oral administration by gavage, pharmacokinetic samples were collected from SD rats through the jugular vein. The collection time points were: before administration, 15 min, 30 min, 1 h, 2 h, 4 h, 6 h, 8 h and 24 h after administration. Three whole blood samples were collected at each time point, with a collection volume of about 0.2 mL, and anticoagulated with sodium heparin. After blood samples were collected, they were immediately placed on ice and centrifuged to separate plasma within 1 hour (centrifugation conditions: 6800 rpm, 6 min, 2-8°C). The collected plasma was stored in a -80°C refrigerator before analysis.

Table 7: Pharmacokinetic test results of some compounds

| Example | Dose | Cmax (ng/ml) | AUC (ng/ml*h) |
|---|---|---|---|
| 34 | 10 mg/kg | 25 | 75 |
| 45 | 10 mg/kg | 21 | 94 |
| 86 | 10 mg/kg | 21 | 52 |
| 118 | 10 mg/kg | 31 | 224 |
| 131 | 10 mg/kg | 13 | 57 |

(continued)

| Example | Dose | Cmax (ng/ml) | AUC (ng/ml*h) |
|---|---|---|---|
| 132 | 10 mg/kg | 29 | 240 |
| 143 | 10 mg/kg | 32 | 410 |
| 170 | 10 mg/kg | 39 | 533 |
| MRTX1133 | 10 mg/kg | 1-2 | No parameter calculation due to a too low blood drug concentration |
| | 100 mg/kg | 26 | 70 |
| 43 | 100 mg/kg | 164 | 537 |
| 108 | 100 mg/kg | 157 | 2086 |
| 118 | 100 mg/kg | 115 | 1290 |
| 140 | 100 mg/kg | 113 | 444 |
| 142 | 100 mg/kg | 96 | 404 |
| 146 | 100 mg/kg | 143 | 483 |
| 149 | 100 mg/kg | 148 | 1192 |
| 153 | 100 mg/kg | 156 | 458 |
| 156 | 100 mg/kg | 138 | 598 |
| 161 | 100 mg/kg | 193 | 1080 |
| 163 | 100 mg/kg | 388 | 2748 |
| 187 | 100 mg/kg | 109 | 567 |
| 188 | 100 mg/kg | 149 | 862 |
| 190 | 100 mg/kg | 66 | 939 |
| 192 | 100 mg/kg | 96 | 284 |
| 198 | 100 mg/kg | 141 | 219 |
| 210 | 100 mg/kg | 145 | 246 |
| 211 | 100 mg/kg | 97 | 225 |
| 215 | 100 mg/kg | 128 | 471 |
| 220 | 100 mg/kg | 38 | 140 |
| 222 | 100 mg/kg | 82 | 258 |
| 223 | 100 mg/kg | 260 | 404 |
| 225 | 100 mg/kg | 54 | 380 |
| 227 | 100 mg/kg | 52 | 245 |
| 234 | 100 mg/kg | 182 | 1190 |
| 237 | 100 mg/kg | 362 | 1508 |

The oral pharmacokinetics of MRTX1133 was extremely poor, while the Cmax and AUC of the compounds in some embodiments of the present disclosure were significantly better than those of MRTX1133, indicating great development potential.

The above compound provided in the present disclosure is useful in the treatment of cancer caused by KRAS mutation, wherein the cancer caused by KRAS mutation is one or more of the cancers caused by KRAS G12C, KRAS G12V, KRAS G12A, and G12D mutations, and is useful as, in particular, a G12D inhibitor. The compound provided by the present disclosure has high inhibitory activity and good availability, is useful in the manufacture of a medicament for preventing and/or treating a disease mediated by KRAS G12D, and has promising applications.

The above are only preferred embodiments of the present disclosure. It should be noted that the above preferred embodiments should not be regarded as limiting the present disclosure, and the protection scope of the present disclosure

should be based on the scope defined by the claims. For ordinary technicians in this technical field, several improvements and modifications can be made without departing from the spirit and scope of the present disclosure, and these improvements and modifications should also be regarded as the protection scope of the present disclosure.

## Claims

1. A compound represented by formula (I), or a tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof:

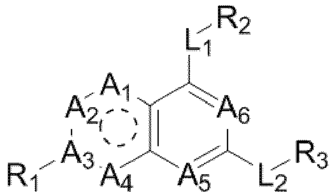

(I);

wherein,

$A_1$, $A_2$, $A_3$, $A_4$, $A_5$ and $A_6$ are each independently selected from the group consisting of C-$R_4$ and N,
$R_1$ is selected from the group consisting of substituted or unsubstituted aryl, and substituted or unsubstituted heteroaryl,
$R_2$ is selected from the group consisting of substituted or unsubstituted cycloalkyl, and substituted or unsubstituted heterocycloalkyl,
$R_3$ is selected from the group consisting of substituted or unsubstituted cycloalkyl, and substituted or unsubstituted heterocycloalkyl,
$R_4$ in $A_1$, $A_2$, $A_3$, $A_4$, $A_5$ and $A_6$ is independently selected from the group consisting of hydrogen, deuterium, halogen, substituted or unsubstituted alkyl or heteroalkyl, substituted or unsubstituted cycloalkyl or heterocycloalkyl, substituted or unsubstituted unsaturated cyclyl or heterocyclyl, substituted or unsubstituted aryl or heteroaryl, hydroxy, cyano, amino, ester, nitro, thiol, amido, sulfonyl, phosphoryl, alkylphosphinoyl, alkylsulfonyl and alkylsulfinyl;
$L_1$ and $L_2$ are absent or independently selected from the group consisting of -CH=CH-, -N(Ra)-, -N(Ra)-(CRaRb)n -, -O-(CRaRb)n-, and -(CRaRb)n-;
Ra and Rb are each independently selected from the group consisting of hydrogen, deuterium, C1-C6 alkyl, C1-C6 heteroalkyl, and C3-C6 cycloalkyl.

2. The compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 1, having a structure represented by formula (II),

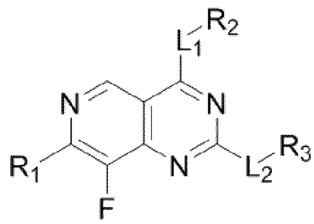

(II);

wherein,

$R_1$ is selected from the group consisting of substituted or unsubstituted aryl, and substituted or unsubstituted heteroaryl,

$R_2$ is selected from the group consisting of substituted or unsubstituted cycloalkyl, and substituted or unsubstituted heterocycloalkyl,

$R_3$ is selected from the group consisting of substituted or unsubstituted cycloalkyl, and substituted or unsubstituted heterocycloalkyl,

$L_1$ is absent or selected from the group consisting of -N(Ra)- and -N(Ra)-(CRaRb)n-, $L_2$ is selected from the group consisting of -O-(CRaRb)n-, -CH=CH-, and -(CRaRb)n-, and Ra and Rb are each independently selected from the group consisting of hydrogen, deuterium, C1-C6 alkyl, C1-C6 heteroalkyl, and C3-C6 cycloalkyl.

**3.** The compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 2, having a structure represented by formula (III) or (IV),

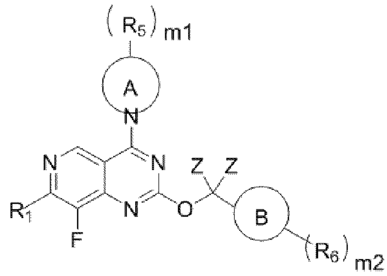

(III),

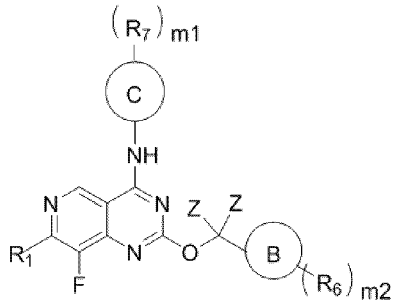

(IV),

wherein,

ring A is a 4- to 12-membered heterocyclic ring with N and/or O as a heteroatom;

ring B is a 4 to 12-membered heterocyclic ring with N as a heteroatom,

the ring A and ring B are each independently selected from the group consisting of a saturated or partially saturated monocycle, fused ring, bridged ring, and spiro ring,

Z is selected from the group consisting of hydrogen and deuterium;

$R_1$ is selected from the group consisting of substituted or unsubstituted aryl, and substituted or unsubstituted heteroaryl,

$R_5$, $R_6$, and $R_7$ are each independently selected from the group consisting of hydrogen, deuterium, halogen, substituted or unsubstituted alkyl or cycloalkyl, substituted or unsubstituted heteroalkyl or heterocycloalkyl, hydroxy, cyano, amino, ester, amido, aryl, heteroaryl, acylguanidino, sulfonyl, phosphoryl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidino, sulfonyl, phosphoryl, sulfonate, and phosphate,

m1 is an integer of 0 to 6; and

m2 is an integer of 0 to 6.

**4.** The compound, or the tautomer, mesomer, racemate, enantiomer, diastereomer or a mixture thereof, metabolite,

metabolic precursor, isotope-substituted form, pharmaceutically acceptable salt, hydrate, solvate, polymorph or cocrystal thereof according to claim 3, wherein ring B is selected from the group consisting of:

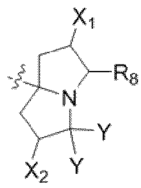
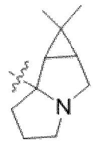
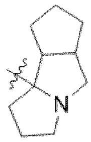
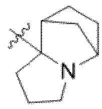
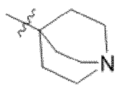
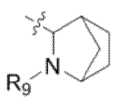
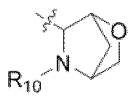
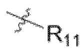
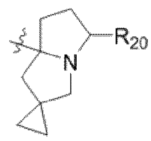
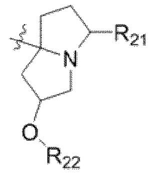

wherein,

$X_1$ and $X_2$ are independently selected from the group consisting of hydrogen, deuterium, and halogen; Y is selected from the group consisting of hydrogen and deuterium;

$R_8$, $R_{20}$, and $R_{21}$ are each independently selected from the group consisting of hydrogen, substituted or unsubstituted aryl or heteroaryl, substituted or unsubstituted alkyl or cycloalkyl, and substituted or unsubstituted heteroalkyl or heterocycloalkyl;

$R_9$, $R_{10}$, and $R_{22}$ are each independently selected from the group consisting of hydrogen, substituted or unsubstituted alkyl or cycloalkyl, substituted or unsubstituted heteroalkyl or heterocycloalkyl, ester, amido, aryl, heteroaryl, acylguanidinyl, sulfonyl, phosphonyl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidinyl, sulfonyl, phosphonyl, sulfonate, and phosphate; and

$R_{11}$ is substituted monocyclic heteroalkyl with nitrogen and/or oxygen as a heteroatom.

**5.** The compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 3, wherein ring A is selected from the group consisting of:

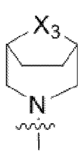
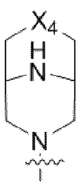
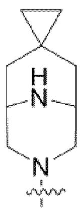
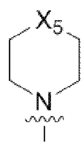
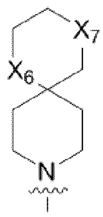
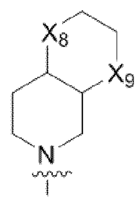
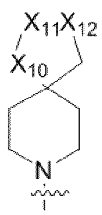
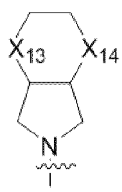

ring C

is 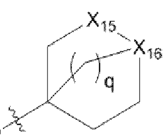 ;

wherein,

$X_3$ to $X_{16}$ are each independently selected from the group consisting of $-NR_{12}-$, -O-, -CO-, and $-CR_{14}R_{15}-$;

$R_{12}$, $R_{14}$, and $R_{15}$ are each independently selected from the group consisting of hydrogen, deuterium, halogen, substituted or unsubstituted alkyl or cycloalkyl, substituted or unsubstituted heteroalkyl or heterocycloalkyl,

hydroxy, cyano, amino, ester, amido, aryl, heteroaryl, acylguanidino, sulfonyl, phosphoryl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidino, sulfonyl, phosphoryl, sulfonate, and phosphate; and

q is an integer of 0 to 3, and q=0 indicates this chemical bond is absent.

**6.** The compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 3, wherein $R_1$ is selected from the group consisting of:

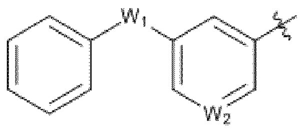
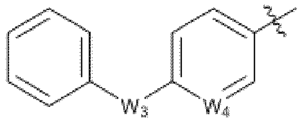
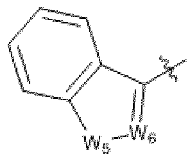
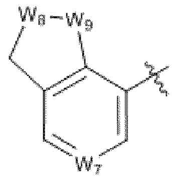

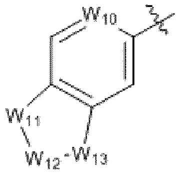
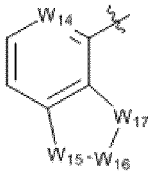
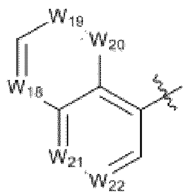
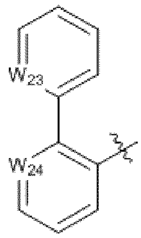

and

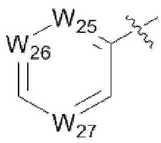
;

wherein, $W_1$, $W_3$, $W_5$, $W_8$, $W_9$, $W_{11}$, $W_{12}$, $W_{13}$, $W_{15}$, $W_{16}$, and $W_{17}$ are each independently selected from the group consisting of $-NR_{12}-$, -O-, -CO-, $-CR_{17}R_{18}-$, and $-SO_2-$; $W_2$, $W_4$, $W_6$, $W_7$, $W_{10}$, $W_{14}$, $W_{18}$, $W_{19}$, $W_{20}$, $W_{21}$, $W_{22}$, $W_{23}$, $W_{24}$, $W_{25}$, $W_{26}$, and $W_{27}$ are each independently selected from the group consisting of N atom, and C atom with $R_{16}$ as a substituent; when $W_{23}$ and $W_{24}$ are C atoms with $R_{16}$ as a substituent, the two carbon atoms are optionally connected by a chemical bond to form a saturated or unsaturated 5-membered or 6-membered ring; and

$R_{12}$, $R_{16}$, $R_{17}$, and $R_{18}$ are each independently selected from the group consisting of hydrogen, deuterium, halogen, substituted or unsubstituted alkyl or cycloalkyl, substituted or unsubstituted heteroalkyl or heterocycloalkyl, hydroxy, alkynyl, cyano, amino, ester, amido, aryl, heteroaryl, acylguanidino, sulfonyl, phosphoryl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidino, sulfonyl, phosphoryl, sulfonate, and phosphate.

**7.** The compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 3, having a structure represented by formula (V), formula (VI) or formula (VII):

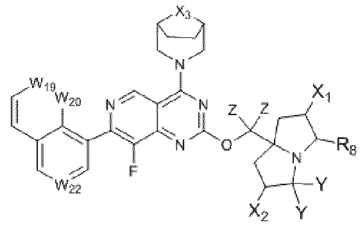

(V)

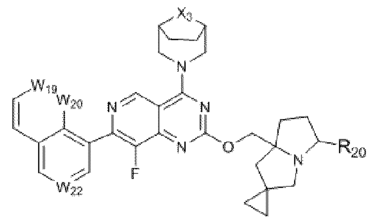

(VI)

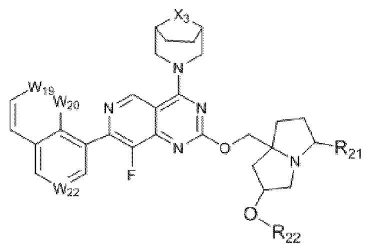

(VII)

wherein,

$X_1$ and $X_2$ are independently selected from the group consisting of hydrogen, deuterium, and halogen; Y is selected from the group consisting of hydrogen and deuterium; Z is selected from the group consisting of hydrogen and deuterium;

$R_8$, $R_{20}$, and $R_{21}$ are each independently selected from the group consisting of hydrogen, substituted or unsubstituted aryl or heteroaryl, substituted or unsubstituted alkyl or cycloalkyl, and substituted or unsubstituted heteroalkyl or heterocycloalkyl;

$R_{22}$ is selected from the group consisting of hydrogen, substituted or unsubstituted alkyl or cycloalkyl, substituted or unsubstituted heteroalkyl or heterocycloalkyl, ester, amido, aryl, heteroaryl, acylguanidinyl, sulfonyl, phosphonyl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidinyl, sulfonyl, phosphonyl, sulfonate, and phosphate;

$X_3$ is selected from the group consisting of $-NR_{12}-$, -O-, -CO-, and $-CR_{14}R_{15}-$; and

$W_{19}$, $W_{20}$, and $W_{22}$ are independently selected from the group consisting of N atom, and C atom with $R_{16}$ as a substituent.

**8.** The compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 7, having a structure represented by formula (VIII), formula (IX) or formula (X):

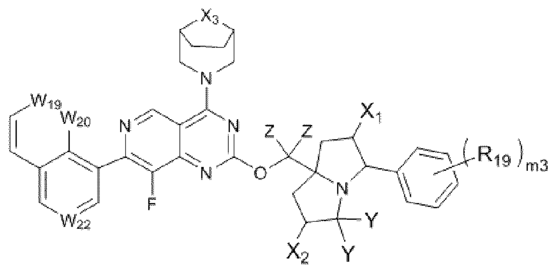

(VIII)

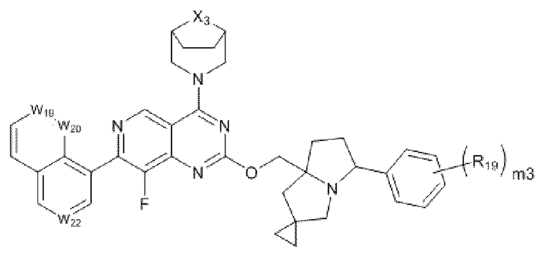

(IX)

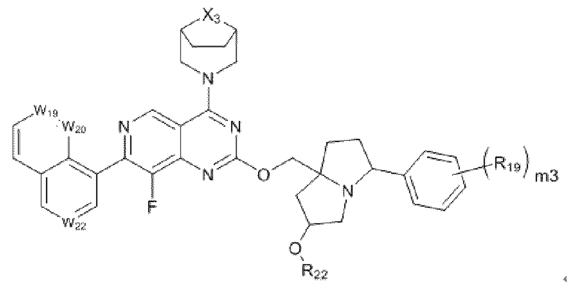

(X)

wherein, $X_1$ and $X_2$ are independently selected from the group consisting of hydrogen, deuterium, and halogen; Y is selected from the group consisting of hydrogen and deuterium; Z is selected from the group consisting of hydrogen and deuterium;

$X_3$ is selected from the group consisting of $-NR_{12}-$, -O-, -CO-, and $-CR_{14}R_{15}-$;

$W_{19}$, $W_{20}$, and $W_{22}$ are each independently selected from the group consisting of N atom, and C atom with $R_{16}$ as a substituent;

$R_{19}$ is selected from the group consisting of -H, -OH, halogen, $-NO_2$, -CN, $-CF_3$, $-C_2F_5$, $-OCF_3$, $-OCHF_2$, $-OCH_2F$, substituted or unsubstituted alkyl or cycloalkyl, substituted or unsubstituted heteroalkyl or heterocycloalkyl, alkoxy, alkynyl, ester, amido, aryl, heteroaryl, acylguanidinyl, sulfonyl, phosphonyl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidinyl, sulfonyl, phosphonyl, sulfonate, and phosphate;

$R_{22}$ is selected from the group consisting of hydrogen, substituted or unsubstituted alkyl or cycloalkyl, substituted or unsubstituted heteroalkyl or heterocycloalkyl, alkoxy, alkynyl, ester, amido, aryl, heteroaryl, acylguanidinyl, sulfonyl, phosphonyl, sulfonate, and phosphate, wherein the substituent is each independently selected from the group consisting of halogen, alkyl, cycloalkyl, heteroalkyl, heterocycloalkyl, hydroxy, cyano, amino, ester, amido, aryl, heteroaryl, acylguanidinyl, sulfonyl, phosphonyl, sulfonate, and phosphate; and

m3 is an interger of 0 to 5, and when m3 is greater than or equal to 2, $R_{19}$ is optionally a 4-to 7-membered ring connected in parallel with a benzene ring.

**9.** The compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 3, having a structure represented by formula (XI) or formula (XII):

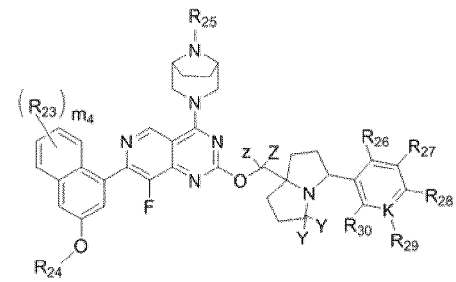

(XI)

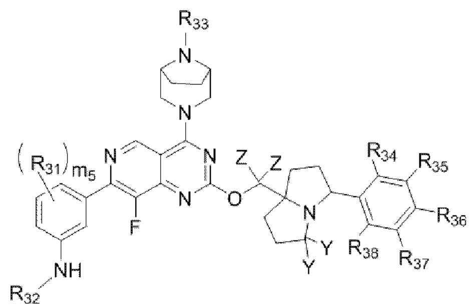

(XII)

wherein Y is selected from the group consisting of hydrogen and deuterium; Z is selected from the group consisting of hydrogen and deuterium;

wherein $R_{23}$ in formula (XI) is independently selected from the group consisting of -H, -F, -Cl, -CN, $-CF_3$, alkenyl, alkynyl, $C_{1-3}$ linear alkyl, and $C_{3-6}$ cycloalkyl;

$R_{24}$ is selected from the group consisting of -H, $C_{1-5}$ alkyl or heteroalkyl, and $-COR_{55}$, and $R_{55}$ is selected from the group consisting of -H, $C_{1-10}$ linear/branched alkyl, $C_{1-10}$ linear/branched heteroalkyl, $C_{3-6}$ cycloalkyl, and $C_{3-6}$ heterocycloalkyl;

$R_{25}$ is selected from the group consisting of -H, -CO($C_{0-10}$ alkyl), -COO($C_{0-10}$ alkyl), $-COO-CH_2-OCO(C_{0-10}$ alkyl), and $-COO-CH(CH_3)-OCO(C_{0-10}$ alkyl);

K is selected from the group consisting of C and N, when K is N, $R_{29}$ is absent;

$R_{26}$, $R_{27}$, $R_{28}$, $R_{29}$ and $R_{30}$ are each independently selected from the group consisting of -H, -OH, halogen, $-NO_2$, -CN, $-CF_3$, $-C_2F_5$, $-OCF_3$, $-OCHF_2$, $-OCH_2F$, phosphate, monomethylphosphate, dimethylphosphate, $C_{1-5}$ linear/branched alkyl, $C_{1-5}$ alkoxy, $C_{3-10}$ cycloalkyl, $C_{3-10}$ heterocycloalkyl, -N($C_{0-10}$ alkyl)($C_{0-10}$ alkyl), -S($C_{0-10}$ alkyl), -OCON($C_{0-10}$ alkyl)($C_{0-10}$ alkyl), $C_{5-6}$ aryl, five-membered heteroaryl, and six-membered heteroaryl; alternatively, $R_{27}$, $R_{28}$ and the carbon atom between $R_{27}$ and $R_{28}$ form saturated or unsaturated $C_{3-8}$ cycloalkyl, saturated or unsaturated $C_{3-8}$ heterocycloalkyl, saturated or unsaturated cyclic lactone, $C_{5-6}$ aryl, bridged cycloalkyl, or spirocycloalkyl, wherein H thereof is optionally substituted with -D, -OH, -F, $-NO_2$, -CN, $-CF_3$, $-C_2F_5$, $C_{1-3}$ alkyl, or amido;

m4 is an interger of 0 to 4;

wherein, $R_{31}$ in formula (XII) is independently selected from the group consisting of -F, -Cl, $-NH_2$, -CN, $-CF_3$, $-C_2F_5$, $-OCF_3$, alkenyl, alkynyl, $C_{1-3}$ linear/branched alkyl, $C_{1-3}$ linear/branched alkoxy, $C_{3-6}$ cycloalkyl, aryl, and heteroaryl;

$R_{32}$ is selected from the group consisting of -H, $C_{1-5}$ linear/branched alkyl, and $C_{3-6}$ cycloalkyl;

$R_{33}$ is selected from the group consisting of -H, -CO($C_{0-10}$ alkyl), -COO($C_{0-10}$ alkyl), $-COO-CH_2-OCO(C_{0-10}$ alkyl), and $-COO-CH(CH_3)-OCO(C_{0-10}$ alkyl);

$R_{34}$, $R_{35}$, $R_{36}$, $R_{37}$, and $R_{38}$ are independently selected from the group consisting of -H, -OH, halogen, $-NO_2$, -CN, $-CF_3$, $-C_2F_5$, $-OCF_3$, $-OCHF_2$, $-OCH_2F$, $C_{1-5}$ linear/branched alkyl, $C_{1-5}$ alkoxy, $C_{3-10}$ cycloalkyl, and $C_{3-10}$ heterocycloalkyl; alternatively, $R_{35}$, $R_{36}$ and the carbon atom between $R_{35}$ and $R_{36}$ form saturated or unsaturated $C_{3-8}$ cycloalkyl or saturated or unsaturated $C_{3-8}$ heterocycloalkyl, wherein H thereof is optionally substituted with -D, -OH, -F, $-NO_2$, -CN, $-CF_3$, $-C_2F_5$, $C_{1-3}$ alkyl, or amido; and

m5 is an integer of 0 to 4.

**10.** The compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 9, having a structure represented by formula (XIII) or formula (XIV):

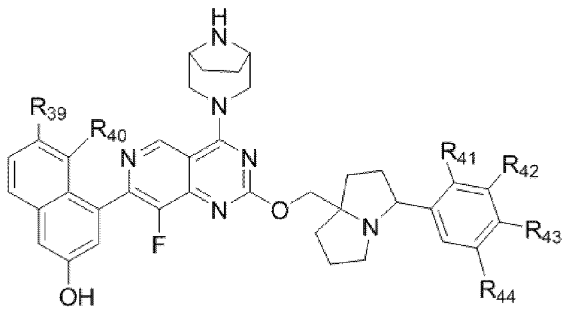

(XIII)

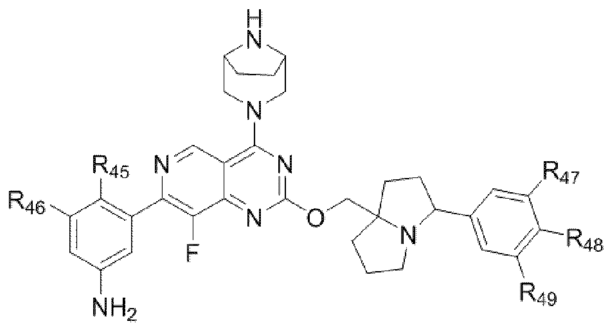

(XIV)

wherein, $R_{39}$ and $R_{40}$ are independently selected from the group consisting of -H, -F, -Cl, -CN, alkynyl, and $C_{1-3}$ linear alkyl; $R_{41}$, $R_{42}$, $R_{43}$, and $R_{44}$ are independently selected from the group consisting of -H, -OH, -F, -Cl, -CN, $-CF_3$, $-C_2F_5$, $-OCF_3$, monomethylphosphoryl, dimethylphosphoryl, $C_{1-5}$ linear/branched alkyl, $C_{1-5}$ alkoxy, $C_{3-10}$ cycloalkyl, $C_{3-10}$ heterocycloalkyl, -S($C_{0-10}$ alkyl), -OCON($C_{0-10}$ alkyl)($C_{0-10}$ alkyl), $C_{5-6}$ aryl, five-membered heteroaryl, and six-membered heteroaryl; alternatively, $R_{42}$, $R_{43}$ and the carbon atom between $R_{42}$ and $R_{43}$ form saturated or unsaturated $C_{3-8}$ cycloalkyl, saturated or unsaturated $C_{3-8}$ heterocycloalkyl, five-membered cyclic lactone, $C_{5-6}$ aryl, bridged cycloalkyl, or spirocycloalkyl, wherein H thereof is optionally substituted with -D, -OH, -F, $-NO_2$, -CN, $-CF_3$, $-C_2F_5$, $C_{1-3}$ alkyl, or amido; and

$R_{45}$ and $R_{46}$ are independently selected from the group consisting of -H, -F, -Cl, $-NH_2$, -CN, $-CF_3$, $-C_2F_5$, $-OCF_3$, alkynyl, $C_{1-3}$ linear alkyl, $C_{1-3}$ alkoxy, $C_{3-6}$ cycloalkyl, aryl, and heteroaryl; $R_{47}$, $R_{48}$, and $R_{49}$ are independently selected from the group consisting of -H, -F, -Cl, -CN, $-CF_3$, and $-C_2F_5$; alternatively, $R_{47}$, $R_{48}$ and the carbon atom between $R_{47}$ and $R_{48}$ form saturated or unsaturated $C_{3-8}$ cycloalkyl or saturated or unsaturated $C_{3-8}$ heterocycloalkyl, wherein H thereof is optionally substituted with -D, -OH, -F, -CN, $-CF_3$, or $C_{1-3}$ alkyl.

**11.** The compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 10, wherein $R_{42}$, $R_{43}$ and the carbon atom between $R_{42}$ and $R_{43}$ on the benzene ring in formula (XIII) form saturated or unsaturated $C_{3-8}$ cycloalkyl, saturated or unsaturated $C_{3-8}$ heterocycloalkyl, five-membered cyclic lactone, $C_{5-6}$ aryl, bridged cycloalkyl, or spirocycloalkyl, selected from the group consisting of:

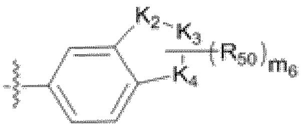 、 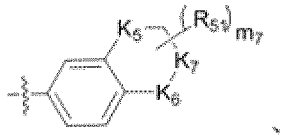 、 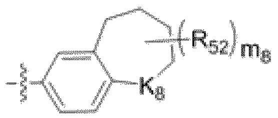 、 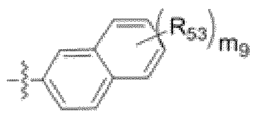

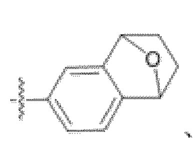 、 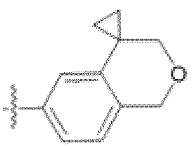 、 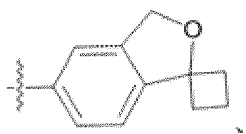 、 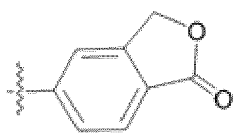 ;

wherein, $K_2$, $K_3$, and $K_4$ are fused with the benzene ring to form a saturated or unsaturated five-membered ring, $K_2$, $K_3$, and $K_4$ are independently selected from the group consisting of C, N, O, and S; $K_5$, $K_6$, and $K_7$ are independently selected from the group consisting of C, N, O, and S; $K_8$ is independently selected from the group consisting of C, N, O, and S; $R_{50}$, $R_{51}$ , $R_{52}$, and $R_{53}$ are independently selected from the group consisting of -H, -D, -OH, -F, $-NO_2$, -CN, $-CF_3$, $-C_2F_5$, $C_{1-3}$ alkyl, $C_{1-3}$ alkoxy, and $C_{3-6}$ cycloalkyl; m6, m7, m8, and m9 are each an integer of 0 to 6;

$R_{47}$, $R_{48}$ and the carbon atom between $R_{47}$ and $R_{48}$ on the benzene ring in formula (XIV) form saturated or unsaturated $C_{3-8}$ cycloalkyl or saturated or unsaturated $C_{3-8}$ heterocycloalkyl, selected from the group consisting of:

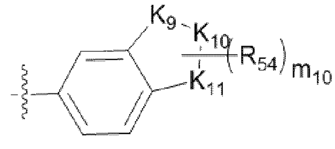

$K_9$, $K_{10}$, and $K_{11}$ are independently selected from the group consisting of C, N, and O; $R_{54}$ is independently selected from the group consisting of -H, -F, -CN, $-CF_3$, $-C_2F_5$, $C_{1-3}$ alkyl, $C_{1-3}$ alkoxy, and $C_{3-6}$ cycloalkyl; and m10 is an integer of 0 to 4.

**12.** The compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to claim 1, wherein the structure represented by formula (I) is selected from the group consisting of:

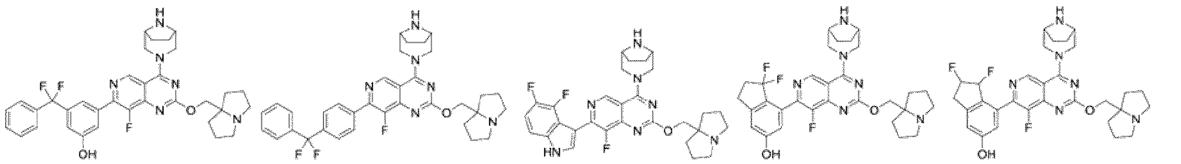

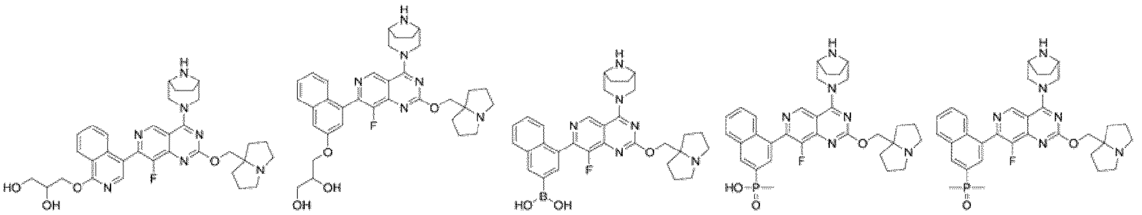

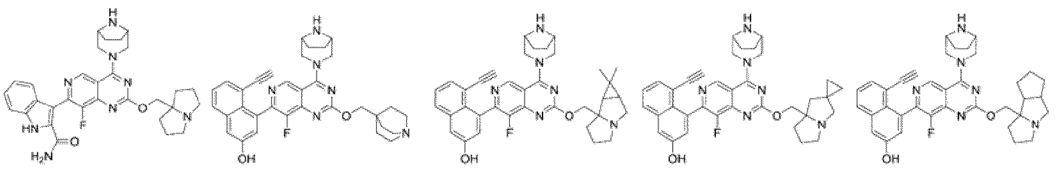

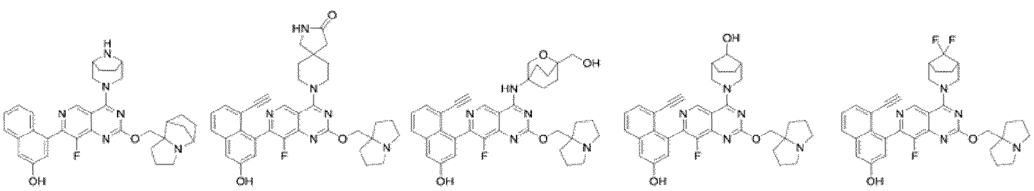

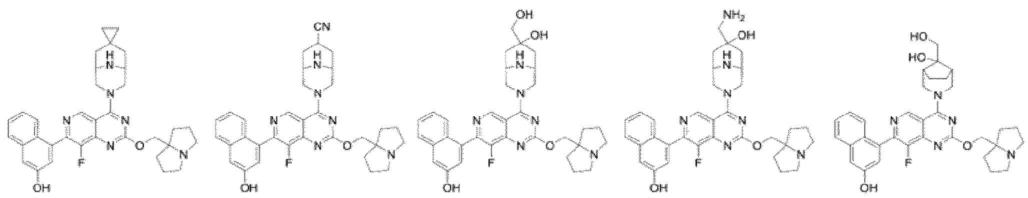

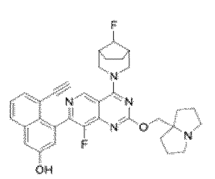 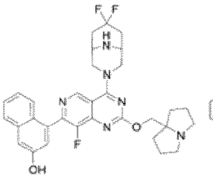 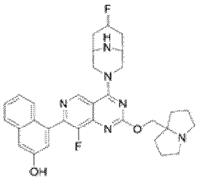 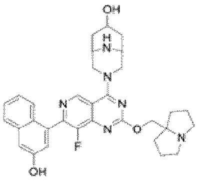 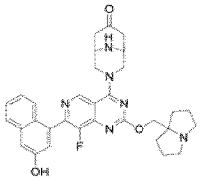

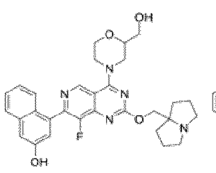 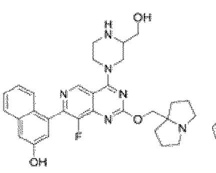 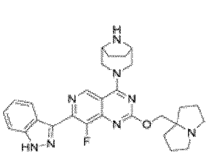 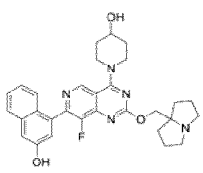 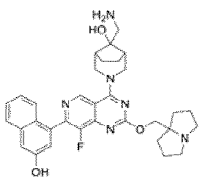

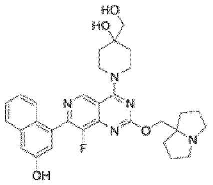 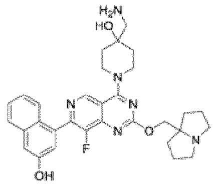 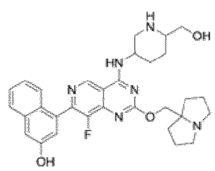 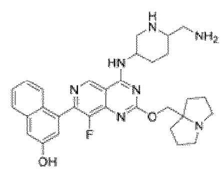 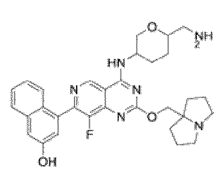

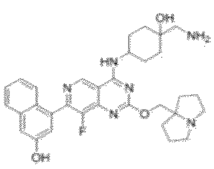 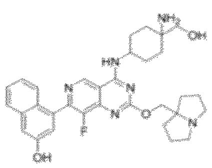 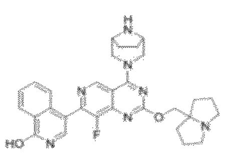 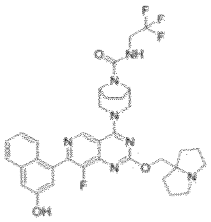 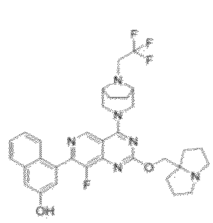

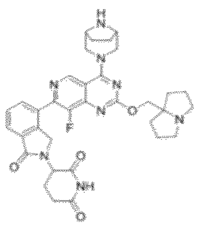 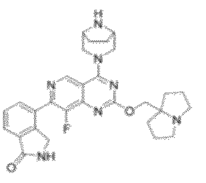 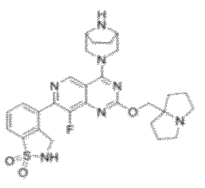 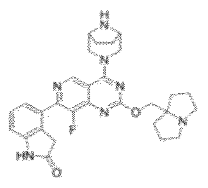 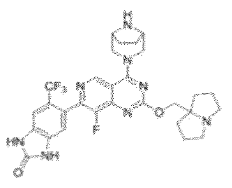

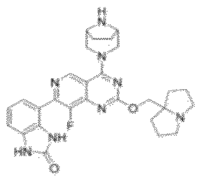 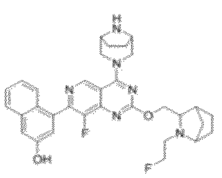 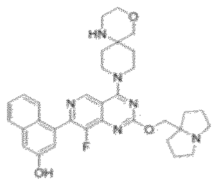 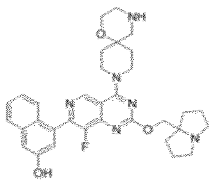 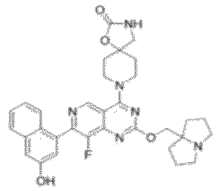

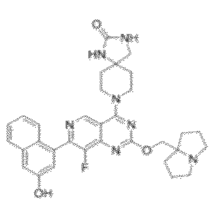 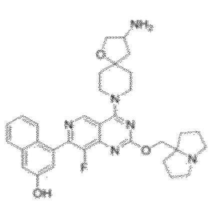 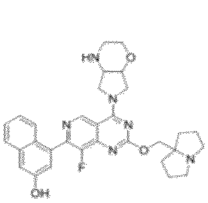 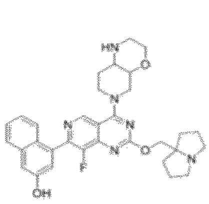 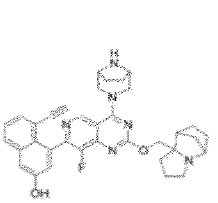

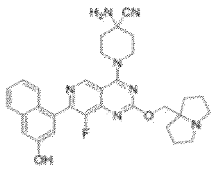 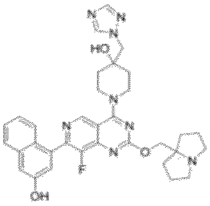 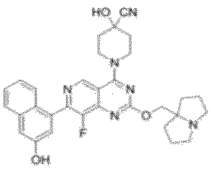 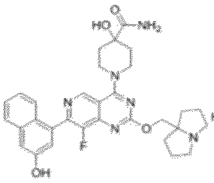 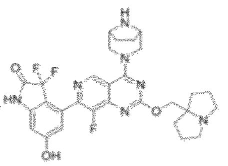

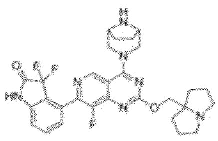 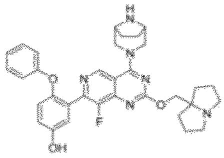 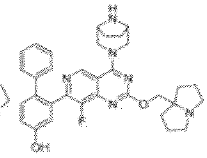 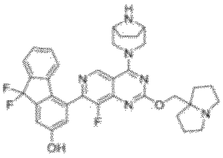 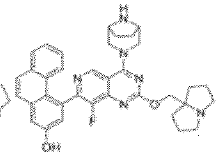

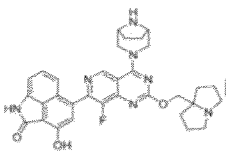 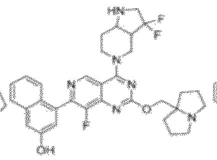 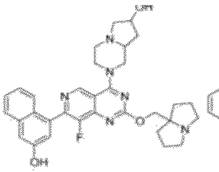 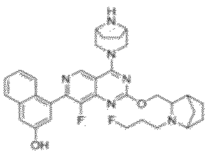 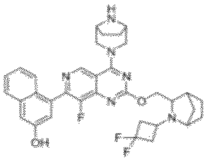

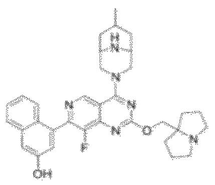 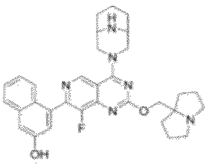 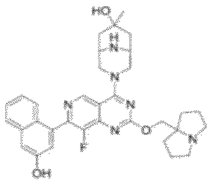 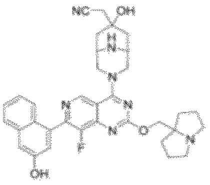 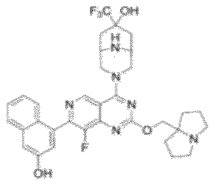

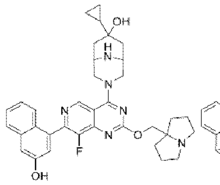 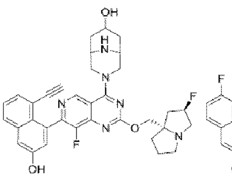 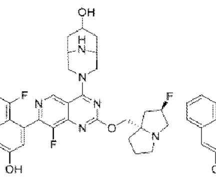 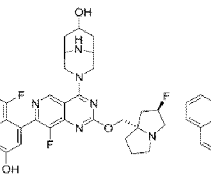 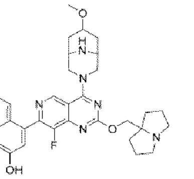

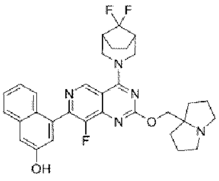 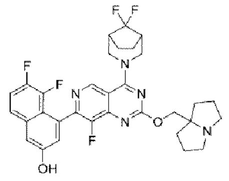 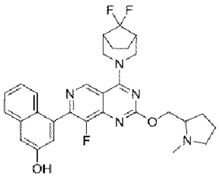 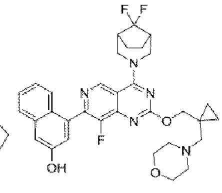 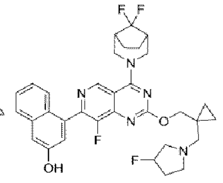

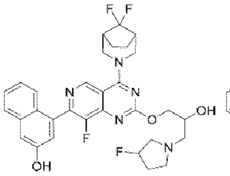 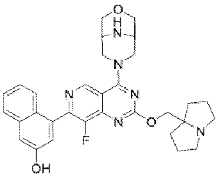 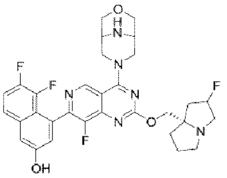 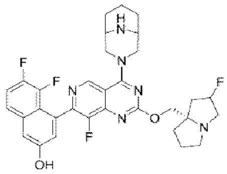 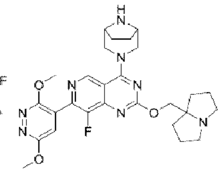

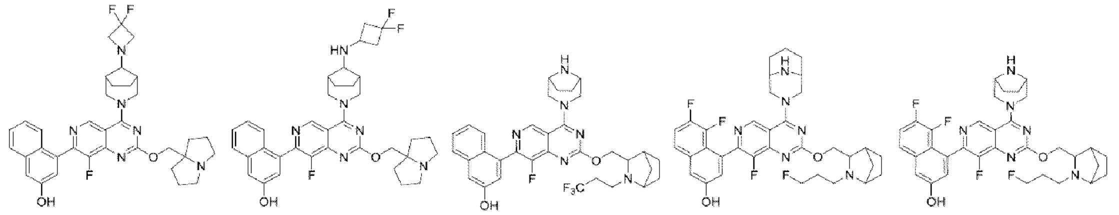

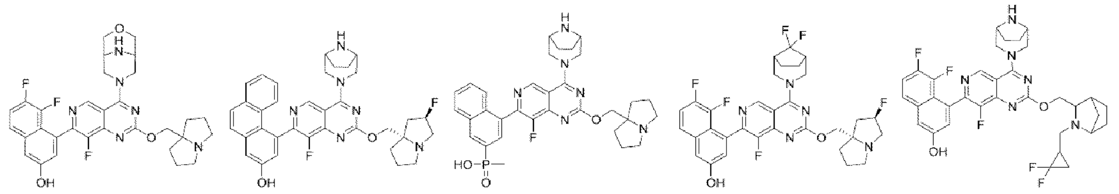

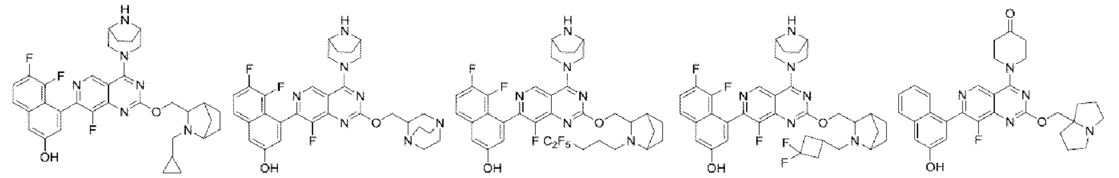

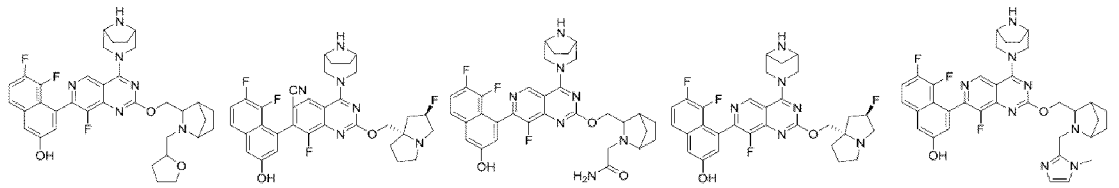

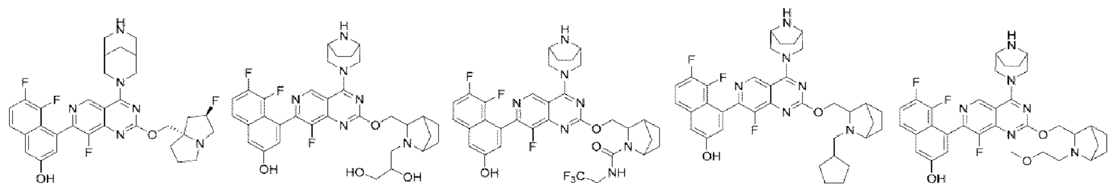

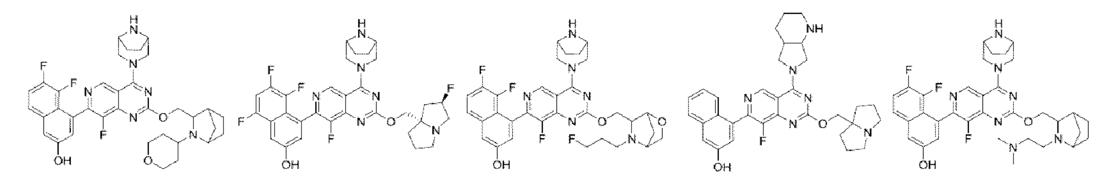

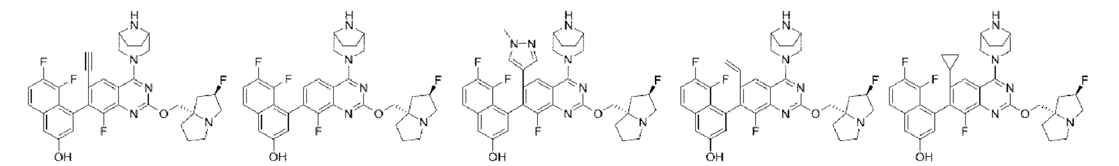

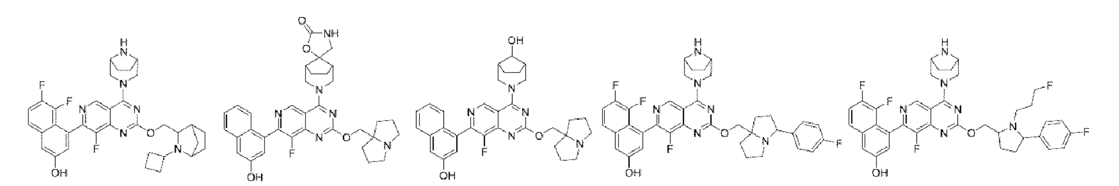

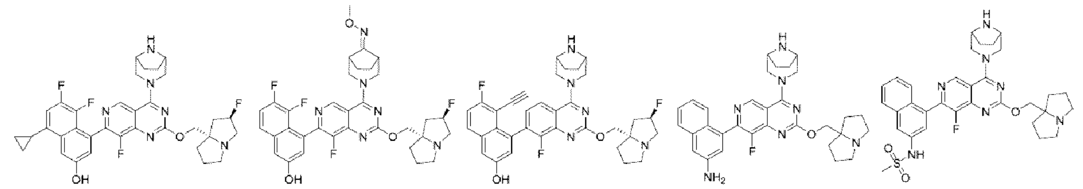

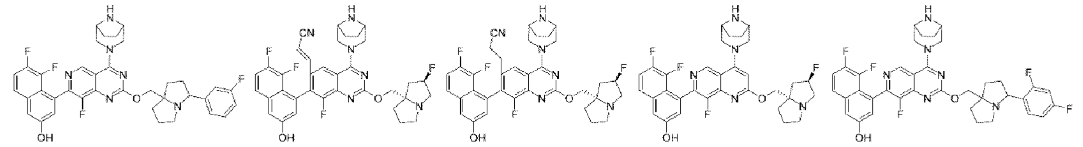

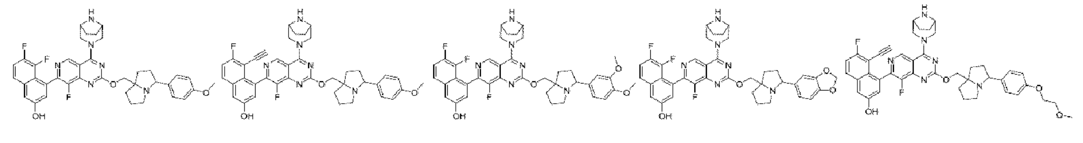

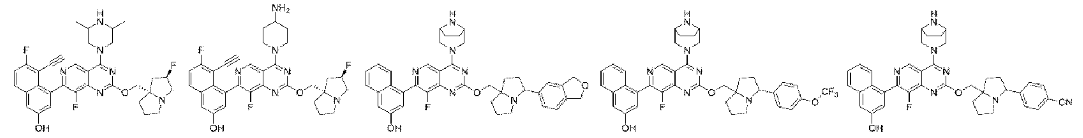

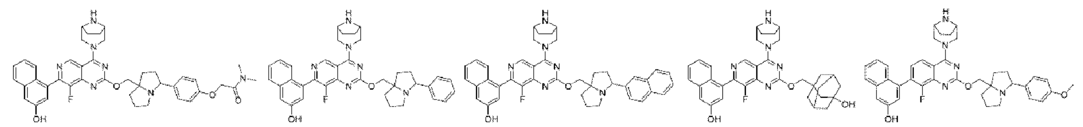

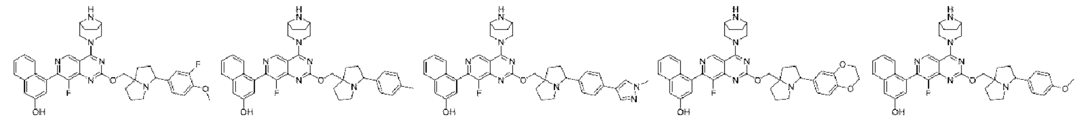

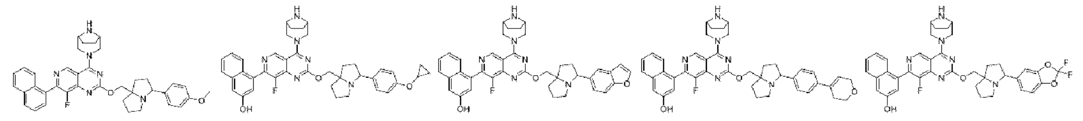

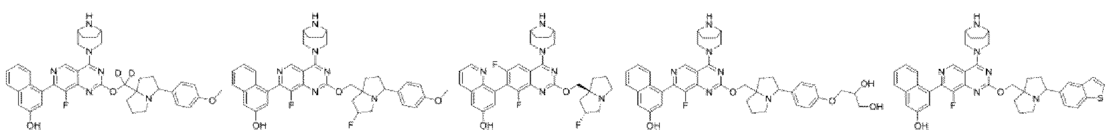

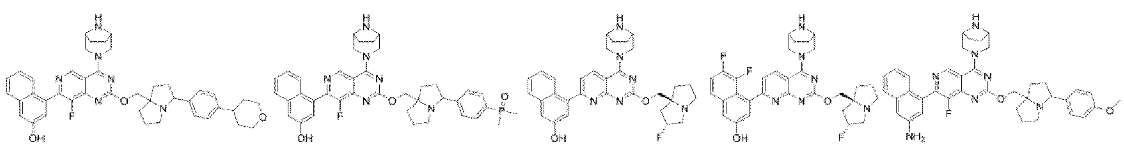

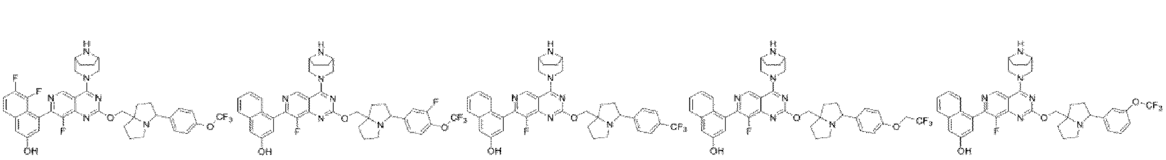

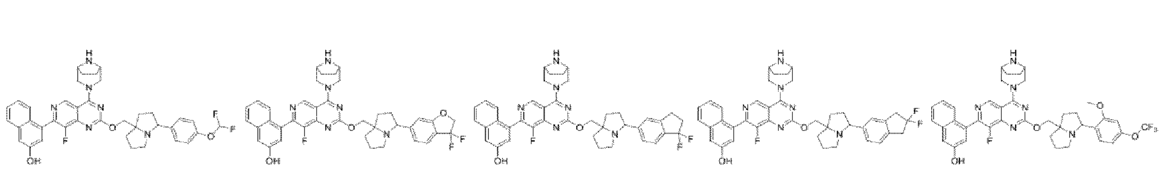

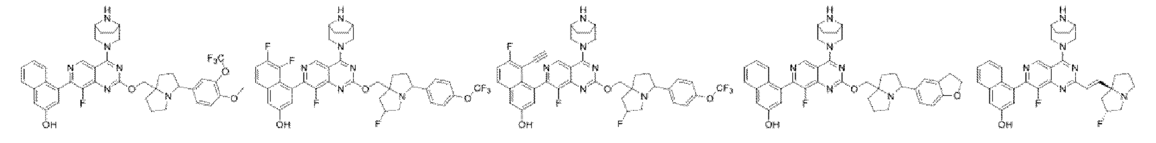

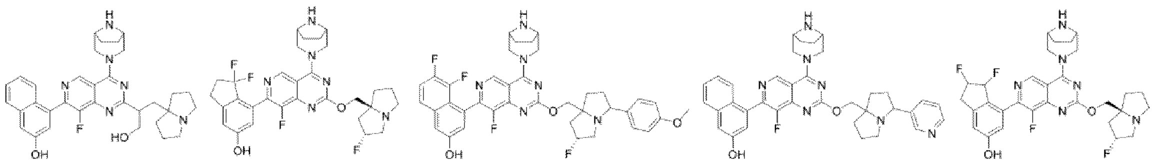

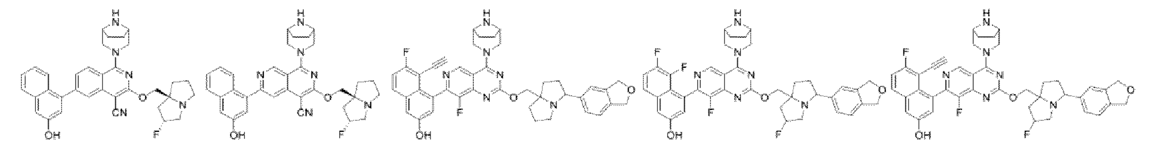

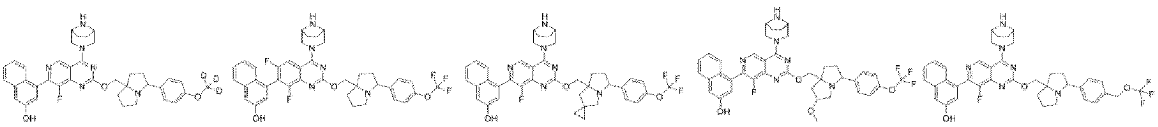

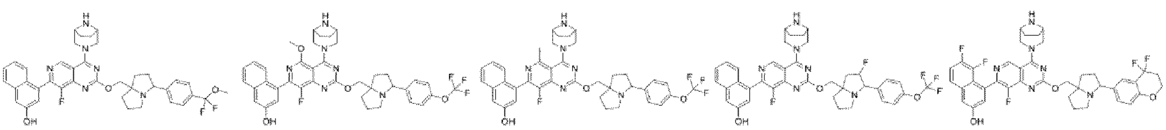

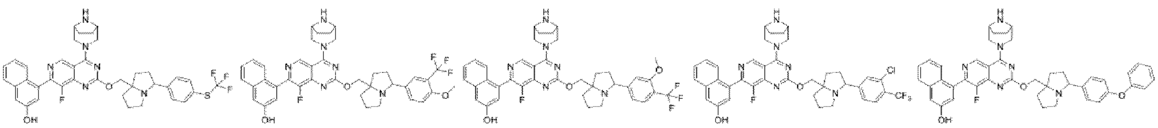

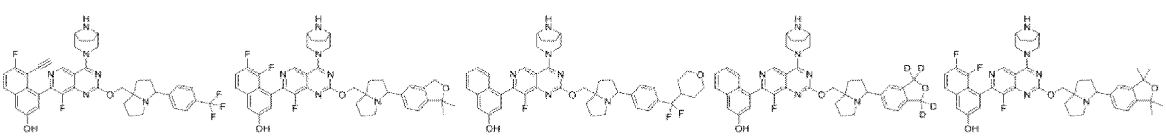

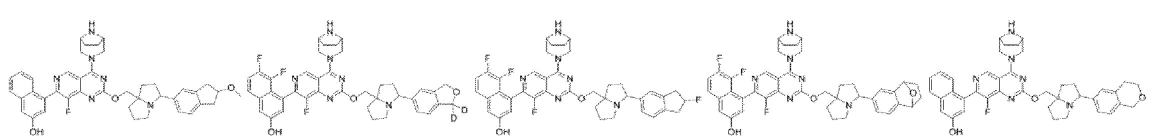

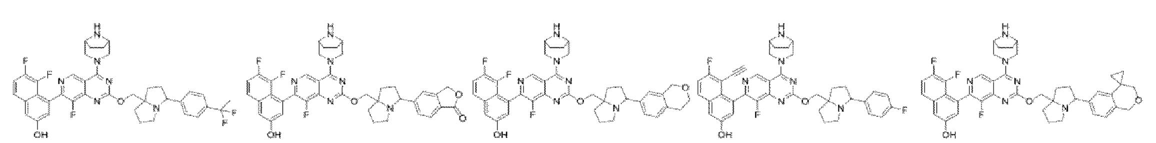

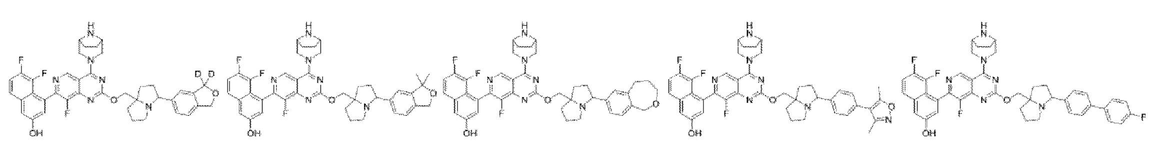

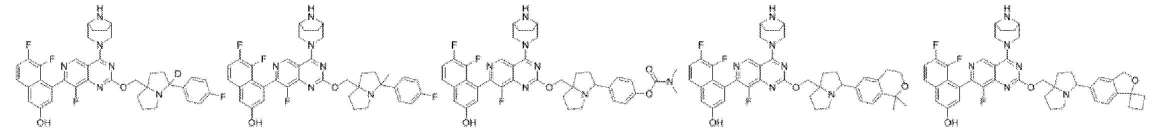

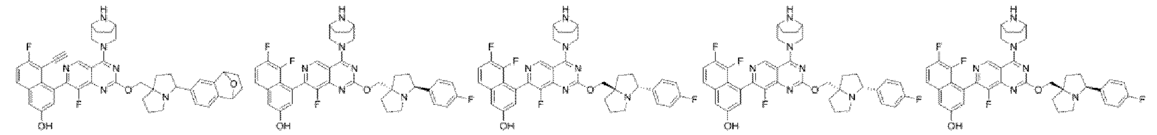

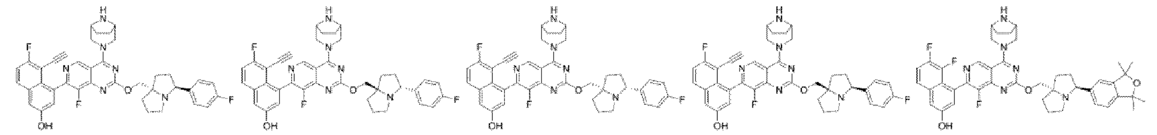

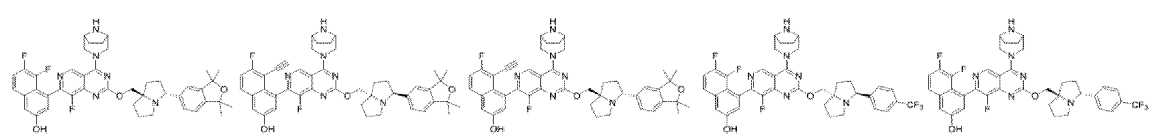

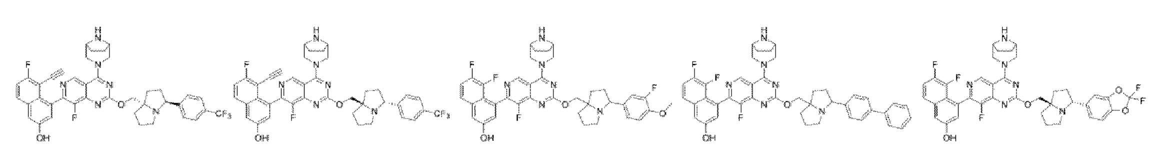

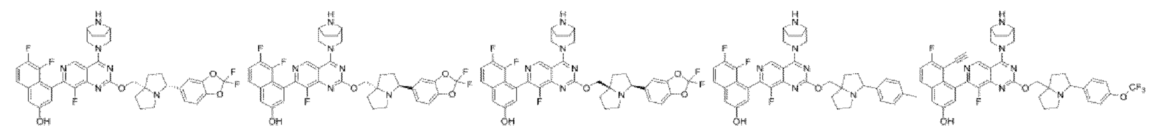

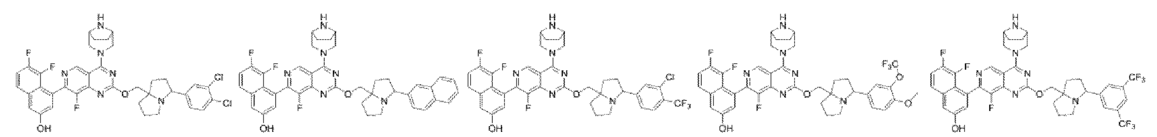

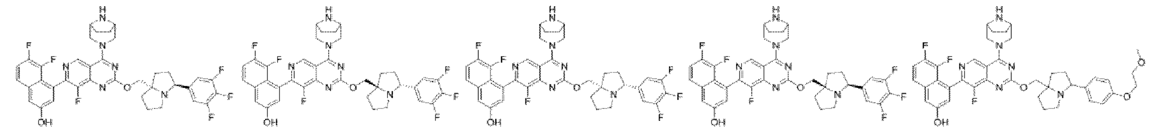

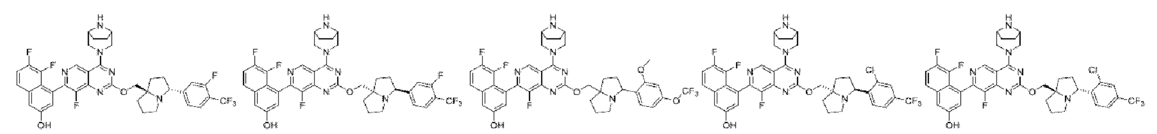

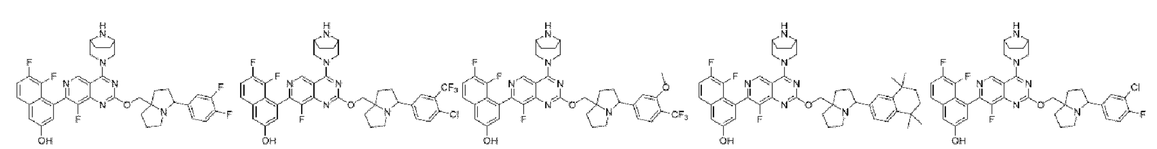

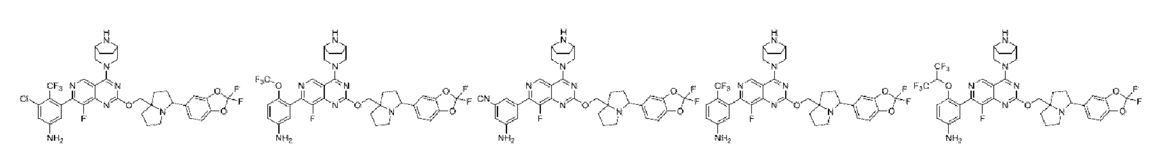

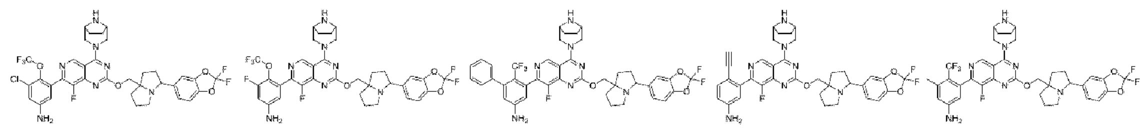

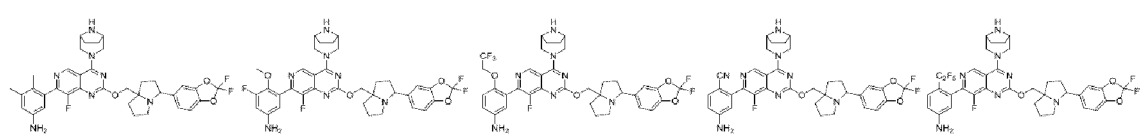

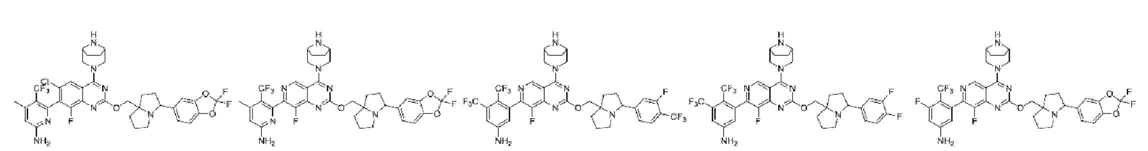

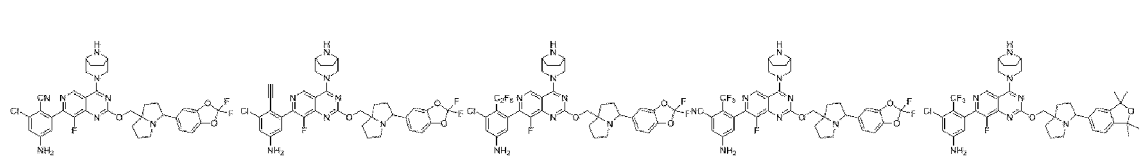

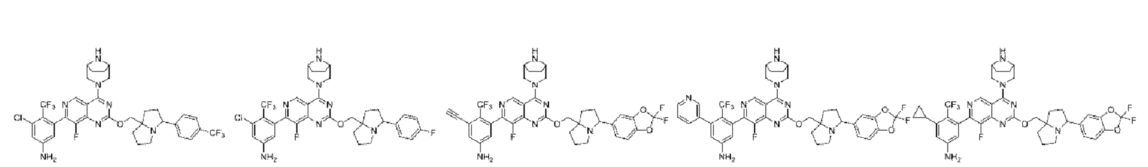

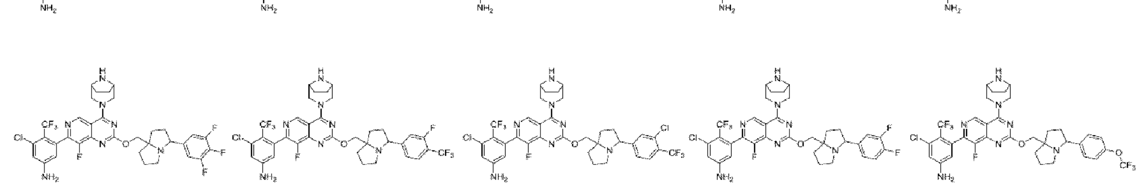

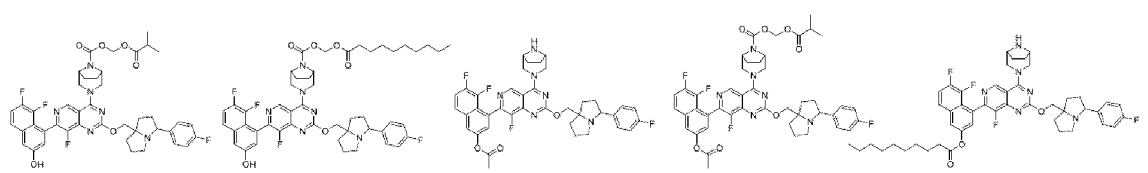

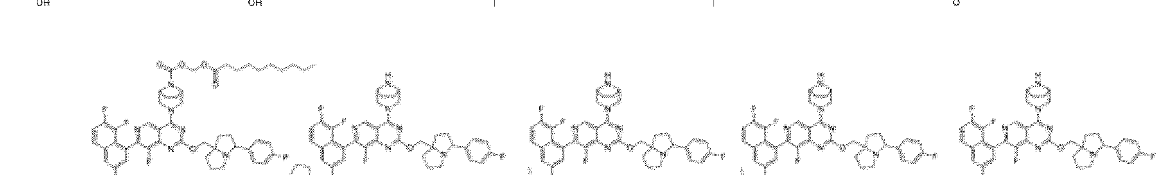

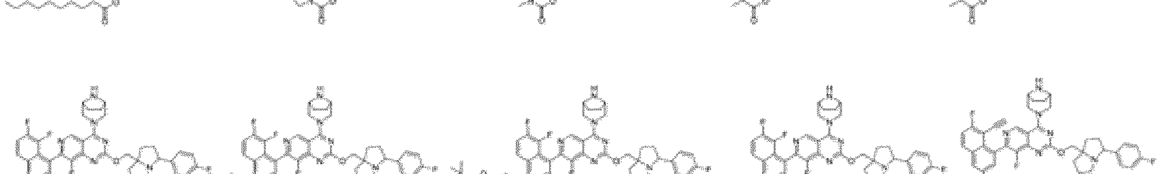

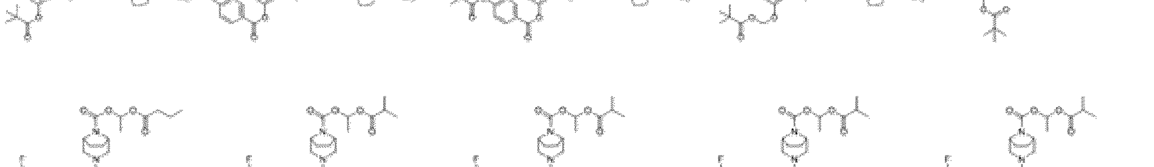

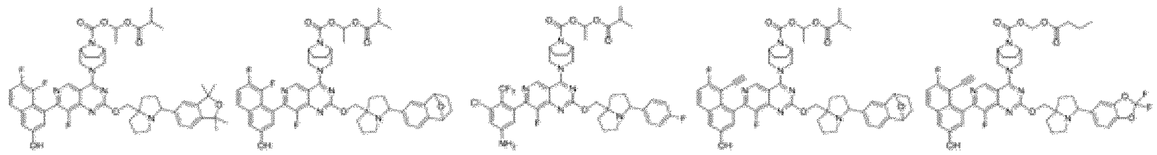

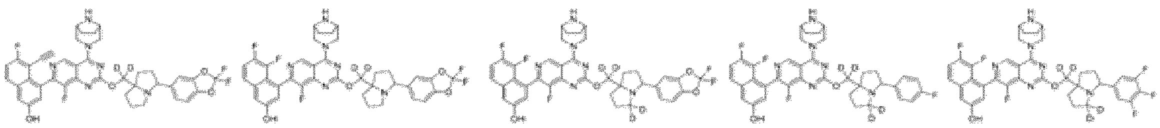

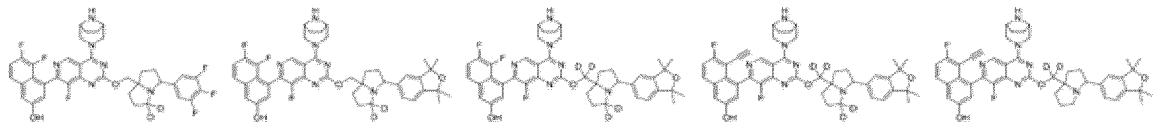

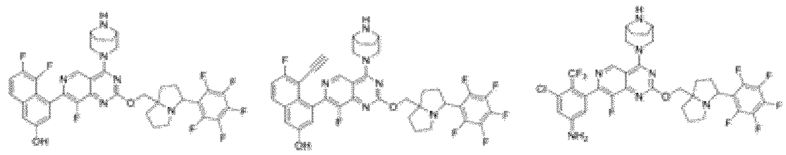

13. A pharmaceutical composition comprising an active compound selected from the group consisting of the compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to any one of claims 1 to 12, and a combination thereof.

14. Use of the compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to any one of claims 1 to 12 in the manufacture of a medicament for a KRAS inhibitor, preferably in the manufacture of a medicament for KRAS G12D mutation.

15. Use of the compound, or the tautomer, mesomer, racemate, enantiomer, diastereomer or a mixture thereof, metabolite, metabolic precursor, isotope-substituted form, pharmaceutically acceptable salt, hydrate, solvate, polymorph or cocrystal thereof according to any one of claims 1 to 12 in the manufacture of a medicament for treating, alleviating or preventing a disease or condition associated with Noonan syndrome, LEOPARD syndrome, leukaemia, neuroblastoma, melanoma, oesophageal cancer, head and neck tumors, breast cancer, lung cancer, pancreatic cancer, pancreatic ductal adenocarcinoma, colorectal and colon cancer.

**INTERNATIONAL SEARCH REPORT**

International application No. **PCT/CN2023/087259**

**A. CLASSIFICATION OF SUBJECT MATTER**

C07D471/00(2006.01)i; C07D471/04(2006.01)i; A61K31/517(2006.01)i; A61K31/519(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D; A61K; A61P

Documentation searched other than minimum documentation to the extent that such documents are included in the fields searched

Electronic data base consulted during the international search (name of data base and, where practicable, search terms used)

DWPI, REGISTRY(STN), CAPLUS(STN), CNKI: 成都海博为药业有限公司, 深圳海博为药业有限公司, KRAS, KRAS G12D, 抑制剂, 肿瘤, 癌, 萘啶, 吡啶并, 嘧啶, 苯并, inhibit, cancer, tumor, naphthyridine, pyrido, pyrimidine, benzo, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2021041671 A1 (MIRATI THERAPEUTICS, INC. et al.) 04 March 2021 (2021-03-04) claims 1, 45, description, page 62 paragraph [0169], pages 65-66 paragraphs [0180]-[0182] | 1-15 |
| X | WO 2022042630 A1 (INVENTISBIO CO LTD. et al.) 03 March 2022 (2022-03-03) description, abstract, claims 1, 78-81, description, pages 102-119, paragraph [0159] | 1-7, 13-15 |
| X | WO 2022015375 A1 (MIRATI THERAPEUTICS, INC. et al.) 20 January 2022 (2022-01-20) description, abstract, claims 30-31, 34, description, pages 26-28, pages 87-104, 106-127, 130-137, 142-147, 150-151, 155-201, 204-258, 260-278, 280-369, 371-469, 475-503 | 1-7, 13-15 |
| X | WO 2022031678 A1 (MIRATI THERAPEUTICS, INC. et al.) 10 February 2022 (2022-02-10) description, abstract, claims 48, 51-53, description pages 21-24 paragraph [0138] | 1-4, 6, 13-15 |
| X | WO 2022061251 A1 (PLEXXIKON INC.) 24 March 2022 (2022-03-24) claims 1 and 74-78, and description, page 195, table IA | 1-7, 13-15 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **12 July 2023** | **20 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/087259**

**C. DOCUMENTS CONSIDERED TO BE RELEVANT**

| Category* | Citation of document, with indication, where appropriate, of the relevant passages | Relevant to claim No. |
|---|---|---|
| X | WO 2022068921 A1 (SHANGHAI PHARMACEUTICALS HOLDING CO., LTD.) 07 April 2022 (2022-04-07)<br>description, abstract, description, page 2 paragraph 3, pages 120-121, page 191 paragraphs 1-5 | 1-5, 13-15 |
| X | WO 2020146613 A1 (MIRATI THERAPEUTICS, INC. et al.) 16 July 2020 (2020-07-16)<br>description, abstract, claims 1-7, description, page 31 | 1-2, 13-15 |
| X | CN 113999226 A (SHANGHAI KECHOW PHARMA INC.) 01 February 2022 (2022-02-01)<br>claims 22-23 | 1-2, 13-15 |

Form PCT/ISA/210 (second sheet) (July 2022)

**INTERNATIONAL SEARCH REPORT**
**Information on patent family members**

International application No.
**PCT/CN2023/087259**

| Patent document cited in search report | Publication date (day/month/year) | Patent family member(s) | Publication date (day/month/year) |
|---|---|---|---|
| WO 2021041671 A1 | 04 March 2021 | None | |
| WO 2022042630 A1 | 03 March 2022 | None | |
| WO 2022015375 A1 | 20 January 2022 | EP 4182313 A1 | 24 May 2023 |
| WO 2022031678 A1 | 10 February 2022 | EP 4192585 A1 | 14 June 2023 |
| WO 2022061251 A1 | 24 March 2022 | US 2023081426 A1 | 16 March 2023 |
| WO 2022068921 A1 | 07 April 2022 | None | |
| WO 2020146613 A1 | 16 July 2020 | None | |
| CN 113999226 A | 01 February 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202210383719
- CN 202310039419
- CN 202310270906
- WO 2021041671 A
- WO 2021106231 A